# EUROPEAN PATENT APPLICATION

(11) **EP 4 361 142 A1**
(43) Date of publication of application: **01.05.2024**
(21) Application number: 22827722.4
(22) Date of filing: 27.06.2022
(51) Int. Cl.: C07D 401/14, C07D 401/12, C07D 221/20, C07D 413/14, C07D 413/12, A61K 31/506, A61K 31/4545, A61K 31/517, A61K 31/438, A61K 31/502, A61K 31/5377, A61K 31/444, A61P 35/00, A61P 31/12, A61P 31/16

(54) **COMPOUND AS KIF18A INHIBITOR**

(30) Priority: 25.06.2021 CN 202110711270; 20.08.2021 CN 202110963377; 08.12.2021 CN 202111493904; 25.03.2022 CN 202210307789
(71) Applicant: Hangzhou Innogate Pharma Co., Ltd., Hangzhou, Zhejiang 311121 (CN)
(72) Inventor: ZHANG, Hancheng, Hangzhou, Zhejiang 311121 (CN); JIA, Wei, Hangzhou, Zhejiang 311121 (CN); CAI, Congcong, Hangzhou, Zhejiang 311121 (CN)
(74) Representative: Lavoix
(86) International application number: PCT/CN2022/101694
(87) International publication number: WO 2022/268230

(57) **Abstract**

The present invention provides a compound as a KIF18A inhibitor. Specifically, the present invention provides a compound having a structure as represented by the following formula (I), or an optical isomer, pharmaceutically acceptable salt, prodrug, deuterated derivative, hydrate and solvate thereof. The compound can be used for treating or preventing diseases or symptoms associated with the activity or expression amount of KIF18A.

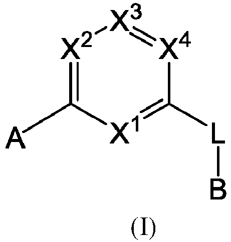

## Description

### FIELD OF THE INVENTION

The present invention relates to the field of medicinal chemistry; specifically, the present invention relates to a class of novel compounds, their synthesis methods and their use as a KIF18A inhibitor in the preparation of drugs for the treatment of related diseases such as tumors.

### Background

Tumors often have uncontrolled cell proliferation. Disrupting one or more genes involved in cell signaling pathways that regulate the cell cycle and centrosome cycle can affect normal cell division. These abnormal genes encode a variety of different tumor suppressor genes or proto-oncogenes, which are involved in a series of physiological processes in the body, resulting in cell cycle progression and cell division that are not controlled by checkpoints. Various kinases and kinesins have been identified to play a key role in the cell cycle and mitosis regulation of normal dividing cells and tumor cells.

Intracellular transport is essential for maintaining normal cell form and function. The members of kinesin superfamily are molecular motors that obtain energy by hydrolyzing ATP and transport organelles, protein complexes and mRNA in cells. During mitosis and meiosis, kinesins are also involved in chromosome and spindle movement. According to sequence homology, the human kinesin superfamily can be divided into 14 subfamilies. A globular domain of about 360 amino acid residues is the hallmark of a kinesin family member, and this globular domain contains the catalytic pocket for hydrolyzing ATP and the region for binding microtubules, also known as the motor domain. The non-motor domain of the protein is responsible for the attachment of transported intracellular "cargo", which includes the organelles, proteins, and chromosomes mentioned before. Kinesins use the energy obtained by ATP hydrolysis to transport "cargo" along the polarized microtubules. Therefore, kinesins are also named plus-end or minus-end directed motors.

In many human malignancies, changes in cell cycle regulation and chromosomal instability (CIN, Chromosomal Instability) are common. Targeting enzymes involved in mitosis has attracted increasing attention, and a variety of anti-mitotic drugs have been developed, such as vinblastine drugs that inhibit the polymerization of microtubules and spindle formation, and paclitaxel chemotherapy drugs that stabilize microtubules and inhibit their depolymerization. But because they also damage normally replicating cells, these chemotherapies have serious side effects, such as myelosuppression and neurotoxicity. KIF18A (Kinesin Family Member 18A), a member of the kinesin-8 subfamily, is a plus-end directed motor. In Hela cervical cancer cells, knocking down KIF18A causes longer spindle, promotes chromosomal instability, and activates mitotic spindle assembly checkpoints. Importantly, in a range of tumor cells with chromosomal instability, KIF18A knockdown triggered the activation of mitotic spindle assembly checkpoints and interfered with tumor cell proliferation, but in normal mammary epithelial cells and near-normal karyotype tumor cells, KIF18A knockdown did not significantly affect cell proliferation. In addition, mice with KIF18A knockout were viable, but had defects in germ cell division, suggesting that KIF18A was not required for normal somatic cell division. In addition, KIF18A is overexpressed in many types of cancer, such as breast, cervical, colon, lung, pancreatic, prostate, bladder, and head and neck cancers. Genetic knockout or knockdown of KIF18A can affect the mitotic spindle of tumor cells, resulting in mitotic arrest and promoting apoptotic cell death.

The above data indicate that the development of anticancer drugs targeting KIF18A can specifically inhibit tumor cells with chromosomal instability, while avoiding affecting normal karyotype cells, reducing the side effects brought by the drug, making it a highly concentrated broad-spectrum cytotoxic drug.

In addition, the expression of KIF18A was significantly increased in host cells infected with influenza virus. Inhibition of ATP hydrolysis activity of KIF18A can hinder the replication of influenza virus.

Therefore, the development of KIF18A inhibitors has very good anti-tumor and antiviral prospects.

### SUMMARY OF THE INVENTION

The object of the present invention is to provide a novel KIF18A inhibitor.

In the first aspect of the present invention, a compound of formula (I), or the optical isomers, pharmaceutically acceptable salts, prodrugs, deuterated derivatives, hydrates, or solvates thereof is provided: 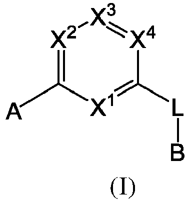 wherein in the formula (I):
A is selected from-Y-R¹; wherein, Y is selected from the bond, -S(O)-, -S(O)₂-, - S(O)(NH)-, -S(O)₂NR^{a}-, -NR^{a}S(O)₂-, -NR^{a}S(O)₂NR^{a}-, -P(O)(R^{q})-, -P(O)(R^{q})NR^{a}-,-NR^{a} P(O)(R^{q})-, -P(O)(R^{q})O-, -O P(O)(R^{q})-, -NR^{a}-, -O-, -S-, -C(O)NR^{a}-, -NR^{a}C(O)-, -C(O)O-, - OC(O)-, -NR^{a}C(O)NR^{a}-, -OC(O)NR^{a}-, -NR^{a}C(O)O-, C₂₋₄ alkenyl, and C₂₋₄ alkynyl; wherein each R^{a} is independently selected from hydrogen and C₁₋₄ alkyl; each R^{q} is independently selected from C₁₋₄ alkyl, C₃₋₆ cycloalkyl, and 3- to 6-membered heterocyclyl; or R^{q} and R¹ together with the attached phosphorus atom form optionally substituted 4-to 8-membered ring structure, while the ring structure may additionally contain 0-1 heteroatoms optionally selected from N, O and S;
or A is selected from formula (Ia): 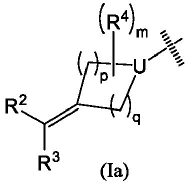
wherein, " 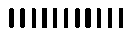 " refers to the site of formula (Ia) which attach to the remaining fragment of the compound of formula (I);
L is selected from -C(O)NR^{b}-, -NR^{b}C(O)-, -NR^{b}C(O)NR^{b}-, -OC(O)NR^{b}-, -C(O)O-, - OC(O)-, -NR^{b}C(O)O-, -S(O)₂NR^{b}-, -NR^{b}S(O)₂-, -NR^{b}-, -O-, 3-to 6-membered heterocyclyl, and heteroaryl; wherein each R^{b} is independently hydrogen or C₁₋₄ alkyl;
B is Formula (Ib) or (Id): 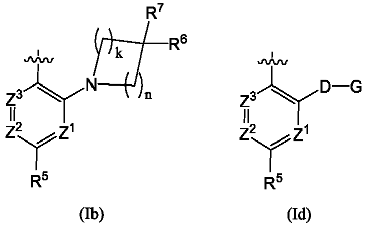
wherein " 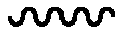 " refers to the site of formula (Ib) or (Id) which attach to the L in a compound of formula (I);
X¹, X², X³ and X⁴ are each independently selected from N or CR^{c}; wherein R^{c} is selected from the group consisting of hydrogen, halogen, C₁₋₄ alkyl, C₁₋₄ haloalkyl, C₂₋₄ alkynyl, C₁₋₄ alkoxy, hydroxy, C₁₋₄ haloalkoxy, CN, NR^{d}R^{d}, C₃₋₆ cycloalkyl, and 3-to 8-heterocyclic group; wherein each R^{d} is independently selected from the group consisting of hydrogen, C₁₋₄ alkyl, and C₁₋₄ haloalkyl;
U is selected from N or CR^{e}; wherein R^{e} is selected from hydrogen, halogen, C₁₋₄ alkyl;
Z¹, Z² and Z³ are each independently selected from N and CR^{m}; wherein R^{m} is selected from the group consisting of hydrogen, halogen, C₁₋₄ alkyl, C₁₋₄ haloalkyl, C₁₋₄ alkoxy, hydroxy, C₁₋₄ haloalkoxy, CN, NR^{d}R^{d}, C₃₋₈ cycloalkyl, and 3- to 8-membered heterocyclyl; wherein, each R^{d} are as defined above; D is selected from the group consisting of bond, - O-, -S-, NR^{d}-, C₁₋₂ alkyl, and C₂₋₄ alkynyl; G is 3-10 membered saturated or unsaturated cyclic structure, which optionally containing 0, 1 or 2 heteroatoms selected from N, O and S, or optionally substituted by R⁶ or R⁷;
R¹ is selected from the group consisting of C₁₋₄ alkyl, C₂₋₄ alkenyl, C₂₋₄ alkynyl, C₃₋₆ cycloalkyl, 3- to 10-membered heterocyclyl; said alkyl, alkenyl, alkynyl, cycloalkyl or heterocyclyl being optionally substituted with one or more groups selected from the group consisting of halogen, C₁₋₄ alkyl, C₁₋₄ haloalkyl, C₂₋₄ alkenyl, C₂₋₄ haloalkenyl, C₂₋₄ alkynyl, C₂₋₄ haloalkynyl, C₃₋₆ cycloalkyl, 3- to 6- membered heterocyclyl, CN, OR^{f}, SR^{f}, NR^{d}R^{d}, -OC₁₋₄ alkyl-OR^{f}, -OC₁₋₄ alkyl-NR^{d}R^{d}, -NR^{d}C₁₋₄ alkyl-OR^{f}, -NR^{d}C₁₋₄ alkyl-NR^{d}R^{d}, -C(O)R^{g}, -C(O)OR^{f}, -OC(O)R^{g}, -C(O)NR^{d}R^{d}, -NR^{d}C(O)R^{g}, -NR^{d}C(O)NR^{d}R^{d}, - OC(O)NR^{d}R^{d}, -NR^{d}C(O)OR^{f}, -OC(O)OR^{f}, -S(O)₂NR^{d}R^{d}, -NR^{d}S(O)₂R^{g}, and - NR^{d}S(O)₂NR^{d}R^{d}; or said cycloalkyl or heterocyclyl is optionally substituted with = M; wherein, R^{f} is selected from hydrogen, C₁₋₄ alkyl, and C₁₋₄ haloalkyl; R^{g} is selected from hydrogen, C₁₋₄ alkyl, C₂₋₄ alkenyl, C₂₋₄ alkynyl, C₃₋₈ cycloalkyl, 4-to 8- membered heterocyclyl, aryl, and heteroaryl; M is selected from O and CR^{h}Rⁱ; wherein, R^{h} and Rⁱ are each independently selected from hydrogen, halogen, and C₁₋₄ alkyl; wherein the alkyl in R^{h} or Rⁱ is optionally substituted with one or more groups selected from hydrogen, halogen, C₁₋₄ alkyl, C₁₋₄ haloalkyl, C₁₋₄ alkoxy, C₁₋₄ haloalkoxy, hydroxy, NR^{d}R^{d}, C₃₋₈ cycloalkyl, 3-to 8-membered heterocyclyl, aryl, and heteroaryl; or R^{h} and Rⁱ, together with the carbon atom to which they are attached, form a 3-to-6-membered ring structure optionally containing 0, 1 or 2 heteroatoms selected from N, O and S; each R^{d} is as defined above;
R² and R³ are each independently selected from the group consisting of hydrogen, fluorine, and C₁₋₄ alkyl; wherein the alkyl is optionally substituted with one or more groups selected from the group consisting of halogen, C₁₋₄ alkyl, C₁₋₄ haloalkyl, C₁₋₄ alkoxy, C₁₋₄ haloalkoxy, hydroxyl, NR^{d}R^{d}, C₃₋₈ cycloalkyl, 3-to 8-membered heterocyclyl, aryl, and heteroaryl; wherein, each R^{d} is defined as above;
each R⁴ is independently selected from hydrogen, halogen, C₁₋₄ alkyl, C₁₋₄ haloalkyl, C₁₋₄ alkoxy, C₁₋₄ alkoxy-C₁₋₄ alkyl, hydroxy, hydroxyl-C₁₋₄ alkyl, C₁₋₄ haloalkoxy, C₁₋₄ haloalkoxy-C₁₋₄ alkyl, and CN;
R⁵ is selected from -Q-R⁸; wherein Q is selected from bond , -S(O)-, -S(O)₂-, - S(O)(NH)-, -S(O)₂NR^{a}-, -NR^{a}S(O)₂-, -NR^{a}S(O)₂NR^{a}-, -NR^{a}-, -O-, -S-, -C(O)NR^{a}-, - NR^{a}C(O)-, -C(O)O-, -OC(O)-, -NR^{a}C(O)NR^{a}-, -OC(O)NR^{a}-, -NR^{a}C(O)O-, C₂₋₄ alkenyl, and C₂₋₄ alkynyl; where each R^{a} is as defined above;
R⁶ and R⁷ are each independently selected from hydrogen, halogen, C₁₋₄ alkyl, C₂₋₄ alkenyl, C₂₋₄ alkynyl, C₃₋₈ cycloalkyl, 4-to 8-membered heterocyclyl; or R⁶ and R⁷ together with the carbon atoms to which they are attached form a 3-to 6-membered ring structure, optionally containing 0, 1, or 2 heteroatoms selected from N, O and S; or R⁶ and R⁷ together with the carbon atom to which they are attached form C=T, wherein T is selected from CR^{j}R^{k}; wherein R^{j} and R^{k} are each independently selected from the group consisting of hydrogen, deuterium, halogen, and C₁₋₄ alkyl; said alkyl is optionally substituted with one or more group selected from halogen, C₁₋₄ haloalkyl, C₂₋₄ alkenyl, C₂₋₄ alkynyl, C₃₋₆ cycloalkyl, 3-to 6-membered heterocyclyl, CN, OR^{f}, SR^{f}, and NR^{d}R^{d}; or R^{j} and R^{k} together with the carbon atom to which they are attached form a 3-to -6 membered ring structure which optionally contains 0 or 1 heteroatom selected from N, O and S; R^{d} and R^{f} are as defined above;
R⁸ is selected from the group consisting of C₁₋₄ alkyl, C₂₋₄ alkenyl, C₂₋₄ alkynyl, C₃₋₆ cycloalkyl, 3-to 10-membered heterocyclyl; said alkyl, alkenyl, alkynyl, cycloalkyl or heterocyclyl being optionally substituted with one or more groups selected from the group consisting of halogen, C₁₋₄ alkyl, C₁₋₄ haloalkyl, C₂₋₄ alkenyl, C₂₋₄ alkynyl, C₃₋₆ cycloalkyl, 3-to 6-membered heterocyclyl, CN, OR^{f}, SR^{f}, NR^{d}R^{d},-OC₁₋₄ alkyl-OR^{f}, -OC₁₋₄ alkyl-NR^{d}R^{d}, -NR^{d}C₁₋₄ alkyl-OR^{f}, -NR^{d}C₁₋₄ alkyl-NR^{d}R^{d}, -C(O)R^{g}, -C(O)OR^{f}, -OC(O)R^{g}, -C(O)NR^{d}R^{d}, -NR^{d}C(O)R^{g}, -NR^{d}C(O)NR^{d}R^{d}, -OC(O)NR^{d}R^{d}, -NR^{d}C(O)OR^{f}, -OC(O)OR^{f}, -S(O)₂NR^{d}R^{d}, -NR^{d}S(O)₂R^{g}, and -NR^{d}S(O)₂NR^{d}R^{d}; or said cycloalkyl or heterocyclyl is substituted by =M; wherein, R^{f} is selected from hydrogen, C₁₋₄ alkyl, and C₁₋₄ haloalkyl; R^{g} is selected from hydrogen, C₁₋₄ alkyl, C₂₋₄ alkenyl, C₂₋₄ alkynyl, C₃₋₈ cycloalkyl, 4-to 8-membered heterocyclyl, aryl, and heteroaryl; M is selected from O and CR^{h}Rⁱ; wherein, R^{h} and Rⁱ are each independently selected from hydrogen, halogen, and C₁₋₄ alkyl; wherein the alkyl in R^{h} or R' is optionally substituted with one or more groups selected from hydrogen, halogen, C₁₋₄ alkyl, C₁₋₄ haloalkyl, C₁₋₄ alkoxy, C₁₋₄ haloalkoxy, hydroxy, NR^{d}R^{d}, C3-8 cycloalkyl, 3-to 8-membered heterocyclyl, aryl, and heteroaryl; or R^{h} and Rⁱ together with the carbon atoms to which they are attached form a 3-to-6-membered ring structure optionally containing 0, 1, or 2 heteroatoms selected from N, O and S; each R^{d} is as defined above;
p and q are each independently selected from 0, 1, 2, 3, 4, 5 and 6; with the proviso that p and q are not simultaneously 0;
m is selected from 0, 1, 2, 3 and 4;
k and n are selected from 1, 2, 3, 4 and 5;
wherein, each of the above mentioned alkyl, alkenyl, alkynyl, cycloalkyl, heterocyclyl, aryl and heteroaryl is optionally and each independently substituted by 1-3 substituents selected from the group consisting of halogen, C₁₋₄ alkyl, C₁₋₄ haloalkyl, C₂₋₄ alkenyl, C₂₋₄ alkynyl, C₃₋₈ cycloalkyl, 3- to 8-membered heterocyclyl, aryl, heteroaryl, CN, NO₂, OR^{f}, SR^{f}, NR^{d} R^{d}, C(O)R^{g}, C(O)OR^{f}, C(O)NR^{d}R^{d}, NR^{d}C(O)R^{g}, NR^{d}S(O)₂R^{g}, and S(O)₂R^{g}, provided that the chemical structure formed is stable and meaningful; wherein R^{d}, R^{f}, and R^{g} are as defined above;
unless otherwise specified, the aryl is aromatic groups having 6 to 12 carbon atoms; the heteroaryl is 5- to 15-membered (preferably 5- to 12-membered) heteroaromatic groups; and the cyclic structure is saturated or unsaturated cyclic groups with or without heteroatoms.

In another preferred embodiment, formula (I) is formula (XIa) or formula (XIb): 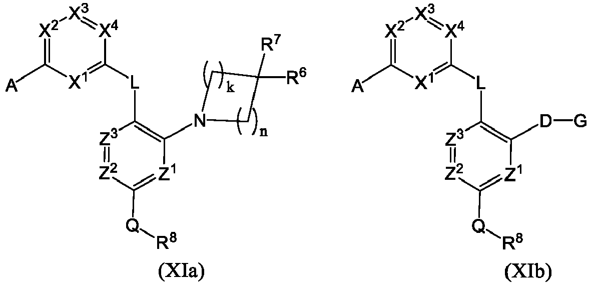 wherein the fragment 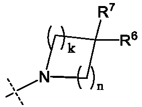 or fragment 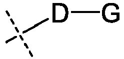 is selected from the following group: 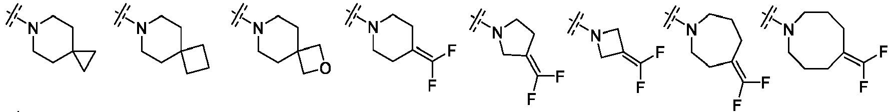 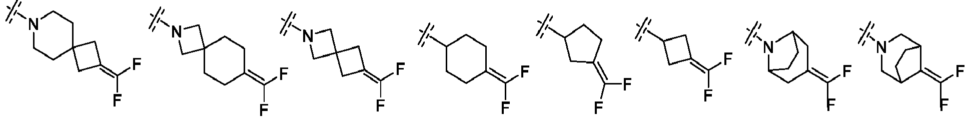 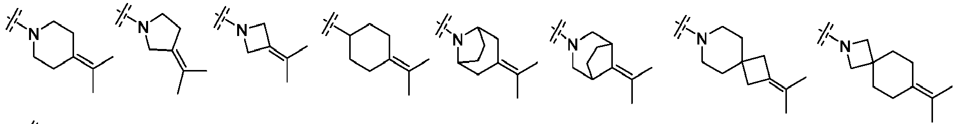 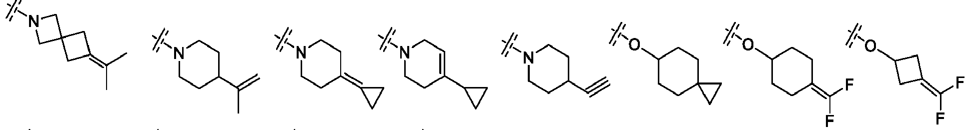 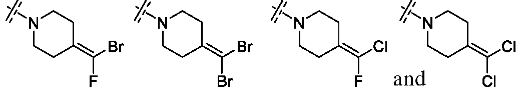 the "-̅-̅-̅-̅-̅-̅" refers to the site of the fragment which attach to the remaining fragment of the formula (XIa) or formula (XIb);
A is a group selected from the group consisting of: 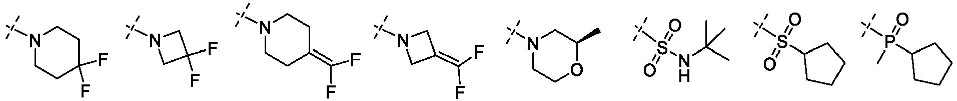 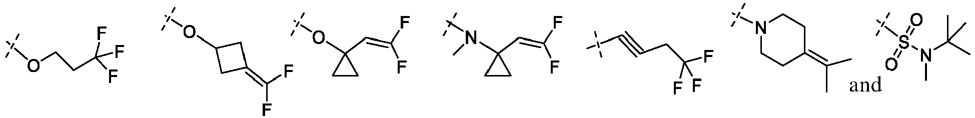
"---" refers to the site of A which attach to the remaining fragment of the formula (XIa) or formula (XIb);
X¹, X², X³ and X⁴ are as defined above;
fragment 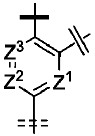 is selected from the group consisting of: 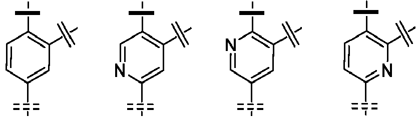 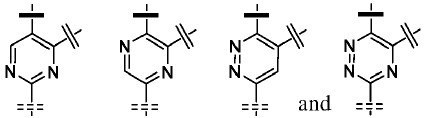
" 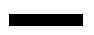 " indicates the site at which the fragment attach to L; " = = = " indicates the site at which attach to Q; 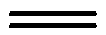 indicates the site at which the fragment attach to 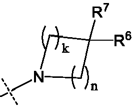 or 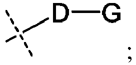
fragment 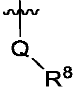 is selected from the group consisting of: 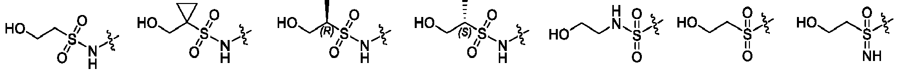 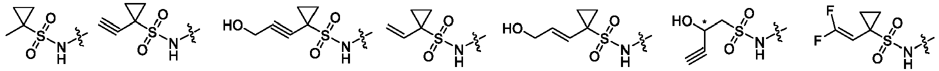 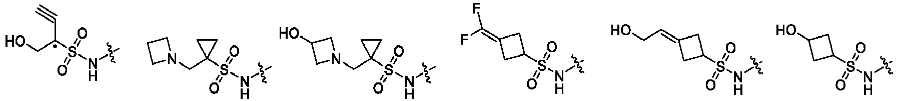 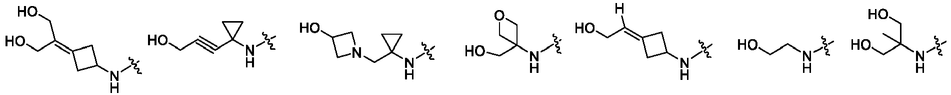 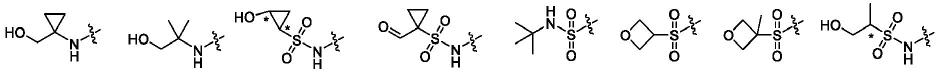 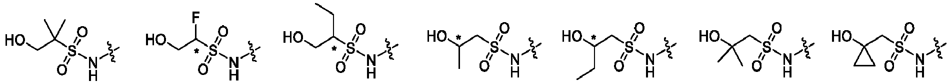 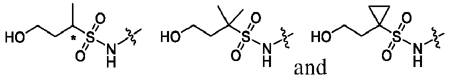
the " 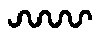 " refers to the site at which the fragment 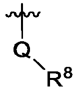 attach to the remaining fragment of the compound of formula (XIa) or formula (XIb);
L is selected from -C(O)NH-, -NHC(O)-, -S(O)₂NH-, and -NHS(O)₂-;
"*" indicates a chiral center.

In another preferred embodiment, the fragment 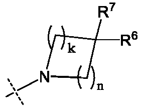 or fragment 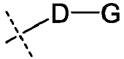 in formula (XIa) or formula (XIb) is selected from the group consisting of: 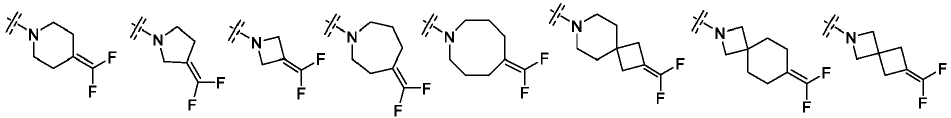 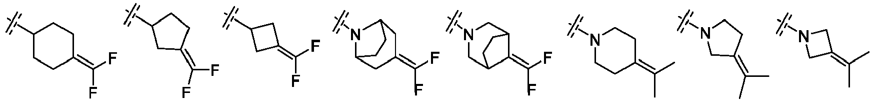 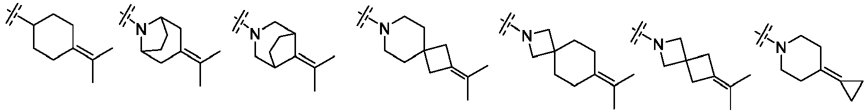 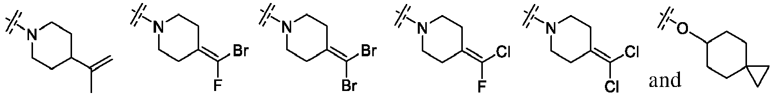 the "-̅-̅-̅-̅-̅-̅" refers to the site at which the fragment attach to the remaining fragment of the compound of formula (XIa) or formula (XIb).

In another preferred embodiment, the fragment 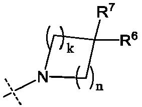 of formula (XIa) is selected from the group consisting of: 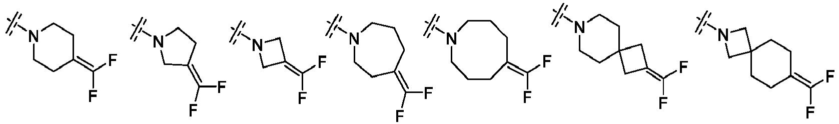 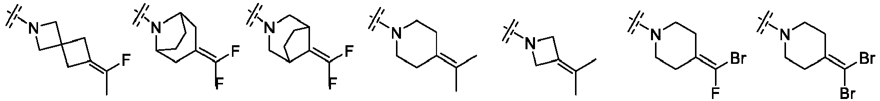 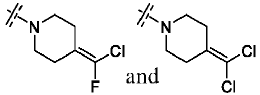 the "-̅-̅-̅-̅-̅-̅" refers to the site at which the fragment attach to the remaining fragment of the compound of formula (XIa).

In another preferred embodiment, formula (I) is formula (XII): 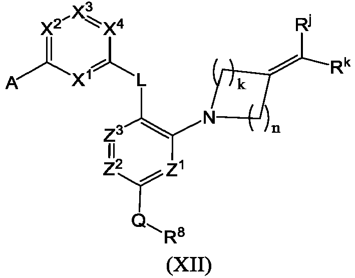
wherein R^{j} and R^{k} are each independently selected from the group consisting of hydrogen, deuterium, fluorine, chlorine, bromine, and C₁₋₄ alkyl;
k and n are selected from 1, 2, 3, 4 and 5;
while the remaining groups are defined as above.

In another preferred embodiment, formula (I) is formula (XIII): 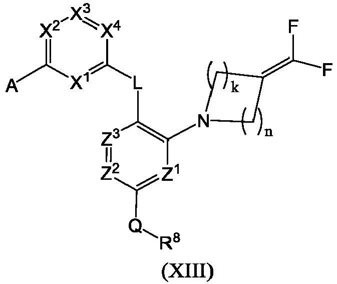
k and n are selected from 1, 2, 3 and 4;
A is a group selected from: 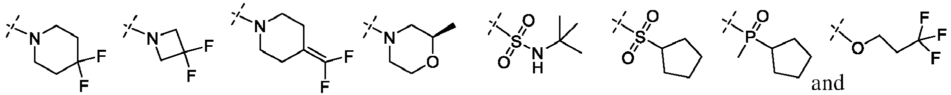
"---" refers to the site of A at which attach to the remaining fragment of the formula (XIII) compound;
fragment 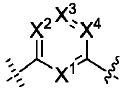 is selected from the group consisting of: 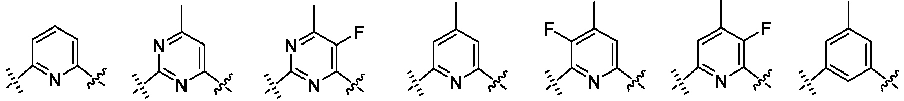 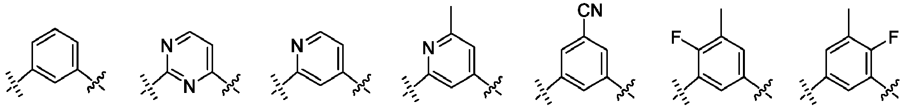 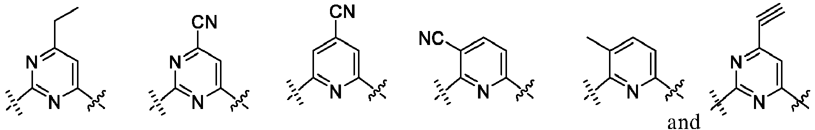
" 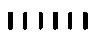 " indicates the site at which the fragment attach to A; " 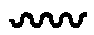 " indicates the site where the fragment attach to L;
fragment 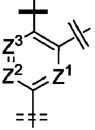 is selected from the group consisting of: 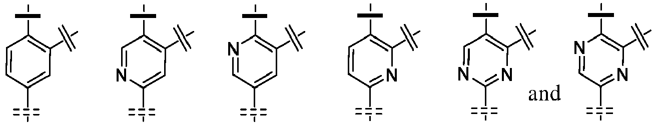
"-" indicates the site at which the fragment attach to L; " = = = " indicates the site attaching to Q; 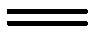 indicates the site at which the fragment attach to 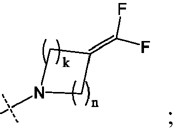
fragment 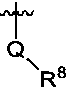 is selected from the group consisting of: 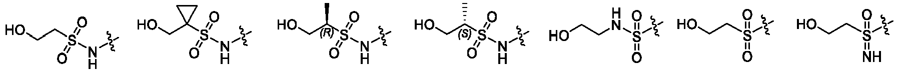 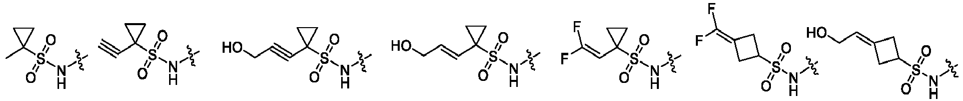 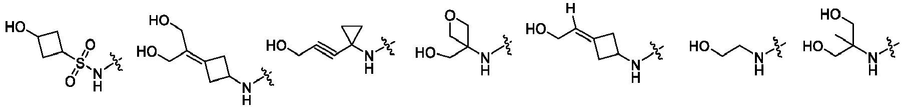 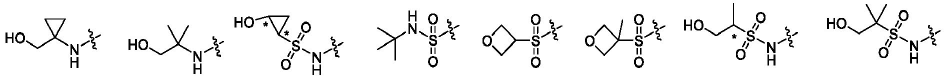 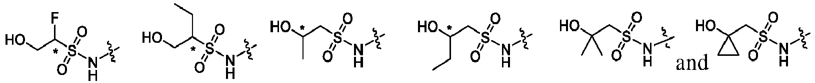
" 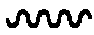 " refers to the site of fragment which attach to the remaining fragment of the formula (XIII) compound;
L is selected from -C(O)NH-, -NHC(O)-, -S(O)₂NH-, and -NHS(O)₂-;
"*" indicates a chiral center.

In another preferred embodiment, formula (I) is formula (XIV): 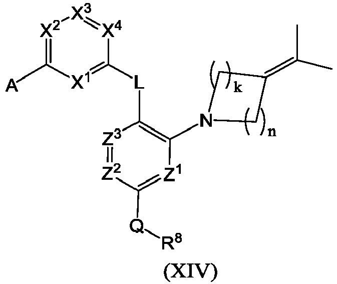 while the groups are defined as above.

In another preferred embodiment, formula (I) is formula (XV): 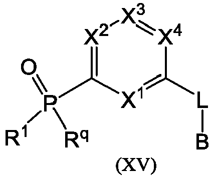 while the groups are defined as above.

In another preferred embodiment, formula (I) is formula (XVII): 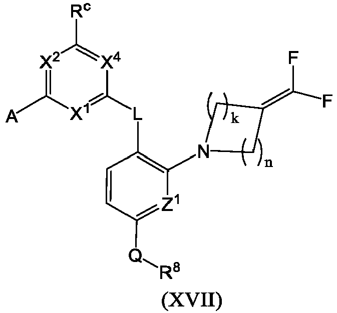
k and n are each independently selected from 1 and 2;
X¹ selected from N, C-H, and C-F;
X² is selected from N, C-H, C-F, C-CN, and C-Me;
X⁴ is selected from C-H, C-F, and N;
Z¹ is selected from C-H, C-F, and N;
R^{c} is selected from H, halogen, C₁₋₄ alkyl, and CN;
L is selected from -C(O)NH- and -NHC(O)-;
While the remaining groups are defined as above.

In another preferred embodiment, formula (I) is formula (XVIII): 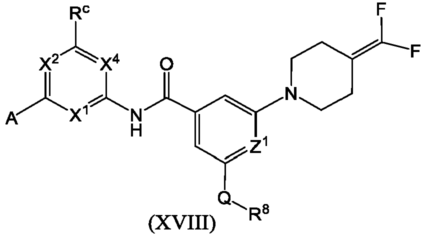
X¹ selected from N, C-H, and C-F;
X² is selected from N, C-H, C-F, C-CN, and C-Me;
X⁴ is selected from C-H, C-F, and N;
Z¹ is selected from C-H, C-F, and N;
R^{c} is selected from hydrogen, halogen, methyl, ethyl, and CN;
A is a group selected from: 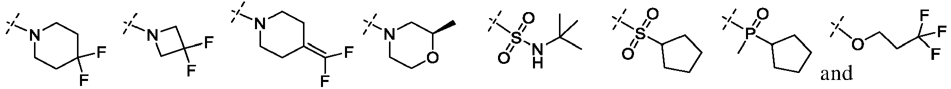
fragment 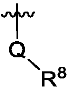 is selected from the group consisting of:

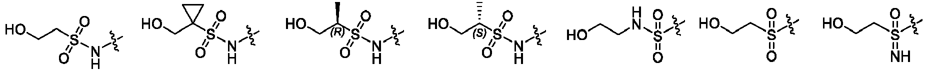

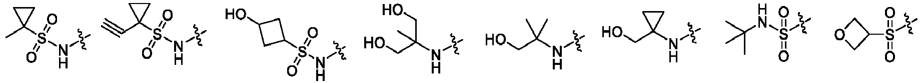

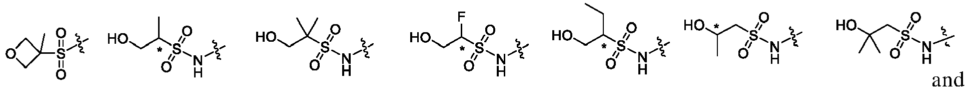

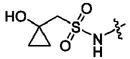

In another preferred embodiment, the fragment A in formula (XIa) or formula (XIb) is selected from the group consisting of: 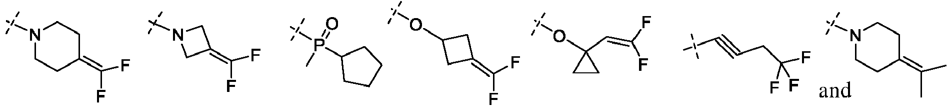 "---" refers to the site of A which attach to the remaining fragment of the formula (XIa) or formula (XIb);

In another preferred embodiment, the compound of formula (I) is selected from the group consisting of: 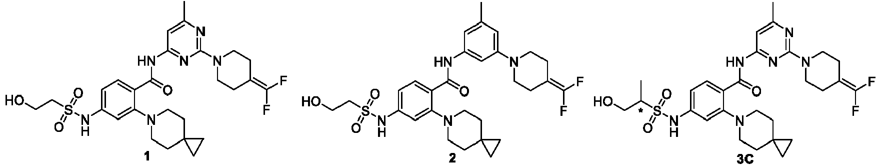 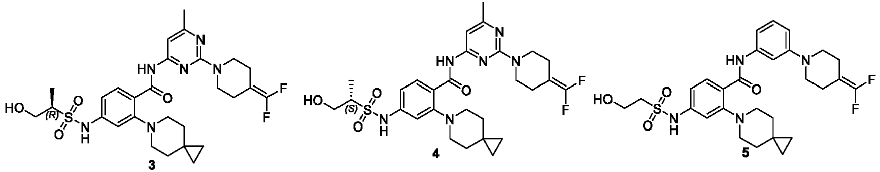 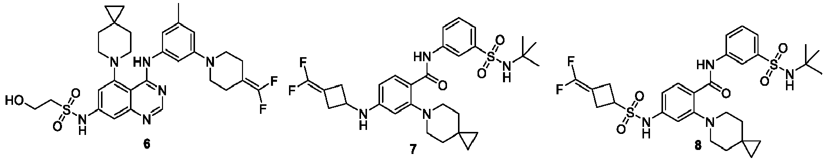 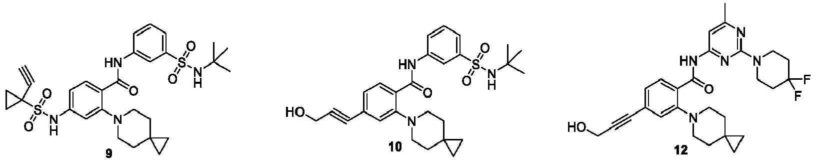 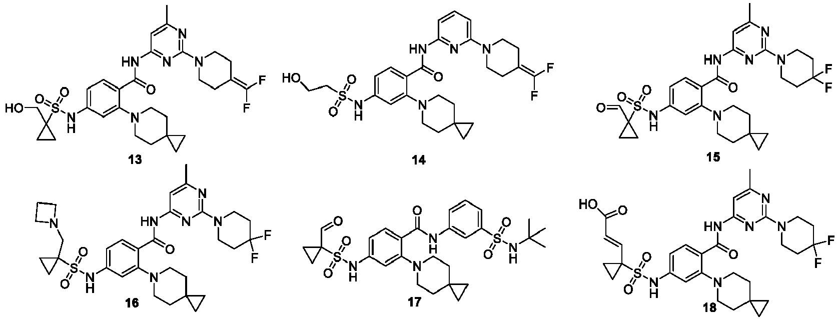 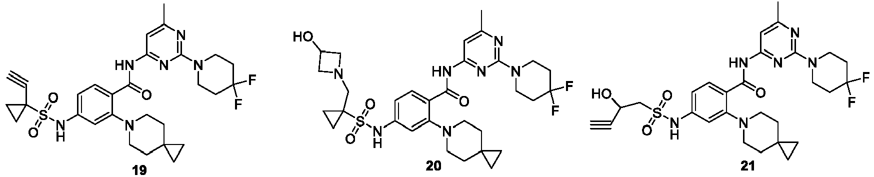 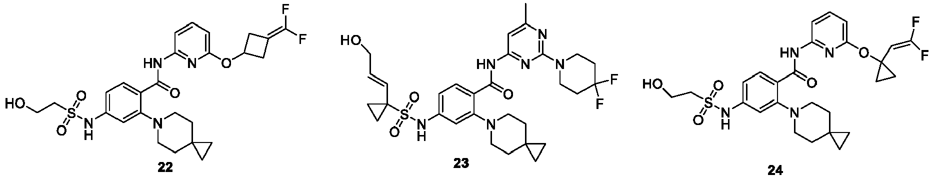 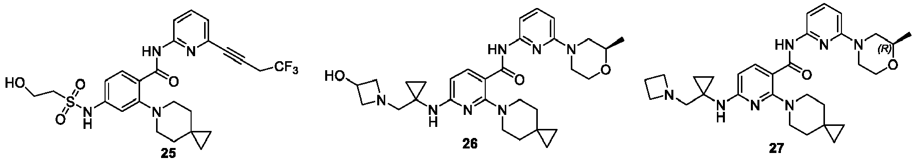 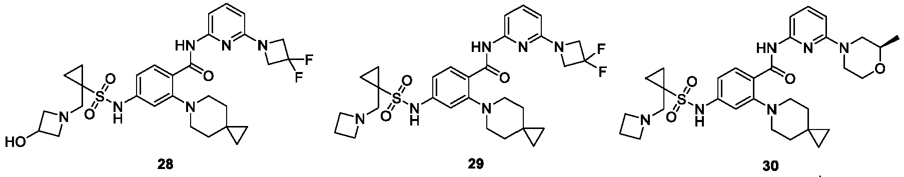 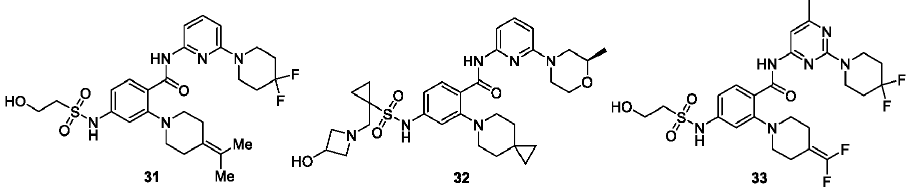 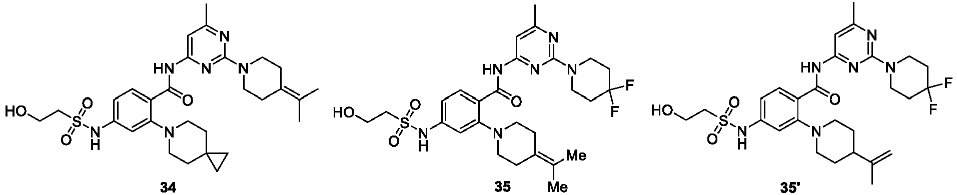 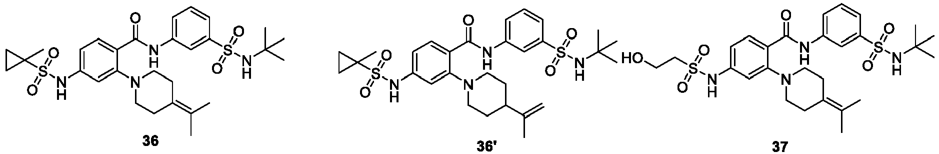 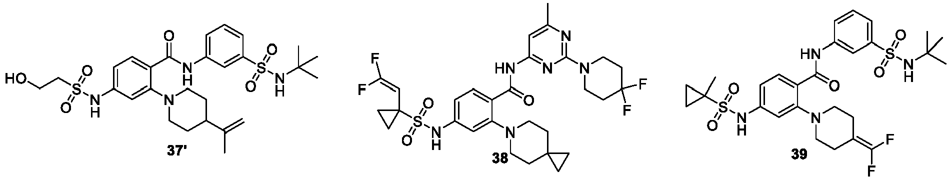 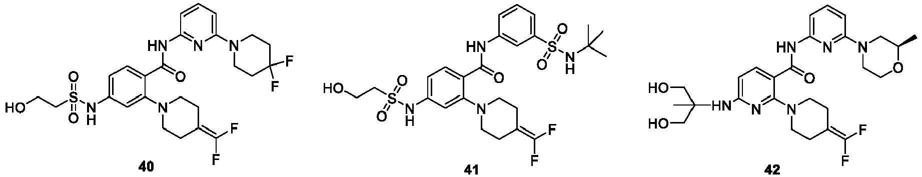 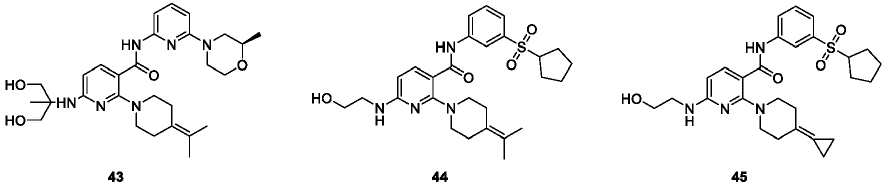 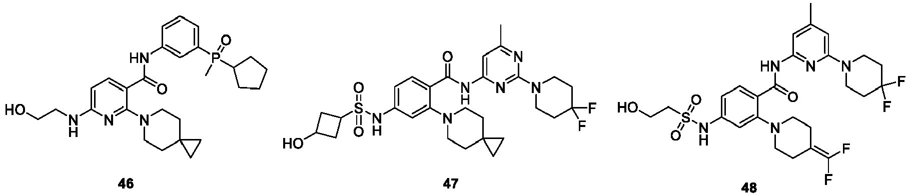 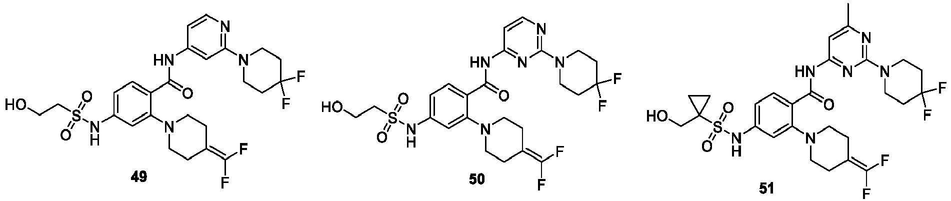 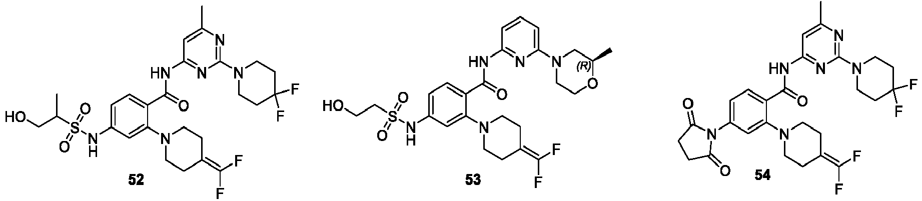 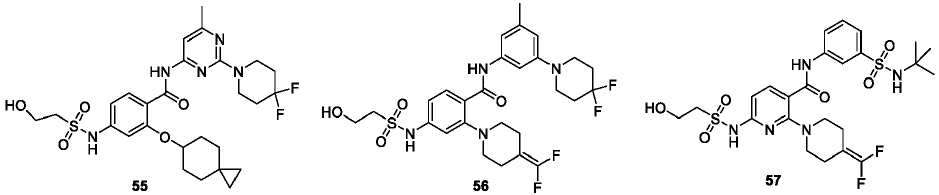 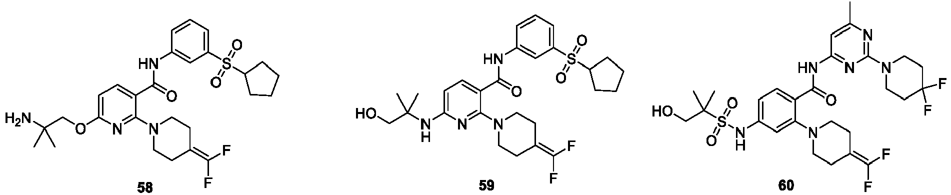 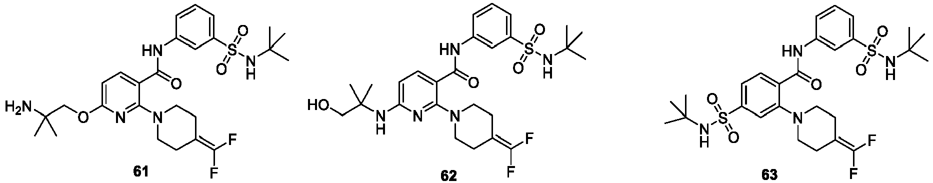 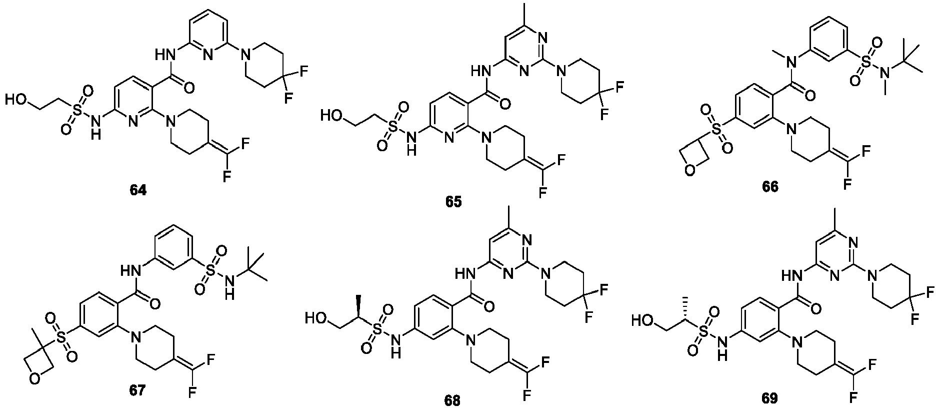 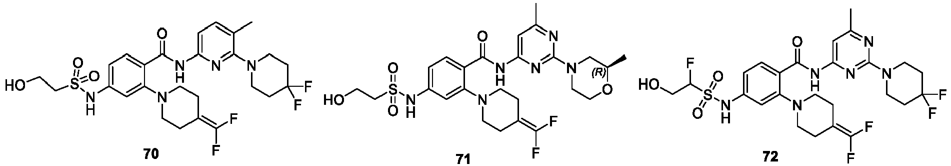 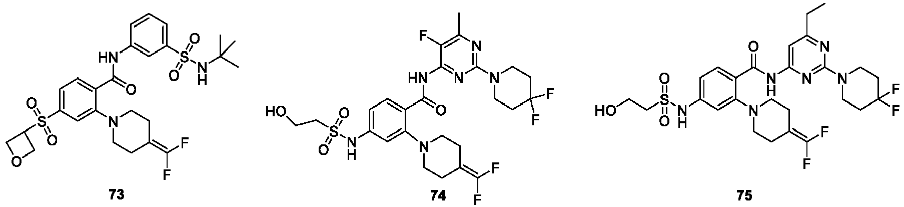 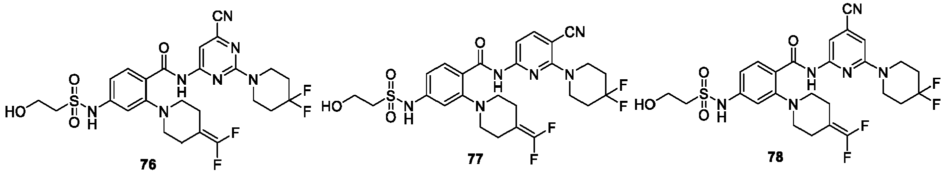 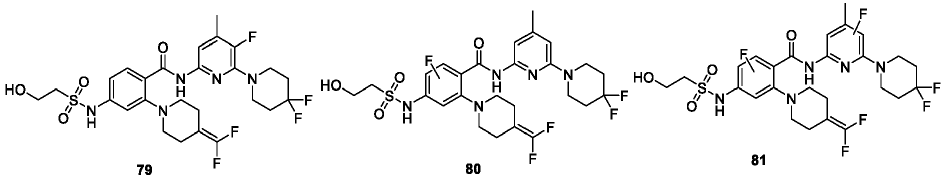 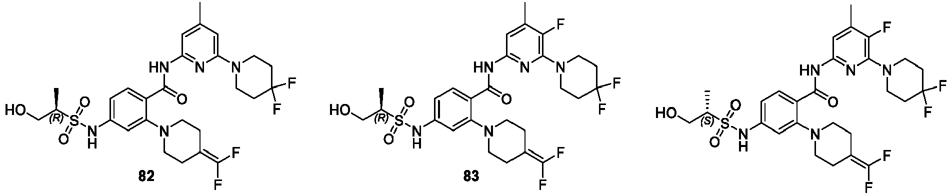 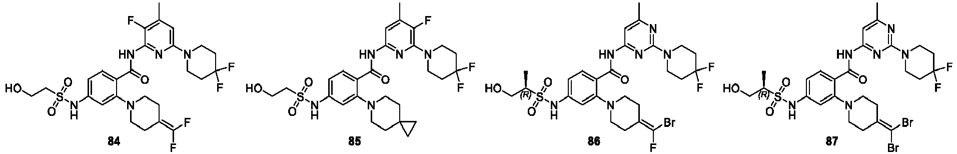 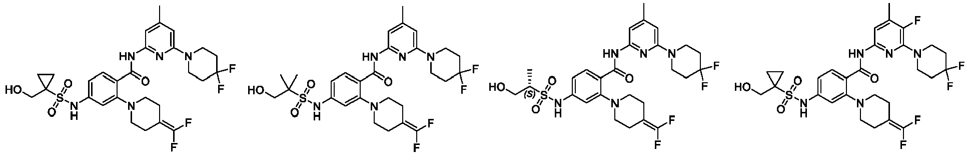 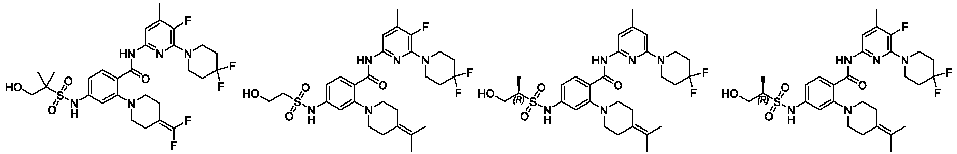 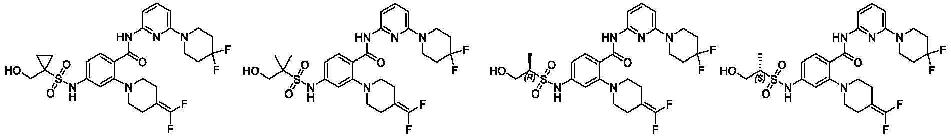 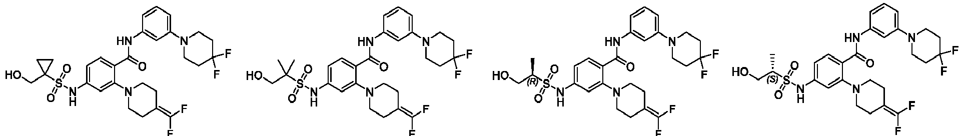 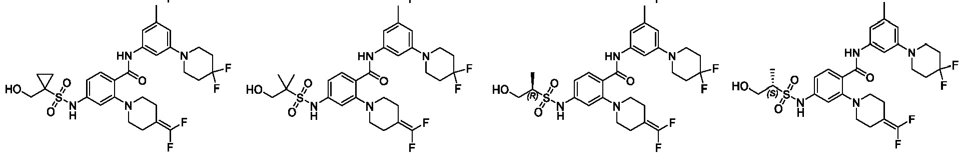 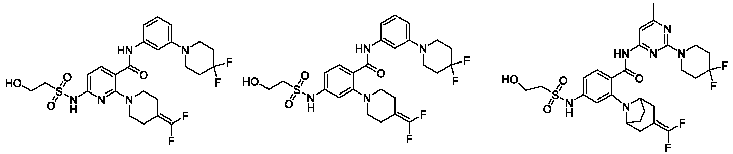 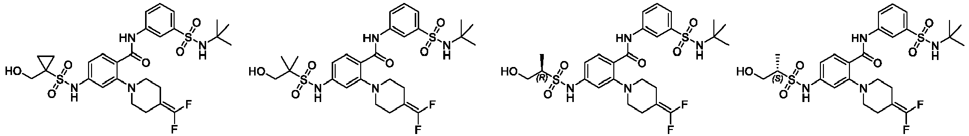 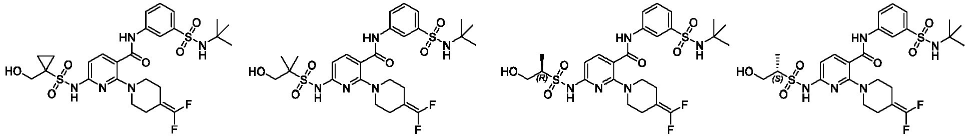 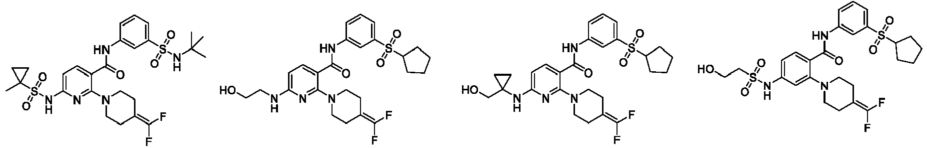 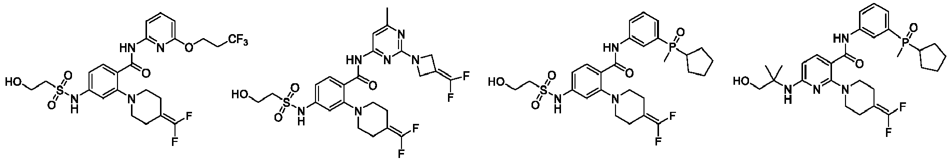 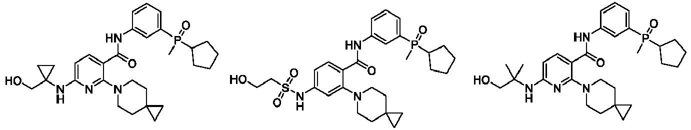 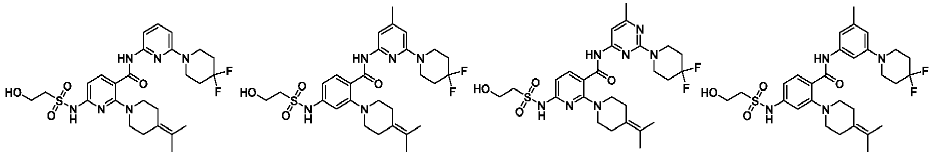 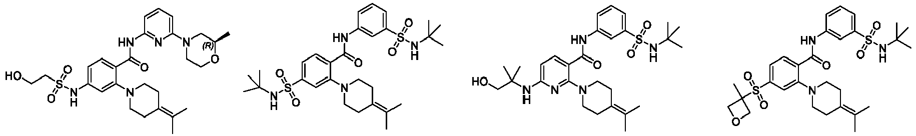 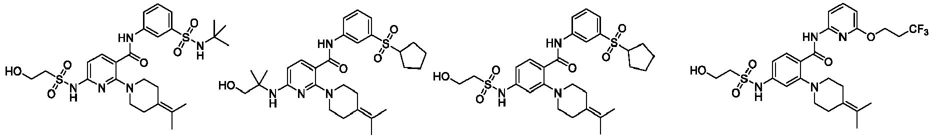 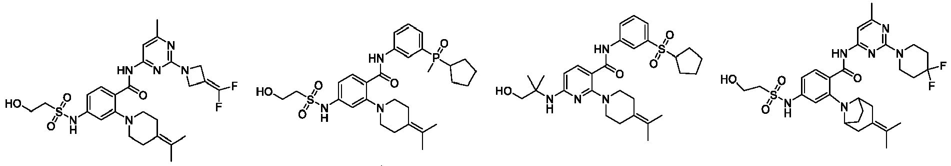 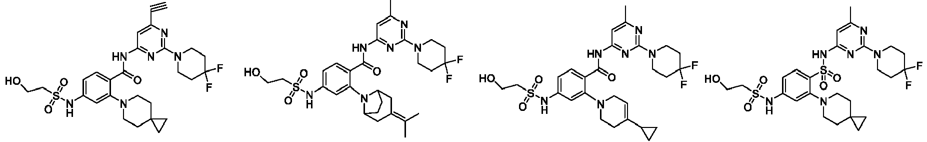 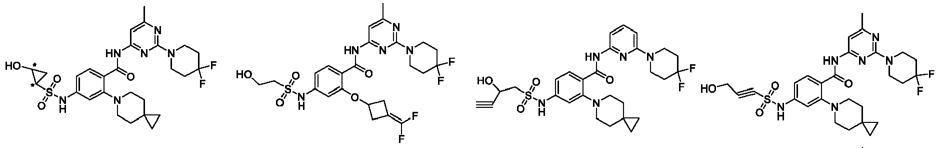 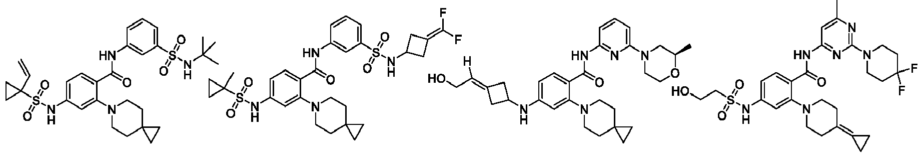 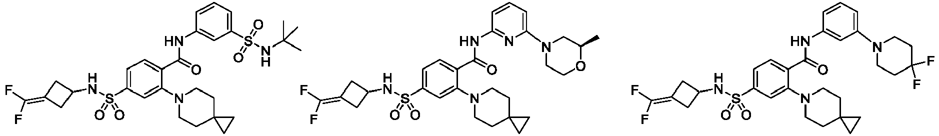 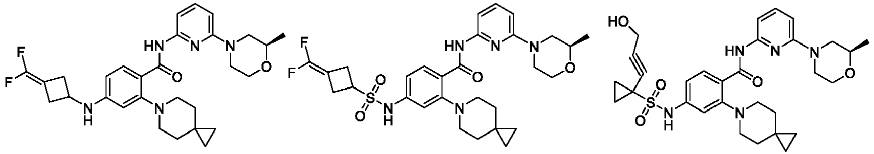 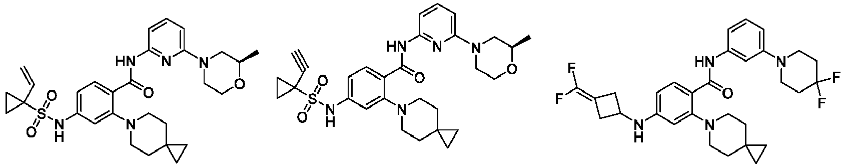 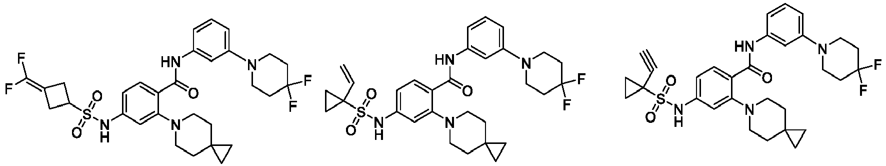 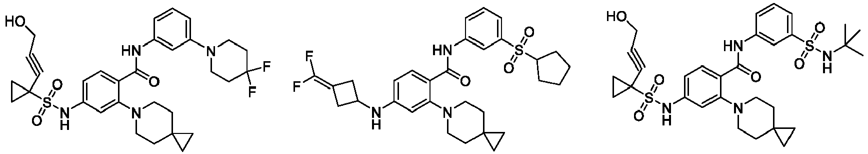 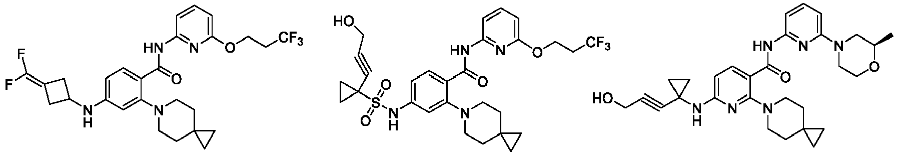 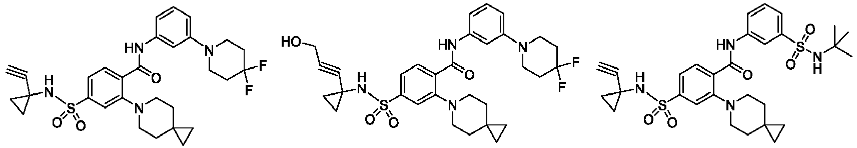 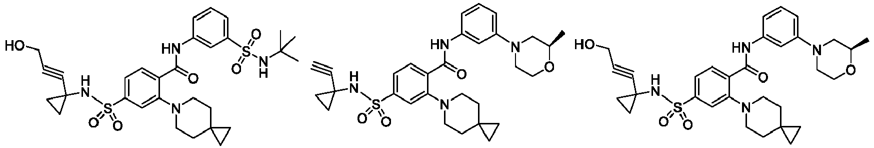 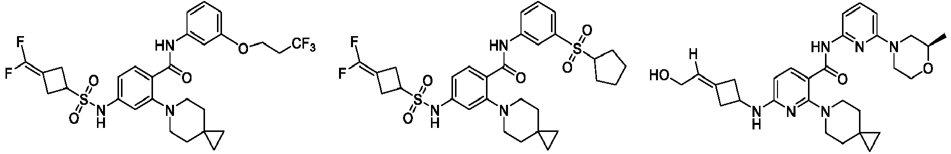 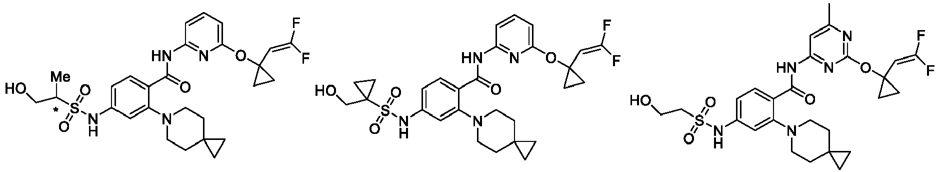 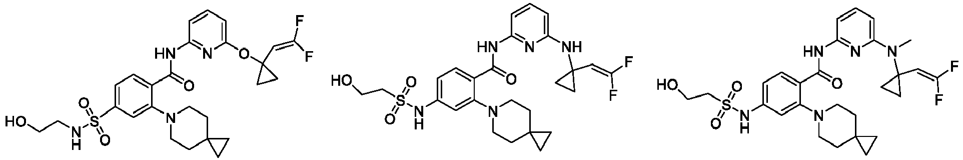 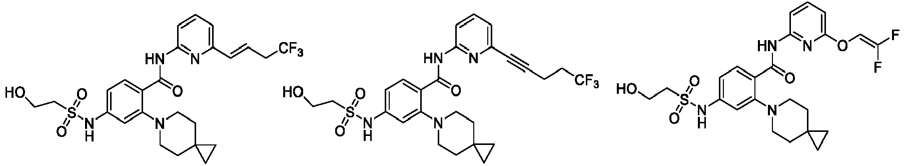 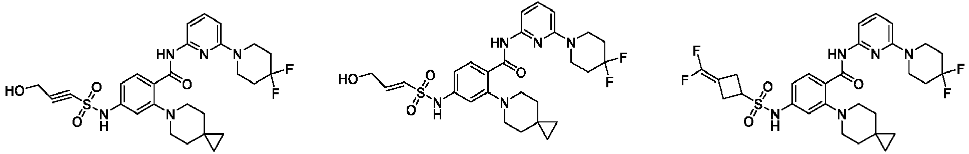 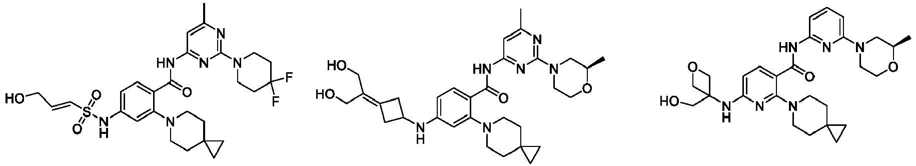 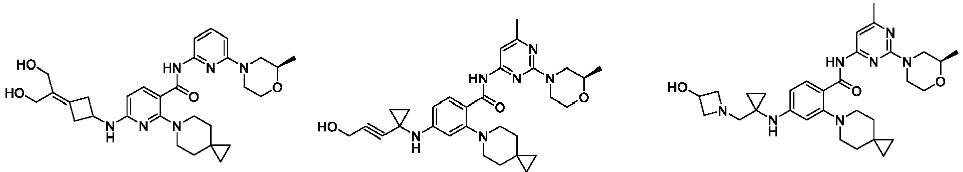 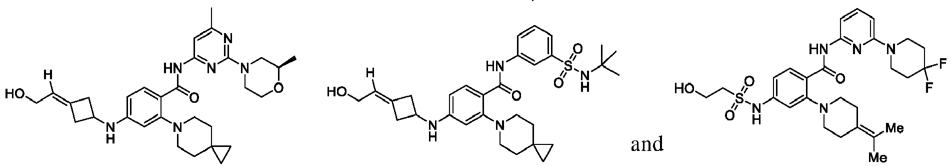 "*" indicates a chiral center.

In the second aspect of the present invention, a pharmaceutical composition is provided, which comprises: (i) compound of the first aspect of the invention, or optical isomers, pharmaceutically acceptable salts, prodrugs, deuterated derivatives, hydrates, or solvates thereof and pharmaceutically acceptable carrier.

In a third aspect of the present invention, a use of the compound according to the first aspect of the present invention, or an optical isomer, a pharmaceutically acceptable salt, a prodrug, a deuterated derivative, a hydrate, or a solvate thereof is provided, for preparing a pharmaceutical composition for treating a disease, disorder, or condition associated with KIF18A activity or expression amount.

In a third aspect of the present invention, the disease, disorder or condition is selected from the group consisting of non-small cell lung cancer, small cell lung cancer, lung adenocarcinoma, lung squamous cell carcinoma, pancreatic cancer, colon cancer, thyroid cancer, embryonal rhabdomyosarcoma, skin granulosa cell tumor, melanoma, hepatocellular carcinoma, intrahepatic cholangiocarcinoma, rectal cancer, bladder cancer, throat cancer, breast cancer, prostate cancer, brain tumor, glioma, ovarian cancer, head and neck squamous cell carcinoma, cervical cancer, esophageal cancer, renal cancer, skin cancer, gastric cancer, myeloid leukemia, lymphoblastic leukemia, myeloid leukemia, B-cell lymphoma, T-cell lymphoma, Hodgkin lymphoma, non-Hodgkin lymphoma, monocytic leukemia, polycythemia megalosplenica, multiple eosinophilic leukocytosis syndrome, bone marrow cancer and other solid tumors and hematological tumors, as well as virus infection such as influenza.

### EMBODIMENTS FOR CARRYING OUT THE INVENTION

After long-term and in-depth research, the present inventors have unexpectedly discovered a class of structurally novel KIF18A inhibitors, as well as their preparation methods and applications. The compounds of the present invention can be applied to the treatment of various diseases associated with the activity of the ATP hydrolase. The present invention is completed on this basis.

### Terminology

Unless otherwise stated, "or" as used herein has the same meaning as "and/or" (refers to "or" and "and").

Unless otherwise specified, among all compounds of the present invention, each chiral carbon atom (chiral center) may optionally be in the R configuration or the S configuration, or a mixture of the R configuration and the S configuration.

As used herein, the term "alkyl", alone or as part of another substituent, refers to a straight (ie, unbranched) or branched saturated hydrocarbon group containing only carbon atoms, or a combination of straight and branched chains. When the alkyl group has a carbon number limitation (e.g., C₁₋₁₀), it means that the alkyl group has 1 to 10 carbon atoms. For example, C₁₋₈ alkyl refers to an alkyl group containing from 1 to 8 carbon atoms, including methyl, ethyl, propyl, isopropyl, butyl, isobutyl, sec-butyl, tert-butyl, or the like.

As used herein, the term "alkenyl", when used alone or as part of another substituent, refers to a straight or branched, carbon chain group having at least one carbon-carbon double bond. Alkenyl groups can be substituted or unsubstituted. When the alkenyl group has a carbon number limit (e.g., C₂₋₈), it means that the alkenyl group has 2-8 carbon atoms. For example, C₂₋₈ alkenyl refers to alkenyl groups having 2-8 carbon atoms, including ethenyl, propenyl, 1,2-butenyl, 2,3-butenyl, butadienyl, or the like.

As used herein, the term "alkynyl", when used alone or as part of another substituent, refers to an aliphatic hydrocarbon group having at least one carbon-carbon triple bond. The alkynyl group can be straight or branched, or a combination thereof. When the alkynyl group has a carbon number limitation (e.g., C₂₋₈ alkynyl group), it means that the alkynyl group has 2 to 8 carbon atoms. For example, the term "C₂₋₈ alkynyl" refers to a straight or branched alkynyl group having 2-8 carbon atoms, including ethynyl, propynyl, isopropynyl, butynyl, isobutynyl, secondary Butynyl, tert-butynyl, or the like.

As used herein, when used alone or as part of another substituent, the term "cycloalkyl" refers to a unit ring having a saturated or partially saturated ring, a bicyclic or polycyclic (fused ring, bridged or spiro) ring system.. When a certain cycloalkyl group has a carbon number limitation (e.g., C₃₋₁₀), it means that the cycloalkyl group has 3 to 10 carbon atoms. In some preferred embodiments, the term "C3-8 cycloalkyl" refers to a saturated or partially saturated monocyclic or bicyclic alkyl group having from 3 to 8 carbon atoms, including cyclopropyl, cyclobutyl, cyclopentyl, cycloheptyl, or the like. "Spirocycloalkyl" refers to a bicyclic or polycyclic group that shares a carbon atom (called a spiro atom) between the monocyclic rings. These may contain one or more double bonds, but none of the rings have fully conjugated π electrons system.

"Fused cycloalkyl" means an all-carbon bicyclic or polycyclic group in which each ring of the system shares an adjacent pair of carbon atoms with other rings in the system, wherein one or more of the rings may contain one or more Key, but none of the rings have a fully conjugated π-electron system. "Bridge cycloalkyl" refers to an all-carbon polycyclic group in which two rings share two carbon atoms that are not directly bonded, which may contain one or more double bonds, but none of the rings have a fully conjugated pi-electron system. The atoms contained in the cycloalkyl group are all carbon atoms. Some examples of cycloalkyl groups are as follows, and the present invention is not limited to the following cycloalkyl groups.

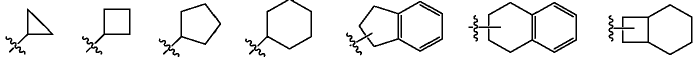

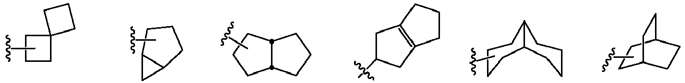

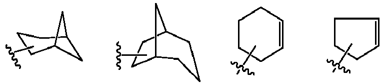

Unless otherwise stated, the following terms used in the specification and claims have the following meanings. "Aryl" means an all-carbon monocyclic or fused polycyclic (ie, a ring that shares a pair of adjacent carbon atoms) groups having a conjugated π-electron system, such as phenyl and naphthyl. The aryl ring may be fused to other cyclic groups (including saturated and unsaturated rings), but may not contain heteroatoms such as nitrogen, oxygen, or sulfur, while the point of attachment to the parent must be on the carbon atoms of the ring in a conjugated pi-electron system. The aryl group can be substituted or unsubstituted. The following are some examples of aryl groups, and the present invention is not limited to the aryl groups described below.

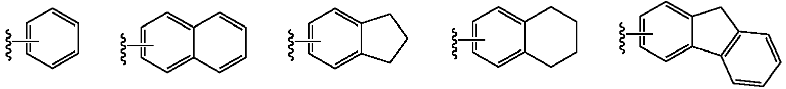

"Heteroaryl" means an aromatic monocyclic or polycyclic group comprising one to more heteroatoms selected from nitrogen, oxygen and sulfur, or a polycyclic group comprising a heterocyclic group comprising one to more heteroatoms selected from nitrogen, oxygen and sulfur in combination with an aryl group, where the linkage site is located on the aryl group.The heteroaryl group can be optionally substituted or unsubstituted. The following are some examples of heteroaryl groups, and the present invention is not limited to the following heteroaryl groups.

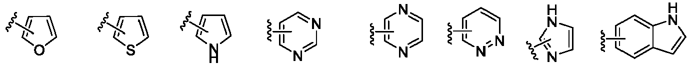

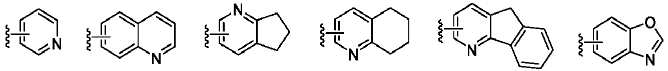

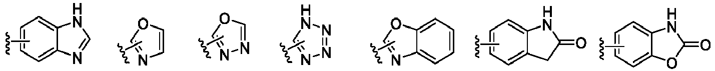

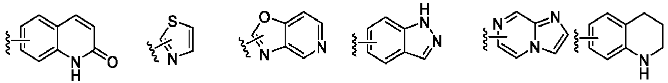

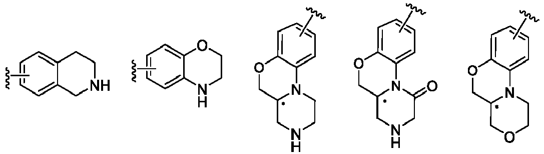

"Heterocyclyl" means a saturated or partially unsaturated monocyclic or polycyclic cyclic hydrocarbon substituent wherein one or more of the ring atoms are selected from nitrogen, oxygen or sulfur and the remaining ring atoms are carbon. Non-limiting examples of monocyclic heterocyclic groups include pyrrolidinyl, piperidinyl, piperazinyl, morpholinyl, thiomorpholinyl, homopiperazinyl. Polycyclic heterocyclic group refers to a heterocyclic group including a spiro ring, a fused ring, and a bridged ring. "Spirocyclic heterocyclyl" refers to a polycyclic heterocyclic group in which each ring of the system shares an atom (referred to as a spiro atom) with other rings in the system, wherein one or more of the ring atoms is selected from the group consisting of nitrogen and oxygen. Or sulfur, the remaining ring atoms are carbon. "Fused ring heterocyclyl" refers to a polycyclic heterocyclic group in which each ring of the system shares an adjacent pair of atoms with other rings in the system, and one or more rings may contain one or more double bonds, but none One ring has a fully conjugated pi-electron system, and wherein one or more ring atoms are selected from nitrogen, oxygen or sulfur, and the remaining ring atoms are carbon. "Bridged heterocyclyl" refers to a polycyclic heterocyclic group in which any two rings share two atoms which are not directly bonded, these may contain one or more double bonds, but none of the rings have a fully conjugated pi-electron system, and wherein one or more of the ring atoms are selected from nitrogen, oxygen or sulfur, and the remaining ring atoms are carbon. If a heterocyclic group has both a saturated ring and an aromatic ring (for example, the saturated ring and the aromatic ring are fused together), the point attached to the parent must be on the saturated ring. Note: When the point attached to the parent is on the aromatic ring, it is called a heteroaryl group and is not called a heterocyclic group. Some examples of the heterocyclic group are as follows, and the present invention is not limited to the following heterocyclic group.

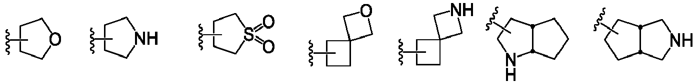

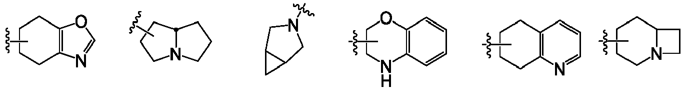

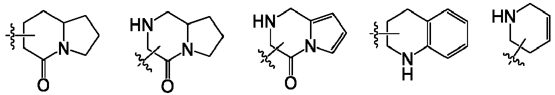

As used herein, the term "halogen", when used alone or as part of another substituent, refers to F, Cl, Br, and I.

As used herein, the term "substituted" (when with or without "optionally") means that one or more hydrogen atoms on a particular group are replaced by a particular substituent. Particular substituents are the substituents described above in the corresponding paragraphs, or the substituents which appear in the examples. Unless otherwise stated, an optionally substituted group may have a substituent selected from a particular group at any substitutable position of the group, and the substituents may be the same or different at each position. A cyclic substituent, such as a heterocyclic group, may be attached to another ring, such as a cycloalkyl group, to form a spirobicyclic ring system, i.e., the two rings have a common carbon atom. Those skilled in the art will appreciate that the combinations of substituents contemplated by the present invention are those that are stable or chemically achievable. The substituents are, for example but not limited to, C₁₋₈ alkyl, C₂₋₈ alkenyl, C₂₋₈ alkynyl, C₃₋₈ cycloalkyl, 3- to 12-membered heterocyclic, aryl, Heteroaryl, halogen, hydroxy, carboxy (-COOH), C₁₋₈ aldehyde, C₂₋₁₀ acyl, C₂₋₁₀ ester, amino.

For convenience and in accordance with conventional understanding, the term "optionally substituted" or "optionally substituted" applies only to sites which are capable of being substituted by a substituent, and does not include those which are not chemically achievable.

As used herein, unless otherwise specified, the term "pharmaceutically acceptable salt" refers to a salt that is suitable for contact with the tissue of a subject (eg, a human) without causing unpleasant side effects. In some embodiments, a pharmaceutically acceptable salt of a compound of the invention includes a salt (eg, a potassium salt, a sodium salt, a magnesium salt, a calcium salt) of a compound of the invention having an acidic group or is basic A salt of a compound of the invention (e.g., a sulfate, a hydrochloride, a phosphate, a nitrate, a carbonate).

### The Use

The present invention provides the use of a class of compounds of formula (I), or their deuterated derivatives, their salts, isomers (enantiomers or diastereomers, if present), hydrates, pharmaceutically acceptable carriers or excipients for the inhibition of KIF18A.

The compounds of the present invention are useful as a KIF18A inhibitor.

The present invention is a single inhibitor of KIF18A, which can prevent, alleviate or cure diseases by modulating the activity of KIF18A. The disease comprises but are not limited to: non-small cell lung cancer, small cell lung cancer, lung adenocarcinoma, lung squamous cell carcinoma, pancreatic cancer, colon cancer, thyroid cancer, embryonal rhabdomyosarcoma, skin granulosa cell tumor, melanoma, hepatocellular carcinoma, intrahepatic cholangiocarcinoma, rectal cancer, bladder cancer, throat cancer, breast cancer, prostate cancer, brain tumor, glioma, ovarian cancer, head and neck squamous cell carcinoma, cervical cancer, esophageal cancer, renal cancer, skin cancer, gastric cancer, myeloid leukemia, lymphoblastic leukemia, myeloid leukemia, B-cell lymphoma, T-cell lymphoma, Hodgkin lymphoma, non-Hodgkin lymphoma, monocytic leukemia, polycythemia megalosplenica, multiple eosinophilic leukocytosis syndrome, bone marrow cancer and other solid tumors and hematological tumors, as well as virus infection such as influenza.

The compounds of the invention and deuterated derivatives thereof, and the pharmaceutically acceptable salts or isomers thereof, if present, or hydrates thereof, and/or compositions thereof, may be formulated with a pharmaceutically acceptable excipient or carrier, and the resulting compositions may be administered in vivo to mammals, such as men, women and animals, for the treatment of disorders, conditions and diseases. The compositions may be: tablets, pills, suspensions, solutions, emulsions, capsules, aerosols, sterile injectable solutions, sterile powder, etc. In some embodiments, pharmaceutically acceptable excipients include microcrystalline cellulose, lactose, sodium citrate, calcium carbonate, dibasic calcium phosphate, mannitol, hydroxypropyl-β-cyclodextrin, β-cyclodextrin (increase), glycine, disintegrants (such as starch, croscarmellose sodium, complex silicates, and macromolecular polyethylene glycols), granulation binders (such as polyvinylpyrrolidone, sucrose, gelatin, and acacia), and lubricants (such as magnesium stearate, glycerin, and talc). In a preferred embodiment, the pharmaceutical composition is in a dosage form suitable for oral administration, including but not limited to tablets, solutions, suspensions, capsules, granules, powders. The amount of a compound or pharmaceutical composition of the present invention administered to a patient is not fixed and is generally administered in a pharmaceutically effective amount. Also, the amount of the compound actually administered may be determined by a physician in the light of the circumstances, including the condition to be treated, the chosen route of administration, the actual compound administered, the individual condition of the patient, and the like. The dosage of the compound of the present invention will depend on the particular use of the therapy, the mode of administration, the condition of the patient, and the judgment of the physician. The proportion or concentration of the compound of the present invention in the pharmaceutical composition depends on a variety of factors, including dosage, physicochemical properties, route of administration, and the like.

It should be understood that, in the present invention, each of the technical features specifically described above and below (such as those in the Examples) can be combined with each other, thereby constituting new or preferred technical solutions which need not be specified again herein

### PHARMACEUTICAL COMPOSITION AND THE ADMINISTRATION THEREOF

Since the compound of the present invention has excellent inhibitory activity on KIF18A, the compound of the present invention and its various crystal forms, pharmaceutically acceptable inorganic or organic salts, hydrates or solvates, and pharmaceutical compositions containing the compound of the present invention as a main active ingredient can be used for the treatment, prevention and alleviation of diseases related to the activity or expression of KIF18A.

The pharmaceutical composition of the invention comprises the compound of the present invention or the pharmaceutically acceptable salts thereof in a safe and effective dosage range and pharmaceutically acceptable excipients or carriers. Wherein the "safe and effective dosage" means that the amount of compound is sufficient to significantly ameliorate the condition without causing significant side effects. Generally, the pharmaceutical composition contains 1-2000 mg polymorphs of the invention per dose, preferably, 5-200mg polymorphs of the invention per dose. Preferably, the "dose" is a capsule or tablet.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc.

There is no special limitation of administration mode for the compound or pharmaceutical compositions of the present invention, and the representative administration mode includes (but is not limited to): oral, intratumoral, rectal, parenteral (intravenous, intramuscular or subcutaneous), and topical administration.

Solid dosage forms for oral administration include capsules, tablets, pills, powders and granules. In these solid dosage forms, the active compounds are mixed with at least one conventional inert excipient (or carrier), such as sodium citrate or CaHPO₄, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or the combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

The dosage forms for topical administration of compounds of the invention include ointments, powders, patches, aerosol, and inhalants. The active ingredients are mixed with physiologically acceptable carriers and any preservatives, buffers, or propellant if necessary, under sterile conditions.

Compounds of the present invention can be administrated alone, or in combination with any other pharmaceutically acceptable compounds.

When the pharmaceutical compositions are used, a safe and effective amount of compound of the present invention is applied to a mammal (such as human) in need of, wherein the dose of administration is a pharmaceutically effective dose. For a person weighed 60 kg, the daily dose is usually 1-2000 mg, preferably 5-500mg. Of course, the particular dose should also depend on various factors, such as the route of administration, patient healthy status, which are well within the skills of an experienced physician.

### The main advantages of the present invention are:

1. Provided a compound of formual I.
2. Provided a KIF18A inhibitor with a novel structure, preparation and use thereof, wherein the inhibitor can inhibit the activity of KIF18A at a very low concentration.
3. Provided a KIF18A inhibitor of good oral absorbability.
4. Provided a class of pharmaceutical compositions for the treatment of diseases associated with KIF18A activity.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

Some representative compounds of the present invention can be prepared by the following synthetic methods, the following reaction formula, the reagents and conditions of each step can be used in the field for this type of preparation method conventional reagents or conditions, after the structure of the compound of the present invention is disclosed, the above selection can be carried out by the skilled person in the art according to the knowledge of the field.

### Abbreviations

Ac = Acetyl
Boc = tert-butoxycarbonyl
t-BuOK = potassium tert-butoxide
m-CPBA = m-chloroperbenzoic acid
DAST = Diethylamine sulfur trifluoride
DCM = dichloromethane
DIPEA or DIEA = diisopropylethylamine
DIBAL-H = diisobutyl aluminium hydride
DMF = dimethylform amide
DMAP = 4-dimethylaminopyridine
DMSO = dimethyl sulfoxide
EtOAc or EA = ethyl acetate
HATU = 1-[Bis(dimethylamino)methylene]-1 H-1,2,3 -triazolo [4,5-b]pyridinium 3-oxid hexafluorophosphate
LiHMDS = lithium bis(trimethylsily)amide
Me = Methyl
NMP = N-methylpyrrolidone
Ph = Benzyl
i-PrOH = isopropyl alcohol
TBAF = tetrabutylammonium fluoride solution
TIPS = triisopropylsilyl group
TEA = triethylamine
Tf = trifluoromethanesulfonyl
TFA = Trifluoroacetic acid
THF = Tetrahydrofuran

### Example 1: Preparation of Compound 1

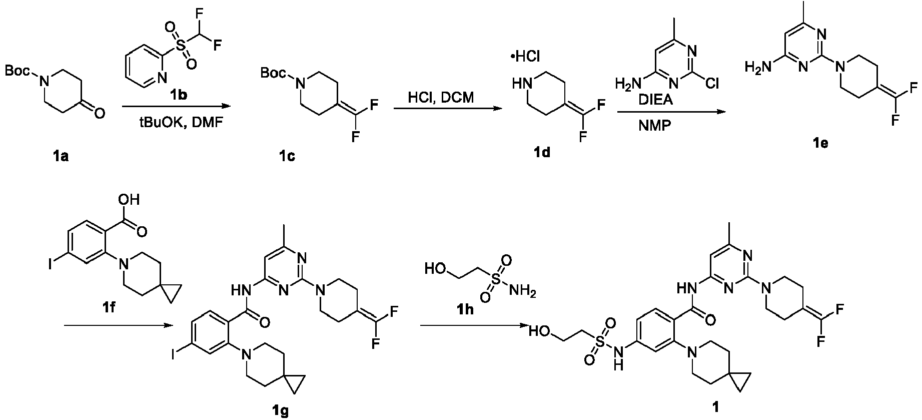

At -50 °C to -60 °C, to a solution of tBuOK (1.7 g, 15.05 mmol) in DMF (15 mL) was added a solution of **1a** (2 g, 10.03 mmol) and **1b** (1.63 g, 8.36 mmol) in DMF (10 mL). The reaction mixture was stirred for 30 min. Then sat.NaHCO₃ (5 mL, sat.: saturated,) and 3 M HCl (13 mL) was added at -50 °C. Then the reaction solution was raised to RT (room temperature) naturally. Water (80.0 mL) was added and extracted with EA (150 mL). The collected organic phase was dried with anhydrous sodium sulfate, filterated and concentrated to give a colorless oil compound **1c** (1.75 g, 75% yield).

To a solution of **1c** (1 g, 4.28 mmol) in DCM (10 mL) was added 4 M HCl/dioxane solution (3 mL) at RT. The mixture was stirred for 4 h at RT. The mixture was concentrated and the crude was washed with DCM, dried to give a white solid (520.8 mg, 90% yield). ¹H NMR (500 MHz, DMSO-*d*₆) δ 9.32 (br, 2H), 3.07 (t, *J* = 5.5 Hz, 4H), 2.38 (t, *J* = 5.5 Hz, 4H) ppm.

The 2-Chloro-4-amino-6-methylpyrimidine (200 mg, 1.39 mmol) was dissolved in NMP (2 mL). Then **1d** (307 mg, 1.81 mmol) and diisopropylethylamine (540 mg, 4.18 mmol) were added. The mixture was stirred at 140 °C for 8 h under a sealed tube. After the reaction was cooled to RT, water (5 mL) was added to quench the reaction. The mixture was extracted with EA(15 mL × 3). The collected organic phase was dried with anhydrous sodium sulfate, filterated and concentrated. The crude was purified by column chromatography (PE: EA=10:1) (PE: petroleum ether) to give a white solid compound **1e** (140 mg, 40%). MS *m*/*z* 241.4 [M+H]⁺.

**1f** was synthesized according to WO2020/132648. The compound **1e** (67 mg, 0.28 mmol) was dissolved in MeCN (acetonitrile, 2 mL). **1f** (100 mg, 0.28 mmol), diisopropylethylamine (540 mg, 4.18 mmol) and HATU (160 mg, 0.42 mmol) were added and the reaction was stirred at RT for 12 h. Water (5 mL) was added to quench the reaction. The mixture was extracted with EA (10 mL × 3). The collected organic phase was dried with anhydrous sodium sulfate, filterated and concentrated. The crude was purified by column chromatography (PE: EA=20:1) to give a white solid compound **1g** (40 mg, 25%). MS *m*/*z* 580.5[M+H]⁺

To a solution of **1h** (7.50 mg, 0.06 mmol) in DMF (2 mL) was added sarcosine (0.60 mg, 0.008 mmol), CuI (CuI:Cuprous iodide, 1.40 mg, 0.008 mmol) and tripotassium phosphate (19 mg, 0.09 mmol). The reaction was stirred at 50 °C for 5 min under a sealed tube. Then **1g** (20 mg, 0.03 mmol) was added and the mixture was stirred at 100 °C for 4 h under N₂. The reaction was quenched with sat.NH₄Cl and extracted with EA. The collected organic phase was dried with anhydrous sodium sulfate, filterated and concentrated. The crude was purified by column chromatography (PE: EA = 1:1) to give a white solid compound **1** (5 mg, 24%). ¹H NMR (500 MHz, CD₃OD) δ 8.11 (d, *J* = 8.7 Hz, 1H), 7.40 (s, 1H), 7.30 (d, *J* = 2.0 Hz, 1H), 7.14 (dd, *J* = 8.7, 2.0 Hz, 1H), 3.94 (t, *J* = 6.3 Hz, 2H), 3.90-3.87 (m, 4H), 3.36 (t, *J* = 6.3 Hz, 2H), 3.07 (t, *J* = 5.5 Hz, 4H), 2.34 (s, 3H), 2.23 (t, *J* = 5.5 Hz, 4H), 2.01-1.56 (m, 4H), 0.44 (s, 4H) ppm. MS *m*/*z* 577.6 [M+H]⁺

### Example 2: Preparation of Compound 2

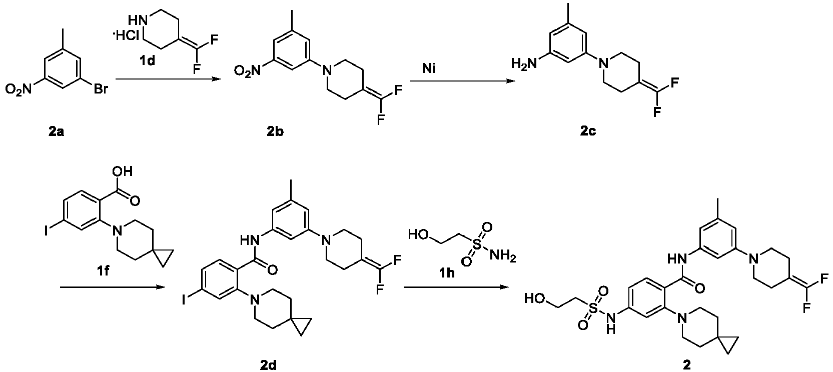

Compound **2a** was used as the raw material to obtain compound **2c** through coupling and reduction reactions. Compound 2d was obtained through compound 2c and compound 1f under acylation reactions, and then compound **2** is obtained through coupling reactions.

### Example 3: Preparation of Compound 3 and 4

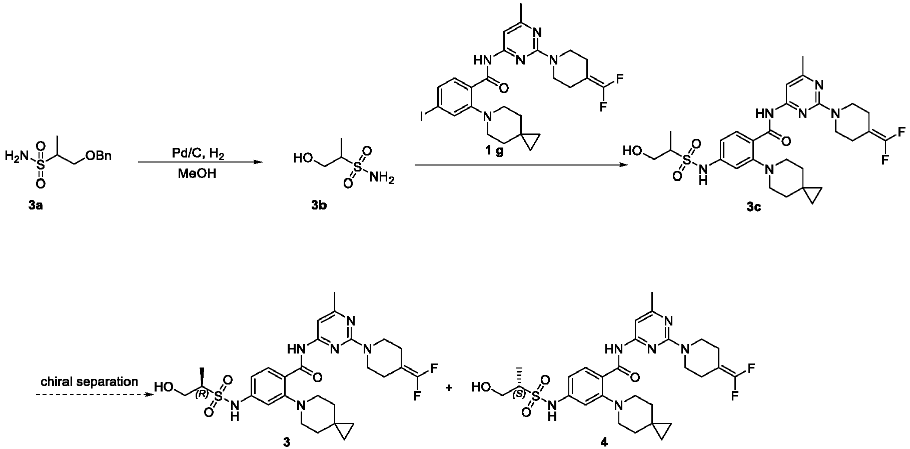

Compound **3a** was synthesized according to (Organic Letters, 2014, 16, 6248-6251). The compound **3a** (140 mg, 0.09 mmol) was dissolved in MeOH (10 mL). 10% Pd/C was added and the reaction was stirred at RT for 12 h under H₂. The mixture was filterated and concentrated. The crude was purified by column chromatography (DCE: MeOH = 15:1) (DCE: 1.2-Dichloroethane and MeOH: methanol) to give a colorless oil compound **3b** (50 mg, 59%).

To a solution of **3b** (4.2 mg, 0.03 mmol) in DMF (0.5 mL) was added sarcosine (0.45 mg, 0.005 mmol), CuI (0.80 mg, 0.005 mmol) and tripotassium phosphate (12 mg, 0.06 mmol). The reaction was stirred at 50 °C for 5 min under a sealed tube. Then **1g** (14 mg, 0.02 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by column chromatography (PE: EA = 1:1) to give a white solid compound **3c** (6.2 mg, 43%). ¹H NMR (500 MHz, DMSO- *d*₆) δ 13.40 (s, 1H), 7.98 (d, *J* = 8.5 Hz, 1H), 7.36 (s, 1H), 7.20 (s, 1H), 7.06 (d, *J* = 8.5 Hz, 1H), 3.88-3.82 (m, 4H), 3.81-3.78 (m, 1H), 3.49-3.46 (m, 1H), 3.23-3.16 (m, 1H), 3.03-2.92 (m, 4H), 2.29 (s, 3H), 2.22-2.12 (m, 4H), 2.03-1.95 (m, 1H), 1.87-1.49 (m, 4H), 1.26 (d, *J* = 6.9 Hz, 3H), 0.39 (s, 4H) ppm. MS *m*/*z* 591.7 [M+H]⁺. Compound **3** and **4** can be obtained by chiral separation of **3c.**

### Example 4: Preparation of Compound 5

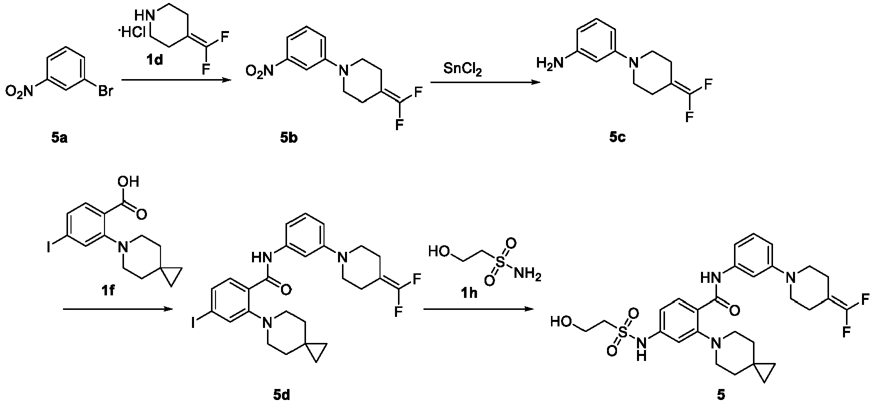

The compound **5a** (101 mg, 0.5 mmol), **1d** (66.5 mg, 0.5 mmol), 2-Dicyclo hexylphosphate-2,4,6-triisopropylbiphenyl (47 mg, 0.1 mmol), tri(dibenzylidene acetone) dipalladium (46 mg, 0.05 mmol) and tBuOK (112 mg, 1.0 mmol) were dissolved in toluene under N₂. The mixture was stirred at 90 °C for 16 h. The reaction was concentrated. The crude was purified by column chromatography (PE: EA= 90:10) to give compound **5b** (110 mg, 87%). MS *m*/*z* 255.3[M+H]⁺.

The compound **5b** (110 mg, 0.44 mmol) was dissolved in EA (1 mL). Stannous dichloride (415 mg, 2.2 mmol) was added and the reaction was stirred at 50 °C for 5 h. After the reaction was completed, sat.NaHCO₃ was added. The mixture was extracted with EA. The collected organic phase was washed with brine, dried with anhydrous sodium sulfate, filterated and concentrated. The crude was purified by column chromatography (PE: EA = 30:70) to give compound **5c** (87 mg, 89%). ¹H NMR (500 MHz, CDCl₃) δ 7.04 (t, *J* = 8.0 Hz, 1H), 6.36 (dd, *J* = 8.0, 2.0Hz, 1H), 6.26 (t, *J* = 2.0 Hz, 1H), 6.22 (dd, *J* = 8.0, 2.0 Hz, 1H), 3.22-3.15 (m, 4H), 2.32-2.28 (m, 4H) ppm

The compound **5c** (30 mg, 0.13 mmol) was dissolved in MeCN (2 mL). **1f** (48 mg, 0.13 mmol), DIPEA (50 mg, 0.39 mmol) and HATU (74 mg, 0.20 mmol) were added and the reaction was stirred at RT for 12 h. Water (5 mL) was added to quench the reaction. The mixture was extracted with EA (10 mL × 3). The collected organic phase was washed with sat.NaCl, dried with anhydrous sodium sulfate, filterated and concentrated. The crude was purified by column chromatography (PE: EA = 20:1) to give a white solid compound **5d** (40 mg, 50%). MS *m*/*z* 564.5[M+H]⁺.

To a solution of **1h** (10 mg, 0.08 mmol) in DMF (0.5 mL) was added sarcosine (0.89 mg, 0.01 mmol), CuI (1.60 mg, 0.01 mmol) and tripotassium phosphate (25 mg, 0.12 mmol). The reaction was stirred at 50 °C for 5 min under a sealed tube. Then **5d** (24 mg, 0.04 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by column chromatography (PE: EA = 1:1) to give a white solid compound **3c** (6.2 mg, 43%). MS *m*/*z* 561.6 [M+H]⁺

### Example 5: Preparation of Compound 6

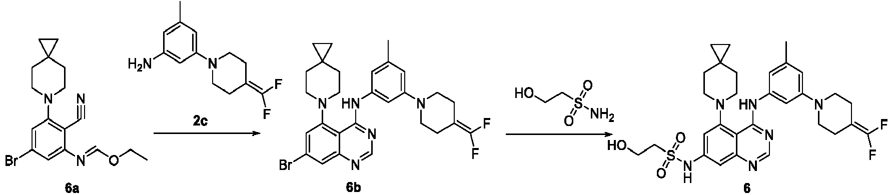

Compound 6a was synthesized according to WO2021/026101. 6a was used as the raw material to obtain compound 6 through ring-closed and coupling reactions.

### Example 6: Preparation of Compound 7

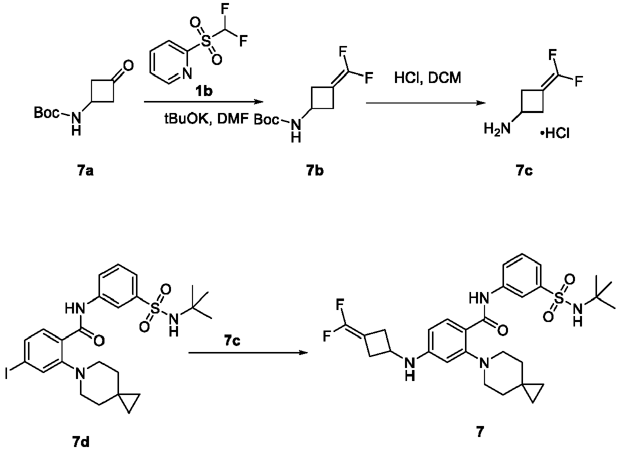

Compound **7b** was obtained through **7a** and **1b** under alkaline conditions, followed by deprotection under acidic conditions to obtain compound **7c.** Compound **7d** was synthesized using the method described in patent WO2021/026098. Compound **7d** and **7c** underwent coupling reaction to obtain compound **7.**

### Example 7: Preparation of Compound 8

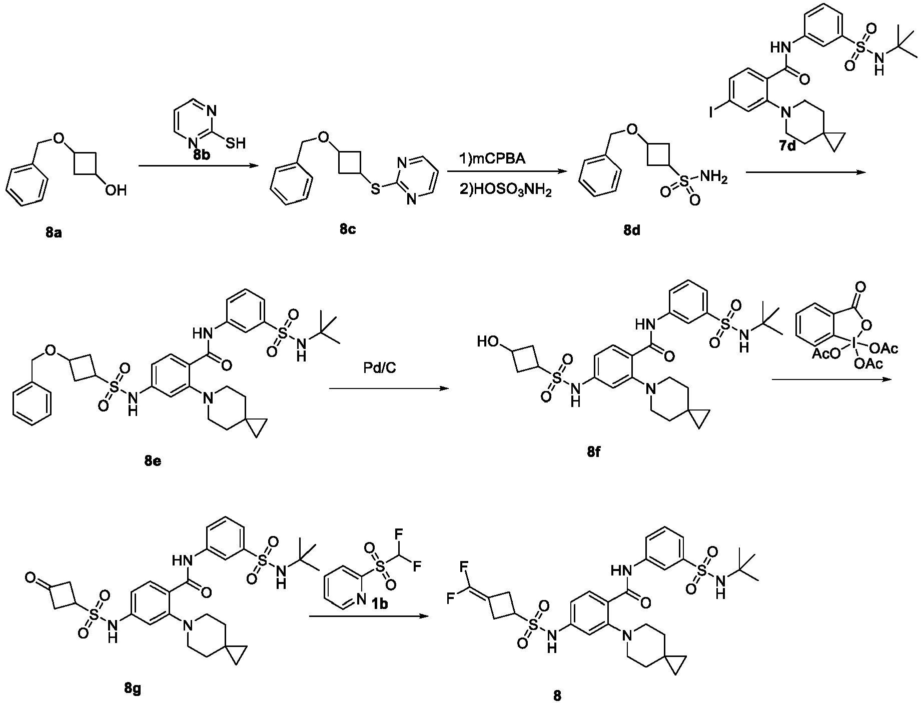

Compound **8a** was used as raw material, compound **8d** was obtained through substitution and oxidation reactions, followed by coupling reaction with compound **7d** to obtain compound **8e.** Compound **8g** was obtained through deprotection and oxidation reactions, followed by reaction with compound **1b** to obtain compound **8.**

### Example 8: Preparation of Compound 9

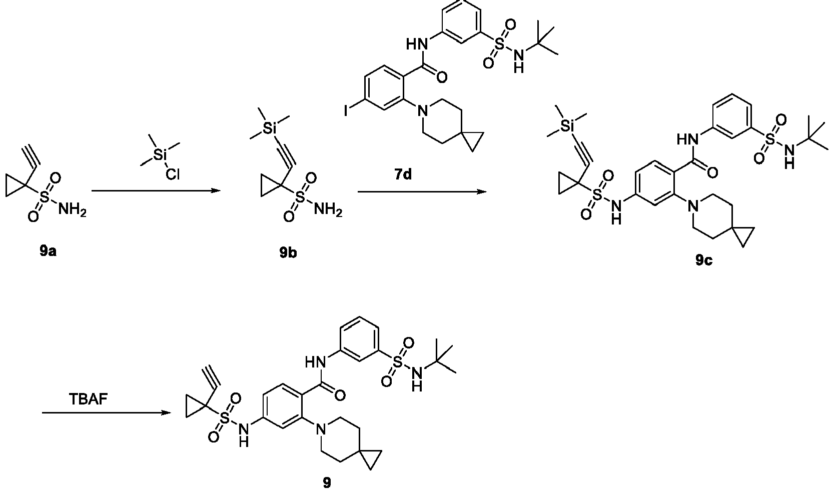

Compound **9a** was protected to obtain compound **9b,** which is then coupled with compound **7d** to obtain compound **9c.** Finally, the protective group is removed to obtain compound **9.**

### Example 9: Preparation of Compound 10

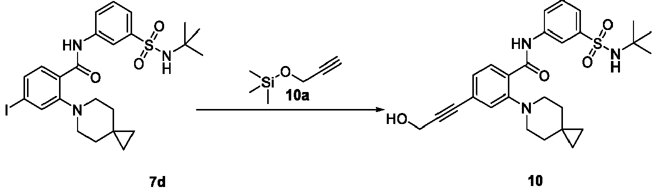

Compound **7d** was synthesized according to WO2021/026098.

To a solution of **7d** (40 mg, 0.07 mmol) in DMF (0.5 mL) was added CuI (1.60 mg, 0.01 mmol), tetra(triphenylphosphine) palladium (16 mg, 0.01 mmol) and NEt₃ (0.3 mL). The reaction was placed in a sealed tube and **10a** (9 mg, 0.07 mmol) was added under N₂. The mixture was stirred at RT for 24 h. The reaction was adjusted to pH = 5-6 with 1M HCl and extracted with EA (5 mL × 3). The collected organic phase was washed with sat.NaCl, dried with anhydrous sodium sulfate, filterated and concentrated. The crude was purified by column chromatography (PE: EA = 3:2) to give a white solid compound **10** (2.74 mg, 8%). ¹H NMR (500 MHz, CD₃OD) δ 8.39 (t, *J* = 2.0 Hz, 1H), 8.01-7.94 (m, 2H), 7.68-7.65 (m, 1H), 7.58 (t, *J* = 8.0 Hz, 1H), 7.45 (d, *J* = 1.5 Hz, 1H), 7.32 (dd, *J* = 8.0, 1.5 Hz, 1H), 4.45 (s, 2H), 3.12 (t, *J* = 5.3 Hz, 4H), 1.63 (br, 4H), 1.24 (s, 9H), 0.42 (s, 4H) ppm. MS m/z 496.6 [M+H]⁺.

### Example 11: Preparation of Compound 12

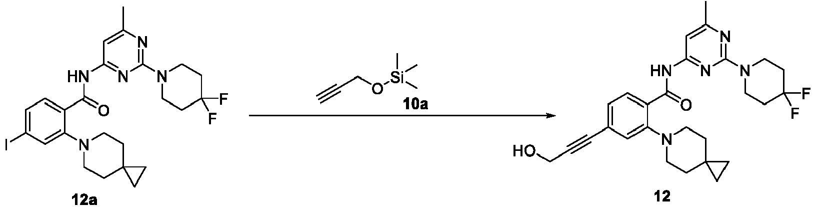

Compound **12a** was synthesized according to WO2020132648.

To a solution of **12a** (30 mg, 0.05 mmol) in DMF (0.3 mL) was added CuI (0.2 mg, 0.01 mmol), tetra (triphenylphosphine) palladium (24 mg, 0.004 mmol) and NEt₃ (0.5 mL). The reaction was placed in a sealed tube and **10a** (14 mg, 0.10 mmol) was added under N₂. The mixture was stirred at RT for 24 h. The reaction was adjusted to pH = 5-6 with 1M HCl and extracted with EA (5 mL×3). The collected organic phase was washed with sat.NaCl, dried with anhydrous sodium sulfate, filterated and concentrated. The crude was purified by column chromatography (PE: EA = 1:1) to give a white solid compound **12** (11 mg, 42%). ¹H NMR (500 MHz, CD₃OD) δ 8.14 (d, *J* = 8.0 Hz, 1H), 7.51 (d, *J* = 1.5 Hz, 1H), 7.45 (s, 1H), 7.35 (dd, *J* = 8.0, 1.5 Hz, 1H), 4.43 (s, 2H), 4.03-3.95 (m, 4H), 3.08 (t, *J* = 5.3 Hz, 4H), 2.36 (s, 3H), 2.01-1.94 (m, 4H), 1.35-1.29 (m, 4H), 0.43 (s, 4H) ppm. MS m/z 496.6 [M+H]⁺.

### Example 12: Preparation of Compound 13

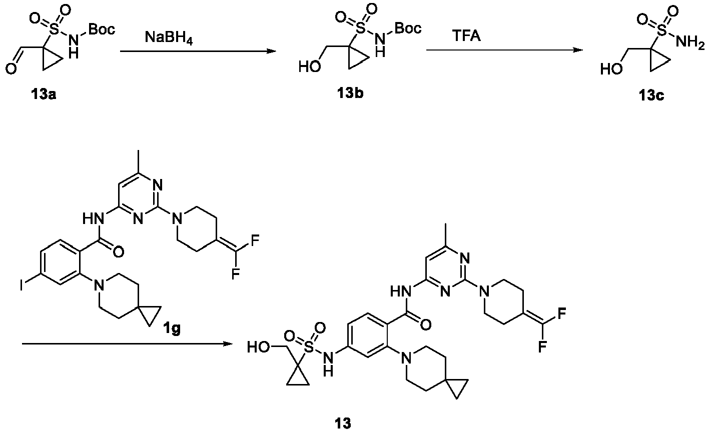

Compound **13a** was synthesized according to US9527885.

The **13a** (750 mg, 3.0 mmol) was dissolved in MeOH (5 mL). Then Sodium borohydride (222 mg, 6.0 mmol) was added at 0 °C and the mixture was stirred at 0 °C for 1 h. Water was added to quench the reaction. The mixture was extracted with EA. The left water phase was freeze-dried to give a yellow crude **13b** (750 mg). The crude was used to the next step directly.

The **13b** (350 mg, 1.4 mmol) was dissolved in TFA (2 mL). The mixture was stirred at RT for 1 h. Water was added to quench the reaction. The mixture was concentrated and purified by column chromatography (DCM: MeOH = 5:95 to 15:85) to give a white solid compound **13c** (122 mg, two step yield 27%). ¹H NMR (500 MHz, DMSO-*d*₆) *δ* 6.69 (s, 2H), 4.90 (t, *J* = 6.2 Hz, 1H), 3.79 (d, *J* = 6.2 Hz, 2H), 1.06 (q, *J* = 4.4 Hz, 2H), 0.91 (q, *J* = 4.4 Hz, 2H) ppm.

To a solution of **13c** (9 mg, 0.06 mmol) in DMF (2 mL) was added sarcosine (0.60 mg, 0.008 mmol), CuI (1.40 mg, 0.008 mmol) and tripotassium phosphate (19 mg, 0.09 mmol). The reaction was stirred at 50 °C for 5 min under a sealed tube. Then **1g** (20 mg, 0.03 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by column chromatography (PE: EA = 1:1) to give a white solid compound **13** (7 mg, 34%). ¹H NMR (500 MHz, DMSO- *d*₆) δ 13.40 (s, 1H), 10.20 (br, 1H), 8.03 (d, *J* = 8.5 Hz, 1H), 7.36 (s, 1H), 7.33 (d, *J* = 1.7 Hz, 1H), 7.15 (dd, *J* = 8.5 Hz, 1.7 Hz, 1H), 4.95 (br, 1H), 3.87-3.80 (m, 4H), 3.74 (s, 2H), 3.02-2.89 (m, 4H), 2.30 (s, 3H), 2.23-2.13 (m, 4H), 1.32-1.25 (m, 4H), 1.18-1.10 (m, 2H), 1.02-0.93 (m, 2H), 0.39 (s, 4H) ppm. MS m/z 603.6 [M+H]⁺.

### Example 13: Preparation of Compound 14

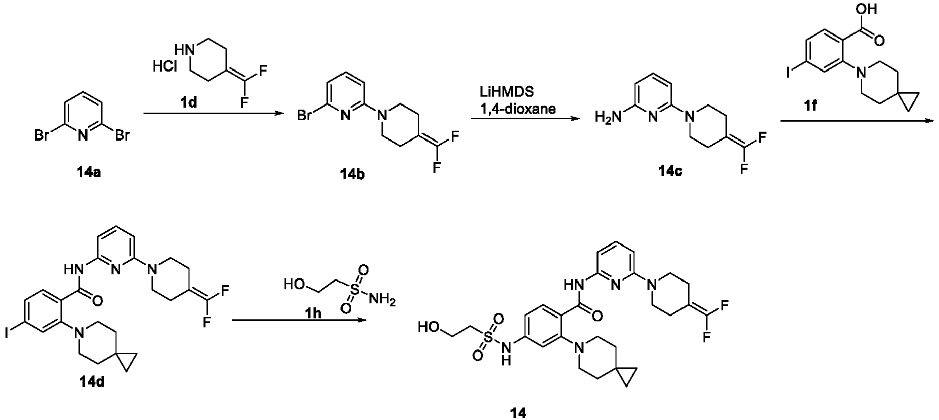

The compound **14a** (465 mg, 1.96 mmol), **1d** (368 mg, 2.16 mmol) and Cs₂CO₃ (Cesium Carbonate, 1.92 g, 5.89 mmol) were dissolved in NMP (8 mL). The mixture was stirred at 140 °C for 3 h. After the reaction was finished, water was added and extracted with EA (20 mL×3). The collected organic phase was washed with sat.NaCl, dried with anhydrous sodium sulfate, filterated and concentrated. The crude was purified by column chromatography (PE: EA=25:1) to give a colorless oil compound **14b** (449 mg, yield 42%). MS m/z 291.3 [M+H]⁺.

The compound **14b** (480 mg, 1.66 mmol), tri(dibenzylidene acetone) dipalladium (46 mg, 0.05 mmol) and 2-(Dicyclohexylphosphine) biphenyl (58 mg, 0.166 mmol) were dissolved in dioxane. Then a solution of lithium bis (trimethylsilylamine) in tetrahydrofuran (1 M, 3.3 mL) was added. The mixture was stirred at 60 °C for 2 h under N₂. The mixture was cooled to RT, filterated and washed. The obtained filtrate is concentrated to give crude product. The crude was purified by column chromatography (PE: EA = 10:1) to give yellow oil compound **14c** (316 mg, 85%). MS *m*/*z* 226.4[M+H]⁺.

The compound **14c** (316 mg, 1.40 mmol), **1f** (501 mg, 1.40 mmol), diisopropylethylamine (542 mg, 4.2 mmol) and N,N,N',N'-Tetramethyl-O- (7-azabenzotriazol-1-yl) hexafluorophosphourea (800 mg, 2.1 mmol) were dissolved in MeCN (6 mL). The reaction was stirred at RT for 1 h under N₂. After the reaction was finished, the mixture was concentrated and purified by column chromatography (PE: EA = 15:1) to give a white solid compound **14d** (715 mg, 90%). ¹H NMR (500 MHz, CDCl₃) δ 12.80 (s, 1H), 8.00 (d, *J* = 8.8 Hz, 1H), 7.71 (d, *J* = 7.8 Hz, 1H), 7.64 (s, 1H), 7.64-7.62 (m, 1H), 7.54 (t, *J* = 8.1 Hz, 1H), 6.44 (d, *J* = 8.3 Hz, 1H), 3.69-3.59 (m, 4H), 3.13-3.01 (m, 4H), 2.29-2.22 (m, 4H), 2.09-1.63 (m, 4H), 0.41 (s, 4H) ppm. MS m/z 565.5 [M+H]⁺.

To a solution of **1h** (30 mg, 0.05 mmol) in DMF (1 mL) was added sarcosine (1.2 mg, 0.01 mmol), CuI (2.4 mg, 0.01 mmol) and tripotassium phosphate (31.8 mg, 0.15 mmol). The reaction was stirred at 50 °C for 5 min under a sealed tube. Then **4d** (9.4 mg, 0.075 mmol) was added and the mixture was stirred at 100 °C for 4 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by column chromatography (PE: EA=1:1) to give a white solid compound **14** (11 mg, yield 34%). ¹H NMR (500 MHz, DMSO- *d*₆) δ 12.89 (s, 1H), 10.20 (s, 1H), 8.07 (d, *J* = 8.6 Hz, 1H), 7.57 (d, *J* = 1.2 Hz, 1H), 7.56 (s, 1H), 7.27 (d, *J* = 2.0 Hz, 1H), 7.12 (dd, *J* = 8.6, 2.0 Hz, 1H), 6.66-6.59 (m, 1H), 4.90 (brs, 1H), 3.76 (t, *J* = 6.5 Hz, 2H), 3.7-3.62 (m, 4H), 3.35 (t, *J* = 6.5 Hz, 2H), 3.06-2.91 (m, 4H), 2.26-2.16 (m, 4H), 1.92-1.49 (m, 4H), 0.39 (s, 4H) ppm. MS *m*/*z* 562.6 [M+H]⁺.

### Example 14: Preparation of Compound 15

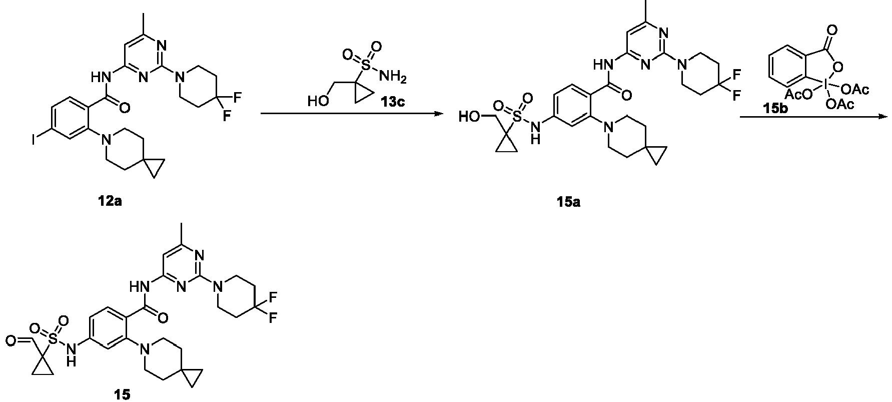

Compound **12a** was synthesized according to WO2020132648.

To a solution of **12a** (50 mg, 0.09 mmol) in DMF (0.3 mL) was added sarcosine (2 mg, 0.02 mmol), CuI (3.52 mg, 0.02 mmol) and tripotassium phosphate (57 mg, 0.27mmol). The reaction was stirred at 50 °C for 5 min under a sealed tube. Then **13c** (27 mg, 0.18 mmol) was added and the mixture was stirred at 100 °C for 4 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by column chromatography (PE: EA=1:1) to give a white solid compound **15a** (10 mg, yield 77%).

The compound **15a** (20 mg, 0.03 mmol) was dissolvesd in DCM (2 mL). Then **15b** (21 mg, 0.05 mmol) was added. The mixture was stirred at RT for 2 h. The mixture was filterated. The obtained filtrate is concentrated to give crude product. The crude was purified by column chromatography (PE: EA=1:1) to give a white solid compound **15** (7 mg, 34%). ¹H NMR (500 MHz, DMSO- *d*₆) δ 13.40 (s, 1H), 10.65 (s, 1H), 9.38 (s, 1H), 8.04 (d, *J* = 8.5 Hz, 1H), 7.39 (s, 1H), 7.30 (d, *J* = 2.0 Hz, 1H), 7.14 (dd, *J* = 8.6, 2.0 Hz, 1H), 3.97-3.85 (m, 4H), 3.02-2.87 (m, 4H), 2.31 (s, 3H), 2.03-1.94 (m, 4H), 1.86-1.57 (m, 8H), 0.39 (s, 4H) ppm. MS *m*/*z* 589.6 [M+H]⁺.

### Example 15: Preparation of Compound 16

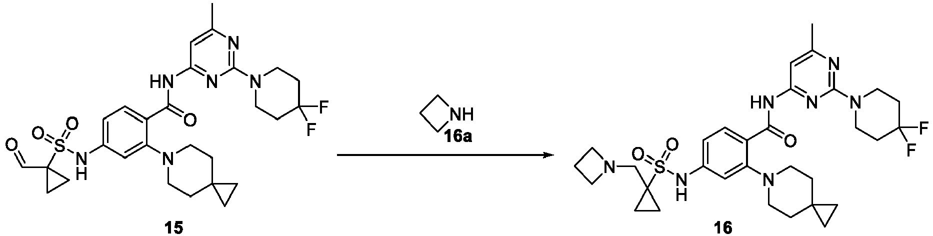

The compound **15** (10 mg, 0.017 mmol) was dissolved in MeOH (0.5 mL). Then **16a** (2 mg, 0.03 mmol) and one drop HOAc (acetic acid) were added. The mixture was stirred at RT for 1 h. Then NaBH₃CN (2 mg, 0.03 mmol) was added and the mixture was stirred for another 12 h. The mixture was concentrated to give crude product. The crude was purified by column chromatography (DCM: MeOH = 20:1) to give a white solid compound **16** (7.03 mg, 66%). ¹H NMR (500 MHz, DMSO- *d*₆) δ 13.47 (s, 1H), 8.04 (d, *J* = 8.5 Hz, 1H), 7.40 (s, 1H), 7.34 (d, *J* = 2.0 Hz, 1H), 7.19 (dd, *J* = 8.5 Hz, 2.0 Hz, 1H), 3.99-3.83 (m, 4H), 3.61-3.48 (m, 2H), 3.15-3.01 (m, 2H), 3.01-2.91 (m, 4H), 2.88-2.74 (m, 2H), 2.31 (s, 3H), 2.07-1.93 (m, 4H), 1.94-1.88 (m, 2H), 1.82-1.55 (m, 4H), 1.22-1.11 (m, 2H), 1.01-0.88 (m, 2H), 0.39 (s, 4H) ppm. MS *m*/*z* 630.8 [M+H]⁺.

### Example 16: Preparation of Compound 17

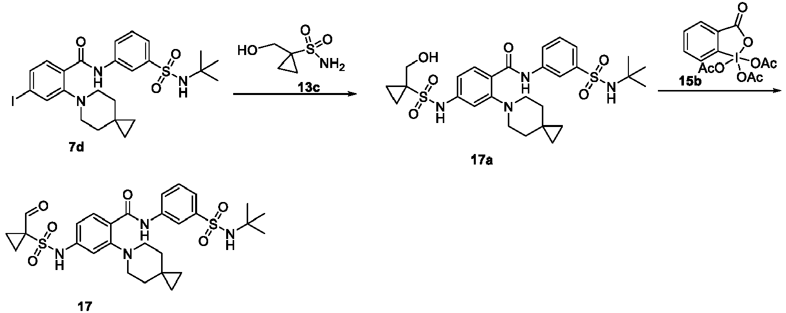

The compound **7d** (45.5 mg, 0.08 mmol), **13c** (17 mg, 0.11 mmol), sarcosine (7.2 mg, 0.08 mmol) and CuI (8.0 mg, 0.04 mmol) were dissolved in DMF (1 mL). The reaction was stirred at 100 °C for 3 h under N₂. The mixture was concentrated and purified by column chromatography (PE: EA = 50:50) to give a white solid compound **17a** (20 mg, 42%). MS *m*/*z* 591.7 [M+H]⁺.

The compound **17a** (15mg, 0.025mmol) was dissolvesd in DCM (1 mL). Then **15b** (21 mg, 0.05 mmol) was added. The mixture was stirred at RT for 2 h. The mixture was filterated with diatomite. The obtained filtrate is concentrated to give crude product. The crude was purified by column chromatography (PE: EA= 60:40) to give a white solid compound **17** (15 mg, 99%). MS *m*/*z* 589.6 [M+H]⁺.

### Example 17: Preparation of Compound 18

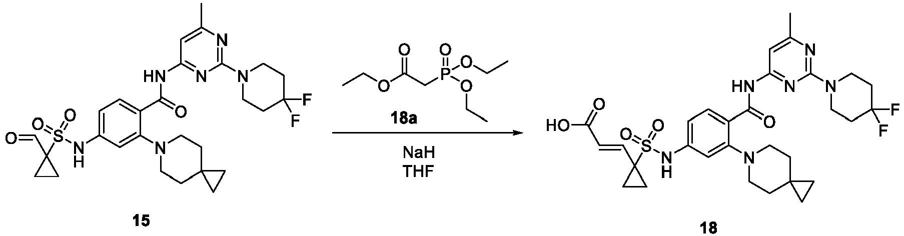

The compound **18a** (6 mg, 0.03 mmol) was dissolved in THF (1.5 mL) at 0 °C. Then 60% NaH (1.2 mg, 0.03 mmol) was added. The mixture was stirred at 0 °C for 20 min. Then **15** (10 mg, 0.02 mmol) was added to the mixture. The reaction was stirred at RT for 2 h. The mixture was concentrated and purified by column chromatography (DCM: MeOH = 8:1) to give a white solid compound **18** (6 mg, 93%). MS m/z 631.7 [M+H]⁺

### Example 18: Preparation of Compound 19

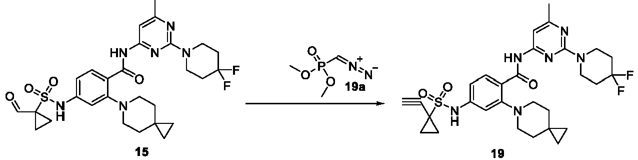

The compound **15** (10 mg, 0.017 mmol) was dissolved in MeOH (0.5 mL). Then **19a** (4 mg, 0.025 mmol) and K₂CO₃ (7 mg, 0.05 mmol) was added. The reaction was stirred at RT for 2 h. The mixture was concentrated and purified by column chromatography (PE: EA = 1:1) to give a white solid compound **19** (6.62 mg, 66%). MS *m*/*z* 585.6 [M+H]⁺.

### Example 19: Preparation of Compound 20

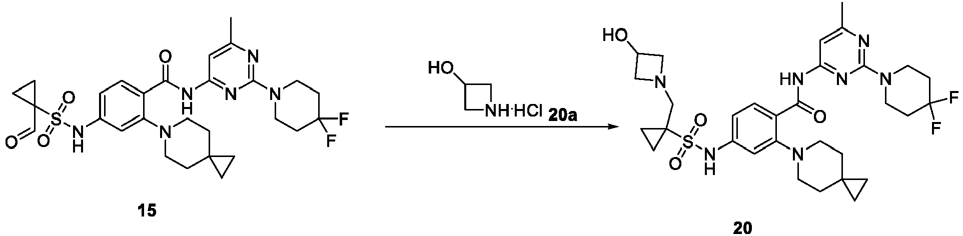

The compound **15** (8 mg, 0.01 mmol), **20a** (2.23 mg, 0.02 mmol), NaBH₃CN (2.56 mg, 0.05 mmol) and ZnCl₂ (3.70 mg, 0.03 mmol) was dissolved in MeOH (1 mL). The reaction was stirred at 70 °C for 2 h. The mixture was concentrated and purified by column chromatography (DCM: MeOH=20:1) to give a white solid compound **20** (2.7 mg, 31%). ¹H NMR (500 MHz, DMSO-*d*₆) δ 13.49 (s, 1H), 8.04 (d, *J* = 8.7 Hz, 1H), 7.40 (s, 1H), 7.34 (d, *J* = 1.8 Hz, 1H), 7.17 (dd, *J* = 8.7, 1.8 Hz, 1H), 5.30 (br, 1H), 4.03-3.94 (m, 1H), 3.94-3.88 (m, 4H), 3.40-3.10 (s, 2H, in H₂O), 3.02-2.89 (m, 4H), 2.85-2.73 (m, 2H), 2.70-2.59 (m, 2H), 2.31 (s, 3H), 2.05-1.93 (m, 4H), 1.89-1.52 (m, 4H), 1.27-1.20 (m, 2H), 1.20-1.14 (m, 2H), 0.39 (s, 4H) ppm. MS *m*/*z* 646.7 [M+H]⁺.

### Example 20: Preparation of Compound 21

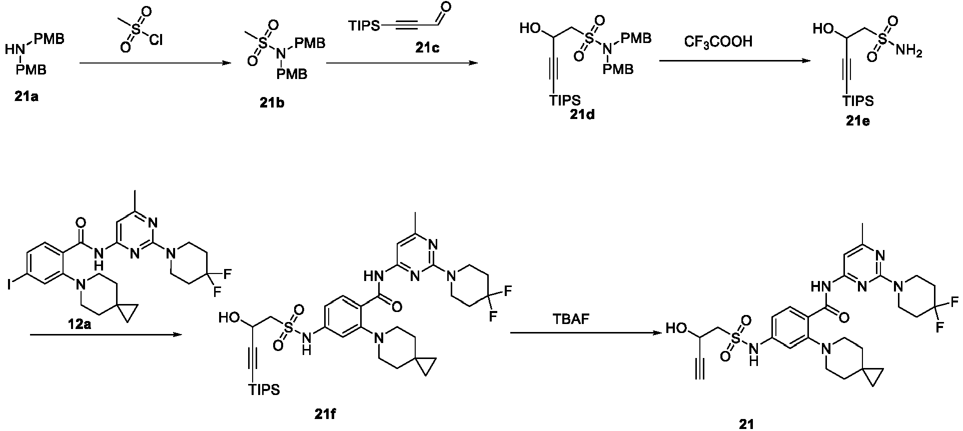

The Bis-(4-methoxybenzyl)-amine (2.5 g, 9.73 mmol) and DIPEA (3.8 g, 29.18 mmol) were dissolved in DCM (40 mL). Methanesulfonyl chloride (1.3 g, 11.68 mmol) were added under ice bath. Then the reaction was stirred for 0.5 h. The mixture was concentrated and purified by column chromatography to give a white solid compound **21b** (2.0 g, 68%). MS *m*/*z* 336.3 [M+H]⁺.

The compound **21b** (100 mg, 0.30 mmol) was dissolved in THF (2 mL). The mixture was cooled to -70 °C and nBuLi (2.5 M, 0.15 mL) was added. The reaction was stirred at this temperature for 10 min. Then s solution of **21c** (91 mg, 0.36 mmol) in THF (0.5 mL) was added. The reaction was stirred at this temperature for 1h. The mixture warmed to 0 °C. The reaction was quenched with sat.NH₄Cl and extracted with EA (10 mL×3). The collected organic phase was washed with sat.NaCl, dried with anhydrous sodium sulfate, filterated and concentrated. The crude was purified by column chromatography to give compound **21d** (61 mg, 37%). MS *m*/*z* 546.4 [M+H]⁺.

The compound **21d** (61 mg, 0.11 mmol) was dissolved in DCM (2 mL). Then TFA (0.5 mL) was added. The mixture was stirred at RT overnight. The mixture was concentrated and purified by column chromatography to give compound **21e** (30 mg, 89%). ¹H NMR (500 MHz, DMSO-*d*₆) δ 6.79 (s, 2H), 5.93 (d, *J* = 6.2 Hz, 1H), 4.70 (q, *J* = 12.2, 6.2 Hz, 1H), 3.30-3.27 (m, 2H), 1.11-0.97 (m, 21H) ppm. MS *m*/*z* 306.5 [M+H]⁺.

The compound **12a** (20 mg, 0.04 mmol, **21e** (24 mg, 0.08 mmol), sarcosine (0.7 mg, 0.008 mmol), CuI (0.8 mg, 0.004 mmol) and tripotassium phosphate (25 mg, 0.12 mmol) were dissolved in DMF (1 mL). The reaction was stirred at 100 °C for 3 h under N₂. The mixture was filterated, concentrated and purified by Pre-TLC to give compound **21f** (22 mg, 85%). MS *m*/*z* 745.5 [M+H]⁺.

The compound **21f** (22 mg, 0.03 mmol) was dissolved in THF (3 mL). TBAF (16 mg, 0.06 mmol) was added slowly. The reaction was stirred at RT for 1 h. Water was added to quench the reaction. The mixture was extracted with EA (10 mL × 3). The collected organic phase was washed with sat.NaCl, dried with anhydrous sodium sulfate, filterated and concentrated. The crude was purified by Pre-TLC (Preparative TLC) to give a white solid compound **21** (6 mg, yield 50%). ¹H NMR (500 MHz, DMSO-*d*₆) δ 13.41 (s, 1H), 10.35 (br, 1H), 8.05 (d, *J* = 8.7 Hz, 1H), 7.40 (s, 1H), 7.25 (d, *J* = 1.7 Hz, 1H), 7.13 (dd, *J* = 8.6, 1.7 Hz, 1H), 6.01 (br, 1H), 4.69-4.64 (m, 1H), 3.95-3.87 (m, 4H), 3.54-3.43 (m, 3H), 2.97 (br, 4H), 2.31 (s, 3H), 2.04-1.93 (m, 4H), 1.72 (br, 4H), 0.39 (s, 4H) ppm. MS m/z 589.2 [M+H]⁺.

### Example 21: Preparation of Compound 22

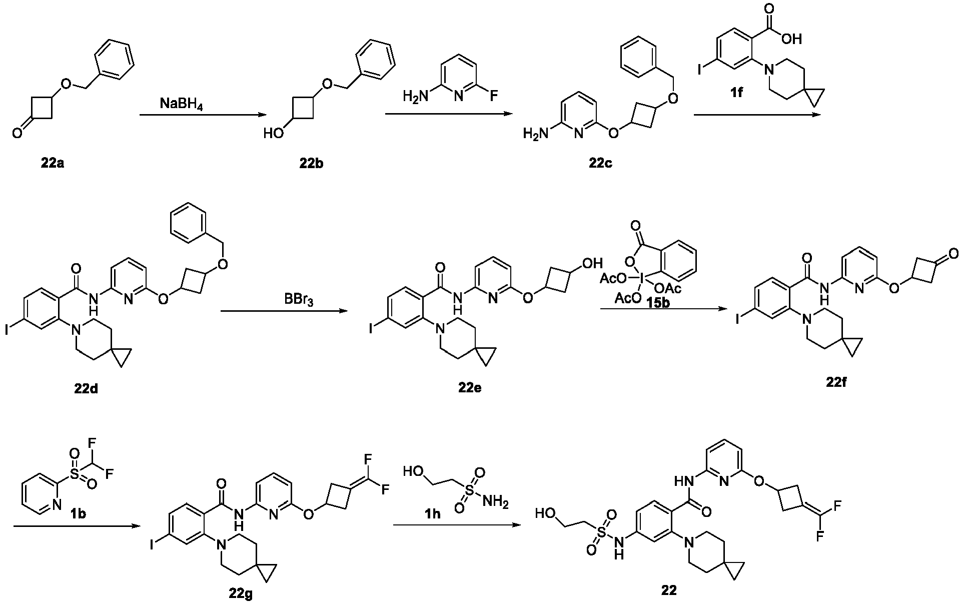

The 3-(benzyloxy) -1-cyclobutanone **22a** (2.0 g, 11.36 mmol) was dissolved in MeOH (20 mL). Then Sodium borohydride (475 mg, 12.50 mmol) was added at 0 °C and the mixture was stirred at RT for 1 h. Water was added to quench the reaction. The mixture was extracted with EA (3×30 mL). The collected organic phase was washed with sat.NaCl, dried with anhydrous sodium sulfate, filterated and concentrated to give compound **22b** (1.9 g, yield 94%). The crude was used to the next step directly.

The compound **22b** (1.0 g, 5.62 mmol) and 2-Amino-6-fluoropyridine (629 mg, 5.62 mmol) were dissolved in dioxane (10 mL) at 0 °C. Then 60% NaH (270 mg, 6.74 mmol, NaH: Sodium hydride) was added. The mixture was stirred at 90 °C for 2 h. the mixture was quenched with water under ice bath. The mixture was extracted with EA (3×30 mL). The collected organic phase was washed with sat.NaCl, dried with anhydrous sodium sulfate, filterated and concentrated. The crude was purified by column chromatography to give compound **22c** (860 mg, yield 53%). MS *m*/*z* 271.5 [M+H]⁺.

The compound **22c** (159 mg, 0.59 mmol) and **1f** (337 mg, 0.59 mmol) were dissolved in MeCN (5 mL). Diisopropylethylamine (228 mg, 1.77 mmol) and N,N,N',N'-Tetramethyl-O-(7-azabenzotriazol-1-yl) hexafluorophosphourea (337 mg, 0.89 mmol) were added. The reaction was stirred at RT for 3 h. After the reaction was finished, the mixture was concentrated and purified by column chromatography to give a white solid compound **22d** (216 mg, yield 60%). MS *m*/*z* 610.7 [M+H]⁺.

The compound **22d** (216 mg, 0.35 mmol) was dissolved in DCM (5 mL) and cooled to -78 °C. BBr₃ (131 mg, 0.53 mmol) was added slowly. The reaction was stirred at RT for 15 min. Sat.NaHCO₃ was added to quench the reaction. The mixture was extracted with EA (20 mL×3). The collected organic phase was washed with sat.NaCl, dried with anhydrous sodium sulfate, filterated and concentrated to give compound **22e** (180 mg). The crude was used to the next step directly. MS *m*/*z* 520.4 [M+H]⁺.

The compound **22e** (180 mg, 0.35 mmol) was dissolved in DCM (3 mL). Then **15b** (223 mg, 0.53 mmol) was added. The mixture was stirred at RT for 1 h. The mixture was filterated. The obtained filtrate is concentrated to give crude product. The crude was purified by column chromatography to give a white solid compound **22f** (127 mg, 70%). MS *m*/*z* 518.6 [M+H]⁺.

The compound **22f** (60 mg, 0.12 mmol), **1b** (46 mg, 0.24 mmol) and hexamethylphosphoric triamide (43 mg, 0.24 mmol) were dissolved in THF (3 mL). The mixture was cooled to -78 °C under N₂ and bis (trimethylsilyl) amino lithium (1M, 0.24mL) was added. Then the reaction warmed to RT and stirred at RT for 3 h. Con.HCl was added to the mixture. The reaction was stirred for another 1 h. The mixture was extracted with EA (3×10 mL). The collected organic phase was washed with sat.NaCl, dried with anhydrous sodium sulfate, filterated and concentrated. The crude was purified by column chromatography to give compound **22g** (10 mg, yield 16%). MS *m*/*z* 552.3 [M+H]⁺.

The compound **22g** (97 mg, 0.02 mmol), 2-hydroxy-1-sulfonamide (5 mg, 0.04 mmol), sarcosine (0.4 mg, 0.004 mmol), CuI (0.4 mg, 0.002 mmol) and tripotassium phosphate (13 mg, 0.06 mmol) were dissolved in DMF (0.5 mL). The reaction was stirred at 100 °C for 3 h under N₂. The mixture was filterated, concentrated and purified by Pre-TLC to give compound **22** (97 mg, 0.23 mmol). ¹H NMR (500 MHz, CD₃OD) δ 8.13 (d, *J* = 8.6 Hz, 1H), 7.94 (dd, *J* = 7.9, 0.4 Hz, 1H), 7.69 (t, *J* = 8.0 Hz, 1H), 7.32 (d, *J* = 2.2 Hz, 1H), 7.15 (dd, *J* = 8.6, 2.2 Hz, 1H), 6.54 (dd, *J* = 8.1, 0.6 Hz, 1H), 5.50-5.42 (m, 1H), 3.95 (t, *J* = 6.2 Hz, 2H), 3.37 (t, *J* = 6.2 Hz, 2H), 3.29-3.23 (m, 2H), 3.10 (t, *J* = 5.2 Hz, 4H), 2.89-2.81 (m, 2H), 2.05-1.65 (br, 4H), 0.45 (s, 4H) ppm. MS *m*/*z* 549.6 [M+H]⁺.

### Example 22: Preparation of Compound 23

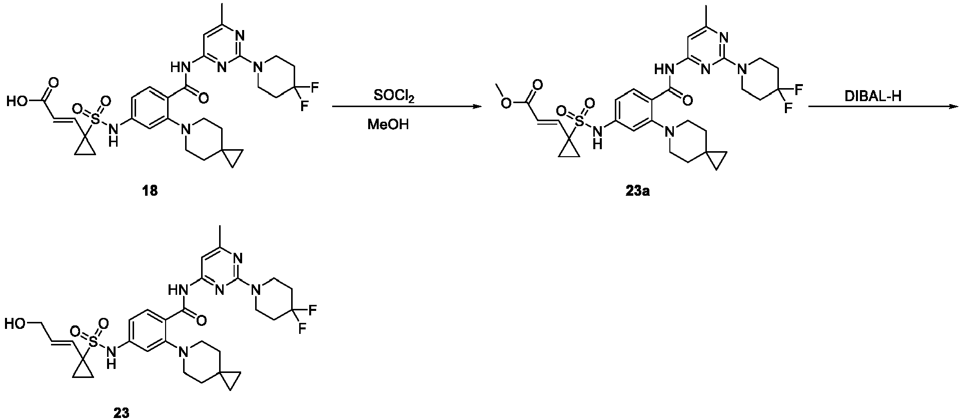

The compound **18** (3.5 mg, 0.005 mmol) was dissolved in MeOH (2 mL). Then SOCl₂ (0.2 mL, SOCl₂: Thionyl chloride) was added and the mixture was stirred for 2 h under reflux. The mixture was cooled to RT and concentrated to give crude product **23a.** MS m/z 645.7 [M+H]⁺.

The crude **23a** was dissolved in THF (1.5 mL). The mixture was cooled to -78 °C under N₂ and and diisobutyl aluminum hydride in n-hexane solution (1 M, 0.4 mL) was added. The mixture was stirred at -78 °C and warmed to 0 °C slowly for 1 h. Water was added to quench the reaction. The mixture was extracted with EA (3×5 mL). The collected organic phase was washed with sat.NaCl, dried with anhydrous sodium sulfate, filterated and concentrated. The crude was purified by column chromatography (DCM: MeOH = 20:1) to give a white solid compound **23** (0.6 mg, two step yield 17%). MS m/z 617.8 [M+H]⁺

### Example 23: Preparation of Compound 24

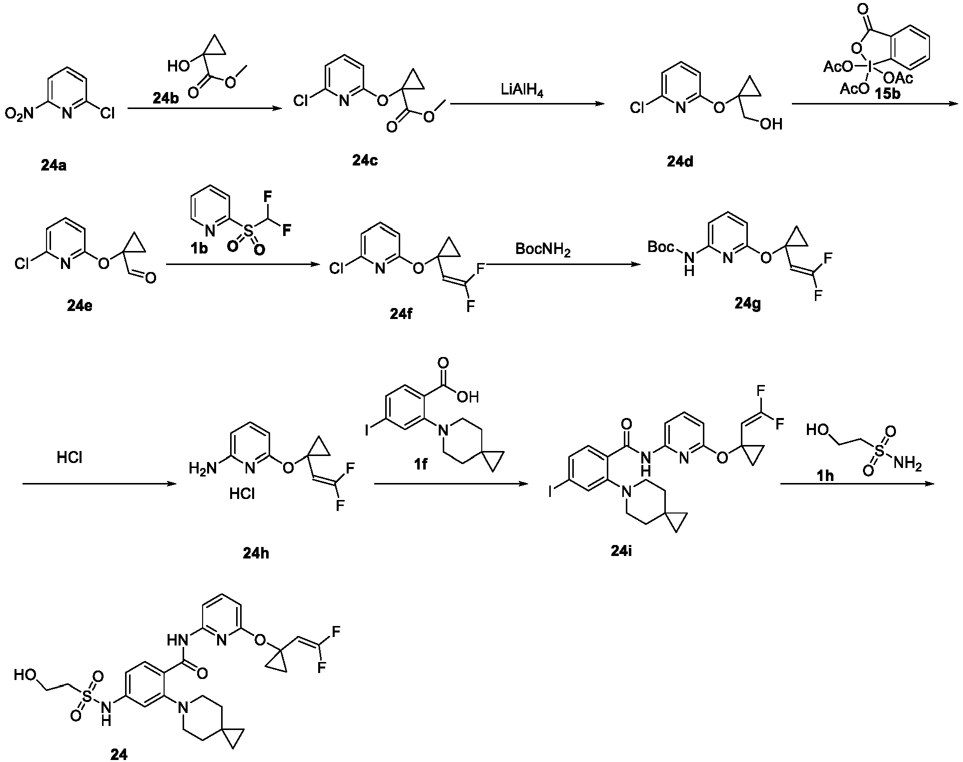

The 2-chloro-6-nitropyridine **24a** (1.4 g, 8.81 mmol) and 1-hydroxy-1-cyclopropanecarboxylic acid methyl ester **24b** (1.0 g, 8.81 mmol) were dissolved in DMF (15 mL) at 0 °C. Then tBuOK (2.0 g, 17.62 mmol) was added. The mixture was stirred at RT for 1 h. Water was added to the reaction. The mixture was extracted with EA (3×50 mL). The collected organic phase was washed with sat.NaCl, dried with anhydrous sodium sulfate, filterated and concentrated. The crude was purified by column chromatography to give product **24c** (1.1 g, yield 58%).

The compound **24c** (205 mg, 0.90 mmol) was dissolved in THF (5 mL). Then LiAlH₄ (51 mg, 1.35 mmol) was added and the mixture was stirred at RT for 1 h. Water (0.05 mL), 15% NaOH (0.05 mL) and water (0.15 mL) was added to the reaction under ice bath. The mixture was stirred at RT for 15 min. Then anhydrous sodium sulfate was added and stirred for another 15 min. The mixture was filterated through diatomite and concentrated to give crude product **24b** (180 mg). The crude was used to the next step directly.

The compound **24d** (180 mg, 0.90 mmol) was dissolved in DCM (4 mL). Then **15b** (572 mg, 1.35 mmol) was added. The mixture was stirred at RT for 1 h. The mixture was filterated. The obtained filtrate is concentrated to give crude product. The crude was purified by column chromatography to give product **24e** (99 mg, 56%).

The compound **24e** (99 mg, 0.50 mmol), **1b** (193 mg, 1.00 mmol) and hexamethylphosphoric triamide (179 mg, 1.00 mmol) were dissolved in THF (3 mL). The mixture was cooled to -78 °C under N₂ and bis (trimethylsilyl) amino lithium (1M, 1.0 mL) was added. Then the reaction warmed to RT and stirred at RT for 3 h. Con.HCl (Con.: Concentrated) was added to the mixture. The reaction was stirred for another 1 h. The mixture was extracted with EA (3×10 mL). The collected organic phase was washed with sat.NaCl, dried with anhydrous sodium sulfate, filterated and concentrated. The crude was purified by column chromatography to give product **24f** (40 mg, yield 34%). MS *m*/*z* 232.1 [M+H]⁺.

The compound **24f** (40 mg, 0.17 mmol), tert-Butyl carbamate (30 mg, 0.26 mmol), Pd(OAc)₂ (Palladium acetate, 4 mg, 0.02 mmol), 2-Dicyclohexylphosphate-2 ', 4', 6 '-triisopropylbiphenyl (19 mg, 0.04 mmol) and Cs₂CO₃ (166 mg, 0.51 mmol) were dissolved in dioxane (2 mL). The reaction was stirred at 110 °C for 2 h under N₂. The mixture was filterated through diatomite, concentrated and purified by column chromatography to give product **24g** (19 mg, yield 35%).

The compound **24g** (19 mg, 0.06 mmol) was dissolved in MeCN (2 mL). HCl/dioxane (4 M, 0.2 mL) was added. The reaction was stirred at RT for 4 h. The mixture was concentrated to give product **24h** (16 mg). The crude was used to the next step directly.

The compound **24h** (16 mg, 0.06 mmol) and **1f** (21 mg, 0.06 mmol) were dissolved in MeCN (5 mL). Diisopropylethylamine (23 mg, 0.18 mmol) and N,N,N',N'-Tetramethyl-O-(7-azabenzotriazol-1-yl) hexafluorophosphourea (34 mg, 0.09 mmol) were added. The reaction was stirred at RT for 3 h. The mixture was concentrated and purified by column chromatography to give yellow oil product **24i** (12 mg, yield 36%). MS *m*/*z* 552.3 [M+H]⁺.

The compound **24i** (12 mg, 0.02 mmol), 2-hydroxy-1-sulfonamide (5 mg, 0.04 mmol), sarcosine (0.4 mg, 0.004 mmol), CuI (0.4 mg, 0.002 mmol) and tripotassium phosphate (13 mg, 0.06 mmol) were dissolved in DMF (0.5 mL). The reaction was stirred at 100 °C for 3 h under N₂. The mixture was filterated, concentrated and purified by Pre-TLC to give product **24** (6 mg, yield 50%). ¹H NMR (500 MHz, CD₃OD) δ 8.09 (d, *J* = 8.6 Hz, 1H), 7.99 (d, *J* = 8.0 Hz, 1H), 7.67 (t, *J* = 8.0 Hz, 1H), 7.30 (d, *J* = 2.0 Hz, 1H), 7.12 (dd, *J* = 8.6, 2.0 Hz, 1H), 6.48 (d, *J* = 8.0 Hz, 1H), 5.17 (dd, *J* = 25.3, 2.2 Hz, 1H), 3.94 (t, *J* = 6.2 Hz, 2H), 3.36 (t, *J* = 6.2 Hz, 2H), 3.13-3.06 (m, 4H), 1.78 (br, 4H), 1.20-1.16 (m, 2H), 1.13-1.08 (m, 2H), 0.42 (s, 4H) ppm. MS *m*/*z* 549.6 [M+H]⁺.

### Example 24: Preparation of Compound 25

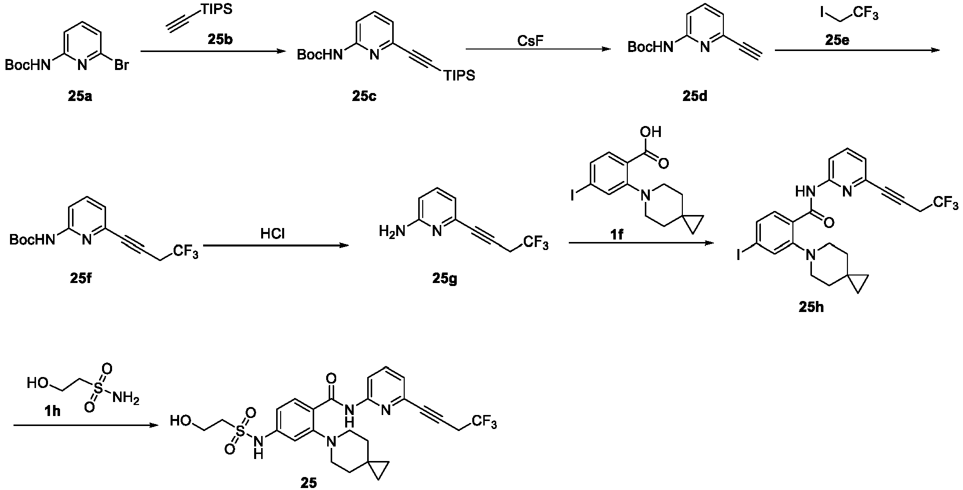

The compound **25a** (2.2 g, 8.05 mmol), **25b** (1.47 g, 8.05 mmol), Di (triphenylphosphine) palladium dichloride (565 mg, 0.81 mmol), CuI (307 mg, 1.61 mmol) and DIPEA (3.12 g, 24.16 mmol) were dissolved in DMF (12 mL). The mixture was stirred at 90 °C for 2 h under N₂. After the reaction was finished, water was added to the reaction. The mixture was extracted with EA (3×30 mL). The collected organic phase was washed with sat.NaCl, dried with anhydrous sodium sulfate, filterated and concentrated. The crude was purified by column chromatography (PE: EA = 30:1) to give a colorless oil compound **25c** (2.71 g, yield 90%). MS m/z 375.5 [M+H]⁺.

The compound **25c** (2.71 g, 7.23 mmol) was dissolved in DMF (10 mL) and Cesium Fluoride (5.49 g, 36.17 mmol) was added. The mixture was stirred at RT for 1 h. After the reaction was finished, water was added to the reaction. The mixture was extracted with EA (3×30 mL). The collected organic phase was washed with sat.NaCl, dried with anhydrous sodium sulfate, filterated and concentrated. The crude was purified by column chromatography (PE: EA = 20:1) to give a white solid product **25d** (1.31 g, yield 83%). MS m/z 241.4 [M+Na]⁺.

The compound **25d** (150 mg, 0.69 mmol), Tri(dibenzylidene acetone) dipalladium (73 mg, 0.07 mmol), Bis (2-Diphenylphosphophenyl) ether (148 mg, 0.28 mmol) and 1,4-Diazobicyclic [2.2.2] octane (308 mg, 2.75 mmol) were dissolved in toluene (5 mL). The mixture was purged with N₂ for 3 times and **25e** (577 mg, 2.75 mmol) was added. The reaction was stirred at 80 °C for 2 h under N₂. After the reaction wascooled to RT, the mixture was filterated and concentrated. The crude was purified by column chromatography (PE: EA=20:1) to give a yellow oil **25f** (233 mg, yield 100%). MS m/z 323.4 [M+Na]⁺.

The compound **25f** (223 mg, 0.74 mmol) was dissolved in MeCN (3 mL). HCl/dioxane (4 M, 3 mL) was added. The reaction was stirred at RT for overnight. The mixture was concentrated and the crude was dissolved in MeOH. And an appropriate amount of ammonia was added to adjust the pH to 7. The mixture was concentrated and the crude was purified by column chromatography (DCM: MeOH = 50:1) to give a yellow solid product **25g** (102 mg, yield 69%). MS m/z 201.3 [M+H]⁺.

The compound **25g** (40 mg, 0.20 mmol), **1f** (71 mg, 0.20 mmol), diisopropylethylamine (77 mg, 0.60 mmol) and N,N,N',N'-Tetramethyl-O- (7-azabenzotriazol-1-yl) hexafluorophosphourea (114 mg, 0.30 mmol) was dissolved in MeCN (2 mL). The reaction was stirred at RT for 1 h under N₂. After the reaction was finished, the mixture was concentrated and purified by column chromatography (PE: EA = 20:1) to give a colorless oil compound **25h** (44 mg, 41%). MS m/z 540.5 [M+H]⁺.

The compound **1h** (12 mg, 0.10 mmol) was dissolved in DMF (1 mL), and then sarcosine (2.5 mg, 0.02 mmol), CuI (5 mg, 0.02 mmol) and tripotassium phosphate (63 mg, 0.3 mmol) were added. The reaction was stirred at 50 °C for 5 min under a sealed tube. Then **25h** (36 mg, 0.07 mmol) was added and the mixture was stirred at 100 °C for 4 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by column chromatography (PE: EA = 1:1) to give a white solid product **25** (0.8 mg, yield 2%). MS *m*/*z* 537.5 [M+H]⁺.

### Example 25: Preparation of Compound 26

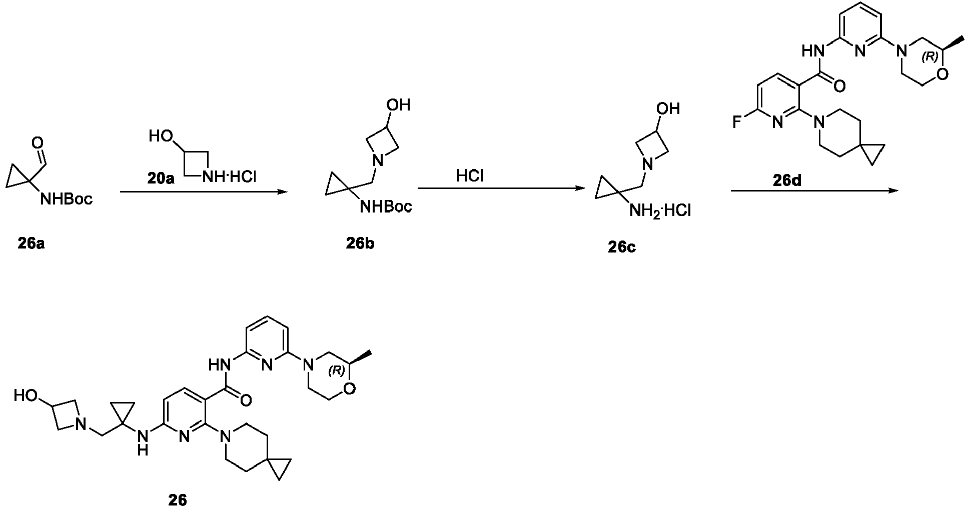

The compound **26a** (200 mg, 1.08 mmol) and **20a** (142 mg, 1.30 mmol) were dissolved in MeOH (2 mL). Then one drop HOAc was added. The mixture was stirred at RT for 1 h. Then NaBH₃CN (203 mg, 3.24 mmol) was added and the mixture was stirred for another 12 h. The mixture was concentrated to give crude product. The crude was purified by column chromatography (DCM: MeOH = 15:1) to give a white solid product **16** (7.03 mg, 66%).

The compound **26b** (35 mg, 0.15 mmol) was dissolved in DCM (1 mL). HCl/dioxane (4 M, 0.1 mL) was added. The reaction was stirred at RT for 3 h. The mixture was concentrated and the crude was used to the next step directly.

The compound **26d** (30 mg, 0.07 mmo, synthesised according to WO2020132653), **26c** (15 mg, 0.08 mmol) and tripotassium phosphate (45 mg, 0.21 mmol) were dissolved in DMSO (1 mL). The reaction was stirred at 110 °C for 12 h under N₂. The reaction was extracted with EA (5 mL×3). The collected organic phase was washed with sat.NaCl, dried with anhydrous sodium sulfate, filterated and concentrated. The crude was purified by Pre-TLC (DCM: MeOH=20:1) to give a white solid product **26** (2.28 mg, yield 6%). ¹H NMR (500 MHz, DMSO-*d*₆) δ 12.15 (s, 1H), 8.08 (d, *J* = 8.9 Hz, 1H), 7.59-7.52 (m, 2H), 6.62 (s, 1H), 6.54 (dd, *J* = 7.1, 1.5 Hz, 1H), 4.87 (br, 1H), 4.17 (d, *J* = 12.2 Hz, 1H), 4.03 (d, *J* = 12.6 Hz, 1H), 3.91 (dd, *J* = 11.3, 2.4 Hz, 1H), 3.87 (br, 1H), 3.62-3.53 (m, 2H), 3.50-3.10 (m, 4H, in H₂O), 3.06 (br, 4H), 2.88-2.78 (m, 2H), 2.60-2.55 (m, 1H), 2.49-2.45 (m, 1H), 1.69 (br, 4H), 1.27-1.21 (m, 4H), 1.17 (d, *J* = 6.2 Hz, 3H), 0.68-0.48 (m, 4H), 0.34 (br, 4H) ppm. MS *m*/*z* 548.4 [M+H]⁺.

### Example 26: Preparation of Compound 27

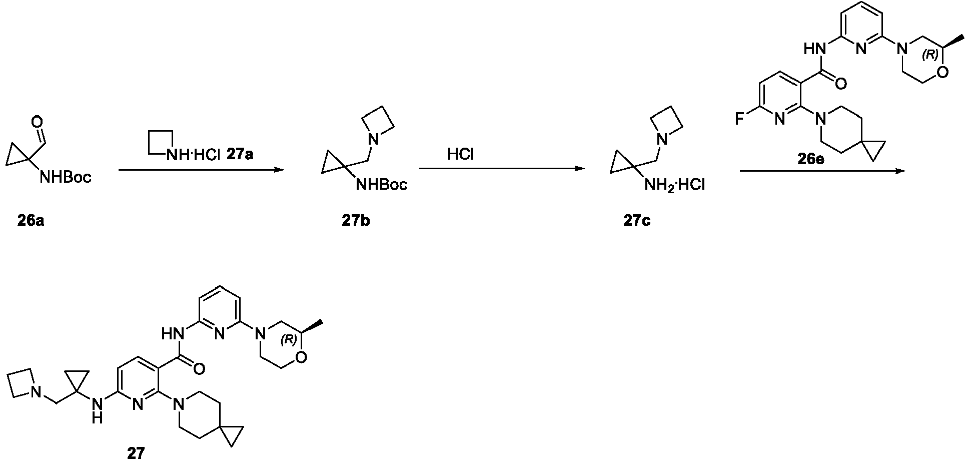

The compound **26a** (200 mg, 1.08 mmol) and **27a** (130 mg, 1.40 mmol) were dissolved in MeOH (2 mL). Then one drop HOAc was added. The mixture was stirred at RT for 1 h. Then NaBH₃CN (203 mg, 3.24 mmol) was added and the mixture was stirred for another 12 h. The mixture was concentrated to give crude product. The crude was purified by column chromatography (DCM: MeOH = 15:1) to give a white solid product **27b** (110 mg, 48%).

The compound **27b** (27 mg, 0.12 mmol) was dissolved in DCM (1 mL). HCl/dioxane (4 M, 0.1 mL) was added. The reaction was stirred at RT for 3 h. The mixture was concentrated and the crude **27c** was used to the next step directly.

The compound **26e** (30 mg, 0.07 mmol, synthesised according to WO2020132653), **27c** (15 mg, 0.08 mmol) and tripotassium phosphate (45 mg, 0.21 mmol) were dissolved in DMSO (1 mL). The reaction was stirred at 110 °C for 12 h under N₂. The reaction was extracted with EA (5 mL×3). The collected organic phase was washed with sat.NaCl, dried with anhydrous sodium sulfate, filterated and concentrated. The crude was purified by Pre-TLC (DCM: MeOH = 20:1) to give a white solid product **27** (8.37 mg, yield 22%). ¹H NMR (500 MHz, DMSO-*d*₆) δ 12.17 (s, 1H), 8.08 (d, *J* = 8.8 Hz, 1H), 7.61-7.48 (m, 2H), 6.57-6.44 (m, 2H), 4.17 (d, *J* = 12.5 Hz, 1H), 4.03 (d, *J* = 12.8 Hz, 1H), 3.91 (dd, *J* = 11.5, 2.7 Hz, 1H), 3.72 (br, 4H), 3.59-3.55 (m, 2H), 3.05 (br, 4H), 2.84-2.79 (m, 1H), 2.71 (t, *J* = 5.7 Hz, 2H), 2.47-2.49 (m, 1H), 1.83 (br, 2H), 1.69 (br, 4H), 1.17 (d, *J* = 6.2 Hz, 3H), 0.62 (t, *J* = 4.7 Hz, 2H), 0.57-0.51 (m, 2H), 0.34 (s, 4H) ppm. MS *m*/*z* 532.4 [M+H]⁺.

### Example 27: Preparation of Compound 28

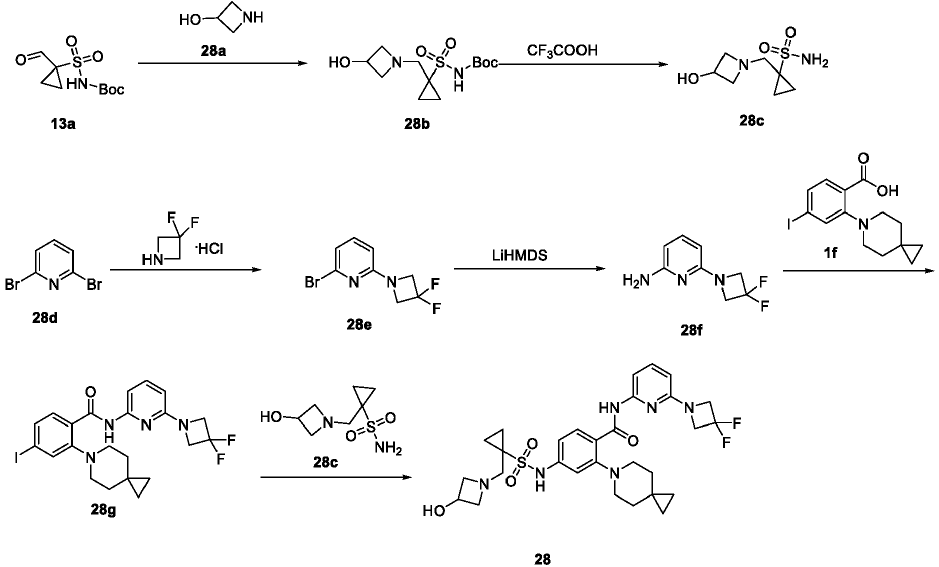

The compound **13a** (500 mg, 2.0 mmol) was dissolved in THF (2 mL) and MeOH (6 mL). Then nitrocyclobutane-3-ol (219 mg, 3.0 mmol) was added. The mixture was stirred at 50 °C for 1 h. Then NaBH₃CN (151 mg, 2.4 mmol) was added and the mixture was stirred at 60 °C for another 16 h. The mixture was concentrated to give crude product. The crude was purified by column chromatography (DCM: MeOH=10:1) to give compound **28b** (300 mg, 50%). ¹H NMR (500 MHz, CD₃OD) δ 4.64-4.57 (m, 1 H), 4.40-4.32 (m, 2 H), 3.84-3.81 (m, 2H), 3.46 (s, 2H), 1.46-1.41 (m, 11H), 1.01-0.99 (m, 2H) ppm.

The compound **28b** (270 mg, 0.86 mmol) was dissolved in TFA (1.0 mL). The mixture was stirred at RT for 0.5 h. The mixture was concentrated to remove the solvent and then dissolved in DCM. And an appropriate amount of ammonia was added to adjust the pH to base. The mixture was dried with anhydrous sodium sulfate, filterated and concentrated. The crude was purified by column chromatography (DCM: MeOH = 4:1) to give product **28c** (140 mg, yield 80%). ¹H NMR (500 MHz, CD₃OD) δ 4.38-4.33 (m, 1H), 3.75-3.72 (m, 2H), 3.04-2.92 (m, 2H), 2.92 (s, 2H), 1.31-1.24 (m, 2H), 0.90-0.88 (m, 2H) ppm.

The compound **28d** (2 g, 8.44 mmol), 3, 3-Difluorotrimethylimine hydrochloride (1.42 g, 10.97 mmol) and Cs₂CO₃ (8.25 g, 25.33 mmol) was dissolved in NMP (50 mL). The mixture was stirred at 140 °C for 3 h. After the reaction was finished, water was added and extracted with EA (50 mL × 3). The collected organic phase was washed with sat.NaCl, dried with anhydrous sodium sulfate, filterated and concentrated. The crude was purified by column chromatography (PE: EA = 25:1) to give a colorless **oil 28e** (1.3g, yield 61.85%). MS m/z 248, 250 [M+H] ⁺.

The compound **28e** (1.24g, 49.66 mmol), tri(dibenzylidene acetone) dipalladium (0.239g, 2.6 mmol) and 2-(Dicyclohexylphosphine) biphenyl (182.7 mg, 5.2 mmol) was dissolved in dioxane (20 mL). Then a solution of lithium bis (trimethylsilylamine) in tetrahydrofuran (1 M, 5 mL) was added. The mixture was stirred at 60 °C for 2 h under N₂. The mixture was cooled to RT, filterated and washed. The obtained filtrate is concentrated to give crude product. The crude was purified by column chromatography (PE: EA = 10: 1) to giveyellow oil compound **28f** (0.6 g, yield 65.3%). MS m/z 186 [M+H]⁺.

The compound **28f** (300 mg, 1.62 mmol), **1f** (636.2 mg, 1.78 mmol), diisopropylethylamine (417.96 mg, 3.24 mmol) and N,N,N',N'-Tetramethyl-O- (7-azabenzotriazol-1-yl) hexafluorophosphourea (672 mg, 1.76 mmol) was dissolved in MeCN (6 mL). The reaction was stirred at RT for 1 h under N₂. After the reaction was finished, the mixture was concentrated and purified by column chromatography (PE: EA = 15:1) to give a white solid product **28g** (460 mg, 54.2%). MS m/z 525 [M+H]⁺.

To a solution of **28c** (18 mg, 0.08 mmol) in DMF (1 mL) was added sarcosine (2.5 mg, 0.02 mmol), CuI (5 mg, 0.02 mmol) and tripotassium phosphate (36 mg, 0.18 mmol). The reaction was stirred at 50 °C for 5 min under a sealed tube. Then **28g** (30 mg, 0.06 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by column chromatography (PE: EA = 2:3) to give a white solid product **28** (9.24 mg, yield 27%). ¹H NMR (500 MHz, DMSO-*d*₆) δ 13.31 (s, 1H), 8.05 (d, *J* = 8.6 Hz, 1H), 7.66 (dt, *J* = 15.6, 7.8 Hz, 2H), 7.33 (d, *J* = 1.9 Hz, 1H), 7.16 (dd, *J* = 8.6, 2.0 Hz, 1H), 6.33 (d, *J* = 7.7 Hz, 1H), 5.22 (s, 1H), 4.39 (t, *J* = 12.4 Hz, 4H), 4.00-3.90 (m, 1H), 3.40-3.10 (s, 2H, in H₂O), 2.97 (br, 4H), 2.76 (s, 2H), 2.64 (s, 2H), 1.75 (br, 4H), 1.22-1.12 (m, 2H), 0.95-0.80 (m, 2H), 0.40 (s, 4H) ppm. MS *m*/*z* 603.3 [M+H]⁺.

### Example 28: Preparation of Compound 29

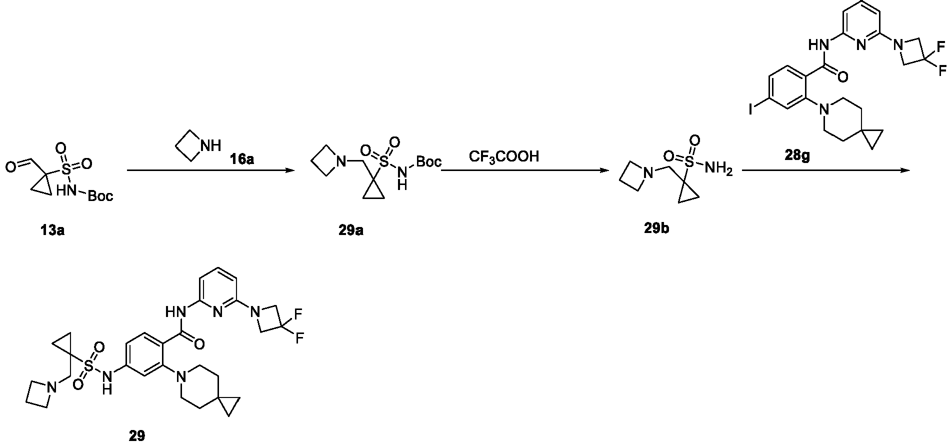

The compound **13a** (500 mg, 2.0 mmol) was dissolved in THF (2 mL) and MeOH (6 mL). Then azetidine (171 mg, 3.0 mmol) was added. The mixture was stirred at 50 °C for 1 h. Then NaBH₃CN (151 mg, 2.4 mmol) was added and the mixture was stirred at 60 °C for another 16 h. The mixture was concentrated to give crude product. The crude was purified by column chromatography (DCM: MeOH=10:1) to give product **29a** (400 mg, 69%).¹H NMR (500 MHz, CD₃OD) δ 4.21 (t, *J* = 8.2 Hz, 4H), 3.50 (s, 2H), 2.54-2.44 (m, 2H), 1.45-1.40 (m, 11H), 1.01-0.98 (m, 2H) ppm.

The compound **29a** (290 mg, 1.0 mmol) was dissolved in TFA (1.0 mL). The mixture was stirred at RT for 0.5 h. The mixture was concentrated to remove the solvent and then dissolved in DCM. And an appropriate amount of ammonia was added to adjust the pH to alkalinity. The mixture was dried with anhydrous sodium sulfate, filterated and concentrated. The crude was purified by column chromatography (DCM: MeOH = 4:1) to give product **29b** (130 mg, yield 68%). ¹H NMR (500 MHz, CD₃OD) δ 3.33 (t, *J* = 7.0 Hz, 4H), 2.85 (s, 2H), 2.13-2.07 (m, 2H), 1.26-1.24 (m, 2H), 0.88-0.86 (m, 2H) ppm.

To a solution of **29b** (16 mg, 0.08 mmol)) in DMF (1 mL) was added sarcosine (2.5 mg, 0.02 mmol), CuI (5 mg, 0.02 mmol) and tripotassium phosphate (36 mg, 0.18 mmol). The reaction was stirred at 50 °C for 5 min under a sealed tube. Then **28g** (30 mg, 0.06 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (DCM: MeOH = 20:1) to give a white solid product **29** (8 mg, yield 24%). ¹H NMR (500 MHz, DMSO-*d*₆) δ 13.30 (s, 1H), 8.05 (d, *J* = 8.6 Hz, 1H), 7.66 (dt, *J* = 15.6, 7.7 Hz, 2H), 7.35 (d, *J =* 1.9 Hz, 1H), 7.17 (dd, *J* = 8.6, 1.9 Hz, 1H), 6.33 (d, *J* = 7.7 Hz, 1H), 4.39 (t, *J* = 12.4 Hz, 4H), 3.03 (t, *J* = 6.9 Hz, 4H), 2.97 (br, 4H), 2.76 (s, 2H), 1.89-1.80 (m, 2H), 1.50 (br, 4H), 1.19-1.12 (m, 2H), 0.90-0.86 (m, 2H), 0.39 (s, 4H) ppm. MS *m*/*z* 587.7 [M+H]⁺.

### Example 29: Preparation of Compound 30

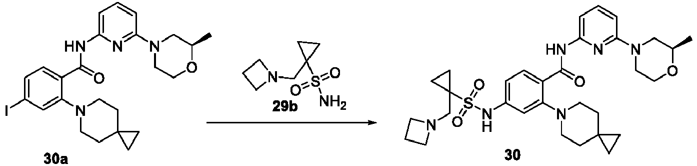

To a solution of **29b** (17 mg, 0.08 mmol) in DMF (1 mL) was added sarcosine (2.5 mg, 0.02 mmol), CuI (5 mg, 0.02 mmol) and tripotassium phosphate (36 mg, 0.18 mmol). The reaction was stirred at 50 °C for 5 min under a sealed tube. Then **30a** (30 mg, 0.06 mmol, synthesised according to WO2020132651) was added and the mixture was stirred at 100 °C for 4 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (DCM: MeOH = 20:1) to give a white solid product **30** (11 mg, yield 33%). ¹H NMR (500 MHz, DMSO-*d*₆) δ 12.88 (s, 1H), 8.04 (d, *J* = 8.6 Hz, 1H), 7.62-7.58 (m, 2H), 7.34 (d, *J* = 1.9 Hz, 1H), 7.16 (dd, *J* = 8.6, 1.9 Hz, 1H), 6.58 (d, *J* = 7.8 Hz, 1H), 4.15 (d, *J* = 12.7 Hz, 2H), 4.08 (d, *J* = 12.7 Hz, 2H), 3.92 (dd, *J* = 11.3, 2.4 Hz, 1H), 3.59-3.57 (m, 2H), 3.03 (t, *J* = 6.9 Hz, 4H), 2.97 (br, 4H), 2.84-2.80 (m, 1H), 2.76 (s, 2H), 2.52-2.49 (m, 1H, in DMSO-*d*₆), 1.88-1.79 (m, 2H), 1.75 (br, 4H), 1.17 (d, *J* = 6.2 Hz, 3H), 1.15 (t, *J* = 3.1 Hz, 2H), 0.90-0.86 (m, 2H), 0.38 (s, 4H) ppm. MS *m*/*z* 595.4 [M+H]⁺.

### Example 30: Preparation of Compound 31

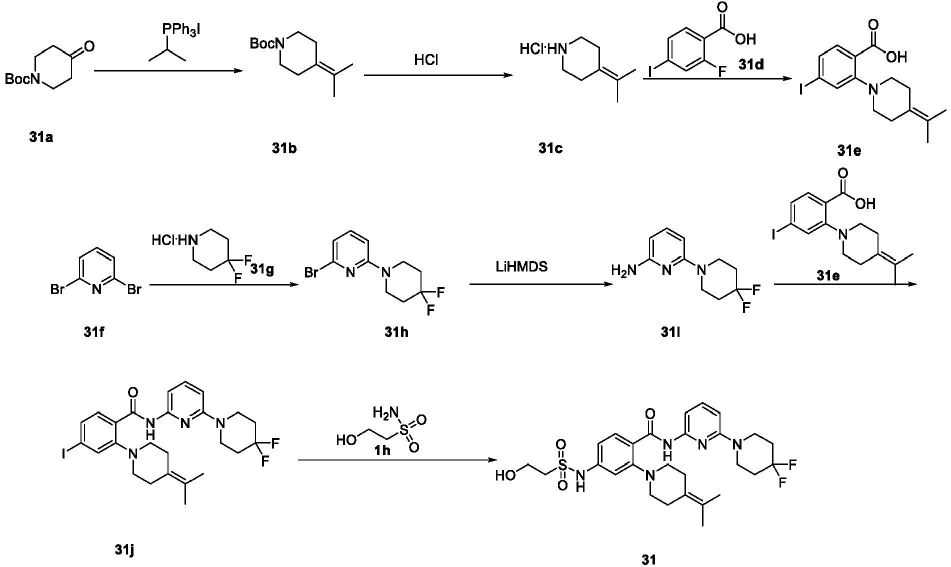

Isopropyltriphenylphosphine iodide (2.17 g, 5.02 mmol) was dissolve in toluene (5 mL), and bis (trimethylsilylamine) potassium (1M, 5 mL) was added under N₂ at room temperature. The mixture was stirred at RT for 10 min and then **31a** (500 mg, 2.51 mmol) was added. The reaction was stirred at 110 °C for 2 h under N₂. The mixture was filterated and concentrated. The crude was purified by column chromatography (PE: EA = 10:1) to give colorless oil compound 31b (400 mg, yield 71%).

The compound **31b** (400 mg, 1.78 mmol) was dissolved in DCM (5 mL). HCl/dioxane (4 M, 0.5 mL) was added. The reaction was stirred at RT for 3 h. The mixture was concentrated and the crude product **31c** was used to the next step directly.

The compound **31c** (40 mg, 0.15 mmol), **31d** (32 mg, 0.19 mmol) and K₂CO₃ (Potassium carbonate, 1.92 g, 5.89 mmol) was dissolved in DMSO (1 mL). The mixture was stirred at 140 °C for 36 h. After the reaction was finished, 1M HCl was added to adjust pH to 5-6. The mixture was extracted with EA (30 mL×3). The collected organic phase was washed with sat.NaCl, dried with anhydrous sodium sulfate, filterated and concentrated. The crude was purified by column chromatography (PE: EA = 25:1) to give a colorless oil compound **14b** (449 mg, yield 42%). MS m/z 291.3 [M+H]⁺.

The compound **31f** (1 g, 4.22 mmol), **31g** (860 mg, 5.49 mmol) and Cs₂CO₃ (4.13 g, 12.66 mmol) was dissolved in NMP (8 mL). The mixture was stirred at 140 °C for 3 h. After the reaction was finished, water was added and extracted with EA (30 mL×3). The collected organic phase was washed with sat.NaCl, dried with anhydrous sodium sulfate, filterated and concentrated. The crude was purified by column chromatography (PE: EA = 25:1) to give a colorless oil compound **31h** (1 g, yield 85%). MS m/z 277.3, 279.3 [M+H]⁺.

The compound **31h** (250 mg, 0.90 mmol), tri(dibenzylidene acetone) dipalladium (82 mg, 0.09 mmol) and 2-(Dicyclohexylphosphine) biphenyl (63 mg, 0.18 mmol) was dissolved in dioxane (20 mL). Then A solution of lithium bis (trimethylsilylamine) in tetrahydrofuran (1 M, 5 mL) was added. The mixture was stirred at 60 °C for 2 h under N₂. The mixture was cooled to RT, filterated and washed. The obtained filtrate is concentrated to give crude product. The crude was purified by column chromatography (PE: EA = 10: 1) to give yellow oil compound **31i** (130 mg, yield 68%). MS m/z 214.4 [M+H]⁺.

The compound **31e** (12 mg, 0.02 mmol), **31i** (8 mg, 0.03 mmol), diisopropylethylamine (11.7 mg, 0.09 mmol) and N,N,N',N'-Tetramethyl-O- (7-azabenzotriazol-1-yl) hexafluorophosphourea (7 mg, 0.05 mmol) was dissolved in MeCN (2 mL). The reaction was stirred at RT for 1 h under N₂. After the reaction was finished, the mixture was concentrated and purified by column chromatography (PE: EA = 10:1) to give a white solid product **31j** (13 mg, 66%). MS m/z 567.5 [M+H]⁺.

The compound **1h** (4 mg, 0.03 mmol), sarcosine (0.45 mg, 0.005 mmol), CuI (0.95 mg, 0.005 mmol) and tripotassium phosphate (19 mg, 0.06 mmol) were dissolved in DMF (1 mL). The reaction was stirred at 50 °C for 5 min under a sealed tube. Then **31j** (12 mg, 0.02 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA = 1:1)) to give a white solid product **31** (4 mg, yield 33%).¹H NMR (500 MHz, DMSO-*d*₆) δ 12.91 (s, 1H), 10.22 (br, 1H), 8.04 (d, *J* = 8.7 Hz, 1H), 7.60 (d, *J* = 1.6 Hz, 1H), 7.59 (s, 1H), 7.16 (d, *J* = 1.7 Hz, 1H), 7.09 (dd, *J* = 8.6, 1.8 Hz, 1H), 6.69-6.65 (m, 1H), 5.03-4.85 (m, 1H), 3.75 (t, *J* = 6.4 Hz, 2H), 3.65-3.59 (m, 4H), 3.35 (d, *J* = 6.4 Hz, 2H), 2.91 (t, *J* = 5.3 Hz, 4H), 2.66-2.57 (m, 4H), 1.91-1.80 (m, 4H), 1.69 (s, 6H) ppm. MS *m*/*z* 564.5 [M+H]⁺.

### Example 31: Preparation of Compound 32

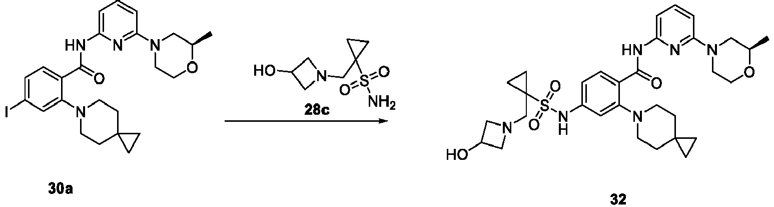

To a solution of **28c** (14 mg, 0.07 mmol) in DMF (1 mL) was added sarcosine (1.33 mg, 0.02 mmol), CuI (2.8 mg, 0.02 mmol) and tripotassium phosphate (38 mg, 0.18 mmol). The reaction was stirred at 50 °C for 5 min under a sealed tube. Then **30a** (30 mg, 0.06 mmol, synthesised according to WO2020132651) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (DCM: MeOH = 20:1) to give a white solid product **32** (8 mg, yield 23%).

### Example 32: Preparation of Compound 33

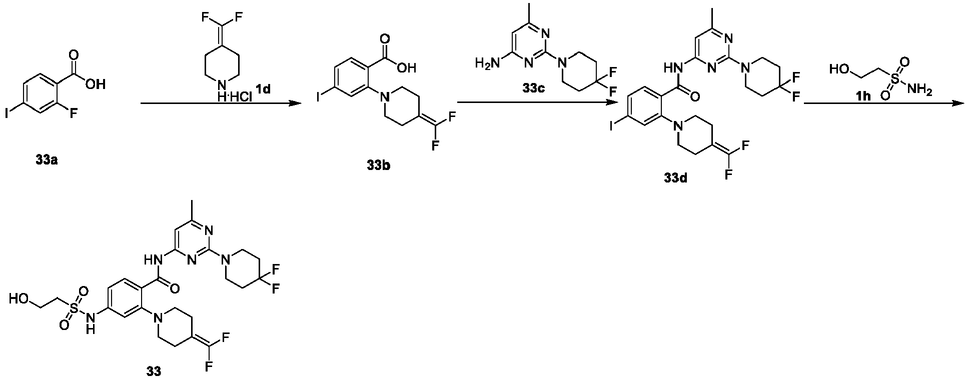

The compound **33a** (1 g, 3.76 mmol), **1d** (830 mg, 4.89 mmol) and potassium carbonate (1.56 g, 11.28 mmol) was dissolved in DMSO (5 mL). The mixture was stirred at 140 °C for 18 h. After the reaction was finished, 1M HCl was added to adjust pH to 5-6. The mixture was extracted with EA (30 mL × 3). The collected organic phase was washed with saturated NaCl, dried with anhydrous sodium sulfate, filterated and concentrated. The crude product was purified by column chromatography (petroleum ether : EA = 5:1) to give a white solid **33b** (350 mg, yield 25%). MS m/z 379.9 [M+H]⁺.

The compound **33b** (50 mg, 0.13 mmol), **33c** (30 mg, 0.13 mmol), N,N,N',N'-tetramethyl-O- (7-azabenzotriazol-1-yl) hexafluorophosphourea (50 mg, 0.39 mmol) and N,N-diisopropylethylamine (50 mg, 0.39 mmol) was dissolved in MeCN (2 mL). The reaction was stirred at 70 °C for 1 h under N₂. After the reaction was finished, the mixture was concentrated and purified by column chromatography (petroleum ether : EA = 10:1) to give a white solid **33d** (50 mg, yield 39%). MS m/z 590.5 [M+H]⁺.

To a solution of **1h** (5 mg, 0.04 mmol) in DMF (1 mL) was added sarcosine (0.70 mg, 0.007 mmol), CuI (1.4 mg, 0.007 mmol) and tripotassium phosphate (19 mg, 0.09 mmol). The reaction was stirred at 50 °C for 5 min under a sealed tube. Then **33d** (15 mg, 0.03 mmol) was added and the mixture was stirred at 100 °C for overnight under N₂. TLC indicated that the reaction was complete. The reaction was quenched with saturated NH₄Cl and extracted with EA (5 mL×3). The collected organic phase was washed with sat.NaCl, dried with anhydrous sodium sulfate, filterated and concentrated. The crude product was purified by Pre-TLC (petroleum ether : EA= 1:1)) to give a white solid **33** (7 mg, yield 47%). ¹H NMR (500 MHz, DMSO-*d*₆) δ 12.93 (s, 1H), 10.31 (br, 1H), 8.02 (d, *J* = 8.6 Hz, 1H), 7.39 (s, 1H), 7.17 (d, *J* = 2.0 Hz, 1H), 7.13 (dd, *J* = 8.6, 2.0 Hz, 1H), 4.96 (br, 1H), 3.86-3.80 (m, 4H), 3.75 (t, *J* = 6.5 Hz, 2H), 3.37 (t, *J* = 6.5 Hz, 2H), 2.99 (t, *J* = 5.4 Hz, 4H), 2.60-2.52 (m, 4H), 2.32 (s, 3H), 1.96-1.86 (m, 4H) ppm. MS *m*/*z* 587.3 [M+H]⁺.

### Example 33: Preparation of Compound 34

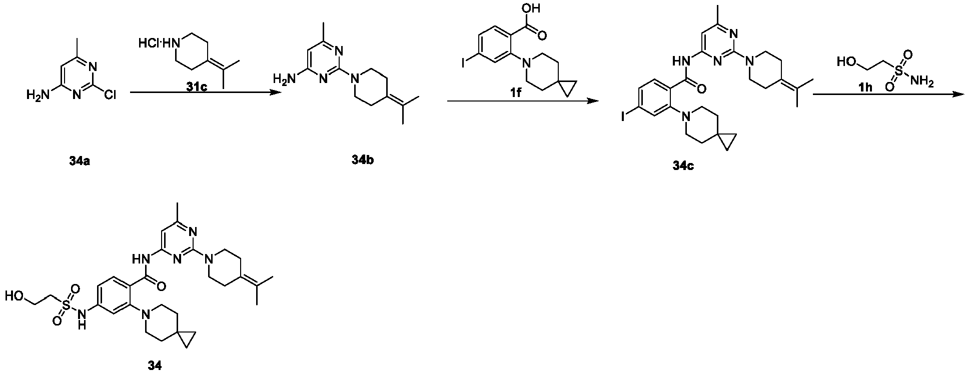

The compound **34a** (100 mg, 0.7 mmol), **31c** (146 mg, 0.9 mmol) and DIPEA (270 mg, 0.21 mmol) was dissolved in NMP (8 mL). The mixture was stirred at 140 °C for 12 h. After the reaction was finished, water was added and extracted with EA (30 mL × 3). The collected organic phase was washed with sat.NaCl, dried with anhydrous sodium sulfate, filterated and concentrated. The crude was purified by column chromatography (PE: EA = 10:1) to give a colorless oil compound **34b** (90 mg, yield 56%). MS m/z 233.5 [M+H]⁺.

The compound **34b** (50 mg, 0.22 mmol), **1f** (82 mg, 0.22 mmol), N,N,N',N'-Tetramethyl-O- (7-azabenzotriazol-1-yl) hexafluorophosphourea (125 mg, 0.33 mmol) and diisopropylethylamine (86 mg, 0.66 mmol) was dissolved in MeCN (2 mL). The reaction was stirred at 70 °C for overnight under N₂. After the reaction was finished, the mixture was concentrated and purified by column chromatography (PE: EA = 10:1) to give a white solid product **34c** (44 mg, yield 36%). MS m/z 572.5 [M+H]⁺.

To a solution of **1h** (7 mg, 0.05 mmol) in DMF (1 mL) was added sarcosine (0.9 mg, 0.01 mmol), CuI (1.89 mg, 0.01 mmol) and tripotassium phosphate (25 mg, 0.12 mmol). The reaction was stirred at 50 °C for 5 min under a sealed tube. Then **33d** (15 mg, 0.03 mmol) was added and the mixture was stirred at 100 °C for overnight under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA = 2:1)) to give a white solid product **34** (2 mg, yield 10%).¹H NMR (500 MHz, DMSO-*d*₆) δ 13.31 (s, 1H), 10.27 (br, 1H), 8.02 (d, *J* = 8.6 Hz, 1H), 7.31 (s, 1H), 7.23 (s, 1H), 7.09 (d, *J* = 8.1 Hz, 1H), 4.95 (br, 1H), 3.79-3.71 (m, 6H), 3.35-3.33 (m, 2H, in H₂O), 2.97 (br, 4H), 2.28 (br, 7H), 2.05-1.94 (m, 4H), 1.68 (s, 6H), 0.39 (s, 4H) ppm. MS *m*/*z* 569.3 [M+H]⁺.

### Example 34: Preparation of Compound 35 and 35'

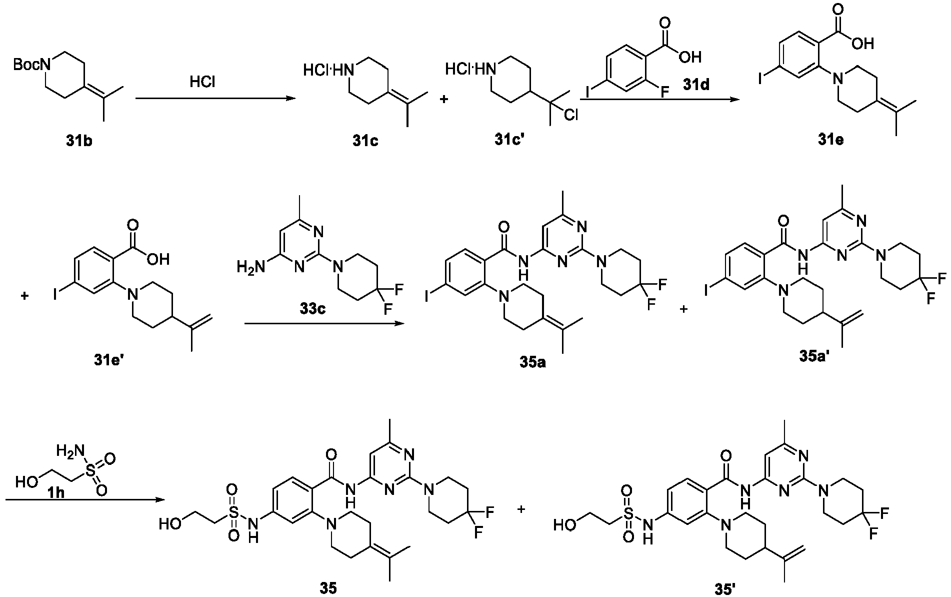

The compound **31b** (400 mg, 1.78 mmol) was dissolved in DCM (5 mL). HCl/dioxane (4 M, 0.5 mL) was added. The reaction was stirred at RT for overnight. The mixture was concentrated to afford product **31 and 31c'** and the crude was used to the next step directly. MS m/z 126.1, 162.1 [M+H]⁺. ¹H NMR (500 MHz, DMSO-*d*6) δ 9.22 (br, 2H), 8.82 (br, 2H), 3.30 (d, *J* = 12.4 Hz, 4H), 2.82 (q, *J* =11.4 Hz, 4H), 1.99-1.91 (m, 4H), 1.88-1.82 (m, 2H), 1.63-1.54 (m, 15H) ppm.

The compound **31d** (1 g, 3.76 mmol), a mixture of **31c and 31c' (31c: 31c'** ≈ 1:1) (790 mg, 4.89 mmol) and K₂CO₃ (1.56 g, 11.28 mmol) was dissolved in NMP (8 mL). The mixture was stirred at 140 °C for 48 h. After the reaction was finished, 1M HCl was added to adjust pH to 5-6, and extracted with EA (30 mL×3). The collected organic phase was washed with sat.NaCl, dried with anhydrous sodium sulfate, filterated and concentrated. The crude was purified by column chromatography (DCM: MeOH = 10:1) to give a mixture of product **31e** and **31e' (31e: 31e'** ≈ 1:1.5) (0.8 g, yield 57%) as white solid. MS m/z 372.3 [M+H]⁺. ¹H NMR (500 MHz, DMSO-*d*6) δ 8.03 (d, *J* = 1.6 Hz, 1H), 7.96 (d, *J* = 1.6 Hz, 1H), 7.77-7.67 (m, 5H), 4.78 (d, *J* = 9.7 Hz, 1H), 3.16-3.08 (m, 6H), 3.04 (t, *J* = 5.7 Hz, 4H), 2.43 (t, *J* = 5.7 Hz, 4H), 2.17-2.11 (m, 1.5H), 1.93-1.87 (m, 3H), 1.75 (s, 4.5H), 1.69 (s, 6H), 1.56-1.48 (m, 3H) ppm.

The mixture compound **31e** and **31e' (31e: 31e**' ≈ 1:1.5) (30 mg, 0.08 mmol), **33c** (18 mg, 0.08 mmol), N,N,N',N'-Tetramethyl-O- (7-azabenzotriazol-1-yl) hexafluorophosphourea (46 mg, 0.12 mmol) and diisopropylethylamine (31 mg, 0.24 mmol) was dissolved in MeCN (2 mL). The reaction was stirred at RT for 1 h under N₂. After the reaction was finished, the mixture was concentrated and purified by column chromatography (PE: EA = 10:1) to give a mixture of product **31a** and **31a'(35a: 35a'** ≈ 1:1.5) (10 mg, yield 22%) as white solid (44 mg, yield 36%). MS m/z 582.5 [M+H]⁺.

The compound **1h** (3.2 mg, 0.02 mmol), sarcosine (0.38 mg, 0.004 mmol), CuI (0.80 mg, 0.004 mmol) and tripotassium phosphate (11 mg, 0.05 mmol) were dissolved in DMF (1 mL). The reaction was stirred at 50 °C for 5 min under a sealed tube. Then **35** and **35a'** (12 mg, 0.017 mmol) was added and the mixture was stirred at 100 °C for 4 h under N₂. TCL showed the reaction was completed. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA=1:1) to give a mixture of product **35** and **35' (35: 35'** ≈ 1:1.5) (4 mg, yield 33%) as white solid. MS *m*/*z* 579.2 [M+H]⁺.

### Example 35: Preparation of Compound 36

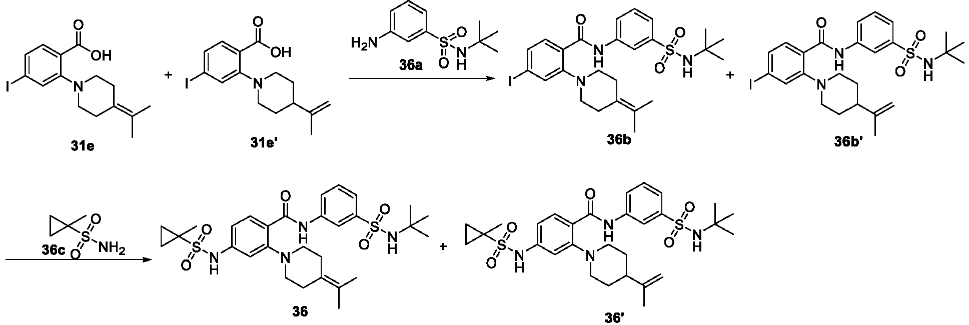

The mixture compound **31e** and **31e' (31e: 31e'** ≈ 1:1.5) (example **34, 200** mg, 0.54 mmol), **36a** (123 mg, 0.54 mmol), N,N,N',N'-Tetramethyl-O- (7-azabenzotriazol-1-yl) hexafluorophosphourea (307.8 mg, 0.81 mmol) and diisopropylethylamine (210 mg, 1.62 mmol) was dissolved in MeCN (5 mL). The reaction was stirred at RT for 1 h under N₂. After the reaction was finished, the mixture was concentrated and purified by column chromatography (PE: EA = 15:1) to give a mixture of product **36b** and **36b'** (210 mg, yield 61%) as white solid (44 mg, yield 36%). MS m/z 582.5 [M+H]⁺.

The mixture compound **36b** and **36b'(30mg,** 0.05 mmol), **36c** (10 mg, 0.08 mmol), (1R, 2R)-N, N-dimethyl-1,2-cyclohexanediamine (1.7 mg, 0.01 mmol), CuI (2.4 mg, 0.01 mmol) and tripotassium phosphate (32 mg, 0.15 mmol) were dissolved in DMF (1 mL). The reaction was stirred at 100 °C for 12 h in a sealed tube under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (DCM: MeOH = 20:1) to give a mixture of product **36** and **36'(36: 36'** ≈ 1:1.5) (20 mg, yield 67%) as white solid. MS m/z 589.2 [M+H]⁺.¹H NMR (500 MHz, DMSO-*d*₆) δ 11.62 (s, 1H), 11.48 (s, 1.5H), 10.15 (s, 2.5H), 8.42 (s, 1.5H), 8.35 (s, 1H), 7.87-7.85 (m, 1H), 7.78-7.72 (m, 3H), 7.58-7.51 (m, 6H), 7.15 (d, *J* = 2.0 Hz, 1.5H), 7.11 (d, *J* = 2.0 Hz, 1H), 7.04-6.98 (m, 2.5H), 6.75 (s, 2.5H), 4.71-4.66 (m, 3H), 3.21 (d, *J* = 11.3 Hz, 3H), 2.95-2.83 (m, 4H), 2.77-2.70 (m, 3H), 2.39-2.33 (m, 4H), 2.04-1.99 (m, 1.5H), 1.76 (d, *J* = 12.7 Hz, 3H), 1.67 (s, 3H), 1.64 (s, 4.5H), 1.57-1.49 (m, 3H), 1.42-1.37 (10.5H), 1.10 (s, 12H), 1.09 (s, 9H), 0.84-0.71 (m, 10H) ppm.

### Example 36: Preparation of Compound 37 and 37'

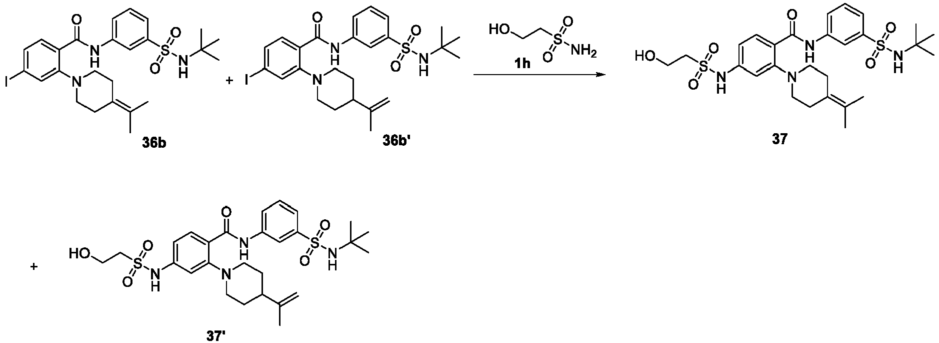

The compound **1h** (9 mg, 0.07 mmol), sarcosine (1 mg, 0.01 mmol), CuI (2 mg, 0.01 mmol) and tripotassium phosphate (32 mg, 0.15 mmol) were dissolved in DMF (1 mL). The reaction was stirred at 50 °C for 5 min under a sealed tube. Then **36** and **36**'(example **35,** 30 mg, 0.05 mmol) was added and the mixture was stirred at 100 °C for 4 h under N₂. TCL showed the reaction was completed. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA=1:1) to give a mixture of product **37** and **37'(37: 37'** ≈ 1:2) (20 mg, yield 67%) as white solid. MS m/z 579.1 [M+H]⁺. ¹H NMR (500 MHz, DMSO-*d*₆) δ 11.54 (s, 1H), 11.37 (s, 2H), 10.10 (s, 3H), 8.42 (s, 2H), 8.35 (s, 1H), 7.88-7.82 (m, 1H), 7.78-7.71 (m, 4H), 7.59-7.51 (m, 7H), 7.08 (d, *J* = 2.1 Hz, 2H), 7.05 (d, *J* = 2.1 Hz, 1H), 7.00-6.97 (m, 3H), 4.97 (br, 3H), 4.72-4.64 (m, 4H), 3.78-3.72 (m, 6H), 3.32-3.29 (m, 6H), 3.23 (d, *J* = 12.0 Hz, 4H), 2.91 (t, *J* = 5.6 Hz, 4H), 2.74 (t, *J* = 11.6 Hz, 4H), 2.38-2.32 (m, 4H), 2.03-1.96 (m, 2H), 1.75 (d, *J* = 12.0 Hz, 4H), 1.66 (s, 6H), 1.63 (s, 6H), 1.55-1.47 (m, 4H), 1.10 (s, 18H), 1.09 (s, 9H) ppm.

### Example 37: Preparation of Compound 38

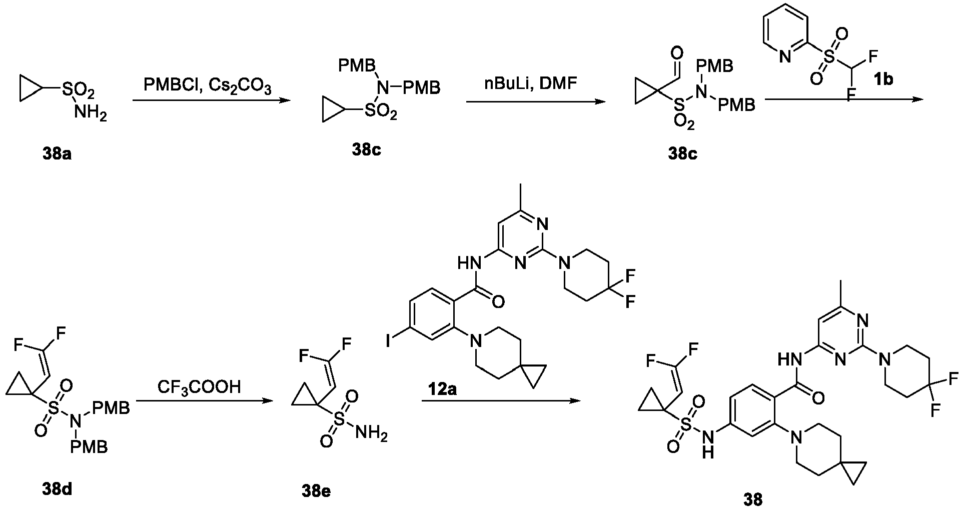

The compound **38a** (1.21g, 10 mmol) was dissolved in DMF (20 mL). Then 4-Methoxybenzyl chloride (3.20 g, 20.5 mmol) and Cs₂CO₃ (6.78 g, 21mmol) were added. The mixture was stirred at 50 °C for 2 h. After the reaction was finished, water was added and extracted with EA. The collected organic phase was washed with sat.NaCl, dried with anhydrous sodium sulfate, filterated and concentrated. The crude was purified by column chromatography (PE: EA = 10:1) to give the product **38b** (2.1 g, yield 57%).

The compound **38b** (361 mg, 1.0 mmol) was dissolved in THF (5.0 mL). The mixture was cooled to -78 °C and nBuLi (2.5 M, 0.8 mL) was added. The reaction was stirred at this temperature for 0.5 h. Then DMF (365 mg, 5 mmol) was added. The mixture was warmed to RT and stirred for 16 h. The reaction was quenched with sat.NH₄Cl and extracted with EA for two times. The collected organic phase was washed with sat.NaCl, dried with anhydrous sodium sulfate, filterated and concentrated. The crude was purified by column chromatography (PE: EA = 10:1) to give product **38c** (260 mg, 57%). ¹H NMR (500 MHz, CDCl₃) *δ* 9.70 (s, 1 H), 7.12 (d, *J* = 8.6 Hz, 4H), 6.86 (d, *J* = 8.6 Hz, 4H), 4.36 (s, 4H), 3.81 (s, 6H), 1.80 (q, *J* = 4.6 Hz, 2H), 1.58 (q, *J* = 4.6 Hz, 2H) ppm.

The compound **38c** (200 mg, 0.51 mmol), **1b** (193 mg, 1.00 mmol) were dissolved in DMF (2 mL). The mixture was cooled to -78 °C under N₂ and tBuOK (86 mg, 0.77 mmol) was added. Then the reaction warmed to RT and stirred at RT for 1 h. Con.HCl was added to the mixture. The reaction was stirred at 60 °C for another 1 h. The mixture was extracted with EA (3×5 mL). The collected organic phase was washed with sat.NaCl, dried with anhydrous sodium sulfate, filterated and concentrated. The crude was purified by column chromatography (PE: EA = 10:1) to give product **38d** (80 mg, yield 37%).

The compound **38d** (80 mg, 0.21 mmol) was dissolved in TFA (1 mL). The reaction was stirred at RT for 12 h. The mixture was concentrated. The crude was purified by column chromatography (DCM: MeOH = 20:1) to give product **38e** (18 mg, yield 48%). ¹H NMR (500 MHz, DMSO-*d*₆) δ 7.01 (s, 2H), 4.92 (d, *J* = 25.0 Hz, 1H), 1.38-1.33 (m, 2H), 1.08-1.04 (m, 2H) ppm.

To a solution of **38e** (5 mg, 0.03 mmol) in DMF (1 mL) was added sarcosine (0.5 mg, 0.01 mmol), CuI (0.9 mg, 0.01 mmol) and tripotassium phosphate (13 mg, 0.06 mmol). The reaction was stirred at 50 °C for 5 min under a sealed tube. Then **12a** (12 mg, 0.02 mmol) was added and the mixture was stirred at 100 °C for overnight under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA = 1:1) to give a white solid product **38** (1.5 mg, yield 11%). MS m/z 623.2 [M+H]⁺.

### Example 38: Preparation of Compound 39

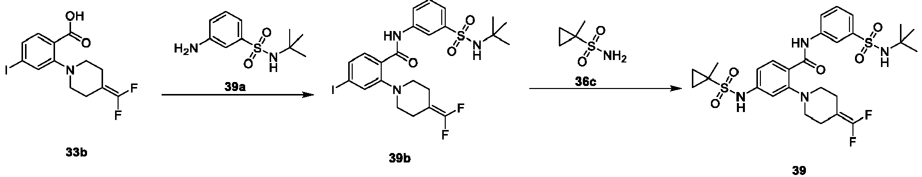

The compound **33b** (50 mg, 0.13 mmol), **39a** (30 mg, 0.13 mmol), N,N,N',N'-Tetramethyl-O- (7-azabenzotriazol-1-yl) hexafluorophosphourea (74 mg, 0.20 mmol) and diisopropylethylamine (50 mg, 0.39 mmol) was dissolved in MeCN (2 mL). The reaction was stirred at RT for overnight under N₂. After the reaction was finished, the mixture was concentrated and purified by column chromatography (PE: EA = 10:1) to give a white solid product **39b** (30 mg, yield 39%). MS m/z 590.5 [M+H]⁺.

To a solution of **36c** (4 mg, 0.03 mmol) in DMF (1 mL) was added sarcosine (0.5 mg, 0.01 mmol), CuI (0.9 mg, 0.01 mmol) and tripotassium phosphate (13 mg, 0.06 mmol). The reaction was stirred at 50 °C for 5 min under a sealed tube. Then **39b** (15 mg, 0.03 mmol) was added and the mixture was stirred at 100 °C for overnight under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (DCM: EA = 1:1) to give a white solid product **39** (1.5 mg, yield 11 %). ¹H NMR (500 MHz, DMSO-*d₆*)δ 11.32 (br, 1H), 10.18 (br, 1H), 8.31 (s, 1H), 7.91 (d, *J* = 7.4 Hz, 1H), 7.67 (d, *J* = 8.4 Hz, 1H), 7.59 (s, 1H), 7.56-7.50 (m, 2H), 7.04 (s, 1H), 6.98 (d, *J* = 8.0 Hz, 1H), 2.94 (t, *J* = 5.2 Hz, 4H), 2.28 (br, 4H), 1.39 (s, 3H), 1.17 (t, *J* = 5.0 Hz, 2H), 1.10 (s, 9H), 0.77 (br, 2H) ppm. MS *m*/*z* 597.1 [M+H]⁺.

### Example 39: Preparation of Compound 40

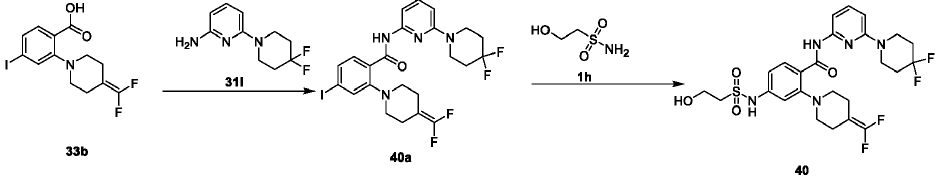

The compound **33b** (50 mg, 0.13 mmol), **31i** (28 mg, 0.13 mmol), N,N,N',N'-Tetramethyl-O- (7-azabenzotriazol-1-yl) hexafluorophosphourea (74 mg, 0.20 mmol) and diisopropylethylamine (50 mg, 0.39 mmol) was dissolved in MeCN (2 mL). The reaction was stirred at RT for overnight under N₂. After the reaction was finished, the mixture was concentrated and purified by column chromatography (PE: EA = 10:1) to give a white solid product **40a** (50 mg, yield 47%). MS m/z 575.5 [M+H]⁺.

To a solution of **1h** (4 mg, 0.03 mmol) in DMF (1 mL) was added sarcosine (0.5 mg, 0.01 mmol), CuI (0.9 mg, 0.01 mmol) and tripotassium phosphate (13 mg, 0.06 mmol). The reaction was stirred at 50 °C for 5 min under a sealed tube. Then **40a** (15 mg, 0.03 mmol) was added and the mixture was stirred at 100 °C for overnight under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (DCM: EA = 1:1) to give a white solid product **40** (7 mg, yield 47%). ¹H NMR (500 MHz, DMSO-*d*₆) δ 12.60 (s, 1H), 10.18 (br, 1H), 8.01 (d, *J* = 8.6 Hz, 1H), 7.63-7.58 (m, 2H), 7.12 (s, 1H), 7.08 (d, *J* = 8.7 Hz, 1H), 6.73-6.67 (m, 1H), 4.97 (br, 1H), 3.75 (t, *J* = 6.5 Hz, 2H), 3.69-3.62 (m, 4H), 3.30 (t, *J* = 6.4 Hz, 2H), 2.99 (t, *J* = 5.3 Hz, 4H), 2.54 (br, 4H), 1.97-1.87 (m, 4H) ppm. MS m/z 572.1 [M+H]⁺.

### Example 40: Preparation of Compound 41

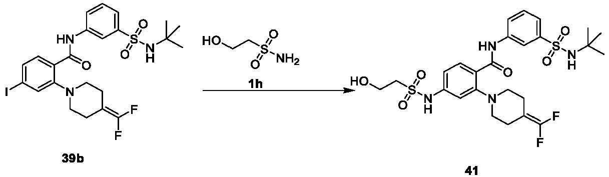

To a solution of **1h** (4 mg, 0.03 mmol) in DMF (1 mL) was added sarcosine (0.5 mg, 0.01 mmol), CuI (0.9 mg, 0.01 mmol) and tripotassium phosphate (13 mg, 0.06 mmol). The reaction was stirred at 50 °C for 5 min under a sealed tube. Then **39b** (15 mg, 0.03 mmol) was added and the mixture was stirred at 100 °C for overnight under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (DCM: EA = 1:1) to give a white solid product **41** (7 mg, yield 43%).

### Example 41: Preparation of Compound 42

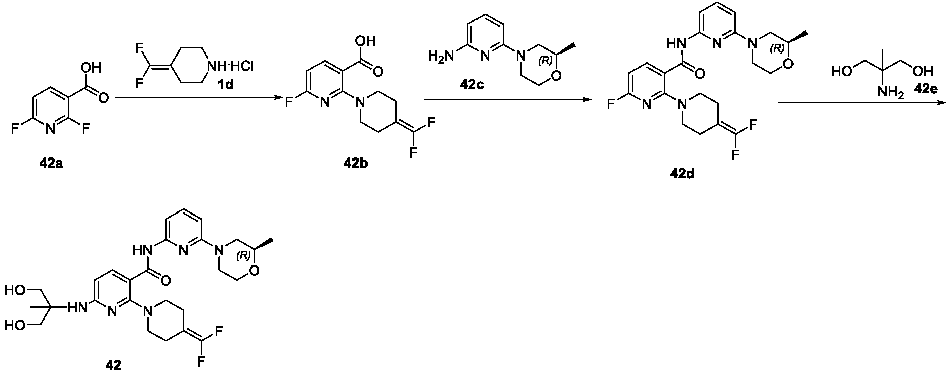

The compound **42a** (60 mg, 0.38 mmol) was dissolved in dioxane (2 mL). Then DIPEA (146.2 mg, 1.13 mmol) and **1d** (70 mg, 0.41 mmol) were added. The mixture was placed in a 25mL single necked bottle and stirred at RT for overnight. After the reaction was finished, water was added and extracted with DCM. The collected organic phase was washed with sat.NaCl, dried with anhydrous sodium sulfate, filterated and concentrated. The crude was purified by column chromatography (DCM: MeOH = 20:1) to give the product product **42b** (70 mg, yield 68%). MS *m*/*z* 273.1 [M+H]⁺.

The compound **42b** (70 mg, 0.26 mmol) was dissolved in DCM (2 mL). The compound **42c** (52 mg, 0.27 mmol, synthesised according to WO2020132653), N,N,N',N'-Tetramethyl-O- (7-azabenzotriazol-1-yl) hexafluorophosphourea (146 mg, 0.38 mmol) and diisopropylethylamine (100 mg, 0.77 mmol) were added. The reaction was stirred at RT for 48h under N₂. After the reaction was finished, water was added and extracted with DCM. The collected organic phase was washed with sat.NaCl, dried with anhydrous sodium sulfate, filterated and concentrated. The crude was purified by column chromatography (PE: EA = 10:1) to give the product product **42d** (60 mg, yield 52%). MS *m*/*z* 448.1 [M+H]⁺.

The compound **42d** (50 mg, 0.11 mmol) was dissolved in DMSO (1 mL). The **42e** (47 mg, 0.45 mmol) and tripotassium phosphate (95 mg, 0.45 mmol) were added. The reaction was stirred at 120 °C in a sealed tube for overnight under N₂. Water was added to quench the reaction. The reaction was extracted with EA (5 mL×3). The collected organic phase was washed with sat.NaCl, dried with anhydrous sodium sulfate, filterated and concentrated. The crude was purified by Pre-TLC (DCM: MeOH = 10:1) to give a white solid product **42** (11 mg, yield 18%). MS *m*/*z* 533.2 [M+H]⁺. ¹H NMR (500 MHz, DMSO-*d*₆) 12.24 (s, 1H), 7.94 (d, *J* = 8.7 Hz, 1H), 7.65-7.46 (m, 2H), 6.66 (d, *J* = 1.8 Hz, 1H), 6.55 (dd, *J* = 7.0, 1.8 Hz, 1H), 6.49 (d, *J* = 8.7 Hz, 1H), 4.69 (t, *J* = 5.8 Hz, 2H), 4.11 (d, *J* = 12.3 Hz, 1H), 3.86 (m, 2H), 3.72-3.59 (m, 4H),3.58-3.49 (m, 2H), 3.04 (t, *J* = 5.7 Hz, 4H), 2.81-2.76 (m, 1H), 2.60-2.52 (m, 4H), 2.40-2.35 (m, 1H), 1.30 (s, 3H), 1.09 (d, *J* = 6.2 Hz, 3H) ppm.

### Example 42: Preparation of Compound 43

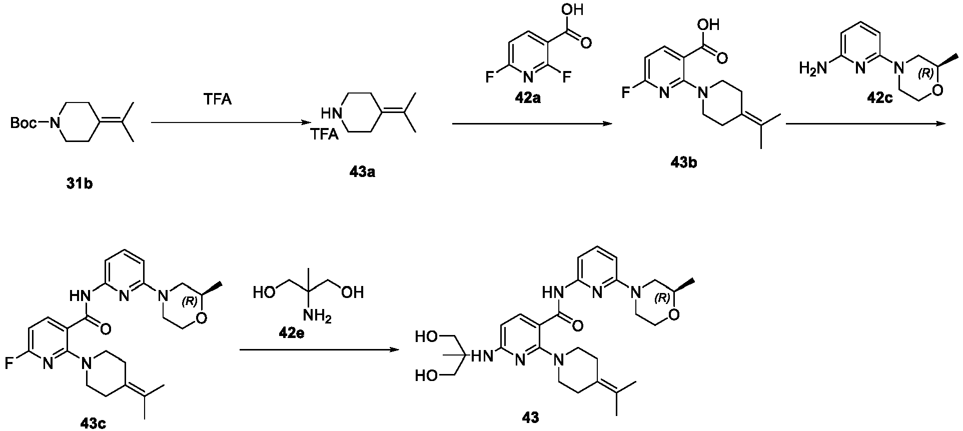

The compound **31b** (1 g, 4.44 mmol) was dissolved in DCM (10 mL). TFA (2 mL) was added. The reaction was stirred at RT for 2 h. The mixture was concentrated to give brown oil **43a** (1.1 g). The crude was used to the next step directly.

The above compound **43a** (1.1 g) was dissolved in dioxane (10 mL). Then DIPEA (3.44 g, 26.64 mmol) and **42a** (706 mg, 4.44 mmol) were added. The mixture was stirred at RT for overnight. The mixture was concentrated. The crude was purified by column chromatography (DCM: MeOH = 15:1) to give yellow solid product **43b** (1 g, yield 85%). MS m/z 265.1 [M+H]⁺.

The compound **43b** (30 mg, 0.11 mmol) was dissolved in DCM (2 mL). The compound **42c** (23 mg, 0.12 mmol), N,N,N',N'-Tetramethyl-O-(7-azabenzotriazol-1-yl) hexafluorophosphourea (63 mg, 0.17 mmol) and diisopropylethylamine (57 mg, 0.44 mmol) were added. The reaction was stirred at RT for 48h under N₂. After the reaction was finished, water was added and extracted with DCM (5 mL × 3). The collected organic phase was washed with sat.NaCl, dried with anhydrous sodium sulfate, filterated and concentrated. The crude was purified by column chromatography (PE: EA = 10:1) to give the product **43c** (30 mg, yield 60%). MS *m*/*z* 440.1 [M+H]⁺.

The compound **43c** (30 mg, 0.07 mmol) was dissolved in DMSO (1 mL). The **42e** (44 mg, 0.42 mmol) and tripotassium phosphate (89 mg, 0.42 mmol) were added. The reaction was stirred at 120 °C in a sealed tube for overnight under N₂. Water was added to quench the reaction. The reaction was extracted with EA (5 mL × 3). The collected organic phase was washed with sat.NaCl, dried with anhydrous sodium sulfate, filterated and concentrated. The crude was purified by Pre-TLC (DCM: MeOH = 10:1) to give a white solid product **43** (12 mg, yield 34%). MS *m*/*z* 525.3 [M+H]⁺. ¹H NMR (500 MHz, DMSO-*d*₆) δ 12.53 (s, 1H), 7.95 (d, *J* = 8.7 Hz, 1H), 7.57-7.52 (m, 2H), 6.64 (s, 1H), 6.55-6.50 (m, 1H), 6.47 (d, *J* = 8.7 Hz, 1H), 4.70 (t, *J* = 5.7 Hz, 2H), 4.11 (d, *J* = 12.3 Hz, 1H), 3.85-3.80 (m,2H), 3.71-3.58 (m, 4H), 3.53-3.42 (m, 2H), 2.97 (t, *J* = 5.7 Hz, 4H), 2.79-2.73 (m, 1H), 2.68-2.56 (m, 4H), 2.37-2.31 (m, 1H), 1.68 (s, 6H), 1.29 (s, 3H), 1.04 (d, *J=* 6.2 Hz, 3H) ppm.

### Example 43: Preparation of Compound 44

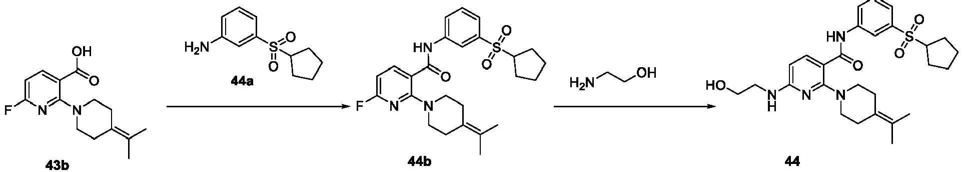

Compound **44a** was synthesized according to (Helvetica Chimica Acta, 1981, 64, 1849-1853). The compound **43b** (75 mg, 0.28 mmol) was dissolved in DCM (3 mL). The compound **42c** (23 mg, 0.12 mmol), N,N,N',N'-Tetramethyl-O-(7-azabenzotriazol-1-yl) hexafluorophosphourea (162 mg, 0.43 mmol) and diisopropylethylamine (150 mg, 1.16 mmol) were added. The reaction was stirred at RT for 96 h under N₂. After the reaction was finished, water was added and extracted with DCM (5 mL×3). The collected organic phase was washed with sat.NaCl, dried with anhydrous sodium sulfate, filterated and concentrated. The crude was purified by column chromatography (PE: EA = 10:1) to give the product **44b** (70 mg, yield 52%). MS *m*/*z* 472.2 [M+H]⁺.

The compound **44b** (28 mg, 0.06 mmol) was dissolved in DMSO (1 mL). The ethanolamine (22 mg, 0.36 mmol) and tripotassium phosphate (76 mg, 0.36 mmol) were added. The reaction was stirred at 120 °C in a sealed tube for overnight under N₂. Water was added to quench the reaction. The reaction was extracted with EA (5 mL × 3). The collected organic phase was washed with sat.NaCl, dried with anhydrous sodium sulfate, filterated and concentrated. The crude was purified by Pre-TLC (DCM: MeOH = 10:1) to give a white solid product **44** (13 mg, yield 43%). MS *m*/*z* 513.2 [M+H]⁺. ¹H NMR (500 MHz, DMSO-*d*₆) δ 11.14 (s, 1H), 8.42 (t, *J* = 2.0 Hz, 1H), 7.90 (dt, *J* = 8.2, 1.4 Hz, 1H), 7.71 (d, *J* = 8.5 Hz, 1H), 7.60 (t, *J* = 7.9 Hz, 1H), 7.52 (dt, *J* = 7.9, 1.4 Hz, 1H), 7.08 (s, 1H), 6.18 (d, *J* = 8.5 Hz, 1H), 4.71 (t, *J* = 5.4 Hz, 1H), 3.74-3.68 (m, 1H), 3.55-3.52 (m, 2H), 3.38-3.33 (m, 2H), 3.13 (t, *J* = 5.6 Hz, 4H), 2.35 (t, *J* = 5.6 Hz, 4H), 1.86-1.73 (m, 4H), 1.63 (s, 6H), 1.63-1.47 (m, 4H) ppm.

### Example 44: Preparation of Compound 45

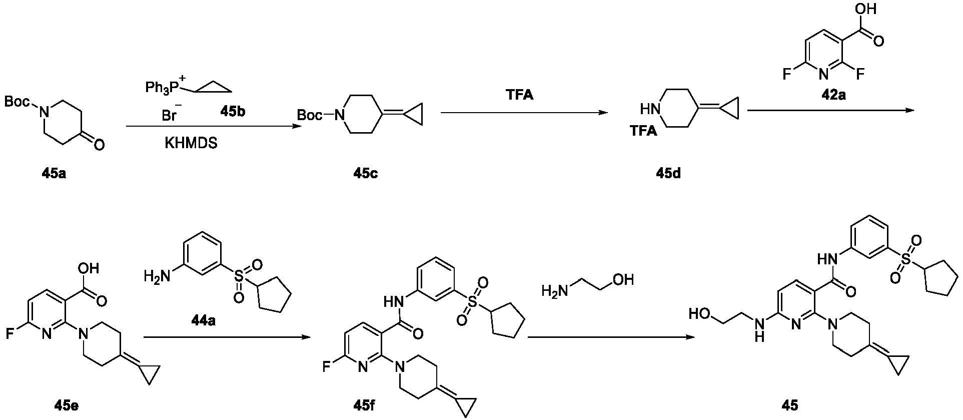

Compound **45b** (1.15 g, 3.0 mmol) was dissolve in toluene (10 mL), and bis (trimethylsilylamine) potassium (1M, 3 mL) was added. The mixture was stirred at RT for 15 min and then **31a** (500 mg, 2.51 mmol) was added after the reaction tutned to yellow. The reaction was stirred at reflux for 2 h. After the reaction was completed, the mixture was quenched in ice cubes. The mixture was filterated and concentrated. The reaction was extracted with EA for 2 times. The collected organic phase was washed with sat.NaCl, dried with anhydrous sodium sulfate, filterated and concentrated. The crude was purified by column chromatography (PE: EA= 20:1) to give the product product **45c** (150 mg, yield 34%). ¹H NMR (500 MHz, DMSO-*d*₆) *δ* 3.36 (m, 4H), 2.23 (t, *J*=5.8 Hz, 4H), 1.41 (s, 9H), 1.03 (s, 4H) ppm.

The compound **45c** (60 mg, 0.27 mmol) was dissolved in DCM (2 mL). TFA (0.5 mL) was added. The reaction was stirred at RT for 2 h. The mixture was concentrated to give the crude product **45d** (59 mg, yield 99%).

The above compound **45d** (59 mg, 0.27 mmol) was dissolved in dioxane (1 mL). Then DIPEA (208 mg, 1.61 mmol) and **42a** (47mg, 0.30 mmol) were added. The mixture was stirred at RT for overnight. Water was added to quench the reaction. The reaction was extracted with EA (5 mL × 3). The collected organic phase was washed with sat.NaCl, dried with anhydrous sodium sulfate, filterated and concentrated. The crude was purified by Pre-TLC (DCM: MeOH = 10:1) to give a yellow solid product **45e** (60 mg, yield 86%). MS *m*/*z* 263.2 [M+H]⁺.

The compound **45e** (45 mg, 0.17 mmol) was dissolved in pyrdine (1 mL). Then **44a** (42 mg, 0.19 mmol) and 1-(3-dimethylaminopropyl)-3-ethylcarbodiimide hydrochloride (66 mg, 0.34 mmol) were added. The mixture was stirred at RT for overnight. Water was added to quench the reaction. The reaction was extracted with EA (5 mL × 3). The collected organic phase was washed with sat.NaCl, dried with anhydrous sodium sulfate, filterated and concentrated. The crude was purified by Pre-TLC (DCM: MeOH = 10:1) to give a yellow solid product **45f** (35 mg, yield 44%). MS *m*/*z* 263.2 [M+H]⁺.

The compound **45f** (25 mg, 0.05 mmol) was dissolved in DMSO (1 mL). The ethanolamine (20 mg, 0.33 mmol) and tripotassium phosphate (70 mg, 0.33 mmol) were added. The reaction was stirred at 120 °C in a sealed tube for overnight under N₂. Water was added to quench the reaction. The reaction was extracted with EA (5 mL × 3). The collected organic phase was washed with sat.NaCl, dried with anhydrous sodium sulfate, filterated and concentrated. The crude was purified by Pre-TLC (DCM: MeOH = 10:1) to give a white solid product **45** (10 mg, yield 37%). MS *m*/*z* 511.1 [M+H]⁺. ¹H NMR (500 MHz, DMSO-*d*₆) δ 11.15 (s, 1H), 8.44 (s, 1H), 7.91 (d, *J* = 8.5 Hz, 1H), 7.72 (d, *J* = 8.5 Hz, 1H), 7.61 (t, *J* = 8.5 Hz, 1H), 7.52 (d, *J* = 8.5 Hz, 1H), 7.12 (br, 2H), 6.20 (d, *J* = 8.5 Hz, 1H), 4.71 (t, *J* = 5.4 Hz, 1H), 3.75-3.68 (m, 1H), 3.55-3.52 (m, 2H), 3.40-3.35 (m, 2H), 3.27-3.14 (m, 4H), 2.46-2.36 (m, 4H), 1.87-1.78 (m, 4H), 1.60-1.50 (m, 4H), 1.02 (s, 4H) ppm.

### Example 45: Preparation of Compound 46

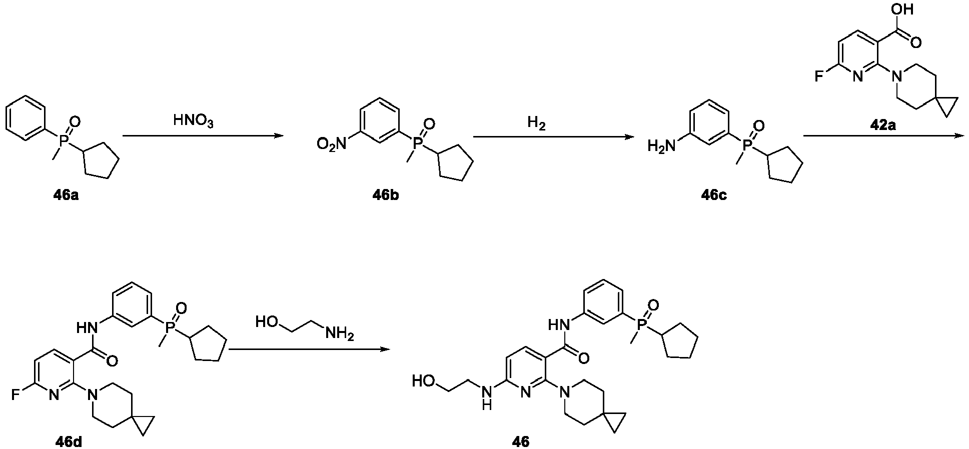

**46a** was synthesized according to (Chemical Communications (Cambridge, United Kingdom) 2021, 57(27). Compound **46a** underwent nitration reaction to obtain compound **46b**, compound **46b** underwent catalytic hydrogenation reaction to obtain compound **46c,** compound **46c** and compound **42a** underwent condensation reaction to obtain compound **46d**, and then underwent substitution reaction to obtain compound **46.**

### Example 46: Preparation of Compound 47

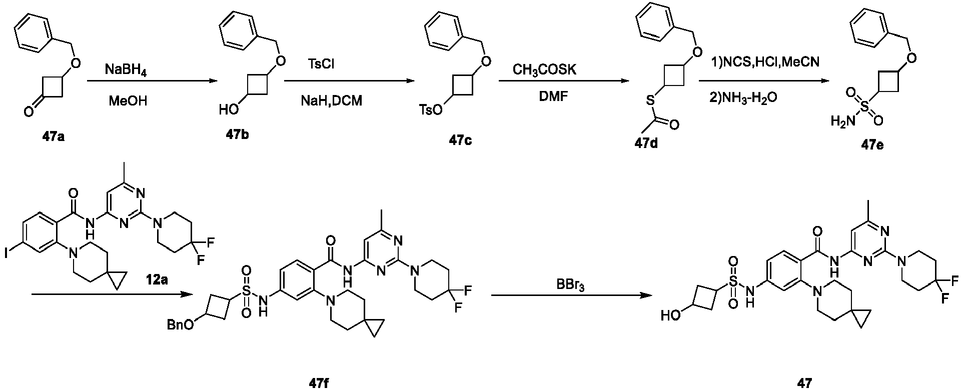

The 3-(benzyloxy) -1-cyclobutanone **47a** (2.0 g, 11.35 mmol) was dissolved in MeOH (20 mL). Then Sodium borohydride (472 mg, 12.43 mmol) was added at 0 °C and the mixture was stirred at RT for 1 h. Water was added to quench the reaction. The mixture was extracted with EA (3×40 mL). The collected organic phase was washed with sat.NaCl, dried with anhydrous sodium sulfate, filterated and concentrated to give product **47b** (2.0 g, yield 99%). The crude was used to the next step directly.

The compound **47b** (600 mg, 3.37 mmol) was dissolved in DCM (10 mL). Then 4-methylbenzenesulfonyl chloride (770 mg, 4.04 mmol) was added. 60% NaH (161 mg, 6.73 mmol) was added under ice bath. The mixture was stirred at 0 °C for 0.5 h. The mixture was quenched with sat.NH₄Cl and extracted with DCM (3×20 mL). The collected organic phase was washed with sat.NaCl, dried with anhydrous sodium sulfate, filterated and concentrated. The crude was purified by column chromatography (PE: EA = 10:1) to give yellow oil compound **47c** (780 mg, yield 70%).

The compound **47c** (255 mg, 0.77 mmol) and potassium thioacetate (96 mg, 0.84 mmol) were dissolved in DMF (2 mL). The mixture was stirred at 70 °C for overnight. The mixture was concentrated. The crude was purified by column chromatography (PE: EA = 5:1) to give yellow oil compound **47d** (156 mg, yield 86%). ¹H NMR (500 MHz, CDCl₃) δ 7.38-7.26 (m, 5H), 4.40 (s, 2H), 4.30-4.23 (m, 1H), 4.02-3.92 (m, 1H), 2.64-2.56 (m, 2H), 2.35-2.22 (m, 5H) ppm.

The chlorosuccinimide (264 mg, 1.98 mmol) was dissolved in MeCN (3 mL). Con.HCl (0.08 mL) was added. The mixture was stirred at RT for 10 min. Then **47d** (156 mg, 0.66 mmol) in 1 mL MeCN was added under ice bath. The mixture was stirred at RT for 0.5 h. Ammonia solution (1 mL) was added to the reaction. The mixture was concentrated. The crude was purified by column chromatography to give white solid product **47e** (142 mg, yield 89%). ¹H NMR (500 MHz, DMSO-*d*₆) δ 7.40-7.27 (m, 5H), 6.80 (s, 2H), 4.38 (s, 2H), 3.98-3.89 (m, 1H), 3.37-3.29 (m, 1H), 2.49-2.42 (m, 2H), 2.21-2.12 (m, 2H) ppm.

To a solution of **47e** (6 mg, 0.03 mmol) in DMF (1 mL) was added sarcosine (0.5 mg, 0.01 mmol), CuI (0.9 mg, 0.01 mmol) and tripotassium phosphate (13 mg, 0.06 mmol). The reaction was stirred at 50 °C for 5 min under a sealed tube. Then **2a** (15 mg, 0.03 mmol) was added and the mixture was stirred at 100 °C for overnight under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA = 5:1) to give a white solid product **47f** (10 mg, yield 55%). MS m/z 681.3 [M+H]⁺.

The compound **47f** (10 mg, 0.01 mmol) was dissolved in DCM (1 mL). Boron tribromide (5 mg, 0.02 mmol) was added at -78 °C. The mixture was stirred at this temperature for 10 min. The mixture was quenched with sat.NaHCO₃ and extracted with EA (5 mL×3). The collected organic phase was washed with sat.NaCl, dried with anhydrous sodium sulfate, filterated and concentrated. The crude was purified by Pre-TLC (PE: EA = 1:1) to give a white solid product **47** (4 mg, yield 46%). MS *m*/*z* 591.2 [M+H]⁺. ¹H NMR (500 MHz, DMSO-*d*₆) δ 13.39 (s, 1H), 10.23 (s, 1H), 8.03 (d, *J* = 8.6 Hz, 1H), 7.40 (s, 1H), 7.27 (d, *J* = 1.8 Hz, 1H), 7.11 (dd, *J* = 8.6, 1.8 Hz, 1H), 5.45 (d, *J* = 6.7 Hz, 1H), 3.99-3.86 (m, 5H), 3.57-3.48 (m, 1H), 3.01-2.92 (m, 4H), 2.42-2.36 (m, 2H), 2.31 (s, 3H), 2.18-2.08 (m, 2H), 2.04-1.93 (m, 4H), 1.74 (br, 4H), 0.39 (s, 4H) ppm.

### Example 47: Preparation of Compound 48

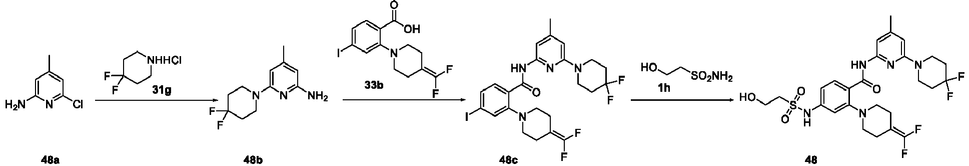

The compound **48a** (150 mg, 1.06 mmol), **31g** (250 mg, 1.59 mmol) and DIPEA (410 mg, 3.18 mmol) were dissolved in NMP (3 mL). The mixture was stirred at 180 °C for 12 h. After the reaction was finished, water was added and extracted with EA (30 mL×3). The collected organic phase was washed with sat.NaCl, dried with anhydrous sodium sulfate, filterated and concentrated. The crude was purified by column chromatography to give a white solid product **48b** (161 mg, yield 67%). MS m/z 228.5 [M+H]⁺.

The **33b** (42 mg, 0.11 mmol) was dissolved in 1,2-dichloroethane (2 mL). Thionyl chloride (20 mg, 0.17 mmol) was added. The mixture was stirred at RT for 0.5h. The above reaction solution was added dropwise to a mixed solution of 1, 2-dichloroethane (1 mL) of compound **48b** (25 mg, 0.11 mmol) and 4-dimethylaminopyridine (41 mg, 0.33 mmol). The mixture was stirred at 80 °C for 0.5h. The mixture was concentrated. The crude was purified by column chromatography to give white solid product **48c** (28 mg, yield 45%). MS m/z 589.6 [M+H]⁺.

The compound **1h** (9 mg, 0.08 mmol), sarcosine (2 mg, 0.02 mmol), CuI (4 mg, 0.02 mmol) and tripotassium phosphate (32 mg, 0.15 mmol) were dissolved in DMF (1 mL). The reaction was stirred at 50 °C for 5 min under a sealed tube. Then **48c** (28 mg, 0.05 mmol) was added and the mixture was stirred at 100 °C for 4 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA=1:2) to give white solid product **48** (8 mg, yield 29%). ¹H NMR (500 MHz, DMSO-*d₆)* δ 12.46 (s, 1H), 10.26 (br, 1H), 8.03 (d, *J* = 8.6 Hz, 1H), 7.48 (s, 1H), 7.16 (d, *J* = 2.0 Hz, 1H), 7.12 (dd, *J* = 8.6, 2.0 Hz, 1H), 6.57 (s, 1H), 4.97 (br, 1H), 3.75 (t, *J* = 6.4 Hz, 2H), 3.67-3.62 (m, 4H), 3.37-3.35 (m, 2H), 3.01-2.96 (m, 4H), 2.55-2.49 (m, 4H), 2.26 (s, 3H), 1.96-1.85 (m, 4H). MS m/z 586.2 [M+H]⁺.

### Example 48: Preparation of Compound 49

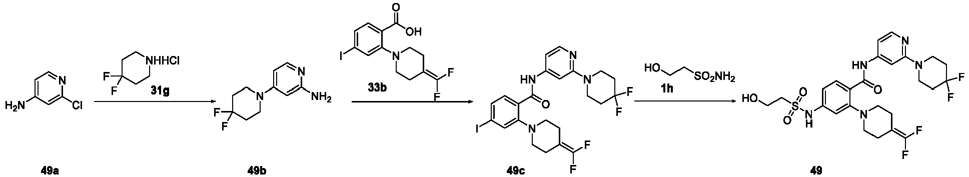

The compound **49a** (150 mg, 1.17 mmol), **31g** (276 mg, 1.76 mmol) and DIPEA (453 mg, 3.51 mmol) were dissolved in NMP (3 mL). The mixture was stirred at 180 °C for 12 h. After the reaction was finished, water was added and extracted with EA (30 mL × 3). The collected organic phase was washed with sat.NaCl, dried with anhydrous sodium sulfate, filterated and concentrated. The crude was purified by column chromatography to give a white solid product **49b** (138 mg, yield 55%). MS m/z 214.4 [M+H]⁺.

The **33b** (40 mg, 0.11 mmol) was dissolved in 1,2-dichloroethane (2 mL). Thionyl chloride (20 mg, 0.17 mmol) was added. The mixture was stirred at RT for 0.5h. The above reaction solution was added dropwise to a mixed solution of 1, 2-dichloroethane (1 mL) of compound **49b** (23 mg, 0.11 mmol) and 4-dimethylaminopyridine (41 mg, 0.33 mmol). The mixture was stirred at 80 °C for 0.5h. The mixture was concentrated. The crude was purified by column chromatography to give white solid product **49c** (25 mg, yield 41%). MS m/z 575.3 [M+H]⁺.

The compound **1h** (8 mg, 0.06 mmol), sarcosine (2 mg, 0.02 mmol), CuI (4mg, 0.02 mmol) and tripotassium phosphate (25 mg, 0.12 mmol) were dissolved in DMF (1 mL). The reaction was stirred at 50 °C for 5 min under a sealed tube. Then **49c** (25 mg, 0.04 mmol) was added and the mixture was stirred at 100 °C for 4 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA = 1:2) to give white solid product **49** (11 mg, yield 44%). ¹H NMR (500 MHz, DMSO-*d*₆) δ 11.05 (s, 1H), 10.14 (br, 1H), 8.05 (d, *J* = 5.5 Hz, 1H), 7.69 (d, *J* = 8.4 Hz, 1H), 7.44 (s, 1H), 7.03 (d, *J* = 2.0 Hz, 1H), 7.00 (dd, *J* = 8.4, 2.0 Hz, 1H), 6.93 (d, *J* = 5.5 Hz, 1H), 4.98 (br, 1H), 3.75 (t, *J* = 6.5 Hz, 2H), 3.68-3.62 (m, 4H), 3.34-3.30 (m, 2H), 2.99-2.92 (m, 4H), 2.33-2.23 (m, 4H), 2.03-1.93 (m, 4H). MS m/z 572.3 [M+H]⁺.

### Example 49: Preparation of Compound 50

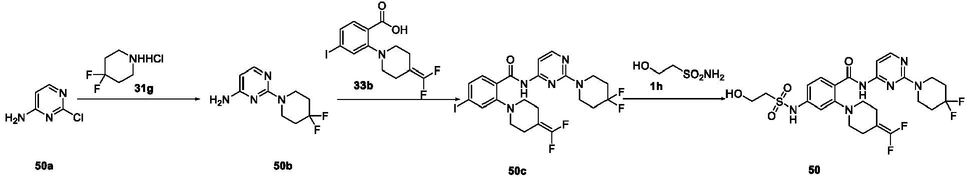

The compound **50a** (150 mg, 1.16 mmol), **31g** (237 mg, 1.15 mmol) and DIPEA (449 mg, 3.48 mmol) were dissolved in NMP (1.5 mL). The mixture was stirred at 180 °C for 12 h. After the reaction was finished, water was added and extracted with EA (30 mL×3). The collected organic phase was washed with sat.NaCl, dried with anhydrous sodium sulfate, filterated and concentrated. The crude was purified by column chromatography (PE: EA = 5:1) to give a white solid product **50b** (150 mg, yield 60%). MS m/z 215.3 [M+H]⁺.

The **33b** (40 mg, 0.11 mmol) was dissolved in 1, 2-dichloroethane (1 mL). Thionyl chloride (20 mg, 0.17 mmol) was added. The mixture was stirred at RT for 0.5 h. The above reaction solution was added dropwise to a mixed solution of 1, 2-dichloroethane (1 mL) of compound **50b** (23 mg, 0.11 mmol) and 4-dimethylaminopyridine (41 mg, 0.33 mmol). The mixture was stirred at 80 °C for 0.5h. The mixture was concentrated. The crude was purified by column chromatography (PE: EA = 10:1) to give white solid product **50c** (30 mg, yield 49%).

The compound **1h** (10 mg, 0.08 mmol), sarcosine (3 mg, 0.03 mmol), CuI (12 mg, 0.06 mmol) and tripotassium phosphate (32 mg, 0.15 mmol) were dissolved in DMF (1 mL) and the mixture was stirred at 100 °C for 18 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA= 1:1) to give white solid product **50** (13 mg, yield 44%). ¹H NMR (500 MHz, DMSO-*d*₆) δ 13.06 (s, 1H), 10.32 (s, 1H), 8.35 (d, *J* = 5.5 Hz, 1H), 8.03 (d, *J* = 8.6 Hz, 1H), 7.47 (d, *J* = 5.5 Hz, 1H), 7.18 (d, *J* = 1.8 Hz, 1H), 7.13 (dd, *J* = 8.6, 2.0 Hz, 1H), 4.97 (s, 1H), 3.83 (br, 4H), 3.76 (t, *J* = 6.4 Hz, 2H), 3.38-3.35 (m, 2H), 3.00 (t, *J* = 5.3 Hz, 4H), 2.54-2.49 (m, 4H), 1.99-1.86 (m, 4H). MS m/z 573.1 [M+H]⁺.

### Example 50: Preparation of Compound 51

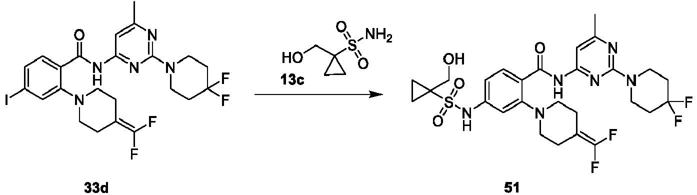

The compound **13c** (10 mg, 0.07 mmol), sarcosine (3 mg, 0.03 mmol), CuI (12 mg, 0.06 mmol) and tripotassium phosphate (32 mg, 0.15 mmol) were dissolved in DMF (1 mL). The reaction was stirred at 50 °C for 5 min under a sealed tube. Then **33d** (30 mg, 0.05 mmol) was added and the mixture was stirred at 100 °C for 18 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA = 1:1) to give white solid product **51** (10 mg, yield 32%). ¹H NMR (500 MHz, DMSO-*d*₆) δ 12.98 (s, 1H), 10.24 (s, 1H), 8.01 (d, *J* = 8.6 Hz, 1H), 7.39 (s, 1H), 7.24 (d, *J* = 2.0 Hz, 1H), 7.14 (dd, *J* = 8.6, 2.0 Hz, 1H), 5.00 (s, 1H), 3.89-3.79 (m, 4H), 3.74 (s, 2H), 2.97 (t, *J* = 5.3 Hz, 4H), 2.54-2.49 (m, 4H), 2.32 (s, 3H), 1.97-1.86 (m, 4H), 1.18-1.11 (m, 2H), 1.02-0.95 (m, 2H). MS m/z 613.2 [M+H]⁺.

### Example 51: Preparation of Compound 52

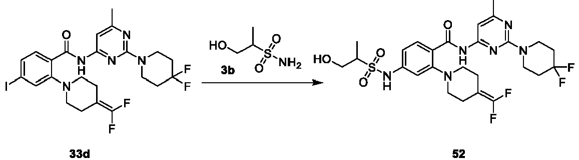

The compound **3b** (7 mg, 0.05 mmol), sarcosine (1.5 mg, 0.01 mmol), CuI (4 mg, 0.02 mmol) and tripotassium phosphate (19 mg, 0.09 mmol) were dissolved in DMF (1 mL). The reaction was stirred at 50 °C for 5 min under a sealed tube. Then **33d** (20 mg, 0.03 mmol) was added and the mixture was stirred at 100 °C for 18 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA = 1:1) to give white solid product **52** (7 mg, yield 36%). ¹H NMR (500 MHz, DMSO-*d*₆)δ 12.91 (s, 1H), 10.32 (s, 1H), 8.02 (d, *J* = 8.6 Hz, 1H), 7.39 (s, 1H), 7.21 (s, 1H), 7.15 (dd, *J* = 8.6, 2.0 Hz, 1H), 5.05 (s, 1H), 3.83 (br, 5H), 3.52-3.45 (m, 1H), 3.31-3.25 (m, 1H), 2.98 (t, *J* = 5.3 Hz, 4H), 2.54-2.49 (m, 4H), 2.31 (s, 3H), 1.97-1.86 (m, 4H), 1.29 (d, *J* = 6.9 Hz, 3H). MS m/z 601.1 [M+H]⁺.

### Example 52: Preparation of Compound 53

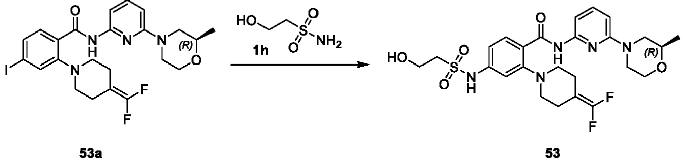

The compound **1h** (7 mg, 0.05 mmol), sarcosine (2 mg, 0.02 mmol), CuI (7 mg, 0.04 mmol) and tripotassium phosphate (25 mg, 0.12 mmol) were dissolved in DMF (1 mL). The reaction was stirred at 50 °C for 5 min under a sealed tube. Then **53a** (synthesised according to **40a**, 24 mg, 0.04 mmol) was added and the mixture was stirred at 100 °C for 18 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA = 1:1) to give white solid product **53** (14 mg, yield 58%). ¹H NMR (500 MHz, DMSO-*d*6) δ 12.66 (s, 1H), 10.27 (s, 1H), 8.06 (d, *J* = 8.6 Hz, 1H), 7.62-7.53 (m, 2H), 7.18 (d, *J* = 2.0 Hz, 1H), 7.12 (dd, *J* = 8.6, 2.1 Hz, 1H), 6.60 (dd, *J* = 6.5, 2.4 Hz, 1H), 4.96 (s, 1H), 4.10 (d, *J* = 12.5 Hz, 1H), 3.89-3.82 (m, 2H), 3.76 (t, *J* = 6.4 Hz, 2H), 3.58-3.48 (m, 2H), 3.39-3.35 (m, 2H), 2.99 (t, *J* = 5.4 Hz, 4H), 2.83-2.75 (m, 1H), 2.61-2.50 (m, 4H), 2.42-2.35 (m, 1H), 1.09 (d, *J* = 6.2 Hz, 3H). MS m/z 552.6 [M+H]⁺.

### Example 53: Preparation of Compound 54

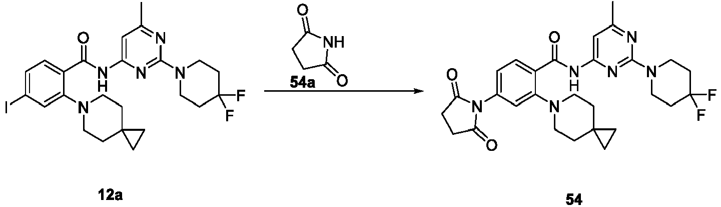

The compound **54a** (6 mg, 0.06 mmol), sarcosine (4.5 mg, 0.05 mmol), CuI (19 mg, 0.1 mmol) and tripotassium phosphate (32 mg, 0.15 mmol) were dissolved in DMF (1 mL). The reaction was stirred at 50 °C for 5 min under a sealed tube. Then **12a** (30 mg, 0.05 mmol) was added and the mixture was stirred at 100 °C for 18 h under N₂. The reaction was quenched with sat.NH₄Cl and extracted with EA (15 mL × 3). The collected organic phase was washed with sat.NaCl, dried with anhydrous sodium sulfate, filterated and concentrated. The crude was purified by Pre-TLC (PE: EA = 2:1) to give white solid product **54** (9 mg, yield 31%). ¹H NMR (500 MHz, DMSO-*d₆*) δ 13.59 (s, 1H), 8.21 (d, *J* = 8.4 Hz, 1H), 7.49 (d, *J* = 1.6 Hz, 1H), 7.42 (s, 1H), 7.32-7.28 (m, 1H), 3.96-3.89 (m, 4H), 3.00 (t, *J* = 5.0 Hz, 4H), 2.81 (br, 4H), 2.33 (s, 3H), 2.03-1.95 (m, 4H), 1.72 (br, 4H), 0.38 (s, 4H). MS m/z 539.2 [M+H]⁺.

### Example 54: Preparation of Compound 55

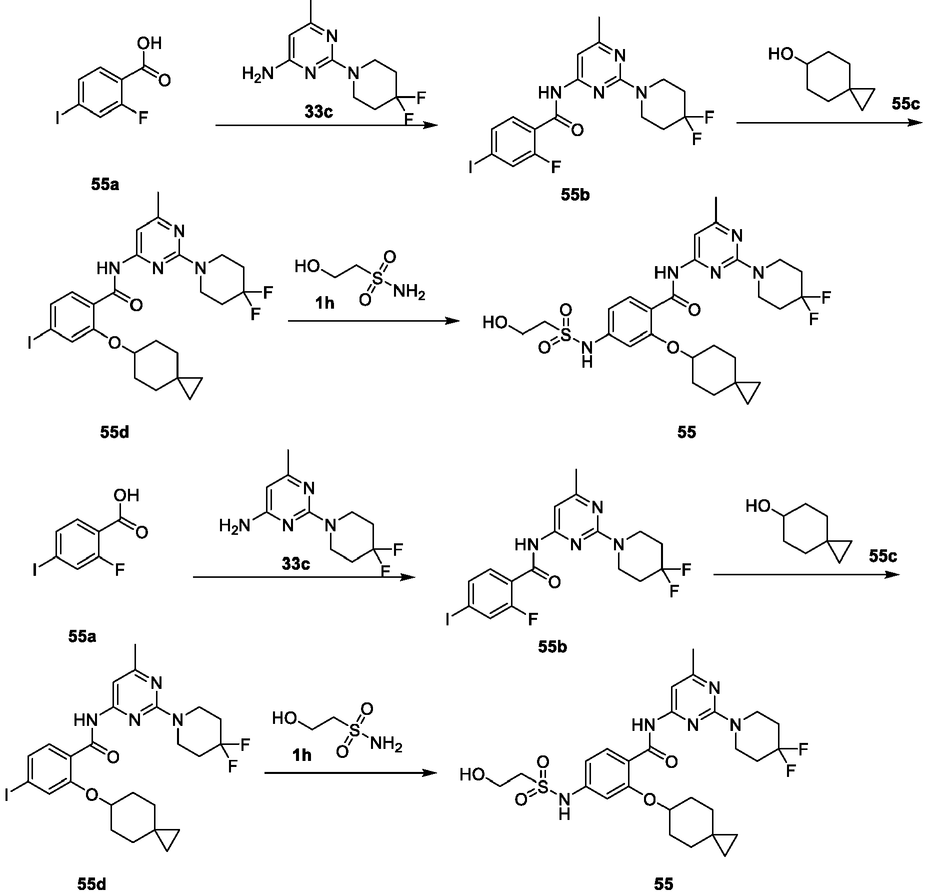

The **55a** (100 mg, 0.38 mmol), **33c** (86 mg, 0.38 mmol), DIPEA (148 mg, 1.14 mmol) and 1-propyl phosphate anhydride (725 mg, 50% DMF solution) were dissolved in 1, 2-dichloroethane (2 mL). The mixture was stirred at 85 °C for 3 h. The mixture was concentrated. The crude was purified by column chromatography (PE: EA=10:1) to give white solid product **55b** (100 mg, yield 56%). MS m/z 477.3 [M+H]⁺.

The compound **55b** (100 mg, 0.21 mmol) and **55c** (27 mg, 0.21 mmol) were dissolved in DCM (10 mL). Then 60% NaH (161 mg, 6.73 mmol) was added under ice bath. The mixture was stirred at 80 °C for 5 h. After the reaction was finished, the mixture was quenched with sat.NH₄Cl and extracted with EA (3×30 mL). The collected organic phase was washed with sat.NaCl, dried with anhydrous sodium sulfate, filterated and concentrated. The crude was purified by column chromatography (PE: EA = 10:1) to give white solid product **55d** (50 mg, yield 41%). MS m/z 583.5 [M+H]⁺.

The compound **1h** (10 mg, 0.08 mmol), sarcosine (3 mg, 0.04 mmol), CuI (13 mg, 0.07 mmol) and tripotassium phosphate (45 mg, 0.21 mmol) were dissolved in DMF (1 mL). The reaction was stirred at 50 °C for 5 min under a sealed tube. Then **53a** (24 mg, 0.04 mmol) was added and the mixture was stirred at 100 °C for 18 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA = 1:1) to give white solid product **55** (14 mg, yield 35%). ¹H NMR (500 MHz, DMSO-*d*₆) δ 10.46 (s, 1H), 10.26 (s, 1H), 7.98 (d, *J* = 8.7 Hz, 1H), 7.39 (s, 1H), 7.06 (d, *J* = 1.6 Hz, 1H), 6.95 (dd, *J* = 8.7, 1.8 Hz, 1H), 4.97 (br, 1H), 4.74-4.65 (m, 1H), 3.93-3.79 (m, 4H), 3.75 (t, *J* = 6.4 Hz, 2H), 3.37 (t, *J* = 6.4 Hz, 2H), 2.31 (s, 3H), 2.10-2.03 (m, 2H), 2.02-1.91 (m, 4H), 1.90-1.80 (m, 2H), 1.53-1.38 (m, 4H), 0.38-0.32 (m, 2H), 0.28-0.22 (m, 2H). MS *m*/*z* 580.2 [M+H]⁺.

### Example 55: Preparation of Compound 56

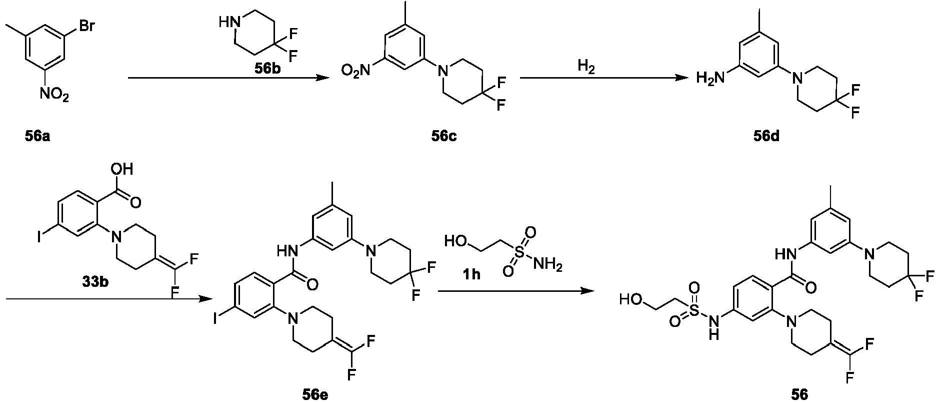

The compound **56a** (1 g, 4.22 mmol), **56b** (1.02 g, 8.44 mmol), tBuONa (1.22 g, 12.66 mmol), tri(dibenzylidene acetone) dipalladium (386 mg, 0.42 mmol) and 2-Dicyclo hexylphosphate-2,4,6-triisopropylbiphenyl (402 mg, 0.84 mmol) were dissolved in toluene (10 mL) under N₂. The mixture was stirred at 100 °C for 2 h. The reaction was filterated and concentrated. The crude was purified by column chromatography (PE: EA = 5:1) to give product **56c** (800 mg, yield 68%).

The compound **56c** (370 mg, 1.44 mmol) was dissolved in MeOH (15 mL). 10% Pd/C (60 mg) was added and the reaction was stirred at RT for 1 h under H₂. The mixture was filterated and concentrated. The crude was purified by column chromatography (PE: EA = 4:1) to give a colorless oil compound **56d** (300 mg, yield 59%). MS m/z 227.3 [M+H]⁺.

The compound **56d** (66 mg, 0.29 mmol) and **33b** (110 mg, 29 mmol) were dissolved in DCM (3 mL). Then diisopropylethylamine (113 mg, 0.87 mmol) and N,N,N',N'-Tetramethyl-O- (7-azabenzotriazol-1-yl) hexafluorophosphourea (138 mg, 0.44 mmol) were added. The reaction was stirred at RT for 1 h under N₂. The mixture was concentrated. The crude was purified by column chromatography to give the product product **56e** (140 mg, yield 82%). MS *m*/*z* 588.5 [M+H]⁺.

The compound **1h** (13 mg, 0.10 mmol), sarcosine (4 mg, 0.04 mmol), CuI (13 mg, 0.08 mmol) and tripotassium phosphate (51 mg, 0.24 mmol) were dissolved in DMF (1 mL). The reaction was stirred at 50 °C for 5 min under a sealed tube. Then **56e** (501 mg, 0.08 mmol) was added and the mixture was stirred at 100 °C for 18 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA = 2:1) to give white solid product **56** (24 mg, yield 48%). ¹H NMR (500 MHz, DMSO-*d*₆) δ 10.82 (s, 1H), 10.09 (s, 1H), 7.69 (d, *J* = 8.4 Hz, 1H), 7.23 (s, 1H), 7.05 (s, 1H), 7.02 (d, *J* = 2.0 Hz, 1H), 6.99 (dd, *J* = 8.4, 2.0 Hz, 1H), 6.58 (s, 1H), 4.98 (s, 1H), 3.75 (t, *J* = 6.5 Hz, 2H), 3.34-3.27 (m, 6H), 2.96 (t, *J* = 5.5 Hz, 4H), 2.30 (br, 4H), 2.25 (s, 3H), 2.11-1.94 (m, 4H). MS *m*/*z* 585.2 [M+H]⁺.

### Example 56: Preparation of Compound 57

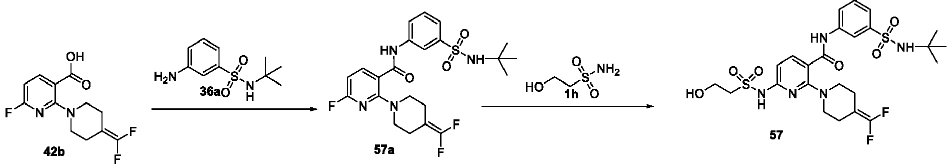

To a solution of compound **42b** (160 mg, 0.59 mmol) in MeCN (3 mL) was added 36a (148 mg, 0.65 mmol), methylimidazole (120 mg, 1.46 mmol) and N,N,N',N'-Tetramethyl-O- (7-azabenzotriazol-1-yl) hexafluorophosphourea (198 mg, 0.71 mmol). The reaction was stirred at RT for 3 h under N₂. After the reaction was finished, water was added and extracted with DCM (5 mL × 3). The collected organic phase was washed with sat.NaCl, dried with anhydrous sodium sulfate, filterated and concentrated. The crude was purified by column chromatography (PE: EA=5:1) to give white solid product **57a** (170 mg, yield 60%). MS m/z 483.0 [M+H]⁺.

The compound **57a** (55 mg, 0.11 mmol) was dissolved in DMSO (1 mL). The compound **1h** (29 mg, 0.23 mmol) and Cs₂CO₃ (74 mg, 0.23 mmol) were added. The reaction was stirred at 100 °C in a sealed tube for overnight under N₂. Water was added to quench the reaction. The reaction was extracted with EA (5 mL × 3). The collected organic phase was washed with sat.NaCl, dried with anhydrous sodium sulfate, filterated and concentrated. The crude was purified by Pre-TLC (DCM: MeOH = 10:1) to give a white solid product **57** (20 mg, yield 30%). MS m/z 588.1 [M+H]⁺. ¹H NMR (500 MHz, CDCl₃) δ 10.76 (s, 1H), 8.31 (d, *J* = 8.3 Hz, 1H), 8.24 (s, 1H), 7.82 (d, *J* = 7.5 Hz, 1H), 7.66 (d, *J* = 8.0 Hz, 1H), 7.49 (t, *J* = 8.0 Hz, 1H), 6.84 (d, *J* = 8.0 Hz, 1H), 4.76 (s, 1H), 4.23 - 4.09 (m, 2H), 3.75 - 3.63 (m, 2H), 3.30 - 3.15 (m, 4H), 2.48 - 2.25 (m, 4H), 1.26 (s, 9H).

### Example 57: Preparation of Compound 58

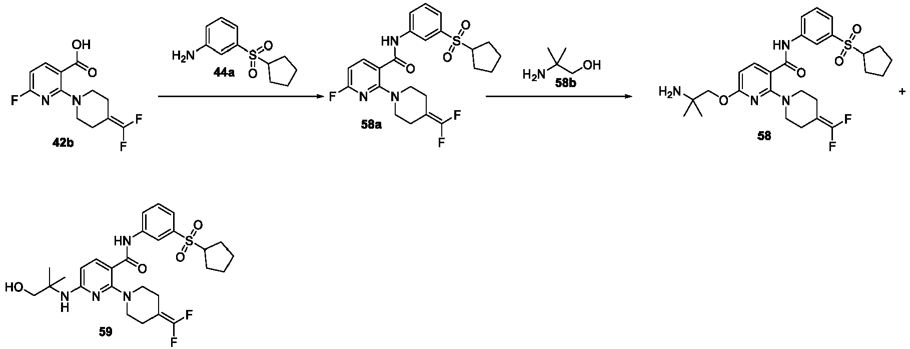

To a solution of compound **42b** (70 mg, 0.26 mmol) in MeCN (2 mL) was added **44a** (64 mg, 0.28 mmol), methylimidazole (53 mg, 0.64 mmol) and N,N,N',N'-Tetramethyl-O-(7-azabenzotriazol-1-yl) hexafluorophosphourea (86 mg, 0.31 mmol). The reaction was stirred at RT for 3 h under N₂. After the reaction was finished, water was added and extracted with DCM (5 mL×3). The collected organic phase was washed with sat.NaCl, dried with anhydrous sodium sulfate, filterated and concentrated. The crude was purified by column chromatography (PE: EA = 5:1) to give white solid product **58a** (75 mg, yield 61%). MS m/z 480.0 [M+H]⁺.

The compound **58a** (75 mg, 0.16 mmol) was dissolved in DMSO (1 mL). The compound **58b** (84 mg, 0.94 mmol) and K₂CO₃ (200 mg, 0.94 mmol) were added. The reaction was stirred at 120 °C in a sealed tube for overnight under N₂. Water was added to quench the reaction. The reaction was extracted with EA (5 mL × 3). The collected organic phase was washed with sat.NaCl, dried with anhydrous sodium sulfate, filterated and concentrated. The crude was purified by Pre-TLC (DCM: MeOH = 10:1) to give white solid product **58** (8 mg, yield 9%) and **59** (2 mg, yield 2%). Compound **58:** MS *m*/*z* 549.3 [M+H]⁺. ¹H NMR (500 MHz, CDCl₃) δ 11.49 (s, 1H), 8.32 (d, *J* = 8.6 Hz, 1H), 8.27-8.25 (m, 1H), 7.90 (d, *J* = 7.8 Hz, 1H), 7.64 (d, *J* = 7.8 Hz, 1H), 7.54 (t, *J* = 7.8 Hz, 1H), 6.70 (d, *J* = 8.6 Hz, 1H), 4.17 (s, 2H), 3.58-3.50 (m, 1H), 3.24-3.19 (m, 4H), 2.44-2.37 (m, 4H), 2.14 -2.04 (m, 2H), 1.95-1.86 (m, 2H), 1.81-1.77 (m, 2H), 1.64-1.61 (m, 2H), 1.30 (s, 6H).

Compound **59:** MS *m*/*z* 549.3 [M+H]⁺. ¹H NMR (500 MHz, CDCl₃) δ 11.86 (s, 1H), 8.30 - 8.24 (m, 1H), 8.21 (d, *J* = 8.6 Hz, 1H), 7.90 - 7.85 (m, 1H), 7.66 - 7.59 (m, 1H), 7.53 (t, *J* = 7.9 Hz, 1H), 6.30 (d, *J* = 8.7 Hz, 1H), 4.81 (s, 1H), 4.44 (s, 1H), 3.74 (s, 2H), 3.59 - 3.48 (m, 1H), 3.17 (t, *J* = 5.3 Hz, 4H), 2.48 - 2.35 (m, 4H), 2.15 - 2.02 (m, 2H), 1.96 - 1.86 (m, 2H), 1.84 - 1.73 (m, 2H), 1.68 - 1.59 (m, 2H), 1.44 (s, 6H).

### Example 58: Preparation of Compound 60

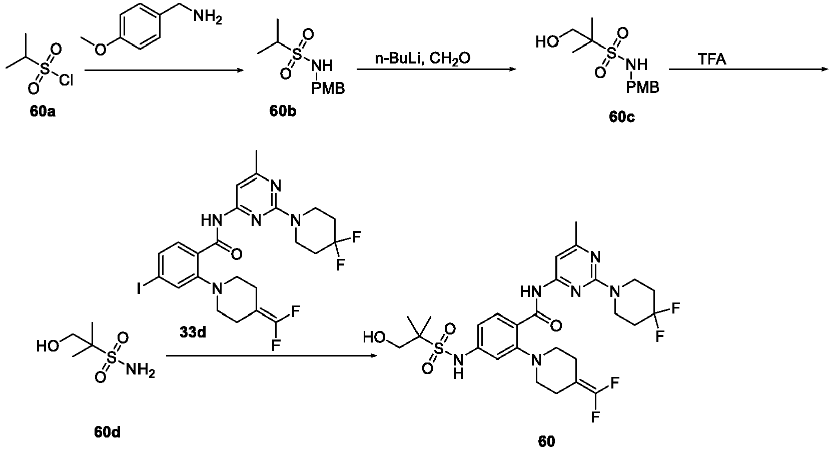

The 4-Methoxybenzylamine (964 mg, 7.04 mmol) was dissolved in DCM (15 mL). Then DIPEA (2.7 g, 21.12 mmol) and isopropyl sulfonyl chloride (1.0 g, 7.04 mmol) were added. The mixture was stirred at RT for 1 h. The mixture was concentrate and the crude was purified by column chromatography to give white solid product **60b** (655 mg, yield 38% PMB: 4-Methoxybenzyl). MS *m*/*z* 244.5 [M+H]⁺.

The compound **60b** (300 mg, 1.23 mmol) was dissolved in THF (6 mL). The mixture was cooled to -78 °C and nBuLi (2.5 M, 1.2 mL) was added. The reaction was stirred at this temperature for 0.5 h. Then paraformaldehyde (74 mg, 2.46 mmol) was added. The mixture was warmed to RT and stirred for 2 h. The reaction was quenched with sat.NH₄Cl and extracted with EA (20 mL×3). The collected organic phase was washed with sat.NaCl, dried with anhydrous sodium sulfate, filterated and concentrated. The crude was purified by column chromatography to give yellow oil compound **60c** (126 mg, 37%). MS *m*/*z* 274.6 [M+H]⁺.

The compound **60c** (126 mg, 0.46 mmol) was dissolved in DCM (4 mL). TFA (2 mL) was added. The reaction was stirred at RT for overnight. The mixture was concentrated. The crude was purified by column chromatography to give white solid product **60d** (36 mg, yield 51%). MS *m*/*z* 154.6 [M+H]⁺.

The compound **60d** (21 mg, 0.14 mmol), sarcosine (3 mg, 0.03 mmol), CuI (6 mg, 0.03 mmol) and tripotassium phosphate (44 mg, 0.21 mmol) were dissolved in DMF (1 mL). The reaction was stirred at 50 °C for 5 min under a sealed tube. Then **33d** (40 mg, 0.07 mmol) was added and the mixture was stirred at 100 °C for 4 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA = 1:2) to give white solid product **60** (21 mg, yield 50%). ¹H NMR (500 MHz, DMSO-*d*₆) δ 12.97 (s, 1H), 10.09 (br, 1H), 7.99 (d, *J* = 8.6 Hz, 1H), 7.38 (s, 1H), 7.33 (d, *J* = 2.0 Hz, 1H), 7.19 (dd, *J* = 8.6, 2.0 Hz, 1H), 5.14 (br, 1H), 3.86-3.79 (m, 4H), 3.55 (s, 2H), 3.02-2.92 (m, 4H), 2.55-2.49 (m, 4H), 2.31 (s, 3H), 1.97-1.85 (m, 4H), 1.24 (s, 6H). MS m/z 615.2 [M+H]⁺.

### Example 59: Preparation of Compound 61 and 62

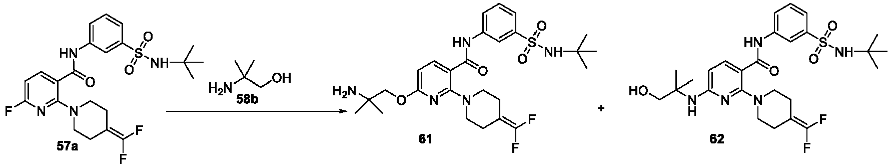

The compound **57a** (70 mg, 0.15 mmol) was dissolved in DMSO (1 mL). The compound **58b** (78 mg, 0.88 mmol) and tripotassium phosphate (185 mg, 0.87 mmol) were added. The reaction was stirred at 120 °C in a sealed tube for overnight under N₂. Water was added to quench the reaction. The reaction was extracted with EA (5 mL × 3). The collected organic phase was washed with sat.NaCl, dried with anhydrous sodium sulfate, filterated and concentrated. The crude was purified by Pre-TLC (DCM: MeOH = 10:1) to give white solid products **61** (6 mg, yield 8%) and **62** (4 mg, yield 5%). Compound **61:** MS m/z 552.3 [M+H]⁺. ¹H NMR (500 MHz, CDCl₃) δ 11.35 (s, 1H), 8.28 (d, *J* = 6.5 Hz, 1H), 8.22 (s, 1H), 7.83 (d, *J* = 8.0 Hz, 1H), 7.62 (d, *J* = 8.0 Hz, 1H), 7.49 - 7.41 (m, 1H), 6.70 (d, *J* = 8.5 Hz, 1H), 4.75 (br, 3H), 4.19 (s, 2H), 3.25 - 3.11 (m, 4H), 2.45 - 2.31 (m, 4H), 1.32 (s, 6H), 1.27 (s, 9H).

Compound **62:** MS m/z 552.3 [M+H]⁺. ¹H NMR (500 MHz, CDCl₃) δ 11.79 (s, 1H), 8.23 (t, *J* = 2.0 Hz, 1H), 8.20 (d, *J* = 8.6 Hz, 1H), 7.88 - 7.75 (m, 1H), 7.67 - 7.55 (m, 1H), 7.46 (t, *J* = 8.0 Hz, 1H), 6.30 (d, *J* = 8.6 Hz, 1H), 4.80 (s, 1H), 4.51 (br, 2H), 3.74 (s, 2H), 3.16 (t, *J* = 5.7 Hz, 4H), 2.45 - 2.41 (m, 4H), 1.44 (s, 6H), 1.26 (s, 9H).

### Example 60: Preparation of Compound 63

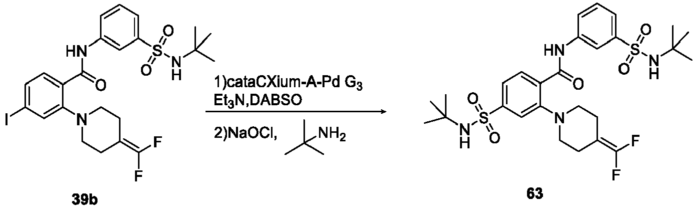

The compound **39b** (42 mg, 0.07 mmol), Methanesulfonic acid[n-Butyldi (1-adamantyl)phosphine](2-amino-1,1'-biphenyl-2-yl)palladium(II) (cataCXium-A- Pd G3, 10 mg, 0.01 mmol), triethylamine (1 mg, 0.21 mmol) and 1,4-Diazobicyclic [2.2.2] Octane-1,4-dionium-1,4-disulfonic acid (DABSO, 10 mg, 0.04 mmol) were dissolved in isopropanol (*i*-PrOH, 1 mL). The reaction was stirred at 85 °C for 3 h in a sealed tube under N₂. Then the reaction was cooled to RT. Tert-Butylamine (10 mg, 0.14 mmol) and sodium hypochlorite (10 mg, 0.14 mmol) were added. The reaction was stirred at RT for overnight. Water was adedd and extracted with EA (5 mL×3). The collected organic phase was washed with sat.NaCl, dried with anhydrous sodium sulfate, filterated and concentrated. The crude was purified by Pre-TLC (PE: EA = 1:2) to give white solid product **63** (2 mg, yield 5%). ¹H NMR (500 MHz, DMSO-*d*₆) δ 10.88 (s, 1H), 8.30 (s, 1H), 7.96-7.92 (m, 1H), 7.74 (d, *J* = 8.2 Hz, 1H), 7.68 (s, 1H), 7.62 (s, 1H), 7.58-7.53 (m, 4H), 3.06-3.01 (m, 4H), 2.24-2.19 (m, 4H), 1.13 (s, 9H), 1.09 (s, 9H). MS *m*/*z* 599.2 [M+H]⁺.

### Example 61: Preparation of Compound 64

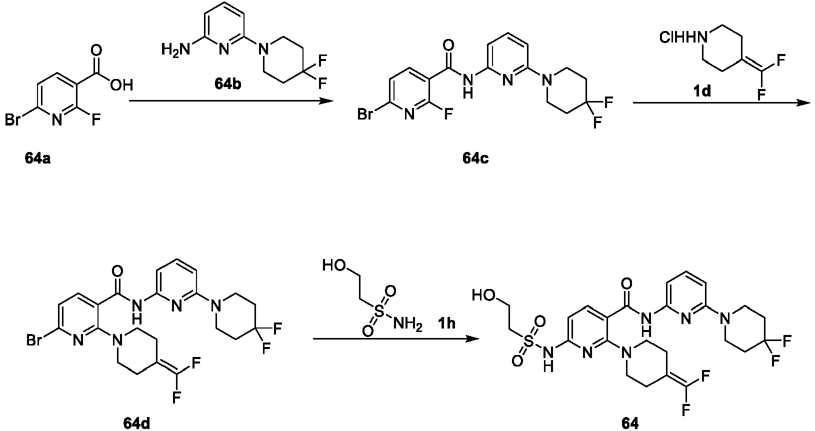

The **64a** (100 mg, 0.45 mmol), **64b** (96 mg, 0.45 mmol), DIPEA (175 mg, 1.35 mmol) and 1-propyl phosphate anhydride (858 mg, 50% DMF solution) were dissolved in 1,2-dichloroethane (2 mL). The mixture was stirred at 85 °C for 3 h. The mixture was concentrated. The crude was purified by column chromatography (PE: EA = 10:1) to give white solid product **64c** (130 mg, yield 69%). MS m/z 415.3, 417.3 [M+H]⁺.

The **64c** (130 mg, 0.31 mmol), **1d** (69 mg, 0.41 mmol) and K₂CO₃ (128 mg, 0.93 mmol) were dissolved in MeCN (3 mL). The mixture was stirred at RT for 12 h. The mixture was filterated and concentrated. The crude was purified by column chromatography (PE: EA = 10:1) to give white solid product **64d** (100 mg, yield 60%). MS m/z 528.0, 530.0 [M+H]⁺.

The mixture compound **1h** (9 mg, 0.07 mmol), (1R, 2R)-N, N-dimethyl-1,2-cyclohexanediamine (4 mg, 0.03 mmol), CuI (11 mg, 0.06 mmol), tripotassium phosphate (38 mg, 0.18 mmol) and **64d** (30 mg, 0.06 mmol) were dissolved in DMF (1 mL). The reaction was stirred at 100 °C for 18 h in a sealed tube under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA = 2:1) to give white solid product **64** (20 mg, yield 61%). ¹H NMR (500 MHz, DMSO-*d*₆) δ 11.22 (s, 1H), 10.91 (s, 1H), 8.10 (d, *J* = 8.1 Hz, 1H), 7.61 (t, *J* = 8.0 Hz, 1H), 7.52 (br, 1H), 6.71 (d, *J* = 8.3 Hz, 1H), 6.62 (d, *J* = 8.3 Hz, 1H), 4.96 (br, 1H), 3.80 (t, *J* = 6.2 Hz, 2H), 3.73 (t, *J* = 6.2 Hz, 2H), 3.67 (br, 4H), 3.20 (t, *J* = 5.5 Hz, 4H), 2.43 (br, 4H), 1.97-1.86 (m, 4H). MS m/z 573.1 [M+H]⁺.

### Example 62: Preparation of Compound 65

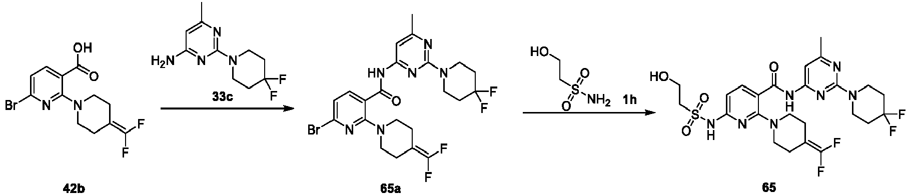

The **42b** (150 mg, 0.45 mmol), **33c** (102 mg, 0.45 mmol), DIPEA (175 mg, 1.35 mmol) and 1-propyl phosphate anhydride (858 mg, 50% DMF solution) were dissolved in 1, 2-dichloroethane (2 mL). The mixture was stirred at 85 °C for 3 h. The mixture was concentrated. The crude was purified by column chromatography (PE: EA = 10:1) to give white solid product **65a** (60 mg, yield 24%). MS m/z 544.3 & 546.3

The mixture compound **1h** (7 mg, 0.06 mmol), (1R, 2R)-N, N-dimethyl-1, 2-cyclohexanediamine (4 mg, 0.03 mmol), CuI (11 mg, 0.06 mmol), tripotassium phosphate (38 mg, 0.18 mmol) and **65a** (25 mg, 0.05 mmol) were dissolved in DMF (1 mL). The reaction was stirred at 100 °C for 18 h in a sealed tube under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA = 2:1) to give white solid product **65** (15 mg, yield 55%). ¹H NMR (500 MHz, DMSO-*d*₆) δ 11.37 (s, 1H), 10.92 (s, 1H), 8.03 (d, *J* = 8.3 Hz, 1H), 7.32 (s, 1H), 6.59 (d, *J* = 8.3 Hz, 1H), 4.96 (br, 1H), 3.84 (br, 4H), 3.80 (t, *J* = 6.3 Hz, 2H), 3.73 (t, *J* = 6.2 Hz, 2H), 3.27-3.16 (m, 4H), 2.39 (br, 4H), 2.31 (s, 3H), 1.99-1.86 (m, 4H). MS m/z 588.1 [M+H]⁺.

### Example 63: Preparation of Compound 66 and 67

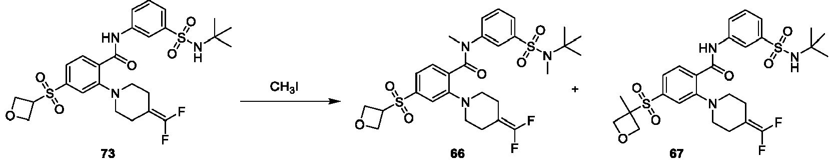

The compound **73** (14 mg, 0.02 mmol) was dissolved in THF (2 mL). The mixtuture was cooled to -78 °C and a solution of lithium bis (trimethylsilylamine) in tetrahydrofuran (1.0 M, 0.08 mL) was added. The reaction was kept in 0.5 h. Then methyl iodide (14 mg, 0.10 mmol) was added. The mixture was stirred at RT for 2 h under N₂. The reaction was quenched with sat.NH₄Cl and extracted with EA (10 mL×3). The collected organic phase was washed with sat.NaCl, dried with anhydrous sodium sulfate, filterated and concentrated. The crude was purified by Pre-TLC to give white solid products **66** (1.1 mg, yield 5%) and **67** (0.6 mg, yield 5%). Compound **66:** MS *m*/*z* 612.1 [M+H]⁺. Compound **67:** ¹H NMR (500 MHz, DMSO-*d*₆) δ 10.89 (s, 1H), 8.30 (s, 1H), 7.94-7.90 (m, 1H), 7.81 (d, *J* = 8.0 Hz, 1H), 7.65 (s, 1H), 7.62 (dd, *J* = 7.9, 1.5 Hz, 1H), 7.58-7.56 (m, 2H), 7.48 (d, *J* = 1.5 Hz, 1H), 5.00 (d, *J* = 7.2 Hz, 2H), 4.50 (d, *J* = 7.2 Hz, 2H), 3.11-3.07 (m, 4H), 2.20-2.16 (m, 4H), 1.58 (s, 3H), 1.09 (s, 9H). MS m/z 598.0 [M+H]⁺.

### Example 64: Preparation of Compound 68

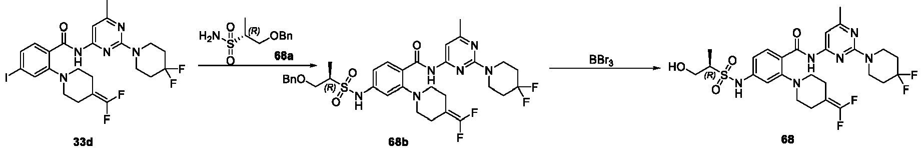

The compound **68a** (47 mg, 0.20 mmol, Organic Letters.2014, 16, 6248-6251), sarcosine (8 mg, 0.09 mmol), cuprous iodide (32 mg, 0.17 mmol) and tripotassium phosphate (108 mg, 0.51 mmol) were dissolved in DMF (1 mL). The reaction was stirred at 50 °C for 5 min under a sealed tube. Then **33d** (100 mg, 0.17 mmol) was added and the mixture was stirred at 100 °C for 18 h under N₂. After the reaction was complete, the reaction mixture was quenched with saturated NH₄Cl and extracted with EA (15 mL×3). The collected organic phase was washed with saturated NaCl, dried with anhydrous sodium sulfate, filterated and concentrated. The crude product was purified by Prearative-TLC (petroleum ether : EA = 2:1) to give white solid compound **68b** (100 mg, yield 85%). MS m/z 691.3 [M+H]⁺.

The compound **68b** (100 mg, 0.14 mmol) was dissolved in DCM (5 mL). Boron tribromide (72 mg, 0.28 mmol) was added at -78 °C. The mixture was stirred at this temperature for 0.5 h. After the reaction was complete, the reaction mixture was concentrated under reduced pressure. The crude product was purified by column chromatography (petroleum ether : EA= 1:1) to give a white solid compound **68** (60 mg, yield 69%). ¹H NMR (500 MHz, DMSO-*d*₆) δ 12.90 (s, 1H), 10.29 (s, 1H), 8.02 (d, *J* = 8.6 Hz, 1H), 7.39 (s, 1H), 7.20 (s, 1H), 7.15 (d, *J* = 8.6 Hz, 1H), 5.03 (br, 1H), 3.83 (br, 5H), 3.54-3.45 (m, 1H), 3.31-3.25 (m, 1H), 2.98 (t, *J* = 5.5 Hz, 4H), 2.54-2.49 (m, 4H), 2.31 (s, 3H), 1.97-1.86 (m, 4H), 1.29 (d, *J* = 6.9 Hz, 3H). MS m/z 601.2 [M+H]⁺.

### Example 65: Preparation of Compound 69

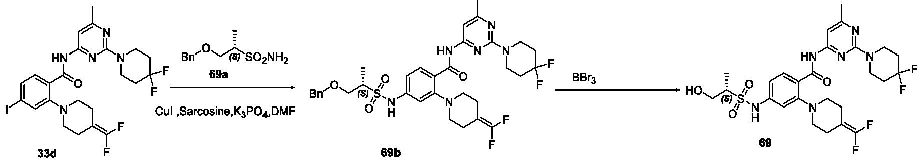

The compound **69a** (75 mg, 0.33 mmol, Organic Letters.2014, 16, 6248-6251), sarcosine (10 mg, 0.11 mmol), cuprous iodide (21 mg, 0.11 mmol) and tripotassium phosphate (140 mg, 0.66 mmol) were dissolved in DMF (3 mL). The reaction was stirred at 50 °C for 5 min under a sealed tube. Then **33d** (130 mg, 0.22 mmol) was added and the mixture was stirred at 100 °C for 18 h under N₂. The reaction was quenched with saturated NH₄Cl and extracted with EA (30 mL×3). The collected organic phase was washed with saturated NaCl, dried with anhydrous sodium sulfate, filterated and concentrated. The crude product was purified by Preparative-TLC (petroleum ether : EA=1:2) to give white solid compound **69b** (103 mg, yield 68%). MS m/z 691.8 [M+H]⁺.

The compound **69b** (103 mg, 0.15 mmol) was dissolved in DCM (2 mL). Boron tribromide (56 mg, 0.22 mmol) was added at -78 °C. The mixture was stirred at this temperature for 20 min. The reaction was quenched with saturated NaHCO₃ and extracted with DCM (5 mL×3). The collected organic phase was washed with saturated NaCl, dried with anhydrous sodium sulfate, filterated and concentrated. The crude product was purified by Pre-TLC (petroleum ether : EA = 1:2) to give white solid compound **69** (80 mg, yield 89%). ¹H NMR (500 MHz, DMSO-*d₆*) δ 12.90 (s, 1H), 10.30 (br, 1H), 8.02 (d, *J* = 8.6 Hz, 1H), 7.39 (s, 1H), 7.21 (d, *J* = 1.5 Hz, 1H), 7.15 (dd, *J* = 8.6, 1.5 Hz, 1H), 5.03 (br, 1H), 3.92-3.77 (m, 5H), 3.53-3.45 (m, 1H), 3.31-3.22 (m, 1H), 3.02-2.92 (m, 4H), 2.56-2.49 (m, 4H), 2.31 (s, 3H), 1.96-1.87 (m, 4H), 1.29 (d, *J* = 6.8 Hz, 3H). MS m/z 601.2 [M+H]⁺.

### Example 65: Preparation of Compound 69

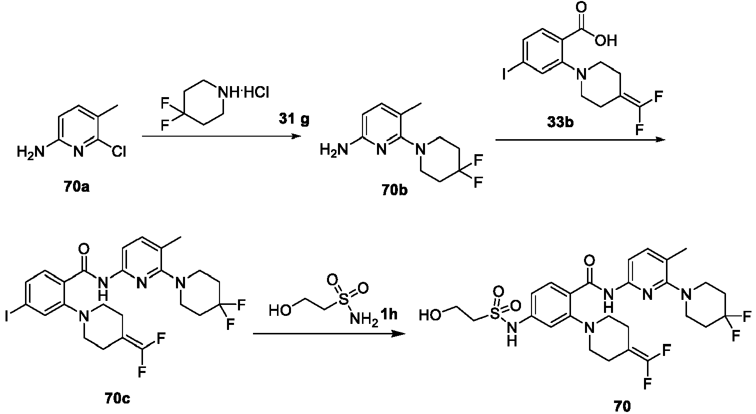

The compound **70a** (300 mg, 2.1 mmol), **31g** (430 mg, 2.74 mmol) and DIPEA (816 mg, 6.31 mmol) were dissolved in NMP (1.5 mL). The mixture was stirred at 180 °C for 72 h. After the reaction was finished, water was added and extracted with EA (30 mL×3). The collected organic phase was washed with sat.NaCl, dried with anhydrous sodium sulfate, filterated and concentrated. The crude was purified by column chromatography (PE: EA = 4:1) to give yellow oil compound **70b** (60 mg, yield 13%).

The compound **70b** (60 mg, 0.26 mmol) and **33b** (110 mg, 26 mmol) were dissolved in DCM (3 mL). Then diisopropylethylamine (101 mg, 078 mmol) and N,N,N',N'-Tetramethyl-O- (7-azabenzotriazol-1-yl) hexafluorophosphourea (124 mg, 0.39 mmol) were added. The reaction was stirred at RT for 1 h under N₂. The mixture was concentrated. The crude was purified by column chromatography to give the product **70c** (130 mg, yield 84%).

To a solution of **1h** (11 mg, 0.09 mmol) in DMF (1 mL) was added sarcosine (4 mg, 0.04 mmol), CuI (13 mg, 0.07 mmol) and tripotassium phosphate (45 mg, 0.21 mmol). The reaction was stirred at 50 °C for 5 min under a sealed tube. Then **70c** (40 mg, 0.07 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by column chromatography (PE: EA = 2:3) to give a white solid product **70** (23 mg, yield 58%). ¹H NMR (500 MHz, DMSO-*d*₆) *δ* 12.70 (s, 1H), 10.28 (s, 1H), 8.05 (d, *J* = 8.6 Hz, 1H), 7.84 (d, *J* = 8.0 Hz, 1H), 7.58 (d, *J* = 8.0 Hz, 1H), 7.17 (d, *J* = 1.9 Hz, 1H), 7.12 (dd, *J* = 8.6, 1.9 Hz, 1H), 4.97 (s, 1H), 3.75 (t, *J* = 6.4 Hz, 2H), 3.37 (t, *J* = 6.4 Hz, 2H), 3.16-3.10 (m, 4H), 2.99 (t, *J* = 5.3 Hz, 4H), 2.55-2.49 (m, 4H), 2.22 (s, 3H), 2.12-2.01 (m, 4H). MS m/z 586.3 [M+H]⁺.

### Example 67: Preparation of Compound 71

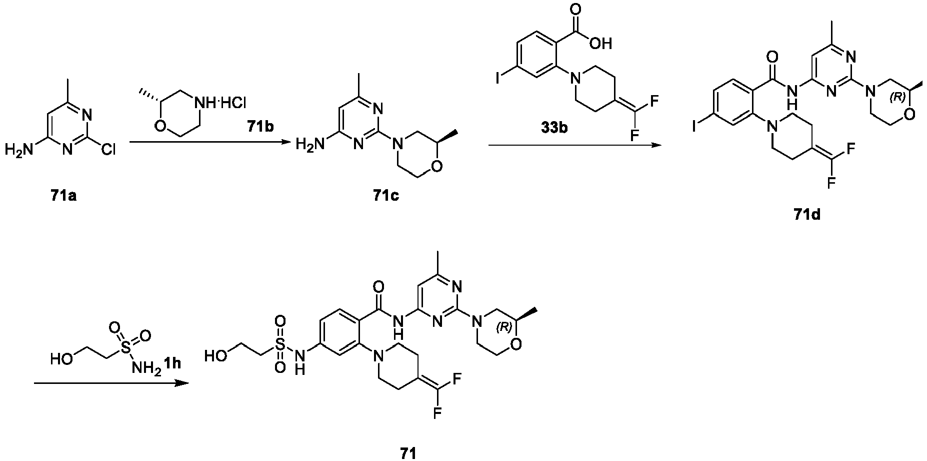

The compound **71a** (200 mg, 1.39 mmol), **71b** (250 mg, 1.81 mmol) and DIPEA (900 mg, 6.97 mmol) were dissolved in NMP (1.5 mL). The mixture was stirred at 150 °C for 18 h. After the reaction was finished, water was added and extracted with EA (30 mL × 3). The collected organic phase was washed with sat.NaCl, dried with anhydrous sodium sulfate, filterated and concentrated. The crude was purified by column chromatography (PE: EA = 4:1) to give yellow oil compound **71c** (200 mg, yield 69%).

The **33b** (100 mg, 0.26 mmol) was dissolved in 1, 2-dichloroethane (2 mL). Thionyl chloride (47 mg, 0.40 mmol) was added. The mixture was stirred at RT for 0.5 h. The above reaction solution was added dropwise to a mixed solution of 1, 2-dichloroethane (1 mL) of compound **71c** (55 mg, 0.26 mmol) and 4-dimethylaminopyridine (96 mg, 0.79 mmol). The mixture was stirred at 85 °C for 0.5 h. The mixture was concentrated. The crude was purified by column chromatography (PE: EA= 10:1) to give white solid product **71d** (60 mg, yield 39%).

The compound **1h** (20 mg, 0.16 mmol), sarcosine (4 mg, 0.05 mmol), CuI (19 mg, 0.10 mmol) and tripotassium phosphate (64 mg, 0.03 mmol) were dissolved in DMF (1 mL). The reaction was stirred at 50 °C for 5 min under a sealed tube. Then **71d** (60 mg, 0.10 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (DCM: MeOH = 20:1) to give white solid product **71** (20 mg, yield 34%). ¹H NMR (500 MHz, DMSO-*d*₆) δ 13.06 (s, 1H), 10.34 (s, 1H), 8.04 (d, *J* = 8.6 Hz, 1H), 7.36 (s, 1H), 7.18 (d, *J* = 1.9 Hz, 1H), 7.14 (d, *J* = 2.0 Hz, 1H), 4.97 (br, 1H), 4.33 (br, 2H), 3.84 (d, *J* = 9.0 Hz, 1H), 3.75 (t, *J* = 6.4 Hz, 2H), 3.50-3.37 (m, 5H), 2.99 (t, *J* = 5.4 Hz, 4H), 2.92-2.83 (m, 1H), 2.59-2.50 (m, 4H), 2.30 (s, 3H), 1.08 (d, *J* = 6.2 Hz, 3H). MS m/z 567.2 [M+H]⁺.

### Example 68: Preparation of Compound 72

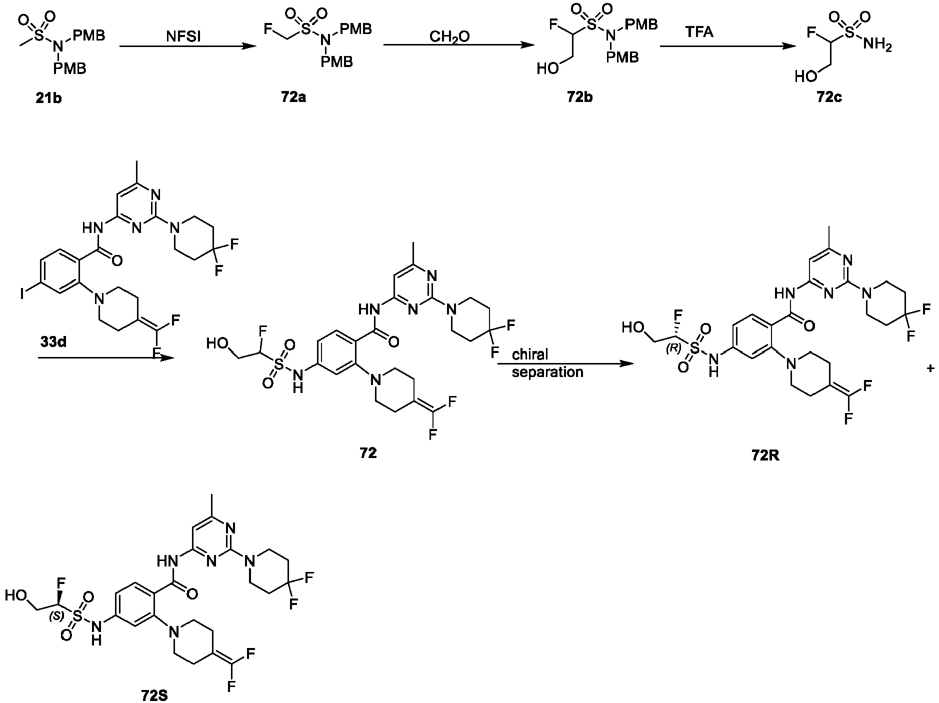

The compound **21b** (1.50 g, 4.48 mmol) was dissolved in THF (20 mL). The mixture was cooled to -78 °C and nBuLi (2.5 M, 1.9 mL) was added. The reaction was stirred at this temperature for 0.5 h. Then N-Fluorobenzenesulfonamide (NFSI, 1.48 g, 4.70 mmol) was added. The mixture was warmed to RT and stirred for 2 h. The reaction was quenched with sat.NH₄Cl and extracted with EA (50 mL × 3). The collected organic phase was washed with sat.NaCl, dried with anhydrous sodium sulfate, filterated and concentrated. The crude was purified by column chromatography to give white solid product **72a** (530 mg, 34%). MS *m*/*z* 354.3 [M+H]⁺.

The compound **72a** (530 mg, 1.50 mmol) was dissolved in THF (10 mL). The mixture was cooled to -78 °C and nBuLi (2.5 M, 0.6 mL) was added. The reaction was stirred at this temperature for 0.5 h. Then paraformaldehyde (48 mg, 1.58 mmol) was added. The mixture was warmed to RT and stirred for 2 h. The reaction was quenched with sat.NH₄Cl and extracted with EA (20 mL × 3). The collected organic phase was washed with sat.NaCl, dried with anhydrous sodium sulfate, filterated and concentrated. The crude was purified by column chromatography to give yellow oil compound **72b** (142 mg, 25%). MS *m*/*z* 384.6 [M+H]⁺.

The compound **72b** (142 mg, 0.37 mmol) was dissolved in DCM (4 mL). TFA (2 mL) was added. The reaction was stirred at RT for overnight. The mixture was concentrated. The crude was purified by column chromatography to give yellow oil compound **72c** (142 mg, yield 25%). MS *m*/*z* 384.6 [M+H]⁺.

The compound**72c** (20 mg, 0.14 mmol), sarcosine (3 mg, 0.03 mmol), CuI (6 mg, 0.03 mmol) and tripotassium phosphate (44 mg, 0.21 mmol) were dissolved in DMF (1 mL). The reaction was stirred at 50 °C for 5 min under a sealed tube. Then **33d** (40 mg, 0.07 mmol) was added and the mixture was stirred at 100 °C for 4 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA = 1:2) to give white solid product **72** (2 mg, yield 5%). MS *m*/*z* 605.1 [M+H]⁺. Compound **72** (2 mg, yield 5%) can be separated to obtain compound **72S** and compound **72R.**

### Example 69: Preparation of Compound 73

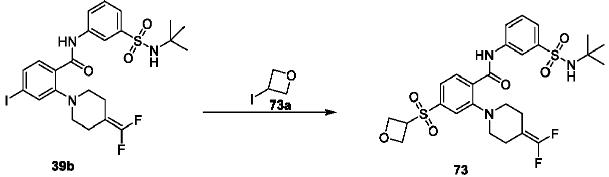

The compound **39b** (100 mg, 0.17 mmol), potassium metabisulfite (75 mg, 0.34 mmol), Tetrabutylammonium bromide (60 mg, 0.19 mmol), Sodium formate (25 mg, 0.37 mmol), triphenylphosphine (9 mg, 0.03 mmol), 1, 10-Phenanthroline (6 mg, 0.03 mmol) and Palladium acetate (4 mg, 0.02 mmol) were dissolved in DMSO (2 mL). The reaction was stirred at 70 °C for 3 h under N₂. Then 3-Iodo heterocyclic butane (69 mg, 0.37 mmol) was added and the mixture was stirred at 120 °C for 4 h. The reaction was diluted with ethyl acetate and filterated through diatomate. The organic was concentrated. The crude was purified by column chromatography to give yellow solid product **73** (18 mg, yield 18%). MS *m*/*z* 584.1 [M+H]⁺.

### Example 70: Preparation of Compound 74

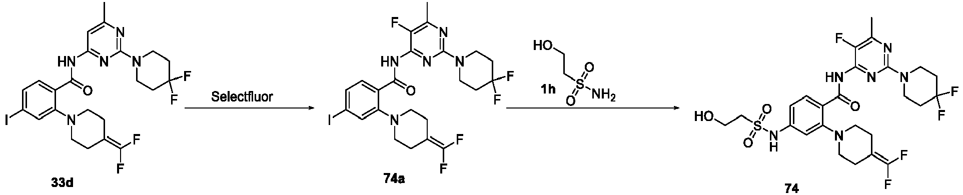

The compound **33d** (108 mg, 0.18 mmol), 1-Chloromethyl-4-fluoro-1,4-diazabicyclic [2.2.2] octanedi(tetrafluoroborate) salt (Selectfluor, 76 mg, 0.22 mmol) and silver carbonate (99 mg, 0.36 mmol) were dissolved in MeCN (3 mL). The mixture was stirred at 70 °C for 2 h. The reaction was filterated through diatomate and concentrated. The crude was purified by column chromatography to give white solid product **74a** (22 mg, yield 20%). MS *m*/*z* 608.5 [M+H]⁺.

The compound **1h** (9 mg, 0.7 mmol), sarcosine (2 mg, 0.02 mmol), CuI (4 mg, 0.02 mmol) and tripotassium phosphate (25 mg, 0.12 mmol) were dissolved in DMF (0.5 mL). The reaction was stirred at 50 °C for 5 min under a sealed tube. Then **74a** (22 mg, 0.04 mmol) was added and the mixture was stirred at 100 °C for 4 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA = 1:2) to give white solid product **74** (2 mg, yield 9%). ¹H NMR (500 MHz, DMSO-*d*₆) δ 11.97 (br, 1H), 10.23 (br, 1H), 7.80 (d, *J* = 8.4 Hz, 1H), 7.10-7.07 (d, 1.8 Hz, 1H), 7.06 (dd, *J* = 8.6, 1.8 Hz, 1H), 5.02-4.96 (m, 1H), 3.82-3.68 (m, 6H), 3.35-3.33 (m, 2H), 2.99-2.88 (m, 4H), 2.42-2.34 (m, 4H), 2.32 (d, *J* = 2.7 Hz, 3H), 1.99-1.87 (m, 4H). MS m/z 605.2 [M+H]⁺.

### Example 71: Preparation of Compound 75

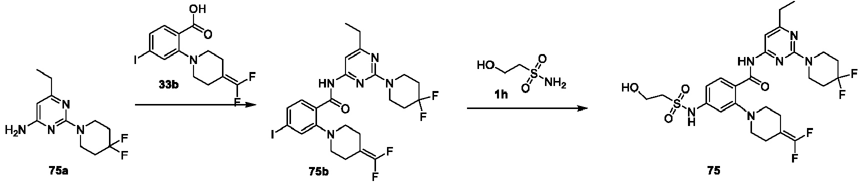

The **33b** (78 mg, 0.21 mmol) was dissolved in DCM (2 mL). Thionyl chloride (37 mg, 0.32 mmol) was added. The mixture was stirred at RT for 0.5 h. Then the mixture was concentrated and dissolved in dioxane (2 mL). The above reaction solution was added dropwise to a mixed solution of dioxane (1 mL) of compound **75a** (50 mg, 0.21 mmol), 4-dimethylaminopyridine (96 mg, 0.79 mmol) and DIPEA (81 mg, 0.63 mmol). The mixture was stirred at 90 °C for 2 h. The mixture was concentrated. The crude was purified by column chromatography to give yellow solid product **75b** (40 mg, yield 32%).

The compound **1h** (12 mg, 0.09 mmol), sarcosine (3 mg, 0.03 mmol), CuI (6 mg, 0.03 mmol) and tripotassium phosphate (38 mg, 0.18 mmol) were dissolved in DMF (1 mL). The reaction was stirred at 50 °C for 5 min under a sealed tube. Then **75b** (38 mg, 0.06 mmol) was added and the mixture was stirred at 100 °C for 4 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA = 1:2) to give white solid product **75** (18 mg, yield 49%). ¹H NMR (500 MHz, DMSO-*d*₆) δ 12.95 (s, 1H), 10.33 (br, 1H), 8.03 (d, *J* = 8.6 Hz, 1H), 7.41 (s, 1H), 7.19 (d, *J* = 2.0 Hz, 1H), 7.14 (dd, *J* = 8.6, 2.1 Hz, 1H), 4.97 (br, 1H), 3.93-3.78 (m, 4H), 3.79-3.72 (m, 2H), 3.37 (t, *J* = 6.5 Hz, 2H), 3.04-2.95 (m, 4H), 2.59 (q, *J* = 7.6 Hz, 2H), 2.56-2.49 (m, 4H), 1.98-1.87 (m, 4H), 1.20 (t, *J* = 7.6 Hz, 3H). MS m/z 601.2 [M+H]⁺

### Example 72: Preparation of Compound 76

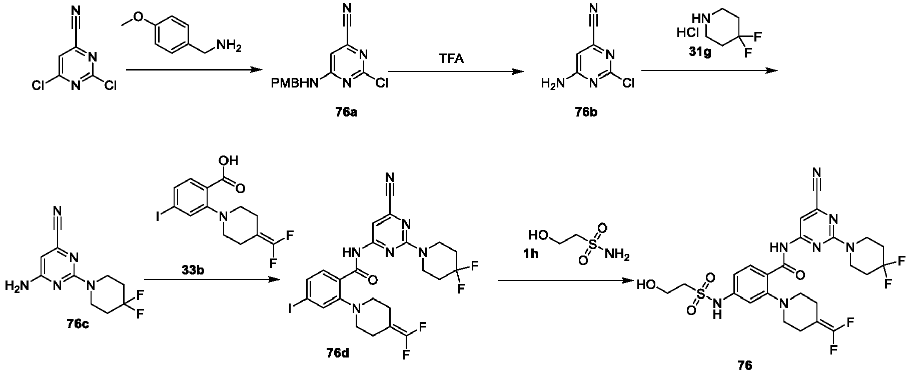

The 2, 6-Dichloro-4-cyanopyrimidine (500 mg, 2.89 mmol) and 4-Methoxybenzylamine (396 mg, 2.89 mmol) were dissolved in DCM (10 mL). Then DIPEA (1.12 g, 8.67 mmol) was added. The mixture was stirred at RT for 10 min. The mixture was concentrated and the crude was purified by column chromatography to give white solid product **76a** (430 mg, yield 54% PMB: 4-Methoxybenzyl). MS m/z 275.4, 277.4 [M+H]⁺.

The compound **76a** (430 mg, 1.57 mmol) was dissolved in 1, 2-dichloroethane (5 mL). TFA (2 mL) was added. The reaction was stirred at 70 °C for 2 h. The mixture was concentrated. The crude was purified by column chromatography to give white solid product **76b** (240 mg, yield 99%). MS m/z 155.5 & 157.5 [M+H]⁺.

The compound **76b** (241 mg, 1.56 mmol), **31g** (367 mg, 2.34 mmol) and DIPEA (604 mg, 4.68 mmol) were dissolved in NMP (3 mL). The mixture was stirred at 150 °C for 2 h. After the reaction was cooled to RT, the mixture was concentrated. The crude was purified by column chromatography to give yellow solid product **76c** (126 mg, yield 34%).

The **33b** (109 mg, 0.29 mmo) was dissolved in DCM (2 mL). Thionyl chloride (52 mg, 0.44 mmol) was added. The mixture was stirred at RT for 0.5 h. Then the mixture was concentrated and dissolved in dioxane (2 mL). The above reaction solution was added dropwise to a mixed solution of dioxane (1 mL) of compound **76c** (69 mg, 0.29 mmol), 4-dimethylaminopyridine (107 mg, 0.87 mmol) and DIPEA (112 mg, 0.87 mmol). The mixture was stirred at 90 °C for 2 h. The mixture was concentrated. The crude was purified by column chromatography to give yellow solid product **76d** (113 mg, yield 66%).

The compound **1h** (21 mg, 0.16 mmol), sarcosine (4 mg, 0.05 mmol), CuI (10 mg, 0.03 mmol) and tripotassium phosphate (70 mg, 0.33 mmol) were dissolved in DMF (1 mL). The reaction was stirred at 50 °C for 5 min under a sealed tube. Then **75b** (38 mg, 0.06 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA = 1:2) to give white solid product **76** (29 mg, yield 43%). ¹H NMR (500 MHz, DMSO-*d*₆) δ 13.51 (s, 1H), 10.36 (br, 1H), 8.04 (d, *J=* 8.6 Hz, 1H), 7.82 (s, 1H), 7.20 (d, *J* = 1.6 Hz, 1H), 7.15 (dd, *J* = 8.6, 1.6 Hz, 1H), 4.94 (br, 1H), 3.86-3.79 (m, 4H), 3.76 (t, *J* = 6.4 Hz, 2H), 3.37 (t, *J* = 6.4 Hz, 2H), 3.04-2.96 (m, 4H), 2.51-2.47 (m, 4H), 2.04-1.91 (m, 4H). MS m/z 598.1 [M+H]⁺.

### Example 73: Preparation of Compound 77

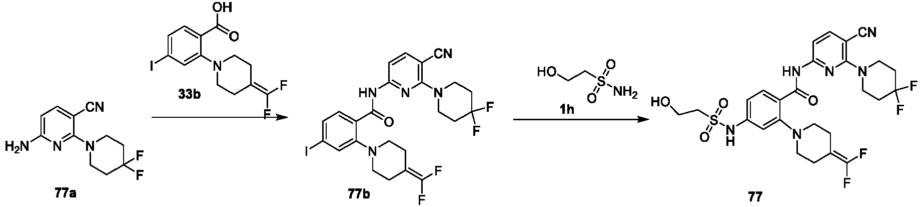

The **33b** (68 mg, 0.29 mmol) was dissolved in DCM (2 mL). Thionyl chloride (51 mg, 0.43 mmol) was added. The mixture was stirred at RT for 0.5 h. Then the mixture was concentrated and dissolved in dioxane (2 mL). The above reaction solution was added dropwise to a mixed solution of dioxane (2 mL) of compound **77a** (108 mg, 0.29 mmol), 4-dimethylaminopyridine (107 mg, 0.87 mmol) and DIPEA (112 mg, 0.87 mmol). The mixture was stirred at 90 °C for 2 h. The mixture was concentrated. The crude was purified by column chromatography to give yellow solid product **77b** (74 mg, yield 43%). MS m/z 600.4 [M+H]⁺.

The compound **1h** (13 mg, 0.11 mmol), sarcosine (3 mg, 0.03 mmol), CuI (7 mg, 0.06 mmol) and tripotassium phosphate (44 mg, 0.21 mmol) were dissolved in DMF (1 mL). The reaction was stirred at 50 °C for 5 min under a sealed tube. Then **77b** (40 mg, 0.07 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA = 1:2) to give white solid product **77** (18 mg, yield 45%) . ¹H NMR (500 MHz, DMSO-*d*₆) δ 13.01 (s, 1H), 10.33 (br, 1H), 8.11 (d, *J =* 8.4 Hz, 1H), 8.05 (d, *J* = 8.6 Hz, 1H), 7.83 (d, *J* = 8.4 Hz, 1H), 7.20 (d, *J* = 2.0 Hz, 1H), 7.14 (dd, *J* = 8.6, 2.1 Hz, 1H), 4.98 (br, 1H), 3.76 (t, *J* = 6.4 Hz, 2H), 3.74-3.68 (m, 4H), 3.37 (t, *J* = 6.5 Hz, 2H), 3.03-2.96 (m, 4H), 2.52-2.46 (m, 4H), 2.10-2.00 (m, 4H). MS *m*/*z* 597.1 [M+H]⁺.

### Example 74: Preparation of Compound 78

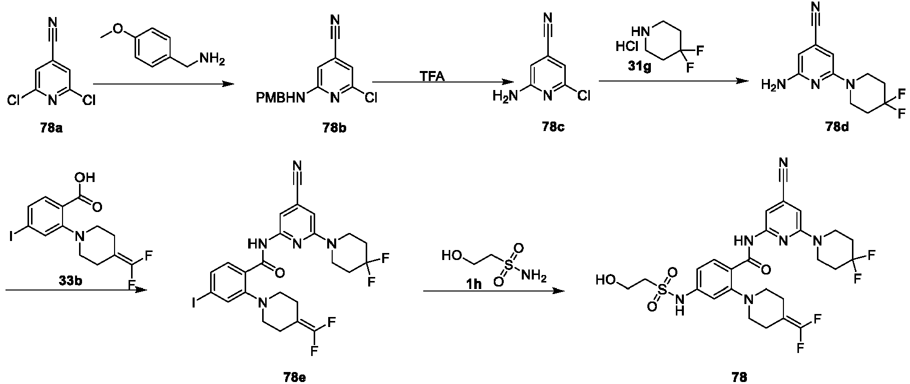

The compound **78a** (500 mg, 2.89 mmol) and 4-Methoxybenzylamine (396 mg, 2.89 mmol) were dissolved in NMP (5 mL). Then DIPEA (1.12 g, 8.67 mmol) was added. The mixture was stirred at 80 °C for 12 h. The reaction was extracted with EA (5 mL × 3) and water (3 mL). The collected organic phase was washed with sat.NaCl, dried with anhydrous sodium sulfate, filterated and concentrated. The crude was purified by column chromatography to give white solid product **78b** (600 mg, yield 76% PMB: 4-Methoxybenzyl). MS m/z 274.1 & 276.1[M+H]⁺.

The compound **78b** (300 mg, 1.10 mmol) was dissolved in 1, 2-dichloroethane (5 mL). TFA (2 mL) was added. The reaction was stirred at 70 °C for 2 h. The mixture was concentrated. The crude was purified by column chromatography to give white solid product **81c** (82 mg, yield 49%). MS m/z 154.0, 156.0[M+H]⁺.

The compound **78c** (82 mg, 0.53 mmol), **31g** (109 mg, 0.69 mmol) and DIPEA (207 mg, 1.59 mmol) were dissolved in NMP (3 mL). The mixture was stirred at 160 °C for 18 h. The reaction was extracted with EA (5 mL × 3) and water (3 mL). The collected organic phase was washed with sat.NaCl, dried with anhydrous sodium sulfate, filterated and concentrated. The crude was purified by column chromatography to give yellow solid product **78d** (30 mg, yield 24%). MS m/z 239.3 [M+H]⁺.

The **33b** (40 mg, 0.53 mmol) was dissolved in DCM (2 mL). Thionyl chloride (19 mg, 0.16 mmol) was added. The mixture was stirred at RT for 0.5 h. Then the mixture was concentrated and dissolved in dioxane (2 mL). The above reaction solution was added dropwise to a mixed solution of dioxane (1 mL) of compound **78d** (25 mg, 0.11 mmol), 4-dimethylaminopyridine (38 mg, 0.31 mmol) and DIPEA (40 mg, 0.31 mmol). The mixture was stirred at 90 °C for 2 h. The mixture was concentrated. The crude was purified by column chromatography to give yellow solid product **78e** (34 mg, yield 53%).

The compound **1h** (9 mg, 0.07 mmol), sarcosine (2 mg, 0.03 mmol), CuI (9 mg, 0.05 mmol) and tripotassium phosphate (32 mg, 0.15 mmol) were dissolved in DMF (1 mL). The reaction was stirred at 50 °C for 5 min under a sealed tube. Then **78e** (32 mg, 0.05 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA = 1:2) to give white solid product *78* (9 mg, yield 28%). ¹H NMR (500 MHz, DMSO-*d*₆) δ 12.93 (s, 1H), 10.29 (br, 1H), 8.04 (d, *J* = 8.6 Hz, 1H), 7.77 (s, 1H), 7.23 (s, 1H), 7.18 (d, *J* = 2.0 Hz, 1H), 7.13 (dd, *J* = 8.6, 2.0 Hz, 1H), 4.95 (br, 1H), 3.76 (t, *J* = 6.5 Hz, 2H), 3.74-3.62 (m, 4H), 3.36 (t, *J* = 6.5 Hz, 2H), 2.99 (t, *J* = 5.2 Hz, 4H), 2.67-2.48 (m, 4H), 2.00-1.85 (m, 4H). MS m/z 597.5 [M+H]⁺.

### Example 75: Preparation of Compound 79, 80 and 81

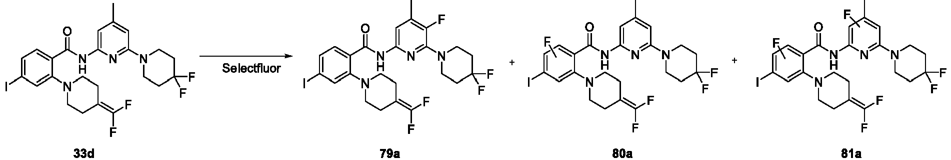

The compound **33d** (240 mg, 0.41 mmol), 1-Chloromethyl-4-fluoro-1, 4-diazabicyclic [2.2.2] octanedi (tetrafluoroborate) salt (218 mg, 0.61 mmol) and silver carbonate (226 mg, 0.82 mmol) were dissolved in MeCN (3 mL). The mixture was stirred at 30 °C for 2 h. The reaction was filterated through diatomate and concentrated. The crude was purified by column chromatography to give white solid **79a** (40 mg, yield 16%), compound **80a** (10 mg, yield 4%) and **81a** (10 mg, yield 4%). The substitution positions of fluorine atoms in the structures of compounds **80a** and **81a** are uncertain

Compound **79a:** MS *m*/*z* 607.1 [M+H]⁺. ¹H NMR (500 MHz, DMSO-*d*₆) δ 12.52 (s, 1H), 7.79 (d, *J* = 8.0 Hz, 1H), 7.75-7.67 (m, 3H), 3.50 (t, *J* = 5.6 Hz, 4H), 3.02 (t, *J* = 5.6 Hz, 4H), 2.50-2.43 (m, 4H), 2.25 (d, *J* = 2.2 Hz, 3H), 2.07-1.96 (m, 4H).

Compound **80a:** MS *m*/*z* 607.1 [M+H]⁺. Compound **81a:** MS *m*/*z* 625.1 [M+H]⁺.

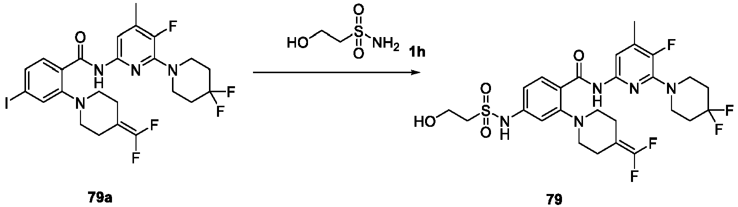

The compound **1h** (9 mg, 0.07 mmol), sarcosine (2 mg, 0.02 mmol), CuI (4 mg, 0.02 mmol) and tripotassium phosphate (25 mg, 0.12 mmol) were dissolved in DMF (0.5 mL). The reaction was stirred at 50 °C for 5 min under a sealed tube. Then**79a** (24 mg, 0.04 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA = 1:2) to give white solid product **79** (10 mg, yield 42%). MS m/z 604.1 [M+H]⁺. ¹H NMR (500 MHz, DMSO-*d*₆) δ 12.52 (s, 1H), 10.23 (br, 1H), 8.03 (d, *J* = 8.6 Hz, 1H), 7.72 (d, *J* = 4.0 Hz, 1H), 7.17 (d, *J* = 2.2 Hz, 1H), 7.12 (dd, *J* = 8.6, 2.2 Hz, 1H), 4.94 (br, 1H), 3.76 (t, *J* = 6.5 Hz, 2H), 3.50 (t, *J* = 5.5 Hz, 4H), 3.35 (t, *J* = 6.5 Hz, 2H), 2.98 (t, *J* = 5.5 Hz, 4H), 2.53-2.48 (m, 4H), 2.25 (d, *J* = 2.2 Hz, 3H), 2.04-1.96 (m, 4H).

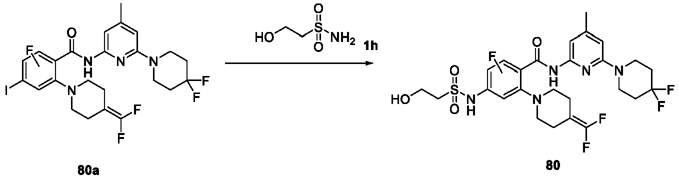

The compound **1h** (4 mg, 0.04 mmol), sarcosine (2 mg, 0.01 mmol), CuI (4 mg, 0.02 mmol) and tripotassium phosphate (12 mg, 0.06 mmol) were dissolved in DMF (0.5 mL). The reaction was stirred at 50 °C for 5 min under a sealed tube. Then **80a** (10 mg, 0.02 mmol) was added and the mixture was stirred at 100 °C for 4 h under N₂. The reaction was quenched with sat.NH₄Cl and extracted with EA (5 mL × 3). The collected organic phase was washed with sat.NaCl, dried with anhydrous sodium sulfate, filterated and concentrated. The crude was purified by Pre-TLC (PE: EA= 1:2) to give white solid **80** (3 mg, yield 30%). The substitution positions of fluorine atoms in the structures of compound **80a** are uncertain. MS m/z 604.1 [M+H]⁺.

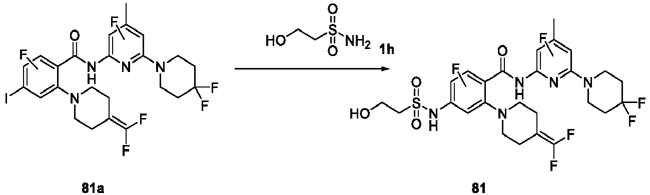

The compound **1h** (4 mg, 0.04 mmol), sarcosine (2 mg, 0.01 mmol), CuI (4 mg, 0.02 mmol) and tripotassium phosphate (12 mg, 0.06 mmol) were dissolved in DMF (0.5 mL). The reaction was stirred at 50 °C for 5 min under a sealed tube. Then **81a** (10 mg, 0.02 mmol) was added and the mixture was stirred at 100 °C for 4 h under N₂. The reaction was quenched with sat.NH₄Cl and extracted with EA (5 mL×3). The collected organic phase was washed with sat.NaCl, dried with anhydrous sodium sulfate, filterated and concentrated. The crude was purified by Pre-TLC (PE: EA = 1:2) to give white solid product **81** (3 mg, yield 30%). The substitution positions of fluorine atoms in the structures of compound **81a** are uncertain. MS m/z 622.1 [M+H]⁺.

### Example 76: Preparation of Compound 82

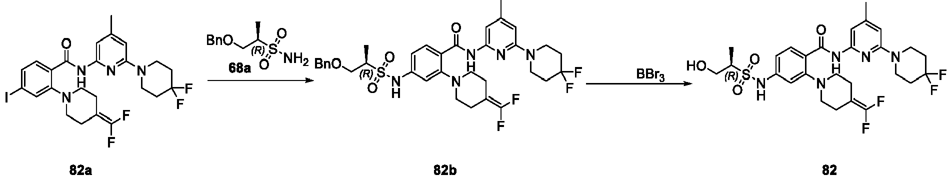

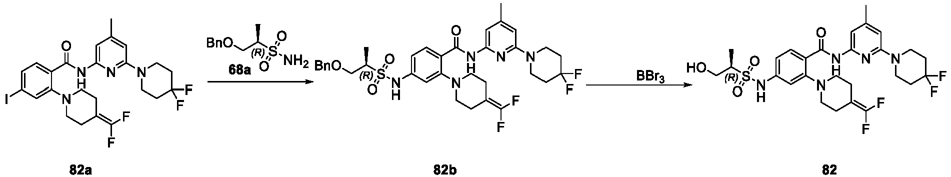

The compound **68a** (18 mg, 0.078 mmol), sarcosine (2.6 mg, 0.03 mmol), CuI (11 mg, 0.06 mmol) and tripotassium phosphate (38 mg, 0.18 mmol) were dissolved in DMF (1 mL). The reaction was stirred at 50 °C for 5 min under a sealed tube. Then **82a** (35 mg, 0.06 mmol) was added and the mixture was stirred at 100 °C for 18 h under N₂. The reaction was quenched with sat.NH₄Cl and extracted with EA (15 mL × 3). The collected organic phase was washed with sat.NaCl, dried with anhydrous sodium sulfate, filterated and concentrated. The crude was purified by Pre-TLC (PE: EA = 1:2) to give white solid product **82b** (19 mg, yield 46%). MS m/z 690.2 [M+H]⁺.

The compound **82b** (19 mg, 0.03 mmol) was dissolved in DCM (5 mL). Boron tribromide (23 mg, 0.09 mmol) was added at -78 °C. The mixture was stirred at this temperature for 30 min. After the reaction was finished, the mixture was concentrated. The crude was purified by Pre-TLC (PE: EA = 1:1) to give white solid product **82** (11 mg, yield 67%). ¹H NMR (500 MHz, DMSO-*d*₆) δ 12.44 (s, 1H), 10.22 (s, 1H), 8.02 (d, *J* = 8.6 Hz, 1H), 7.48 (s, 1H), 7.19 (d, *J* = 2.0 Hz, 1H), 7.14 (dd, *J* = 8.6, 2.0 Hz, 1H), 6.56 (s, 1H), 5.02 (br, 1H), 3.8-3.81 (m, 1H), 3.72-3.57 (m, 4H), 3.54-3.44 (m, 1H), 3.31-3.24 (m, 1H), 2.98 (t, *J* = 5.3 Hz, 4H), 2.54-2.49 (m, 4H), 2.26 (s, 3H), 1.95-1.85 (m, 4H), 1.29 (d, *J* = 6.9 Hz, 3H). MS m/z 600.1 [M+H]⁺.

### Example 77: Preparation of Compound 83

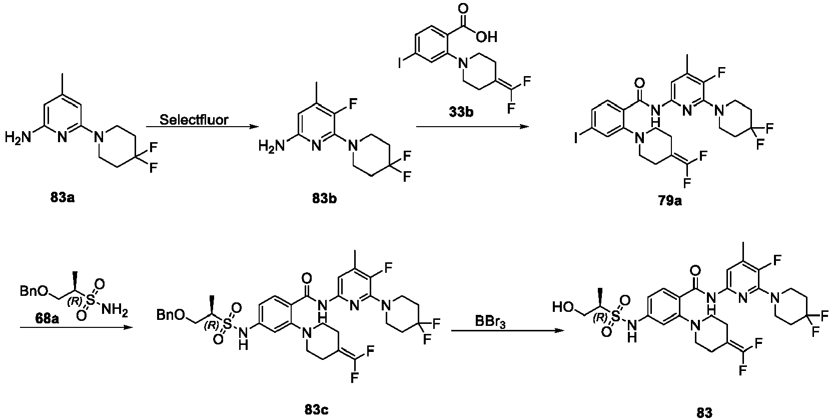

The compound **83a** (500 mg, 2.2 mmol) was dissolved in a mixed solution of chloroform and water (10 mL). 1-Chloromethyl-4-fluoro-1, 4-diazabicyclic [2.2.2] octanedi (tetrafluoroborate) salt (779 mg, 2.2 mmol) was added at RT. The mixture was stirred at RT for 0.5 h. The reaction was concentrated. The crude was purified by column chromatography to give yellow oil **83b** (100 mg, yield 19%). MS m/z 246.2 [M+H]⁺. ¹H NMR (500 MHz, DMSO-*d₆*) δ 5.81 (d, *J* = 3.5 Hz, 1H), 5.45 (s, 2H), 3.41 (t, *J* = 5.6 Hz, 4H), 2.06 (d, *J* = 2.1 Hz, 3H), 2.05-1.97 (m, 4H). NOESY NMR (500 MHz, DMSO-*d*₆) 5.81 (d, *J* = 3.5 Hz, 1H, Ar-*H*) and 5.45 (s, 2H, *NH₂)* have relevant signals, 5.81 (d, *J* = 3.5 Hz, 1H, Ar-*H*) and 2.06 (d, *J* = 2.1 Hz, 3H, *CH₃)* have relevant signals, 3.41 (t, *J* = 5.6 Hz, 4H, *CH₂*×2) and 2.05-1.97 (m, 4H, *CH₂*×2) have relevant signals.

The compound **83b** (45 mg, 0.18 mmol), **33b** (69 mg, 0.18 mmol), N,N,N',N'-Tetramethyl-O- (7-azabenzotriazol-1-yl) hexafluorophosphourea (102 mg, 0.27 mmol) and diisopropylethylamine (70 mg, 0.54 mmol) was dissolved in MeCN (2 mL). The reaction was stirred at RT for 1 h under N₂. After the reaction was finished, the mixture was concentrated and purified by column chromatography (PE: EA = 15:1) to give a white solid product **79a** (70 mg, yield 63%). MS m/z 607.2 [M+H]⁺. ¹H NMR (500 MHz, DMSO-*d*₆) δ 12.52 (s, 1H), 7.79 (d, *J* = 8.0 Hz, 1H), 7.75-7.67 (m, 3H), 3.50 (t, *J* = 5.6 Hz, 4H), 3.02 (t, *J* = 5.6 Hz, 4H), 2.50-2.43 (m, 4H), 2.25 (d, *J* = 2.2 Hz, 3H), 2.07-1.96 (m, 4H).

The compound **68a** (17 mg, 0.075 mmol), sarcosine (2.6 mg, 0.03 mmol), CuI (11 mg, 0.06 mmol) and tripotassium phosphate (38 mg, 0.18 mmol) were dissolved in DMF (1 mL). The reaction was stirred at 50 °C for 5 min under a sealed tube. Then 79a (35 mg, 0.06 mmol) was added and the mixture was stirred at 100 °C for 18 h under N₂. The reaction was quenched with sat.NH₄Cl and extracted with EA (15 mL × 3). The collected organic phase was washed with sat.NaCl, dried with anhydrous sodium sulfate, filterated and concentrated. The crude was purified by Pre-TLC (PE: EA = 2:1) to give a white solid product **83c** (22 mg, yield 54%). MS m/z 708.2 [M+H]⁺.

The compound **83c** (22 mg, 0.03 mmol) was dissolved in DCM (5 mL). Boron tribromide (23 mg, 0.09 mmol) was added at -78 °C. The mixture was stirred at this temperature for 30 min. After the reaction was finished, the mixture was concentrated. The crude was purified by Pre-TLC (PE: EA = 1:1) to give white solid product **83** (12 mg, yield 63%). ¹H NMR (500 MHz, DMSO-*d*₆) δ 12.52 (s, 1H), 10.16 (s, 1H), 8.02 (d, *J* = 8.6 Hz, 1H), 7.71 (d, *J* = 4.0 Hz, 1H), 7.19 (d, *J* = 1.9 Hz, 1H), 7.16-7.07 (m, 1H), 5.07 (br, 1H), 3.84-3.81 (m, 1H), 3.55-3.45 (m, 5H), 3.30-3.24 (m, 1H), 2.97 (t, *J* = 5.3 Hz, 4H), 2.54-2.49 (m, 4H), 2.25 (d, *J* = 2.0 Hz, 3H), 2.06-1.94 (m, 4H), 1.29 (d, *J* = 6.9 Hz, 3H). MS m/z 618.3 [M+H]⁺.

### Example 78: Preparation of Compound 84

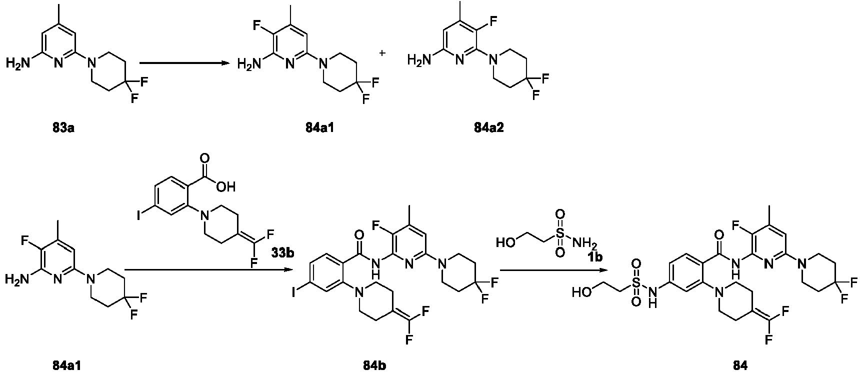

The compound **83a** (2.7 g, 11.88 mmol) was dissolved in a mixed solution of chloroform and water (3:1, 150 mL). 1-Chloromethyl-4-fluoro-1, 4-diazabicyclic [2.2.2] octanedi (tetrafluoroborate) salt (6.3 g, 17.82 mmol) was added. The mixture was stirred at 0 °C for 0.5 h. The reaction was concentrated. The crude was purified by column chromatography to give yellow oil **84a1** (620 mg, yield 21%) and yellow oil product **83b** (1.1 g, yield 38%). Compound **84a1:** ¹H NMR (500 MHz, DMSO-d6) δ 5.88 (d, *J* = 3.2 Hz, 1H), 5.71 (s, 2H), 3.54-3.46 (m, 4H), 2.09 (d, *J=* 1.6 Hz, 3H), 1.98-1.87 (m, 4H). MS m/z 246.2 [M+H]⁺.

The compound **84a1** (50 mg, 0.20 mmol), **33b** (77 mg, 0.20 mmol), N, N, N', N'-Tetramethyl-O- (7-azabenzotriazol-1-yl) hexafluorophosphourea (81 mg, 0.3 mmol) and diisopropylethylamine (78 mg, 0.60 mmol) was dissolved in MeCN (2 mL). The reaction was stirred at RT for 1 h under N₂. After the reaction was finished, the mixture was concentrated and purified by column chromatography (PE: EA = 15:1) to give a white solid product **84b** (40 mg, yield 32%). MS m/z 607.0 [M+H]⁺.

The compound **1b** (12 mg, 0.09 mmo), sarcosine (4 mg, 0.04 mmol), CuI (17 mg, 0.09 mmol) and tripotassium phosphate (57 mg, 0.27 mmol) were dissolved in DMF (1 mL). The reaction was stirred at 50 °C for 5 min under a sealed tube. Then **84b** (40 mg, 0.07 mmol) was added and the mixture was stirred at 100 °C for 18 h under N₂. The reaction was quenched with sat.NH₄Cl and extracted with EA (15 mL × 3). The collected organic phase was washed with sat.NaCl, dried with anhydrous sodium sulfate, filterated and concentrated. The crude was purified by Pre-TLC (PE: EA = 2:1) to give white solid product **84** (20 mg, yield 50%). ¹H NMR (500 MHz, DMSO-d6) δ 11.36 (s, 1H), 10.11 (s, 1H), 7.79 (d, *J* = 6.2 Hz, 1H), 7.08 (s, 1H), 7.06-7.01 (m, 1H), 6.76 (s, 1H), 4.94 (br, 1H), 3.76 (t, *J* = 6.5 Hz, 2H), 3.58 (br, 4H), 3.36-3.32 (m, 2H), 2.97 (br, 4H), 2.45 (br, 4H), 2.24 (s, 3H), 1.99-1.88 (m, 4H). MS m/z 604.1 [M+H]⁺.

### Example 79: Preparation of Compound 85

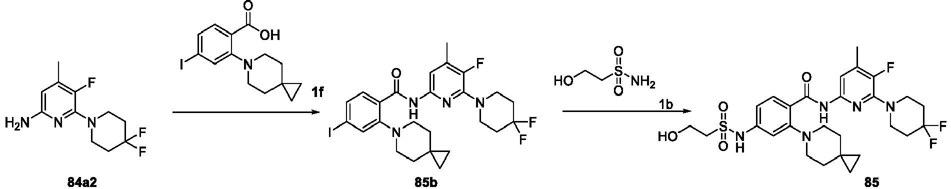

The compound **83b** (50 mg, 0.20 mmol) and **1f** (73 mg, 0.20 mmol) were dissolved in MeCN (2 mL). Then diisopropylethylamine (79 mg, 0.60 mmol) and N,N,N',N'-Tetramethyl-O- (7-azabenzotriazol-1-yl) hexafluorophosphourea (116 mg, 0.30 mmol) were added. The reaction was stirred at RT for 3 h under N₂. The mixture was concentrated. The crude was purified by column chromatography to give yellow oil product **85b** (100 mg, yield 84%). MS *m*/*z* 585.1 [M+H]⁺.

The compound **1b** (13 mg, 0.10 mmol), sarcosine (4 mg, 0.04 mmol), CuI (17 mg, 0.09 mmol) and tripotassium phosphate (57 mg, 0.27 mmol) were dissolved in DMF (1 mL). The reaction was stirred at 50 °C for 5 min under a sealed tube. Then **85b** (40 mg, 0.07 mmol) was added and the mixture was stirred at 100 °C for 18 h under N₂. The reaction was quenched with sat.NH₄Cl and extracted with EA (15 mL × 3). The collected organic phase was washed with sat.NaCl, dried with anhydrous sodium sulfate, filterated and concentrated. The crude was purified by Pre-TLC (PE: EA = 2:1) to give white solid product **85** (11 mg, yield 27%). ¹H NMR (500 MHz, DMSO-d6) δ 12.95 (s, 1H), 8.05 (d, *J* = 8.6 Hz, 1H), 7.74 (d, *J =* 4.0 Hz, 1H), 7.24 (d, *J* = 2.0 Hz, 1H), 7.11 (dd, *J* = 8.6, 2.1 Hz, 1H), 3.75 (t, *J* = 6.5 Hz, 2H), 3.64-3.52 (m, 4H), 3.35 (br, 2H), 2.96 (br, 4H), 2.25 (d, *J* = 1.8 Hz, 3H), 2.14-2.03 (m, 4H), 1.70 (br, 4H), 0.38 (s, 4H). MS m/z 582.0 [M+H]⁺.

### Example 80: Preparation of Compound 86 and 87

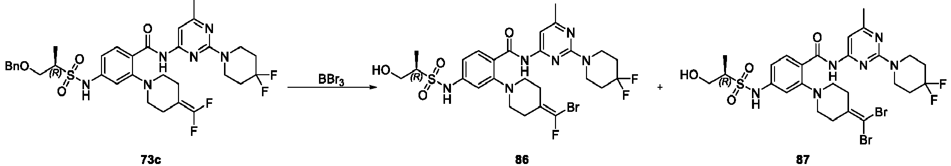

The compound **73c** (1.7 g, 2.46 mmol) was dissolved in DCM (50 mL). Boron tribromide (6.15 mL, 2 M) was added at -20 °C. The mixture was stirred at this temperature for 0.5 h. After the reaction was finished, the reaction was quenched with sat.NaHCO₃ and extracted with DCM (5 mL × 3). The collected organic phase was washed with sat.NaCl, dried with anhydrous sodium sulfate, filterated and concentrated. The crude was purified by Pre-TLC (DCE: MeOH = 20:1) to give white solid product **86** (11.95 mg, yield 0.8%). ¹H NMR (500 MHz, DMSO-d6) δ 12.89 (s, 1H), 8.00 (d, *J* = 8.6 Hz, 1H), 7.39 (s, 1H), 7.19 (d, *J* = 1.9 Hz, 1H), 7.14 (dd, *J* = 8.6, 2.0 Hz, 1H), 3.98-3.90 (m, 2H), 3.84-3.79 (m, 1H), 3.74 (dd, *J* = 12.4, 7.8 Hz, 2H), 3.52-3.45 (m, 1H), 3.29-3.24 (m, 1H), 2.98 (t, *J* = 5.3 Hz, 4H), 2.52 (br, 4H), 2.37-2.21 (m, 7H), 1.28 (d, *J* = 6.9 Hz, 3H). MS m/z 661.2, 663.1 [M+H]⁺. White solid compound **87** (28 mg, yield 1.9 %). ¹H NMR (500 MHz, DMSO-d6) δ 12.92 (s, 1H), 7.97 (d, *J* = 8.6 Hz, 1H), 7.39 (s, 1H), 7.13 (s, 1H), 7.11-7.07 (m, 1H), 3.84-3.77 (m, 5H), 3.48 (dd, *J* = 11.1, 7.3 Hz, 1H), 3.25-3.20 (m, 1H), 2.97 (t, *J* = 5.3 Hz, 4H), 2.54-2.51 (m, overlaps with DMSO), 2.49-2.47 (m, overlaps with DMSO), 2.31 (s, 3H), 1.27 (d, *J =* 6.9 Hz, 3H). MS m/z 721.1, 723.1 [M+H]⁺.

### Example 81: Inhibitory activity of compounds on proliferation of ovarian cancer cell line OVCAR-3, breast cancer cell lines HCC1954 and HCC1806

OVCAR-3, HCC1954 and HCC1806 cells with good growth status were selected and digested with trypsin. Fresh culture medium was added and mixed thoroughly, and centrifuged at 800 rpm for 3 minutes. The cells were seeded into 96-well plates at a density of 3,300 OVCAR-3 cells, 2,500 HCC1954 cells, and 3,000 HCC1806 cells per well, and cultured overnight in a 37°C incubator. The next day, the culture plate was taken out, compounds were diluted in a four-fold gradient, and added into the plate. The blank control wells contain only normal medium, no cells, and no DMSO; the DMSO wells contain cells and 0.5% DMSO. The 96-well plate was placed in a 37°C incubator for 96 hours.

The cell culture plate was placed at room temperature for 30 minutes to equilibrate; 100 µL of CellTiter Glo detection reagent was added to each well and mixed on a shaker for 2 minutes to induce cell lysis; The 96-well plate was placed at room temperature for 10 minutes to stabilize the luminescence signal ; The white bottom film was pasted to the bottom of the culture plate, Enspire was used to detect the chemiluminescence value and inhibition rate was calculated according to the following formula: Inhibition rate (%) = (1 - (compound well -blank control well) / (DMSO control well-blank control well)) ×100%. The IC₅₀ value of each compound was analyzed using the nonlinear regression method of XLFit software. Activity data of representative compounds in inhibiting cell proliferation are shown in Table 1.

**Table 1: Inhibitory Activity of Compounds on Proliferation of OVCAR-3, HCC1954 and HCC1806**

| Compounds | OVCAR-3 | HCC1954 | HCC 1806 |
|---|---|---|---|
| | IC₅₀ (nM) | IC₅₀ (nM) | IC₅₀ (nM) |
| **1** | <500 | | <1000 |
| **3C** | <200 | | <500 |
| **5** | <75 | | <200 |
| **13** | <500 | | <500 |
| **14** | <100 | | <200 |
| **20** | <1000 | | <1000 |
| **21** | <500 | | <1000 |
| **24** | <200 | | <500 |
| **25** | <1500 | | <1500 |
| **29** | <500 | | <1000 |
| **30** | <500 | | <1000 |
| **31** | <50 | | <100 |
| **32** | <1500 | | <1000 |
| **33** | <75 | <75 | <200 |
| **34** | <1000 | | <1500 |
| **35** | <500 | | |
| **36** | <200 | | |
| **37&37'** | <100 | | |
| **38** | <1500 | | |
| **39** | <50 | | |
| **40** | <50 | | |
| **41** | <50 | | |
| **42** | <500 | <500 | |
| **43** | <1000 | | |
| **44** | <2000 | | |
| **45** | <1000 | | |
| **46** | <1500 | | |
| **47** | <50 | <75 | |
| **48** | <50 | <50 | <50 |
| **49** | <200 | | |
| **50** | <100 | | |
| **51** | <50 | <100 | <200 |
| **52** | <50 | | <100 |
| **53** | <50 | <50 | |
| **55** | <1500 | | <3000 |
| **56** | <50 | <75 | <100 |
| **59** | <75 | <75 | <200 |
| **60** | <50 | <75 | <200 |
| **62** | <200 | | <500 |
| **63** | <50 | <50 | <75 |
| **64** | <200 | | <500 |
| **65** | <500 | | <1000 |
| **67** | <500 | | <1000 |
| **68** | <50 | <50 | <100 |
| **69** | <100 | <200 | <200 |
| **70** | <50 | <50 | <75 |
| **71** | <100 | <75 | <500 |
| **72** | <200 | | <500 |
| **73** | <200 | | <500 |
| **74** | <1000 | <1000 | |
| **75** | <200 | | |
| **76** | <200 | | |
| **77** | <50 | | |
| **78** | <50 | | |
| **79** | <50 | | |
| **81** | <500 | | |

### Example 82: Inhibition of KIF18A enzyme activity by compounds

The ADP-Glo method was used to test the enzymatic activity of KIF18A. The compound was diluted 3-fold with DMSO at a starting concentration of 3000 nM, for a total of ten concentrations, and tested in duplicate. Echo was used to transfer 25 nL of the diluted compound to a 384-well plate, and 2.5 µL of enzyme working solution was added. Plate was centrifuged at 1000 rpm for 1 min, and incubated at 25°C for 10 min. 2.5 µL of ATP and substrate solution was added to start the reaction, and plate was incubated at 25°C for 60 min. 4 µL of ADP-Glo solution was added to the 384-well plate, and plate was incubated at 25°C for 40 min. 8 µL of detection solution was added and incubated at 25°C for 40 min, and BMG was used to read the fluorescence value. Inhibition rate was calculate according to the following formula: Inhibition rate (%) = (DMSO well - compound well) / (DMSO well - blank control well) × 100%. The IC₅₀ value of each compound was analyzed using the nonlinear regression method of XLFit 5.5.0 software. The formula is: Y=Bottom + (Top-Bottom)/(1+10^((LogIC50-X)*HillSlope)). The enzyme inhibitory activity data of some representative compounds are shown in Table 2

**Table 2 KIF18A Enzymatic Activity of Compounds**

| Compounds | KIF18A IC₅₀ (nM) |
|---|---|
| **33** | <50 |
| **48** | <50 |
| **56** | <50 |
| **60** | <150 |
| **68** | <50 |
| **69** | <100 |
| **79** | <50 |
| **82** | <50 |
| **83** | <100 |
| **85** | <50 |

### Example 83: Pharmacokinetic study in rats

Instruments: XEVO TQ-S LC/MS instrument was produced by Waters. All measurement data were collected and processed by Masslynx V4.1 software, and the data were calculated and processed by Microsoft Excel. The statistical moment method of WinNonLin 8.0 software was used to calculate the pharmacokinetic parameters. These mainly include kinetic parameters Tmax, T1/2, Cmax, AUC₀₋₂₄ₕ etc. Column: ACQUITY UPLC BEH C18 (2.1 mm × 50 mm, 1.7 µm); column temperature 40°C; mobile phase A is water (0.1% formic acid), mobile phase B is acetonitrile, flow rate is 0.350 mL/min, gradient elution is adopted, the elution gradient is 0.50 min: 10% B; 1.50 min: 90% B; 2.50 min: 90% B; 2.51 min: 10% B; 3.50 min: stop. Injection volume: 1 µL.

Animals: 3 male SD rats with a weight range of 200-220 g were purchased and kept in the laboratory of the Experimental Animal Center for 2 days and then used. They were fasted for 12 hours predose and 4 hours after dosing. Drinking water was free during the test. After the rats were gavage, blood samples were taken according to the established time point.

Solvent: 0.4% ethanol + 0.4% Tween80 + 99.2% (0.5% methylcellulose M450). Preparation of the solution for intragastrical administration: the compounds were accurately weighed and added to the solvent, and ultrasound was used at room temperature for 5 minutes to completely dissolve the drug, and a 0.3 mg/mL medicinal solution was prepared.

Pharmaceutical samples: Generally, multiple samples with similar structures (with a molecular weight difference of more than 2 units) are taken, accurately weighed, and administered together (cassette PK). In this way, multiple compounds can be screened at the same time and their oral absorption rates can be compared. A single administration was also used to study the pharmacokinetics of the drug sample in rats.

After intragastrical administration, blood was taken from the orbit at 0.25, 0.5, 1, 2, 4, 8, 10 and 24 hours. Then 50 µL of the plasma sample was taken and added 200 µL of acetonitrile (including internal standard control verapamil 2 ng/mL), vortexed for 3 min and centrifuged at 20000 rcf, 4°C for 10 min. The supernatant plasma was used for LC-MS/ MS analysis.

The compounds were accurately weighed to prepare different concentrations, and quantitative analysis on mass spectrometry was performed to establish a standard curve, and then the concentration of the above-mentioned compound in the plasma was tested to obtain its concentration at different time points. All measurement data were collected and processed by relevant software, and the statistical moment method was used to calculate the pharmacokinetic parameters (mainly including kinetic parameters Tmax, T_{1/2}, Cmax, AUC₀₋₂₄ₕ etc). The kinetic parameters of some representative compounds are shown in Table 3.

**Table 3: Pharmacokinetic Parameters of Compounds in SD rats**

| Compounds | Oral dosage | T_{1/2} (h) | Tₘₐₓ (h) | Cₘₐₓ (ng/mL) | AUC₀₋₂₄ₕ (h*ng/mL) |
|---|---|---|---|---|---|
| **5** | 3 mg/kg | 4.04 | 3.33 | 64.96 | 465.58 |
| **14** | 3 mg/kg | 12.57 | 8.67 | 23.21 | 383.83 |
| **31** | 3 mg/kg | 2.54 | 1.67 | 22.44 | 123.51 |
| **33** | 3 mg/kg | 7.05 | 8.00 | 717.6 | 10273.88 |
| **48** | 3 mg/kg | 4.91 | 4.00 | 153.57 | 1724.65 |
| **50** | 3 mg/kg | 7.14 | 4.00 | 15.98 | 198.07 |
| **51** | 3 mg/kg | 6.68 | 4.67 | 43.29 | 423.59 |
| **56** | 3 mg/kg | 3.83 | 4.00 | 175.28 | 2051.53 |
| **60** | 3 mg/kg | 7.47 | 6.67 | 90.70 | 1330.47 |
| **68** | 3 mg/kg | 7.18 | 6.67 | 227.19 | 3328.11 |
| **79** | 3 mg/kg | 7.61 | 5.33 | 160.5 | 2241.56 |
| **82** | 3 mg/kg | 5.01 | 4.67 | 363.12 | 4521.67 |
| **83** | 3 mg/kg | 6.99 | 6.00 | 424.35 | 5634.70 |

All references mentioned in the present invention are cited as references in this application, just as each reference is cited separately. In addition, it should be understood that after reading the above lecture content of the present invention, those skilled in the art can make various modifications or modifications to the present invention, and these equivalent forms also fall within the scope of the claims attached to the present application.

## Claims

1. A compound of the following formula (I), or the optical isomers, pharmaceutically acceptable salts, prodrugs, deuterated derivatives, hydrates, or solvates thereof: 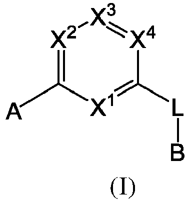 wherein in the formula (I):
A is selected from-Y-R¹; wherein, Y is selected from the bond, -S(O)-, -S(O)₂-, - S(O)(NH)-, -S(O)₂NR^{a}-, -NR^{a}S(O)₂-, -NR^{a}S(O)₂NR^{a}-, -P(O)(R^{q})-, -P(O)(R^{q})NR^{a}-, - NR^{a}P(O)(R^{q})-, -P(O)(R^{q})O-, -OP(O)(R^{q})-, -NR^{a}-, -O-, -S-, -C(O)NR^{a}-, -NR^{a}C(O)-, -C(O)O-, -OC(O)-, -NR^{a}C(O)NR^{a}-, -OC(O)NR^{a}-, -NR^{a}C(O)O-, C₂₋₄ alkenyl, and C₂₋₄ alkynyl; wherein each R^{a} is independently selected from hydrogen and C₁₋₄ alkyl; each R^{q} is independently selected from C₁₋₄ alkyl, C₃₋₆ cycloalkyl, and 3- to 6-membered heterocyclyl; or R^{q} and R¹ together with the attached phosphorus atom form optionally substituted 4- to 8-membered ring structure, while the ring structure may additionally contain 0-1 heteroatoms optionally selected from N, O and S;
or A is Formula (Ia): 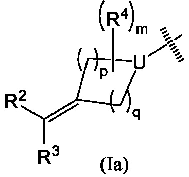
wherein, " 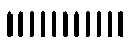 " refers to the site of formula (Ia) which attachs to the remaining fragment of the compound of formula (I);
L is selected from -C(O)NR^{b}-, -NR^{b}C(O)-, -NR^{b}C(O)NR^{b}-, -OC(O)NR^{b}-, -C(O)O-, - OC(O)-, -NR^{b}C(O)O-, -S(O)₂NR^{b}-, -NR^{b}S(O)₂-, -NR^{b}-, -O-, 3- to 6-membered heterocyclyl, and heteroaryl; wherein each R^{b} is independently hydrogen or C₁₋₄ alkyl;
B is Formula (Ib) or (Id): 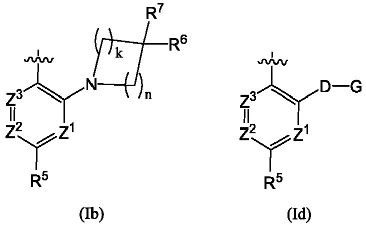
wherein " 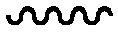 " refers to the site of formula (Ib) or (Id) which attach to the L in a compound of formula (I);
X¹, X², X³ and X⁴ are each independently selected from N or CR^{c}; wherein R^{c} is selected from the group consisting of hydrogen, halogen, C₁₋₄ alkyl, C₁₋₄ haloalkyl, C₂₋₄ alkynyl, C₁₋₄ alkoxy, hydroxy, C₁₋₄ haloalkoxy, CN, NR^{d}R^{d}, C₃₋₆ cycloalkyl, and 3- to 8-heterocyclic group; wherein each R^{d} is independently selected from the group consisting of hydrogen, C₁₋₄ alkyl, and C₁₋₄ haloalkyl;
U is selected from N or CR^{e}; wherein R^{e} is selected from the group consisting of hydrogen, halogen, and C₁₋₄ alkyl;
Z¹, Z² and Z³ are each independently selected from N and CR^{m}; wherein R^{m} is selected from the group consisting of hydrogen, halogen, C₁₋₄ alkyl, C₁₋₄ haloalkyl, C₁₋₄ alkoxy, hydroxy, C₁₋₄ haloalkoxy, CN, NR^{d}R^{d}, C₃₋₈ cycloalkyl, and 3- to 8-membered heterocyclyl; wherein, each R^{d} are as defined above; D is selected from the group consisting of bond, - O-, -S-, NR^{d}-, C₁₋₂ alkyl, and C₂₋₄ alkynyl; G is 3-10 membered saturated or unsaturated cyclic structure, which optionally containing 0, 1 or 2 heteroatoms selected from N, O and S, or optionally substituted by R⁶ or R⁷;
R¹ is selected from the group consisting of C₁₋₄ alkyl, C₂₋₄ alkenyl, C₂₋₄ alkynyl, C₃₋₆ cycloalkyl, 3- to 10-membered heterocyclyl; said alkyl, alkenyl, alkynyl, cycloalkyl or heterocyclyl being optionally substituted with one or more groups selected from the group consisting of halogen, C₁₋₄ alkyl, C₁₋₄ haloalkyl, C₂₋₄ alkenyl, C₂₋₄ haloalkenyl, C₂₋₄ alkynyl, C₂₋₄ haloalkynyl, C₃₋₆ cycloalkyl, 3- to 6- membered heterocyclyl, CN, OR^{f}, SR^{f}, NR^{d}R^{d}, -OC₁₋₄ alkyl-OR^{f}, -OC₁₋₄ alkyl-NR^{d}R^{d}, -NR^{d}C₁₋₄ alkyl-OR^{f}, -NR^{d}C₁₋₄ alkyl-NR^{d}R^{d}, -C(O)R^{g}, -C(O)OR^{f}, -OC(O)R^{g}, -C(O)NR^{d}R^{d}, -NR^{d}C(O)R^{g}, -NR^{d}C(O)NR^{d}R^{d}, - OC(O)NR^{d}R^{d}, -NR^{d}C(O)OR^{f}, -OC(O)OR^{f}, -S(O)₂NR^{d}R^{d}, -NR^{d}S(O)₂R^{g}, and - NR^{d}S(O)₂NR^{d}R^{d}; or said cycloalkyl or heterocyclyl is optionally substituted with =M; wherein, R^{f} is selected from hydrogen, C₁₋₄ alkyl, and C₁₋₄ haloalkyl; R^{g} is selected from hydrogen, C₁₋₄ alkyl, C₂₋₄ alkenyl, C₂₋₄ alkynyl, C₃₋₈ cycloalkyl, 4- to 8- membered heterocyclyl, aryl, and heteroaryl; M is selected from O and CR^{h}Rⁱ; wherein, R^{h} and Rⁱ are each independently selected from hydrogen, halogen, and C₁₋₄ alkyl; wherein the alkyl in R^{h} or Rⁱ is optionally substituted with one or more groups selected from hydrogen, halogen, C₁₋₄ alkyl, C₁₋₄ haloalkyl, C₁₋₄ alkoxy, C₁₋₄ haloalkoxy, hydroxy, NR^{d}R^{d}, C₃₋₈ cycloalkyl, 3-to 8-membered heterocyclyl, aryl, and heteroaryl; or R^{h} and Rⁱ, together with the carbon atom to which they are attached, form a 3- to -6-membered ring structure optionally containing 0, 1 or 2 heteroatoms selected from N, O and S; each R^{d} is as defined above;
R² and R³ are each independently selected from the group consisting of hydrogen, fluorine, and C₁₋₄ alkyl; wherein the alkyl is optionally substituted with one or more groups selected from the group consisting of halogen, C₁₋₄ alkyl, C₁₋₄ haloalkyl, C₁₋₄ alkoxy, C₁₋₄ haloalkoxy, hydroxyl, NR^{d}R^{d}, C₃₋₈ cycloalkyl, 3- to 8-membered heterocyclyl, aryl, and heteroaryl; wherein, each R^{d} is defined as above;
each R⁴ is independently selected from hydrogen, halogen, C₁₋₄ alkyl, C₁₋₄ haloalkyl, C₁₋₄ alkoxy, C₁₋₄ alkoxy-C₁₋₄ alkyl, hydroxy, hydroxyl-C₁₋₄ alkyl, C₁₋₄ haloalkoxy, C₁₋₄ haloalkoxy-C₁₋₄ alkyl, and CN;
R⁵ is selected from -Q-R⁸; wherein Q is selected from bond, -S(O)-, -S(O)₂-, - S(O)(NH)-, -S(O)₂NR^{a}-, -NR^{a}S(O)₂-, -NR^{a}S(O)₂NR^{a}-, -NR^{a}-, -O-, -S-, -C(O)NR^{a}-, - NR^{a}C(O)-, -C(O)O-, -OC(O)-, -NR^{a}C(O)NR^{a}-, -OC(O)NR^{a}-, -NR^{a}C(O)O-, C₂₋₄ alkenyl, and C₂₋₄ alkynyl; where each R^{a} is as defined above;
R⁶ and R⁷ are each independently selected from hydrogen, halogen, C₁₋₄ alkyl, C₂₋₄ alkenyl, C₂₋₄ alkynyl, C₃₋₈ cycloalkyl, 4- to 8-membered heterocyclyl; or R⁶ and R⁷ together with the carbon atom to which they are attached form a 3- to 6-membered ring structure, optionally containing 0, 1, or 2 heteroatoms selected from N, O and S; or R⁶ and R⁷ together with the carbon atom to which they are attached form C=T, wherein T is selected from CR^{j}R^{k}; wherein R^{j} and R^{k} are each independently selected from the group consisting of hydrogen, deuterium, halogen, and C₁₋₄ alkyl; said alkyl is optionally substituted with one or more group selected from halogen, C₁₋₄ haloalkyl, C₂₋₄ alkenyl, C₂₋₄ alkynyl, C₃₋₆ cycloalkyl, 3- to 6-membered heterocyclyl, CN, OR^{f}, SR^{f}, and NR^{d}R^{d}; or R^{j} and R^{k} together with the carbon atom to which they are attached form a 3- to -6 membered ring structure which optionally contains 0 or 1 heteroatom selected from N, O and S; R^{d} and R^{f} are as defined above;
R⁸ is selected from the group consisting of C₁₋₄ alkyl, C₂₋₄ alkenyl, C₂₋₄ alkynyl, C₃₋₆ cycloalkyl, 3- to 10-membered heterocyclyl; said alkyl, alkenyl, alkynyl, cycloalkyl or heterocyclyl being optionally substituted with one or more groups selected from the group consisting of halogen, C₁₋₄ alkyl, C₁₋₄ haloalkyl, C₂₋₄ alkenyl, C₂₋₄ alkynyl, C₃₋₆ cycloalkyl, 3- to 6-membered heterocyclyl, CN, OR^{f}, SR^{f}, NR^{d}R^{d}, -OC₁₋₄ alkyl-OR^{f}, -OC₁₋₄ alkyl-NR^{d}R^{d}, -NR^{d}C₁₋₄ alkyl-OR^{f}, -NR^{d}C₁₋₄ alkyl-NR^{d}R^{d}, -C(O)R^{g}, -C(O)OR^{f}, -OC(O)R^{g}, - C(O)NR^{d}R^{d}, -NR^{d}C(O)R^{g}, -NR^{d}C(O)NR^{d}R^{d}, -OC(O)NR^{d}R^{d}, -NR^{d}C(O)OR^{f}, -OC(O)OR^{f}, - S(O)₂NR^{d}R^{d}, -NR^{d}S(O)₂R^{g}, and -NR^{d}S(O)₂NR^{d}R^{d}; or said cycloalkyl or heterocyclyl is substituted by =M; wherein, R^{f} is selected from hydrogen, C₁₋₄ alkyl, and C₁₋₄ haloalkyl; R^{g} is selected from hydrogen, C₁₋₄ alkyl, C₂₋₄ alkenyl, C₂₋₄ alkynyl, C₃₋₈ cycloalkyl, 4- to 8-membered heterocyclyl, aryl, and heteroaryl; M is selected from O and CR^{h}Rⁱ; wherein, R^{h} and Rⁱ are each independently selected from hydrogen, halogen, and C₁₋₄ alkyl; wherein the alkyl in R^{h} or Rⁱ is optionally substituted with one or more groups selected from hydrogen, halogen, C₁₋₄ alkyl, C₁₋₄ haloalkyl, C₁₋₄ alkoxy, C₁₋₄ haloalkoxy, hydroxy, NR^{d}R^{d}, C₃₋₈ cycloalkyl, 3- to 8-membered heterocyclyl, aryl, and heteroaryl; or R^{h} and Rⁱ together with the carbon atoms to which they are attached form a 3- to -6-membered ring structure optionally containing 0, 1, or 2 heteroatoms selected from N, O and S; each R^{d} is as defined above;
p and q are each independently selected from 0, 1, 2, 3, 4, 5 and 6; with the proviso that p and q are not simultaneously 0;
m is selected from 0, 1, 2, 3 and 4;
k and n are selected from 1, 2, 3, 4 and 5;
wherein, each of the above mentioned alkyl, alkenyl, alkynyl, cycloalkyl, heterocyclyl, aryl and heteroaryl is optionally and each independently substituted by 1-3 substituents selected from the group consisting of halogen, C₁₋₄ alkyl, C₁₋₄ haloalkyl, C₂₋₄ alkenyl, C₂₋₄ alkynyl, C₃₋₈ cycloalkyl, 3- to 8-membered heterocyclyl, aryl, heteroaryl, CN, NO₂, OR^{f}, SR^{f}, NR^{d}R^{d}, C(O)R^{g}, C(O)OR^{f}, C(O)NR^{d}R^{d}, NR^{d}C(O)R^{g}, NR^{d}S(O)₂R^{g}, and S(O)₂R^{g}, provided that the chemical structure formed is stable and meaningful; wherein R^{d}, R^{f}, and R^{g} are as defined above;
unless otherwise specified, the aryl is aromatic groups having 6 to 12 carbon atoms;
the heteroaryl is 5- to 15-membered (preferably 5- to 12-membered) heteroaromatic groups; and the cyclic structure is saturated or unsaturated cyclic groups with or without heteroatoms.

2. The compound of claim 1, wherein formula (I) is formula (XIa) or formula (XIb): 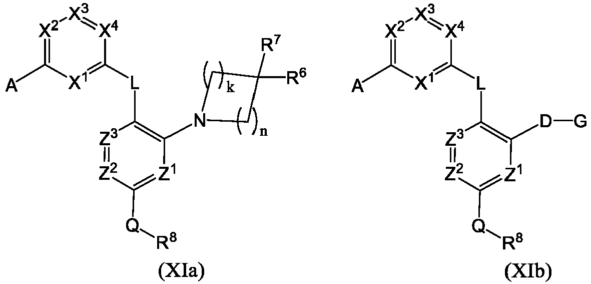 wherein the fragment 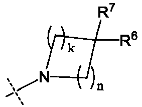 or fragment 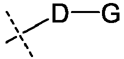 is selected from the 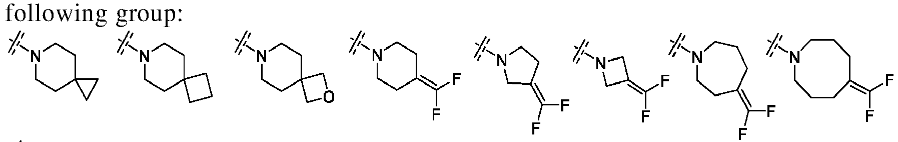 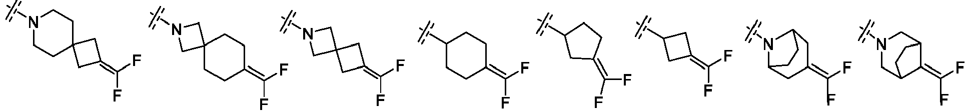 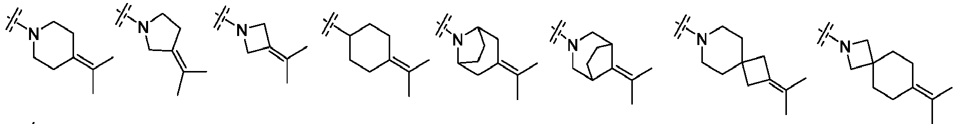 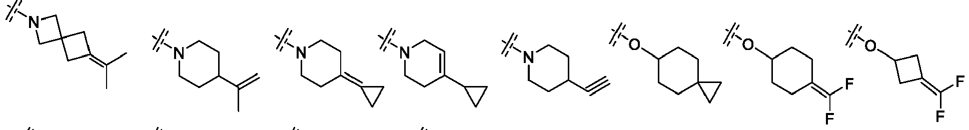 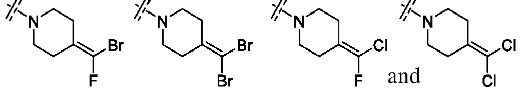 the "-̅ -̅ -̅ -̅ -̅ -̅" refers to the site of the fragment which attach to the remaining fragment of the formula (XIa) or formula (XIb);
A is a group selected from the group consisting of: 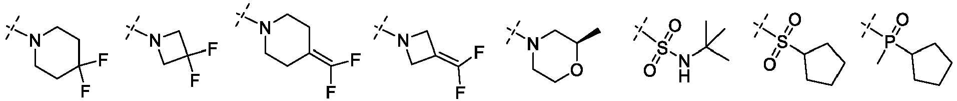 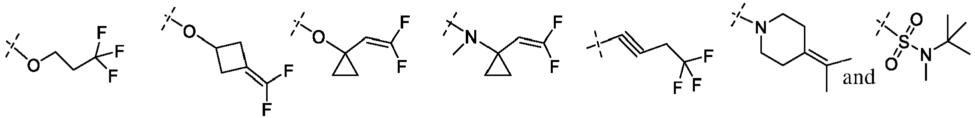
"---" refers to the site of A which attach to the remaining fragment of the formula (XIa) or formula (XIb);
X¹, X², X³ and X⁴ are as defined in claim 1;
fragment 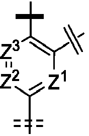 is selected from the group consisting of: 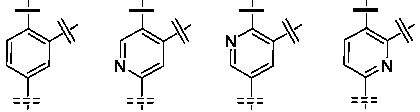 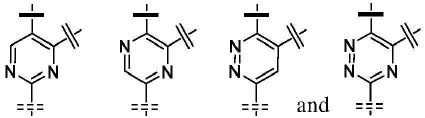
" - " indicates the site at which the fragment attach to L; " = ==" indicates the site at which attach to Q; "=" indicates the site at which the fragment attach to 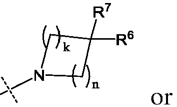 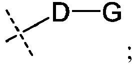
fragment 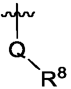 is selected from the group consisting of: 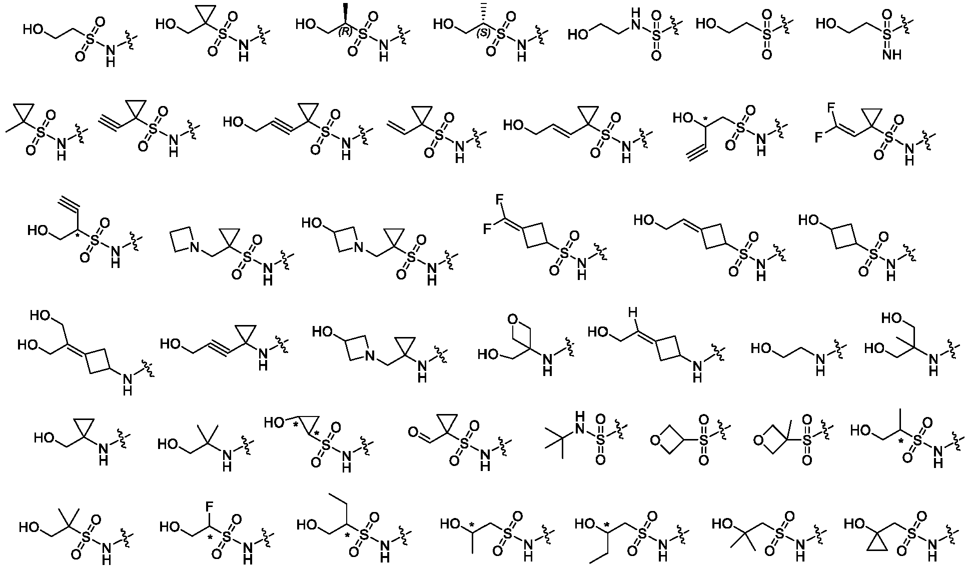 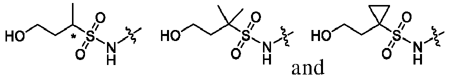
the " 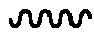 " refers to the site at which the fragment 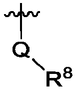 attach to the remaining fragment of the compound of formula (XIa) or formula (XIb);
L is selected from -C(O)NH-, -NHC(O)-, -S(O)₂NH-, and -NHS(O)₂-;
"*" indicates a chiral center.

3. The compound of claim 2, wherein,
the fragment 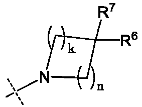 or fragment 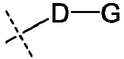 in formula (XIa) or formula (XIb) is selected from the group consisting of: 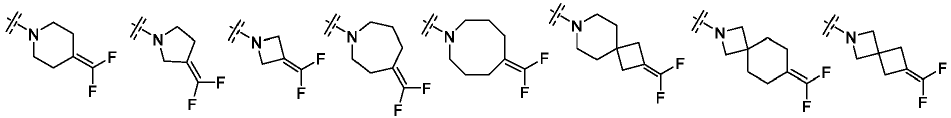 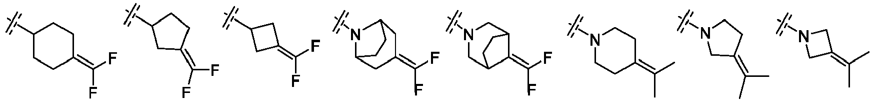 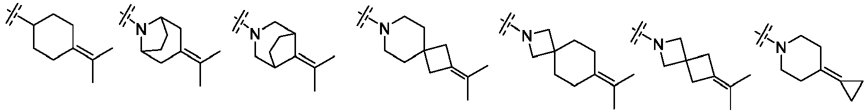 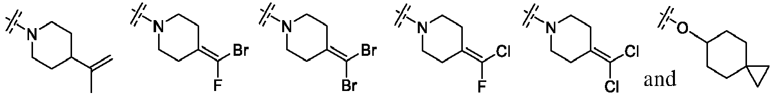
the "-̅ -̅ -̅ -̅ -̅ -̅" refers to the site at which the fragment attach to the remaining fragment of the compound of formula (XIa) or formula (XIb).

4. The compound of claim 2, wherein,
the fragment 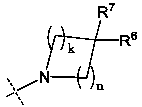 of formula (XIa) is selected from the group consisting of: 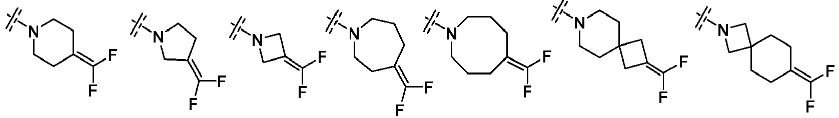 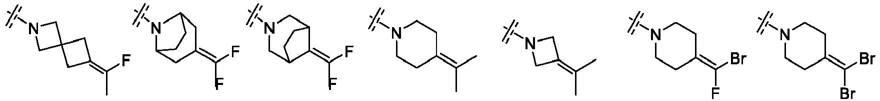 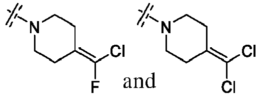
the "-̅ -̅ -̅ -̅ -̅ -̅" refers to the site at which the fragment attach to the remaining fragment of the compound of formula (XIa).

5. The compound of claim 1, wherein the compound (I) is of formula (XII): 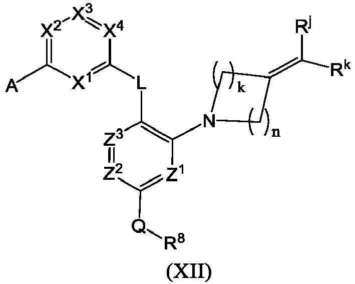
wherein R^{j} and R^{k} are each independently selected from the group consisting of hydrogen, deuterium, fluorine, chlorine, bromine, and C₁₋₄ alkyl;
k and n are selected from 1, 2, 3, 4 and 5;
while the remaining groups are defined as in claim 2.

6. The compound of claim 1, wherein the compound (I) is of formula (XIII): 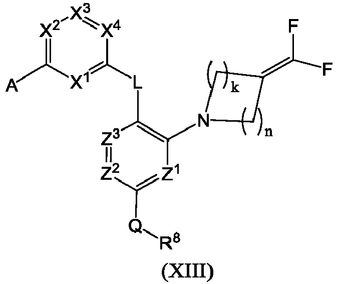
k and n are selected from 1, 2, 3 and 4;
A is a group selected from: 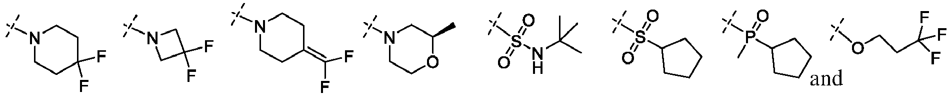
"---" refers to the site of A at which attach to the remaining fragment of the formula (XIII) compound;
fragment 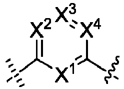 is selected from the group consisting of: 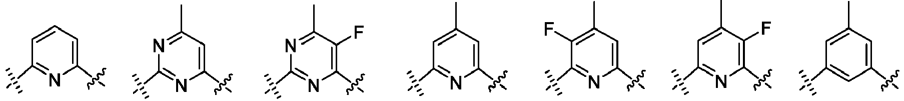 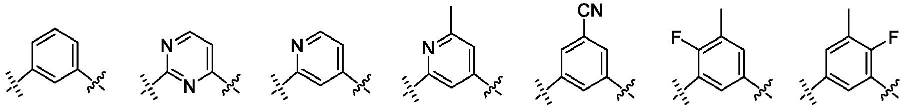 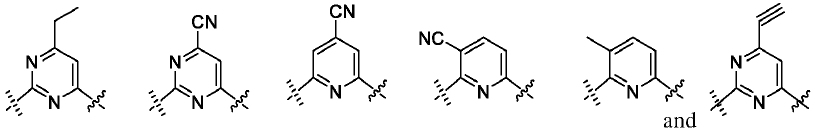
" 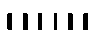 " indicates the site at which the fragment attach to A; " 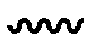 " indicates the site where the fragment attach to L;
fragment 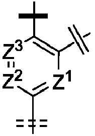 is selected from the group consisting of: 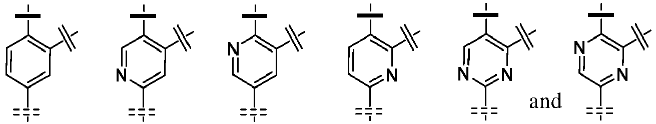
"-"indicates the site at which the fragment attach to L; "- - - " indicates the site attaching to Q; = indicates the site at which the fragment attach to 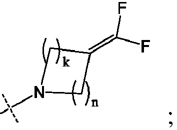
fragment 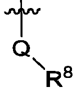 is selected from the group consisting of: 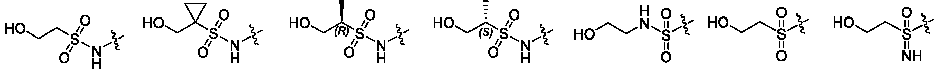 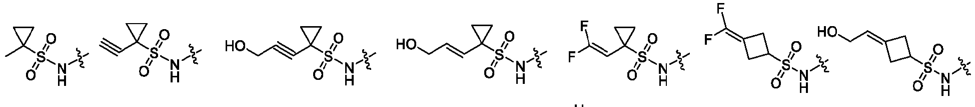 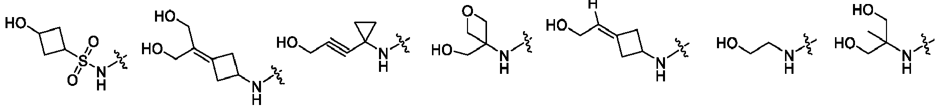 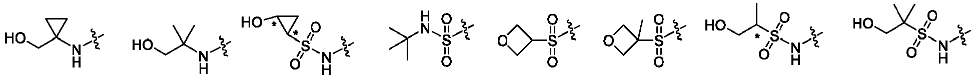 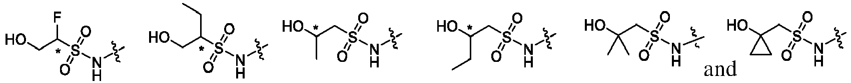
" 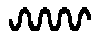 " refers to the site of fragment 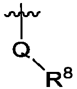 which attach to the remaining fragment of the formula (XIII) compound;
L is selected from -C(O)NH-, -NHC(O)-, -S(O)₂NH-, and -NHS(O)₂-;
"*" indicates a chiral center.

7. The compound of claim 1, wherein the compound (I) is of formula (XIV): 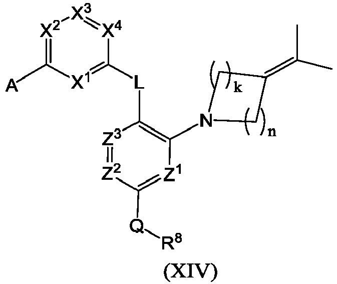 while the groups are defined as in claim 6.

8. The compound of claim 1, wherein the compound (I) is of formula (XV): 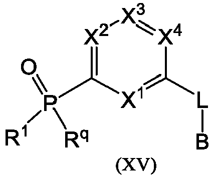 while the groups are defined as in claim 1.

9. The compound of claim 1, wherein the compound (I) is of formula (XVII): 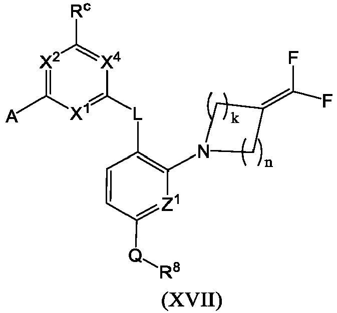
k and n are each independently selected from 1 and 2;
X¹ selected from N, C-H, and C-F;
X² is selected from N, C-H, C-F, C-CN, and C-Me;
X⁴ is selected from C-H, C-F, and N;
Z¹ is selected from C-H, C-F, and N;
R^{c} is selected from H, halogen, C₁₋₄ alkyl, and CN;
L is selected from -C(O)NH- and -NHC(O)-;
while the remaining groups are defined as in claim 6.

10. The compound of claim 1, wherein the compound (I) is of formula (XVIII): 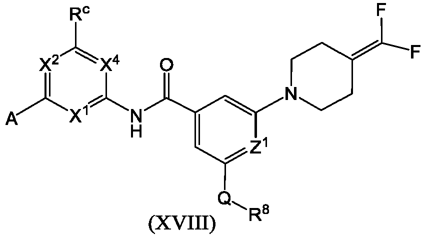
X¹ selected from N, C-H, and C-F;
X² is selected from N, C-H, C-F, C-CN, and C-Me;
X⁴ is selected from C-H, C-F, and N;
Z¹ is selected from C-H, C-F, and N;
R^{c} is selected from hydrogen, halogen, methyl, ethyl, and CN;
A is a group selected from: 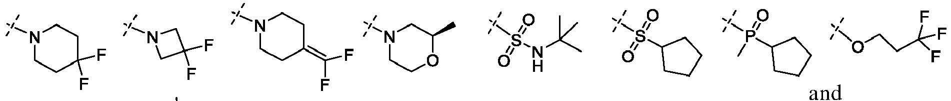
fragment 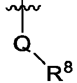 is selected from the group consisting of:

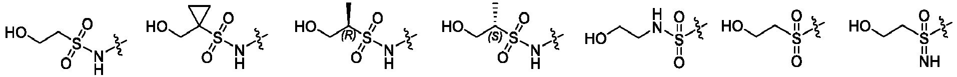

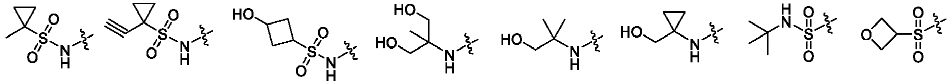

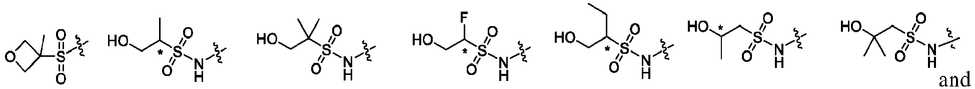

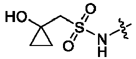

11. The compound of claim 2, wherein,
in the formula (XIa) or formula (XIb), fragment A is a group selected from: 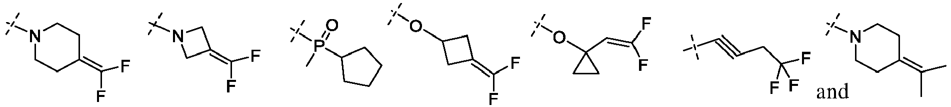
" ---" refers to the site of A which attach to the remaining fragment of the formula (XIa) or formula (XIb);

12. The compound of claim 1, wherein the compound (I) is selected from the following group: 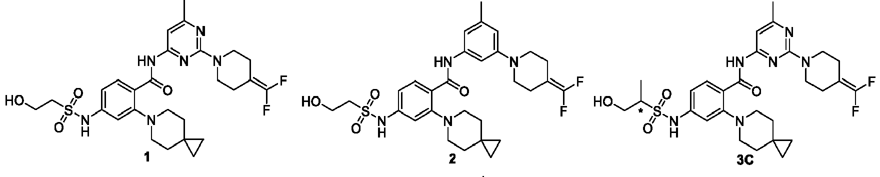 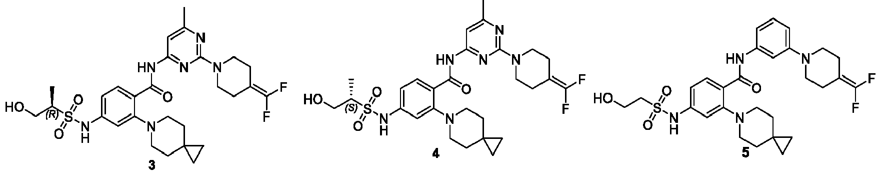 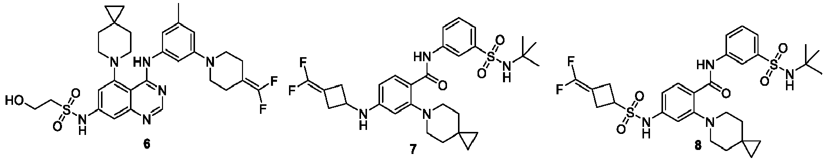 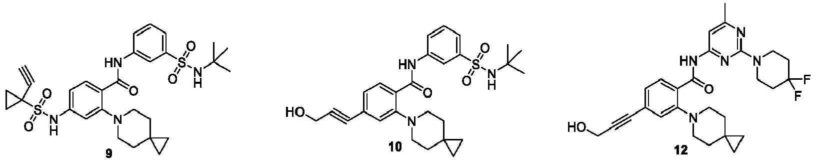 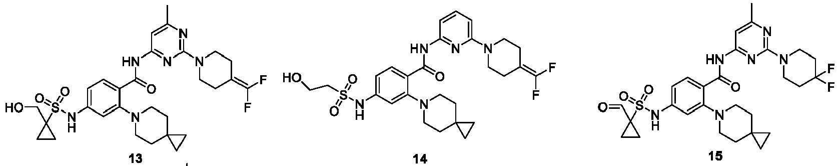 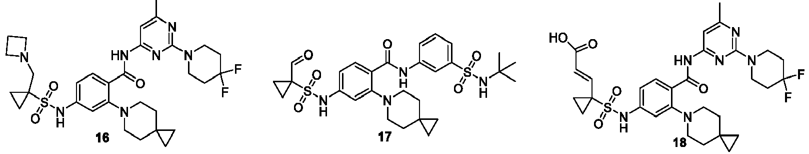 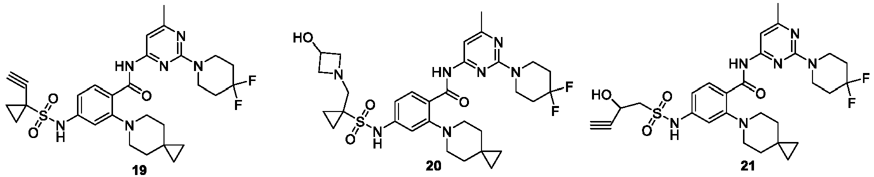 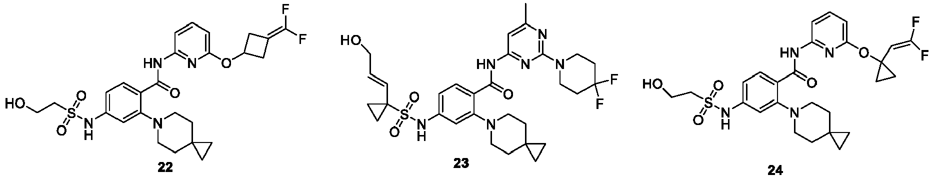 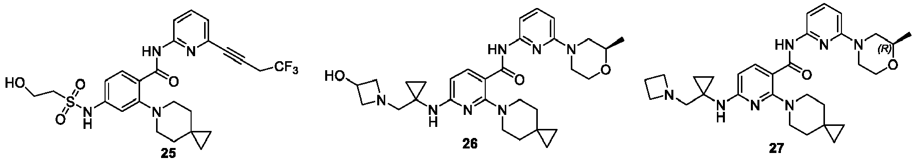 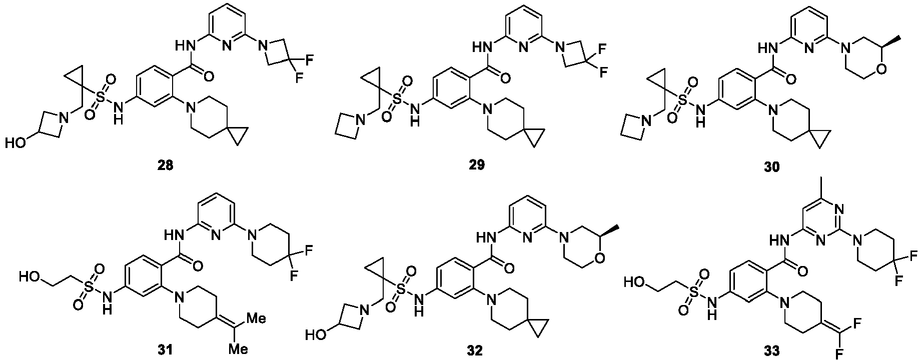 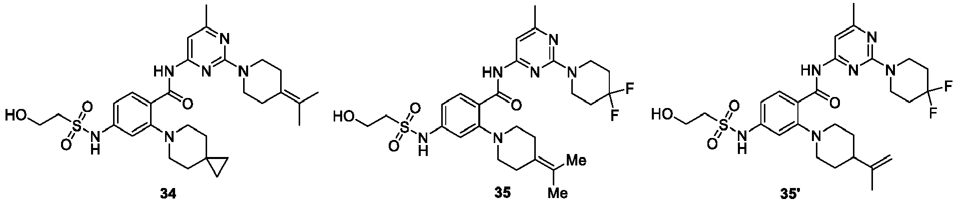 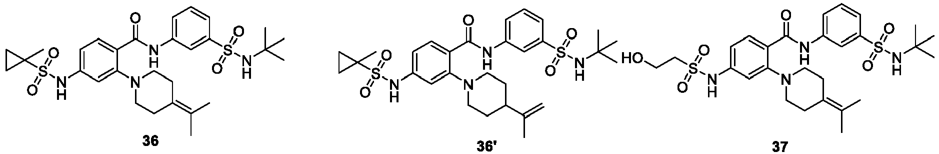 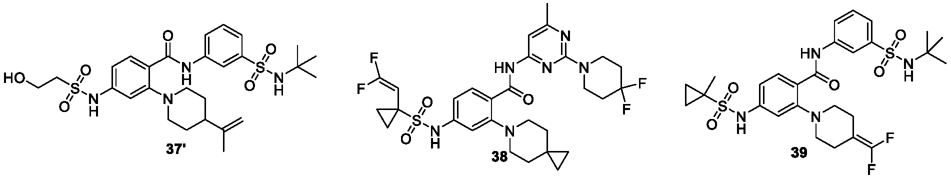 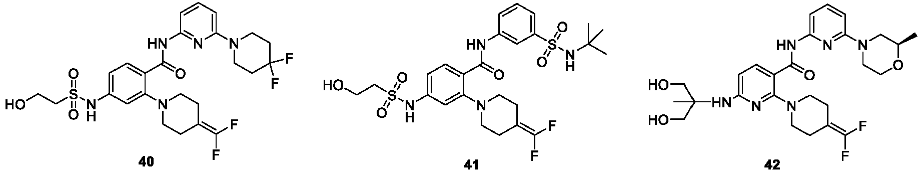 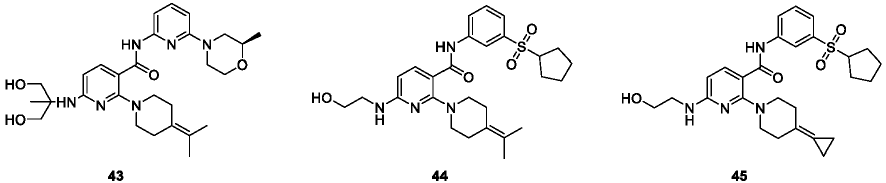 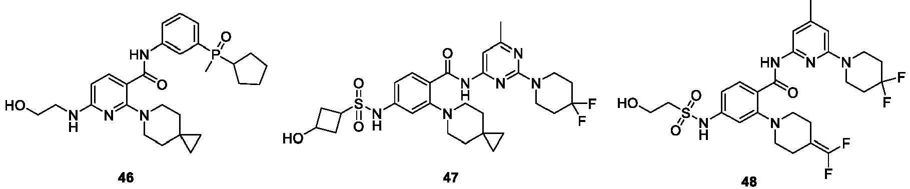 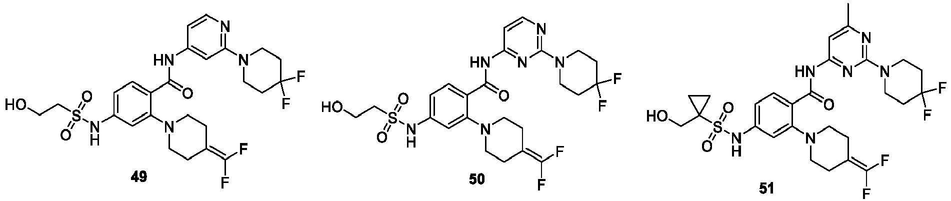 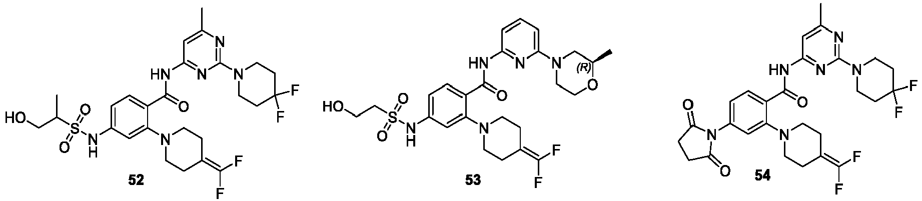 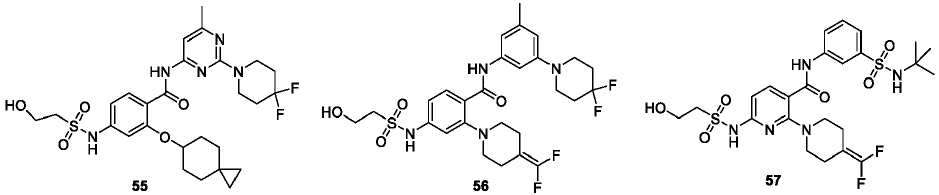 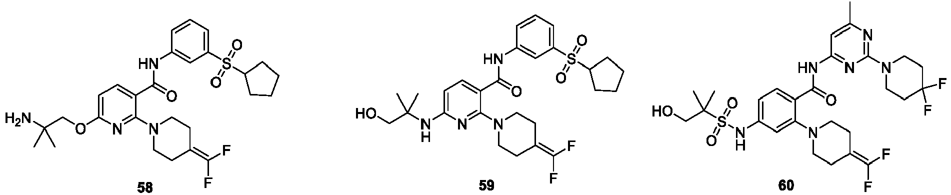 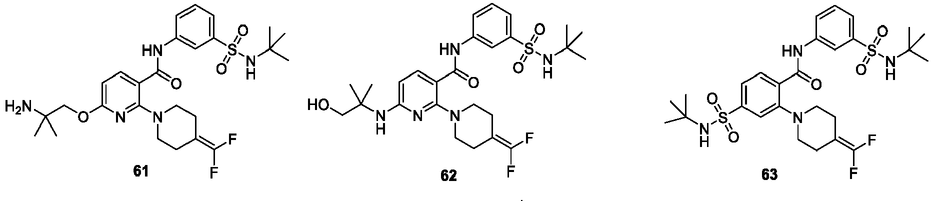 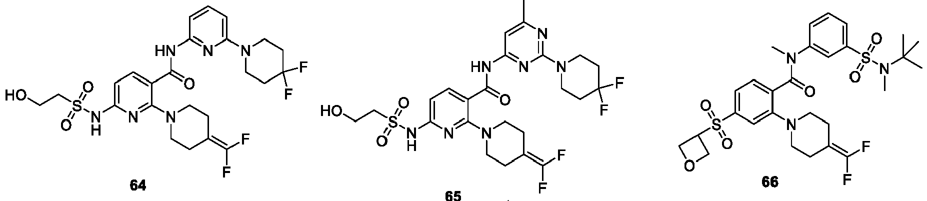 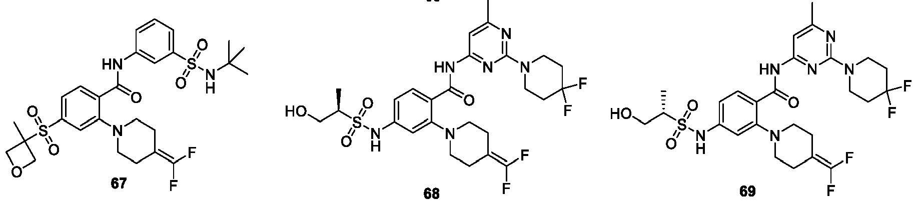 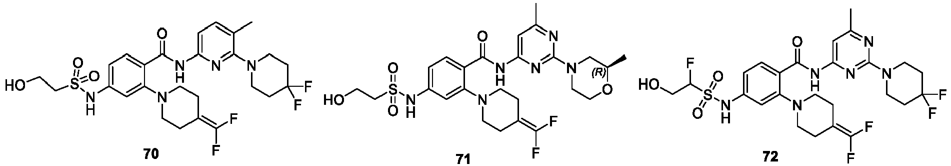 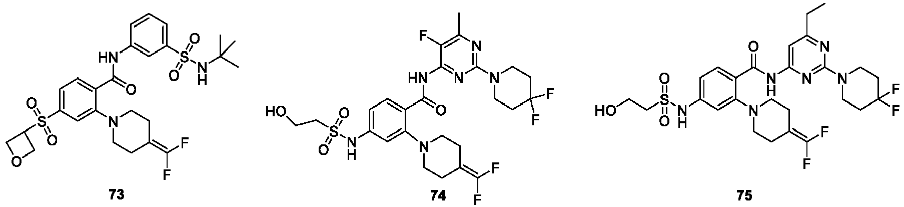 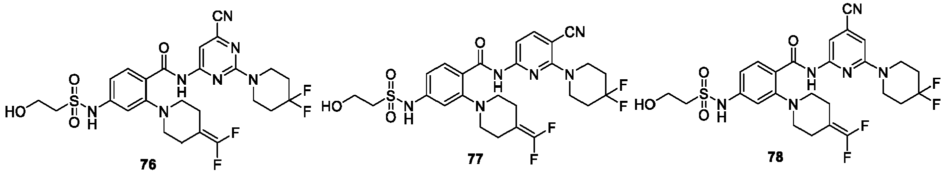 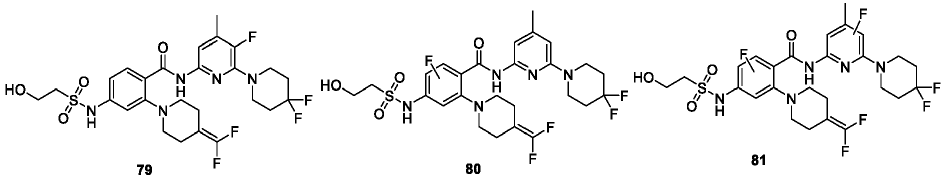 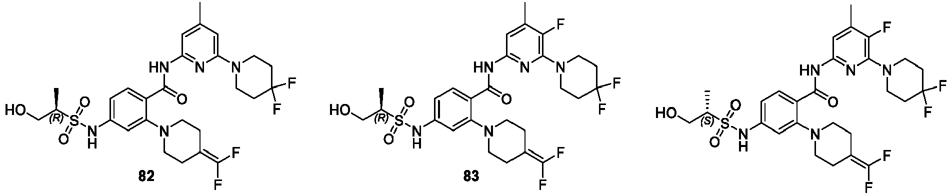 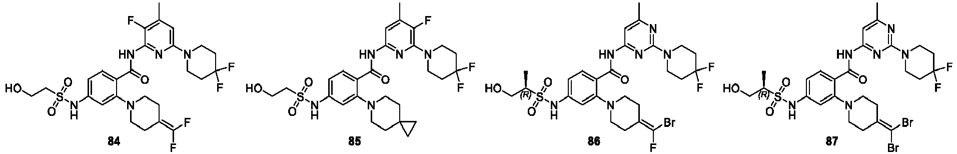 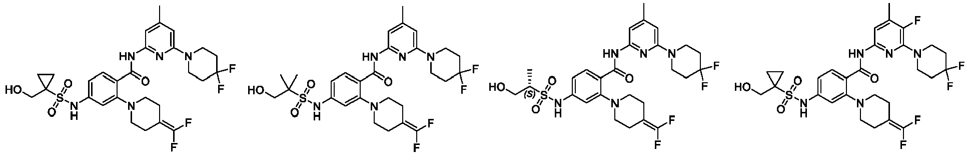 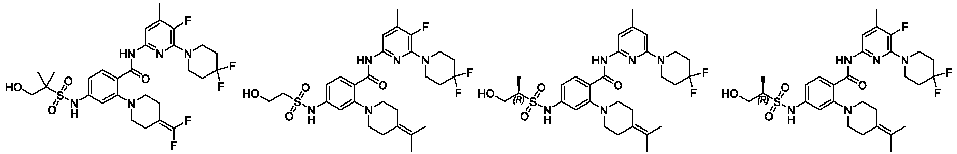 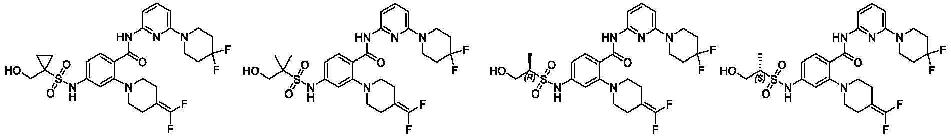 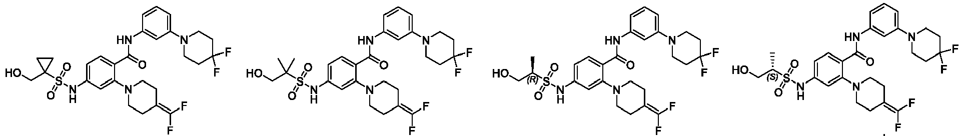 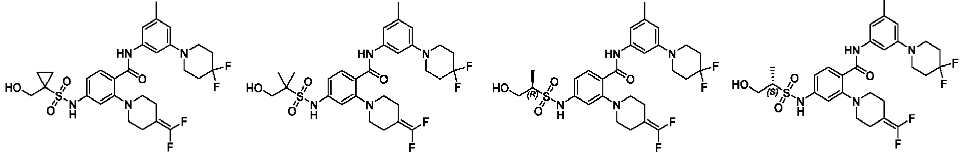 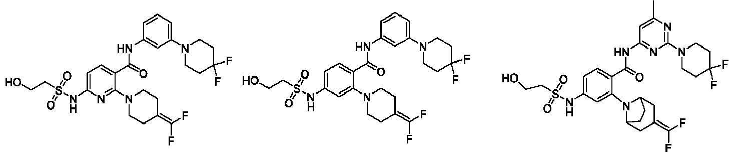 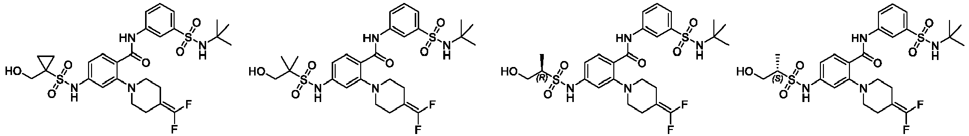 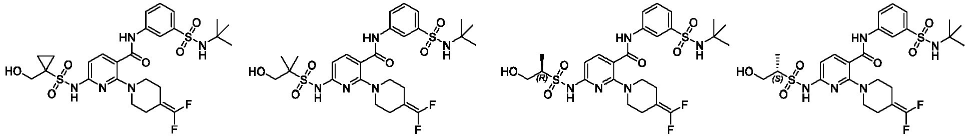 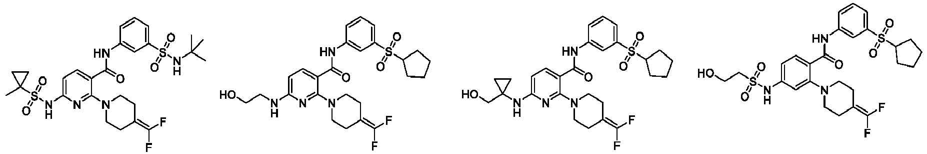 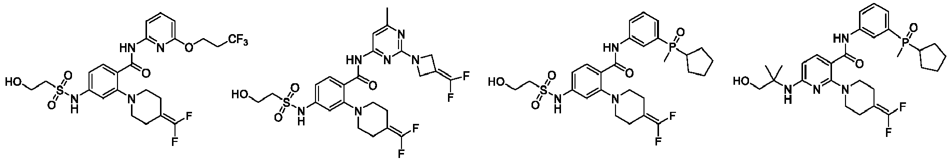 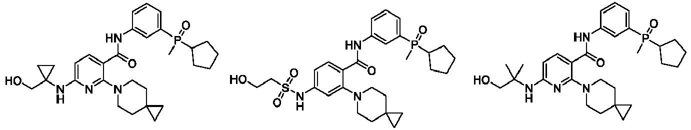 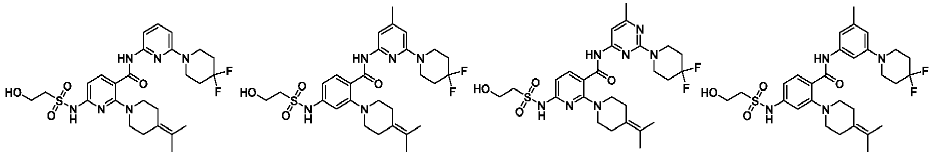 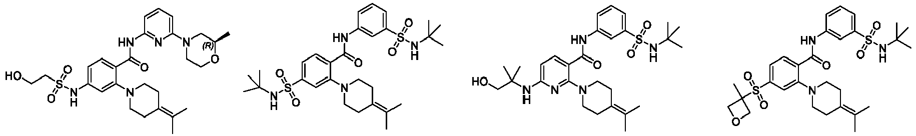 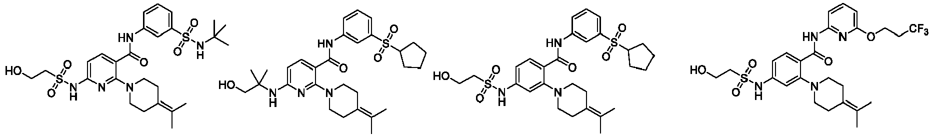 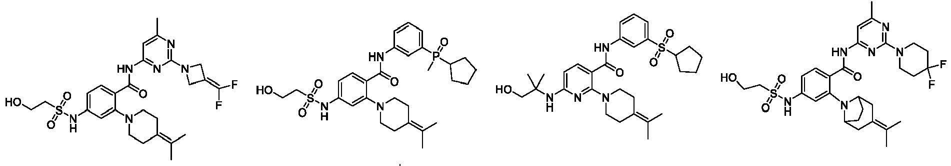 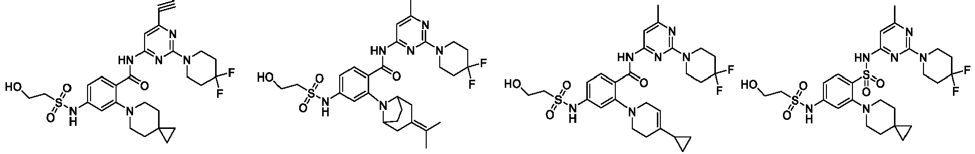 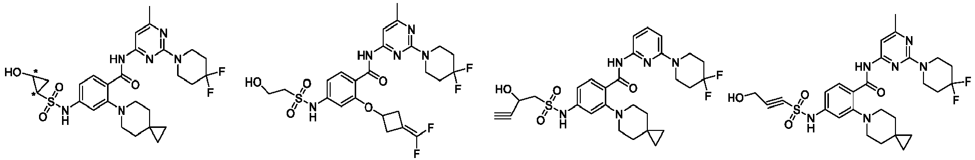 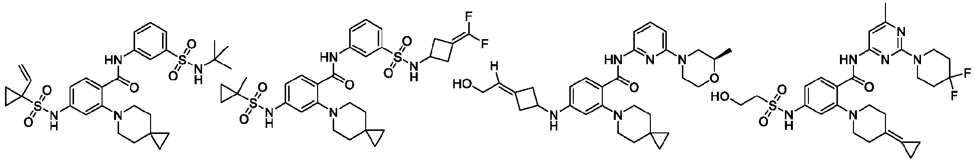 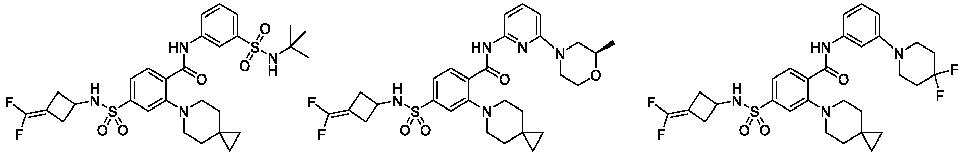 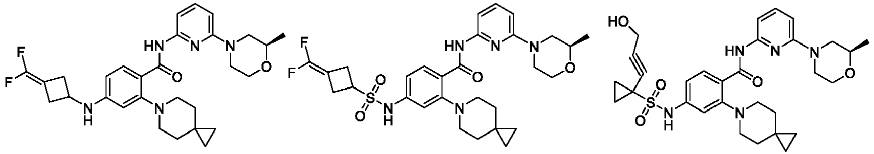 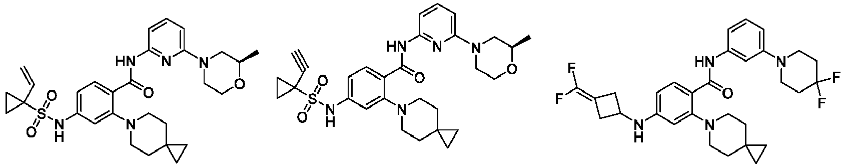 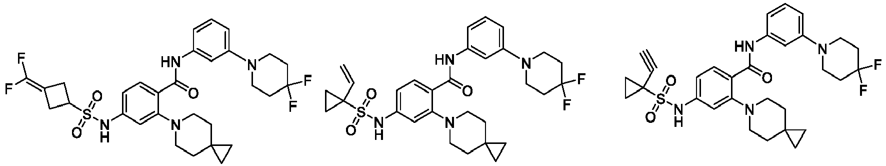 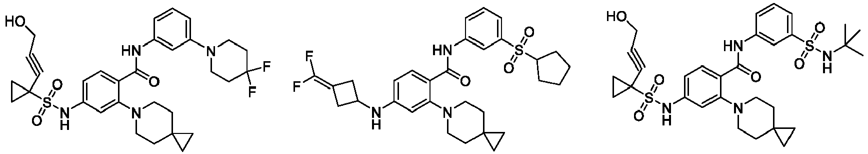 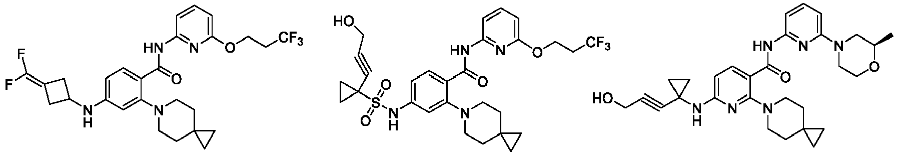 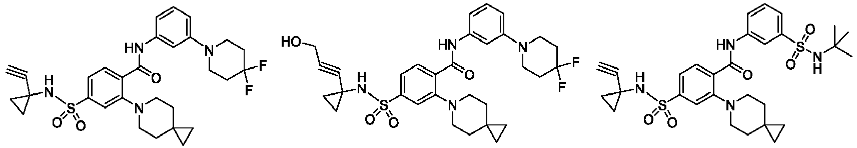 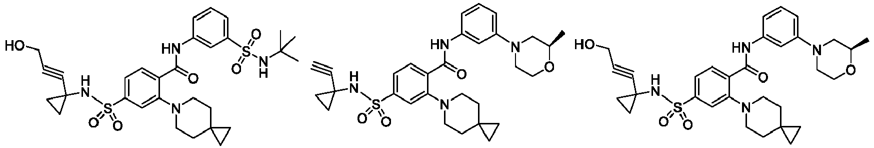 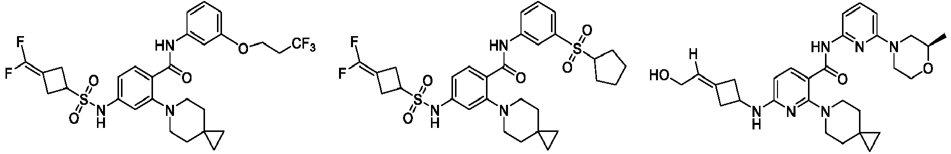 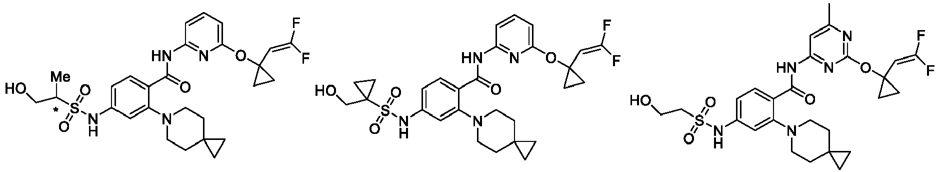 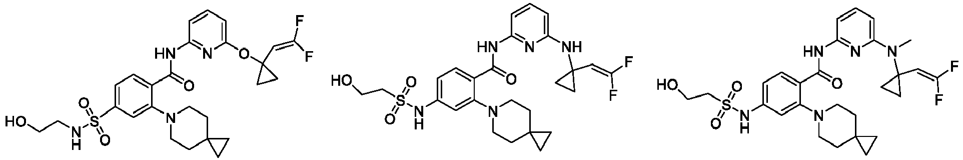 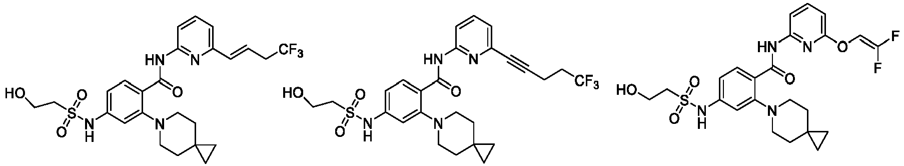 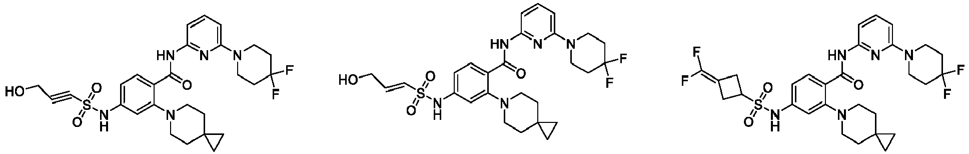 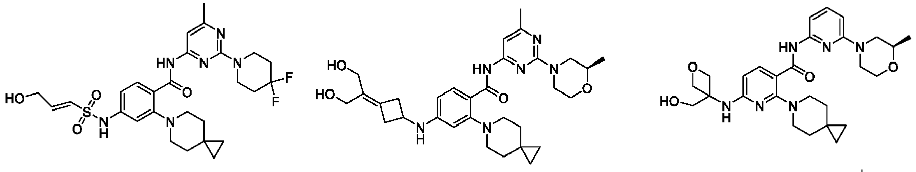 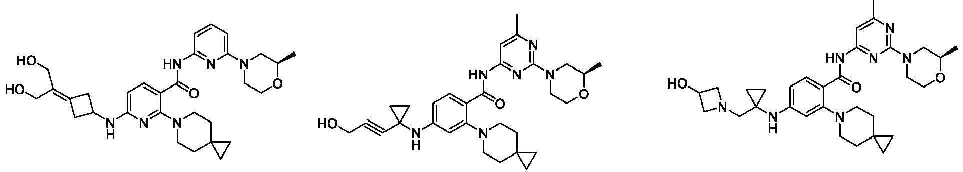 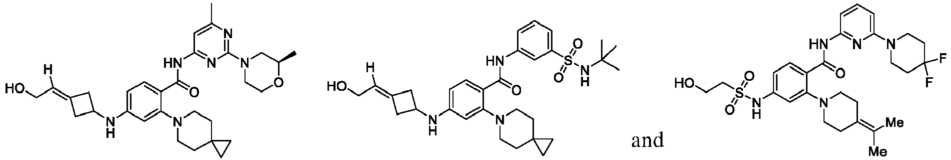 "*" indicates a chiral center.

13. A pharmaceutical composition, wherein comprises the compound of any one of claims 1-12, or the optical, tautomer, or a pharmaceutically acceptable salt, prodrugs, deuterated derivatives, hydrate or solvate thereof, and pharmaceutically acceptable carriers.

14. Use of the compound of any one of claims 1-12, or the optical isomers, pharmaceutically acceptable salts, prodrugs, deuterated derivatives, hydrates or solvates thereof, wherein in the treatment of disease, disorder or condition associated with the activity or expression amount of KIF18A.

15. The use of claim 14, wherein the disease, disorder or condition is selected from the group consisting of non-small cell lung cancer, small cell lung cancer, lung adenocarcinoma, lung squamous cell carcinoma, pancreatic cancer, colon cancer, thyroid cancer, embryonal rhabdomyosarcoma, skin granulosa cell tumor, melanoma, hepatocellular carcinoma, intrahepatic cholangiocarcinoma, rectal cancer, bladder cancer, throat cancer, breast cancer, prostate cancer, brain tumor, glioma, ovarian cancer, head and neck squamous cell carcinoma, cervical cancer, esophageal cancer, renal cancer, skin cancer, gastric cancer, myeloid leukemia, lymphoblastic leukemia, myeloid leukemia, B-cell lymphoma, T-cell lymphoma, Hodgkin lymphoma, non-Hodgkin lymphoma, monocytic leukemia, polycythemia megalosplenica, multiple eosinophilic leukocytosis syndrome, bone marrow cancer and other solid tumors and hematological tumors, as well as virus infection such as influenza.
